(19) Europäisches Patentamt / European Patent Office / Office européen des brevets

(11) EP 3 279 193 B1

(12) EUROPÄISCHE PATENTSCHRIFT

(45) Veröffentlichungstag und Bekanntmachung des Hinweises auf die Patenterteilung:
**08.05.2019 Patentblatt 2019/19**

(51) Int Cl.:
***C07D 403/04*** (2006.01) ***H01L 51/50*** (2006.01)

(21) Anmeldenummer: **16205427.4**

(22) Anmeldetag: **20.12.2016**

# (54) PHTHALIMID UND CARBAZOL ODER DESSEN ANALOGE ENTHALTENDE VERBINDUNGEN ZUR VERWENDUNG IN ORGANISCHEN OPTOELEKTRONISCHEN VORRICHTUNGEN

COMPOUNDS COMPRISING A PHTHALIMIDE GROUP AND CARBAZOLE OR ITS ANALOGUES FOR USE IN ORGANIC OPTOELECTRONIC DEVICES

COMPOSES COMPRENANT UN GROUPE PHTHALIMIDE ET UN GROUPE CARBAZOLE OU SES ANALOGUES UTILES DANS DES DISPOSITIFS ORGANIQUES OPTOELECTRONIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.08.2016 EP 16182868**
**20.09.2016 EP 16189679**

(43) Veröffentlichungstag der Anmeldung:
**07.02.2018 Patentblatt 2018/06**

(73) Patentinhaber: **CYNORA GMBH**
**76646 Bruchsal (DE)**

(72) Erfinder:
- **SEIFERMANN, Stefan**
  **77815 Bühl (DE)**
- **DANZ, Michael**
  **76344 Eggenstein-Leopoldshafen (DE)**
- **VOLZ, Daniel**
  **76137 Karlsruhe (DE)**

(74) Vertreter: **Hoppe, Georg Johannes**
**Darani Anwaltskanzlei**
**Beuckestrasse 20**
**14163 Berlin (DE)**

(56) Entgegenhaltungen:
**WO-A1-2016/042070**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 279 193 B1

## Beschreibung

Die Erfindung betrifft rein organische Moleküle und deren Verwendung in organischen lichtemittierenden Dioden (OLEDs) und in anderen organischen optoelektronischen Vorrichtungen.

## Hintergrund

Organische optoelektronische Vorrichtungen zeichnen sich dadurch aus, dass entweder elektrische Energie in Photonen umgewandelt wird (Organische Lichtemittierende Dioden, OLED oder Lichtemittierende Elektrochemische Zellen, LEEC) oder der umgekehrte Prozess abläuft (Organische Photovoltaik, OPV). Dabei ist es wichtig, dass diese Prozesse möglichst effizient ablaufen. Für den Bereich von OLEDs müssen daher idealerweise Materialien mit möglichst hoher photolumineszenter Quantenausbeute verwendet werden. Begrenzte Effizienzen von OLED-Materialien können durch Verwendung effizienter Materialien, die thermisch aktivierte verzögerte Fluoreszenz (TADF) zeigen, verbessert werden, da im Gegensatz zu rein fluoreszenten Materialien statt 25 % der in einer OLED gebildeten Exzitonen bis zu 100 % der Exzitonen genutzt werden können. Hierbei können auch die entstandenen Triplett-Excitonen in Singulett-Excitonen überführt werden, aus welchem Zustand dann Photonen emittiert werden können. Voraussetzung für eine solche thermische Rückbesetzung ist dabei ein geringer energetischer Abstand zwischen dem niedrigsten angeregten Singulett-($S_1$) und Triplett-Niveau ($T_1$). Dies kann beispielweise durch Verwendung von Kupfer-(I)-Komplexen (siehe hierzu z. B.: H. Yersin, U. Monkowius, T. Fischer, T. Hofbeck, WO 2010/149748 A1) aber auch durch rein organische Materialien (siehe hierzu z. B.: Q. Zhang et al., J. Am. Chem. Soc. 2012, 134, 14706, WO 2013161437 A1) erreicht werden.

Die intensive Forschung in diesem Bereich zeigt, dass es weiterhin einen großen Bedarf an neuen Materialien gibt. So besteht zum Beispiel nach wie vor Bedarf an tiefblauen und himmelblauen TADF-OLEDs. Bestehende blaue TADF-Materialien zeigen oft hohe Exzitonenlebensdauern und/oder geringe Photolumineszenquantenzausbeuten, welche schlecht für effiziente und langlebige OLEDs sind. Als Maß für eine effiziente blaue OLED kann der Quotient aus der Stromeffizienz in cd/A und dem y-Wert der CIE-Farbkoordinate ($CIE_y$) des von der OLED emittierten Lichts verwendet werden, also eine auf den $CIE_{y}$-Wert normierte Effizienz. Neben den erwähnten Eigenschaften der Materialien ist für eine Kommerzialisierung ebenso die Zugänglichkeit relevant. Dies schließt die Verfügbarkeit von Synthesebausteinen, als auch den Aufwand für die eigentliche Synthese des funktionellen Materials, insbesondere dessen Aufreinigung mit ein.

## Beschreibung

Der vorliegenden Erfindung lag die Aufgabe zu Grunde, Moleküle bereitzustellen, die sich zur Verwendung als Emittermaterialien in OLEDs eignen, die blaues Licht emittieren.

Überraschenderweise wurde festgestellt, dass durch Verwendung geeigneter Donoreinheiten in Kombination mit Phthalimid-Akzeptoreinheiten Moleküle mit Emissionsmaxima bei Wellenlängen unterhalb von 491 nm erhalten werden können, welche hohe Quantenausbeuten und geringe Exzitonenlebensdauern aufweisen. Da die Effizienz des Bauteils nach Optimierung des Stack-Designs typischerweise direkt mit der Photolumineszenquantenzausbeute (PLQY) des Emittermaterials korreliert, wurde für die erfindungsgemäßen Moleküle ein technischer Index analog zu dem oben beschriebenen bekannten Effizienzindex für blaue OLEDs ermittelt. Dieser Blaumaterialindex ("blue material index", BMI) ergibt sich als Quotient aus der PLQY (in %) und der $CIE_{y}$-Farbkoordinate des von dem erfindungsgemäßen Molekül emittierten Lichts.

Die erfindungsgemäßen organischen Moleküle weisen eine Struktur der Formel A1 auf oder bestehen aus einer Struktur der Formel A1.

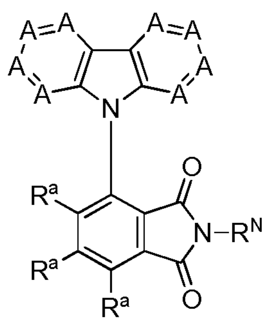

Formel A1

mit

A ist bei jedem Auftreten gleich oder verschieden $CR^b$ oder N;
$R^N$ ist ausgewählt aus der Gruppe bestehend aus Methyl, Phenyl, Xylyl, Mesityl, Naphtyl, Biphenyl, Naphthylphenyl, Terphenyl und 2,4,6-Triphenylphenyl.

$R^a$ ist bei jedem Auftreten unabhängig voneinander ausgewählt aus der Gruppe bestehend aus H, Deuterium, einer Alkylgruppe und einer Arylgruppe;
$R^b$ ist bei jedem Auftreten gleich oder verschieden H, Deuterium, $CF_3$, $C(=O)R^1$, CN, eine unsubstituierte oder mit einem oder mehreren $R^2$ substituierte Alkylgruppe, eine unsubstituierte oder mit einem oder mehreren $R^2$ substituierte Arylgruppe, die optional mit einer oder mehreren unsubstituierten Alkylgruppe und/oder mit einer oder mehreren unsubstituierten oder Alkyl-substituierten Arylgruppe substituiert ist, eine unsubstituierte oder mit einem oder mehreren $R^2$ und/ oder mit einer oder mehreren unsubstituierten Alkylgruppe und/oder mit einer oder mehreren unsubstituierten oder Alkyl-substituierten Arylgruppe substituierte Heteroarylgruppe, eine Gruppe der Unterformel T1 oder eine Gruppe der Unterformel T2:

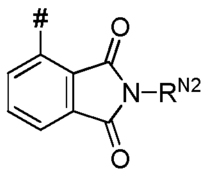

Unterformel T1

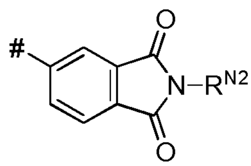

Unterformel T2

mit den folgenden Definitionen:
$R^{N2}$ ist eine Alkylgruppe, eine unsubstituierte oder mit einem oder mehreren $R^{N3}$ substituierte Arylgruppe oder eine unsubstituierte oder mit einem oder mehreren $R^{N3}$ substituierte Heteroarylgruppe.
$R^{N3}$ ist eine Alkylgruppe, eine Arylgruppe oder eine Heteroarylgruppe.
$R^1$ ist bei jedem Auftreten eine unsubstituierte oder mit einem oder mehreren $R^2$ substituierte Arylgruppe.
$R^2$ ist bei jedem Auftreten gleich oder verschieden F, $CF_3$ oder CN;
wobei mindestens ein und maximal vier A gleich N ist oder mindestens ein $R^b$ ausgewählt ist aus der Gruppe bestehend aus, $CF_3$, $C(=O)R^1$, CN, einer mit einem oder mehreren $R^2$ substituierten Alkylgruppe, einer mit einem oder mehreren $R^2$ substituierten Arylgruppe, die optional mit einer oder mehreren unsubstituierten Alkylgruppe und/oder mit einer oder mehreren unsubstituierten oder Alkyl-substituierten Arylgruppe substituiert ist, einer unsubstituierten oder mit einem oder mehreren $R^2$ und/ oder mit einer oder mehreren unsubstituierten Alkylgruppe und/oder mit einer oder mehreren unsubstituierten oder Alkyl-substituierten Arylgruppe substituierten Heteroarylgruppe und einer Gruppe der Unterformel T1 oder eine Gruppe der Unterformel T2:

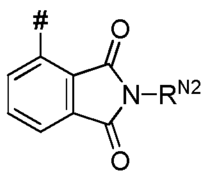

Unterformel T1

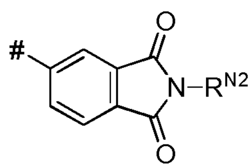

Unterformel T2

mit
$R^{N2}$ ist eine Alkylgruppe, eine unsubstituierte oder mit einem oder mehreren $R^{N3}$ substituierte Arylgruppe oder eine unsubstituierte oder mit einem oder mehreren $R^{N3}$ substituierte Heteroarylgruppe.
$R^{N3}$ ist eine Alkylgruppe, eine Arylgruppe oder Heteroarylgruppe.
In einer Ausführungsform ist mindestens ein und maximal vier A gleich N oder mindestens ein $R^b$ ist ausgewählt aus der Gruppe bestehend aus $CF_3$, $C(=O)R^1$, CN, einer mit einem oder mehreren $R^2$ substituierten Alkylgruppe, einer unsubstituierten oder mit einem oder mehreren $R^2$ substituierten 6-Ring-Arylgruppe, die optional mit einer oder mehreren unsubstituierten Alkylgruppe und/oder mit einer oder mehreren unsubstituierten oder Alkyl-substituierten Arylgruppe substituiert ist, einer unsubstituierten oder mit einem oder mehreren $R^2$ und/oder mit einer oder mehreren unsubstituierten Alkylgruppe und/oder mit einer oder mehreren unsubstituierten oder Alkyl-substituierten Arylgruppe substituierten 6-Ring-Heteroarylgruppe und einer Gruppe der Unterformel T1 oder eine Gruppe der Unterformel T2:

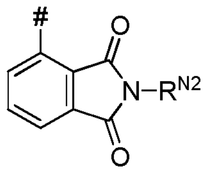

Unterformel T1

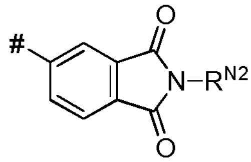

Unterformel T2

mit
$R^{N2}$ ist eine Alkylgruppe, eine unsubstituierte oder mit einem oder mehreren $R^{N3}$ substituierte Arylgruppe oder eine unsubstituierte oder mit einem oder mehreren $R^{N3}$ substituierte Heteroarylgruppe.
$R^{N3}$ ist eine Alkylgruppe, eine Arylgruppe oder eine Heteroarylgruppe.

In einer Ausführungsform ist $R^b$ wie oben definiert mit der Maßgabe, dass $R^b$ nicht gleich Pyridin oder Pyrimidin ist.

In einer Ausführungsform weist das organische Molekül eine Struktur der Formel A2 auf oder hat eine Struktur der Formel A2.

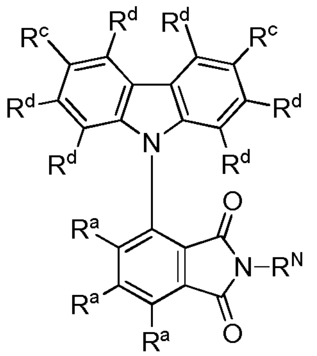

Formel A2

wobei gilt:

$R^N$ ist Methyl, Phenyl, Xylyl, Mesityl, Naphtyl, Biphenyl, Naphthylphenyl, Terphenyl oder 2,4,6-Triphenylphenyl;

$R^a$ ist bei jedem Auftreten gleich oder verschieden H, Deuterium, eine Alkylgruppe oder eine Arylgruppe;

$R^c$ ist bei jedem Auftreten gleich oder verschieden $CF_3$, $C(=O)R^1$, CN, eine mit einem oder mehreren $R^2$ substituierte Alkylgruppe, eine substituierte Arylgruppe, die optional mit einer oder mehreren unsubstituierten Alkylgruppe und/oder mit einer oder mehreren unsubstituierten oder Alkyl-substituierten Arylgruppe substituiert ist, eine unsubstituierte oder mit einem oder mehreren $R^2$ und/ oder mit einer oder mehreren unsubstituierten Alkylgruppe und/oder mit einer oder mehreren unsubstituierten oder Alkyl-substituierten Arylgruppe substituierte Heteroarylgruppe oder eine Gruppe der Unterformel T1 oder eine Gruppe der Unterformel T2:

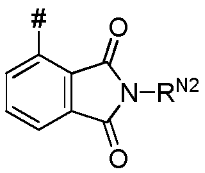

Unterformel T1

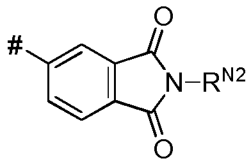

Unterformel T2

mit
$R^{N2}$ ist eine Alkylgruppe, eine unsubstituierte oder mit einem oder mehreren $R^{N3}$ substituierte Arylgruppe oder eine

unsubstituierte oder mit einem oder mehreren $R^{N3}$ substituierte Heteroarylgruppe.

$R^{N3}$ ist eine Alkylgruppe, eine Arylgruppe oder eine Heteroarylgruppe.
$R^d$ ist bei jedem Auftreten gleich oder verschieden H, Deuterium, $CF_3$, $C(=O)R^1$, CN, eine unsubstituierte oder mit einem oder mehreren $R^2$ substituierte Alkylgruppe, eine unsubstituierte oder mit einem oder mehreren $R^2$ und/oder mit einer oder mehreren unsubstituierten Alkylgruppe und/ oder mit einer oder mehreren unsubstituierten oder Alkyl-substituierten Arylgruppe substituierte Arylgruppe oder eine unsubstituierte oder mit einem oder mehreren $R^2$ und/oder mit einer oder mehreren unsubstituierten Alkylgruppe und/ oder mit einer oder mehreren unsubstituierten oder Alkyl-substituierten Arylgruppe substituierte Heteroarylgruppe oder eine Gruppe der Unterformel T1 oder eine Gruppe der Unterformel T2:

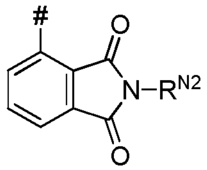

Unterformel T1

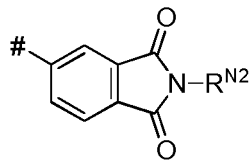

Unterformel T2

mit
$R^{N2}$ ist eine Alkylgruppe, eine unsubstituierte oder mit einem oder mehreren $R^{N3}$ substituierte Arylgruppe oder eine unsubstituierte oder mit einem oder mehreren $R^{N3}$ substituierte Heteroarylgruppe.
$R^{N3}$ ist eine Alkylgruppe, eine Arylgruppe oder eine Heteroarylgruppe.

In einer weiteren Ausführungsform ist $R^c$ bei jedem Auftreten unabhängig voneinander ausgewählt aus der Gruppe bestehend aus $CF_3$, $C(=O)R^1$, CN, einer mit einem oder mehreren $R^2$ substituierten Alkylgruppe, einer mit einem oder mehreren $R^2$ substituierten 6-Ring-Arylgruppe, die optional mit einer oder mehreren unsubstituierten Alkylgruppe und/oder mit einer oder mehreren unsubstituierten oder Alkyl-substituierten Arylgruppe substituiert ist, einer unsubstituierten oder mit einem oder mehreren $R^2$ und/oder mit einer oder mehreren unsubstituierten Alkylgruppe und/ oder mit einer oder mehreren unsubstituierten oder Alkyl-substituierten Arylgruppe substituierten 6-Ring-Heteroarylgruppe und einer Gruppe der Unterformel T1 oder einer Gruppe der Unterformel T2:

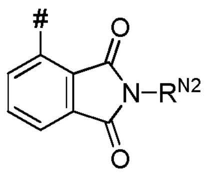

Unterformel T1

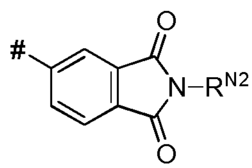

Unterformel T2

mit
$R^{N2}$ ist eine Alkylgruppe, eine unsubstituierte oder mit einem oder mehreren $R^{N3}$ substituierte Arylgruppe oder eine unsubstituierte oder mit einem oder mehreren $R^{N3}$ substituierte Heteroarylgruppe.
$R^{N3}$ ist eine Alkylgruppe, eine Arylgruppe oder eine Heteroarylgruppe.

In einer weiteren Ausführungsform ist $R^c$ wie oben definiert mit der Maßgabe, dass $R^c$ nicht gleich Pyridin oder Pyrimidin ist.

In einer weiteren Ausführungsform ist $R^d$ bei jedem Auftreten gleich H.

In einer Ausführungsform weist das organische Molekül eine Struktur der Formel A3 auf oder hat eine Struktur der Formel A3.

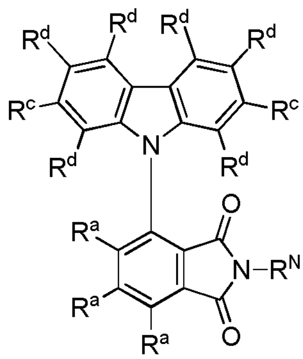

Formel A3

wobei gilt

$R^N$ ist Methyl, Phenyl, Xylyl, Mesityl, Naphtyl, Biphenyl, Naphthylphenyl, Terphenyl oder 2,4,6-Triphenylphenyl;

$R^a$ ist bei jedem Auftreten gleich oder verschieden H, Deuterium, eine Alkylgruppe oder eine Arylgruppe;

$R^c$ ist bei jedem Auftreten unabhängig voneinander ausgewählt aus der Gruppe bestehend aus $CF_3$, $C(=O)R^1$, CN, einer mit einem oder mehreren $R^2$ substituierten Alkylgruppe, einer substituierten Arylgruppe, die optional mit einer oder mehreren unsubstituierten Alkylgruppe und/oder mit einer oder mehreren unsubstituierten oder Alkyl-substituierten Arylgruppe substituiert ist, einer unsubstituierten oder mit einem oder mehreren $R^2$ und/oder mit einer oder mehreren unsubstituierten Alkylgruppe und/oder mit einer oder mehreren unsubstituierten oder Alkyl-substituierten Arylgruppe substituierten Heteroarylgruppe und einer Gruppe der Unterformel T1 oder einer Gruppe der Unterformel T2:

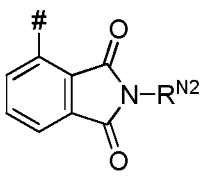

Unterformel T1

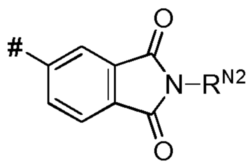

Unterformel T2

mit
$R^{N2}$ ist eine Alkylgruppe, eine unsubstituierte oder mit einem oder mehreren $R^{N3}$ substituierte Arylgruppe oder eine unsubstituierte oder mit einem oder mehreren $R^{N3}$ substituierte Heteroarylgruppe.

$R^{N3}$ ist eine Alkylgruppe, eine Arylgruppe oder eine Heteroarylgruppe.

$R^d$ ist bei jedem Auftreten gleich oder verschieden unabhängig voneinander ausgewählt aus der Gruppe bestehend aus H, Deuterium, $CF_3$, $C(=O)R^1$, CN, einer unsubstituierten oder mit einem oder mehreren $R^2$ substituierten Alkylgruppe, einer unsubstituierten oder mit einem oder mehreren $R^2$ und/oder mit einer oder mehreren unsubstituierten Alkylgruppe und/oder mit einer oder mehreren unsubstituierten oder Alkyl-substituierten Arylgruppe substituierten Arylgruppe, einer unsubstituierten oder mit $R^2$ und/oder mit einer oder mehreren unsubstituierten Alkylgruppe und/ oder mit einer oder mehreren unsubstituierten oder Alkyl-substituierten Arylgruppe substituierten Heteroarylgruppe und einer Gruppe der Unterformel T1 oder einer Gruppe der Unterformel T2:

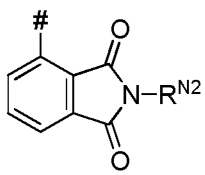

Unterformel T1

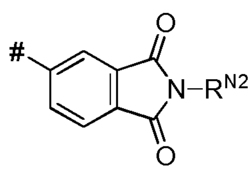

Unterformel T2

mit

$R^{N2}$ ist eine Alkylgruppe, eine unsubstituierte oder mit einem oder mehreren $R^{N3}$ substituierte Arylgruppe oder eine unsubstituierte oder mit einem oder mehreren $R^{N3}$ substituierte Heteroarylgruppe.

$R^{N3}$ ist eine Alkylgruppe, eine Arylgruppe oder eine Heteroarylgruppe.

In einer weiteren Ausführungsform ist $R^c$ bei jedem Auftreten unabhängig voneinander ausgewählt aus der Gruppe bestehend aus $CF_3$, $C(=O)R^1$, CN, einer mit einem oder mehreren $R^2$ substituierten Alkylgruppe, einer mit einem oder mehreren $R^2$ substituierten 6-Ring-Arylgruppe, die optional mit einer oder mehreren unsubstituierten Alkylgruppe und/oder mit einer oder mehreren unsubstituierten oder Alkyl-substituierten Arylgruppe substituiert ist, einer unsubstituierten oder mit einem oder mehreren $R^2$ und/oder mit einer oder mehreren unsubstituierten Alkylgruppe und/ oder mit einer oder mehreren unsubstituierten oder Alkyl-substituierten Arylgruppe substituierten 6-Ring-Heteroarylgruppe und einer Gruppe der Unterformel T1 oder einer Gruppe der Unterformel T2:

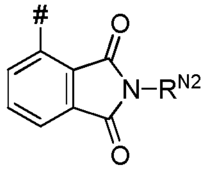

Unterformel T1

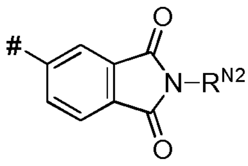

Unterformel T2

mit

$R^{N2}$ ist eine Alkylgruppe, eine unsubstituierte oder mit einem oder mehreren $R^{N3}$ substituierte Arylgruppe oder eine unsubstituierte oder mit einem oder mehreren $R^{N3}$ substituierte Heteroarylgruppe.

$R^{N3}$ ist eine Alkylgruppe, eine Arylgruppe oder eine Heteroarylgruppe.

In einer weiteren Ausführungsform ist $R^c$ wie oben definiert mit der Maßgabe, dass $R^c$ nicht gleich Pyridin oder Pyrimidin ist.

In einer weiteren Ausführungsform ist $R^d$ bei jedem Auftreten gleich H.

In einer weiteren Ausführungsform weist das organische Molekül eine Struktur der Formel A4 auf oder hat eine Struktur der Formel A4

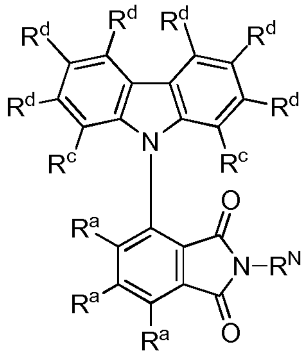

Formel A4

wobei gilt

$R^N$ ist Methyl, Phenyl, Xylyl, Mesityl, Naphtyl, Biphenyl, Naphthylphenyl, Terphenyl oder 2,4,6-Triphenylphenyl;

$R^a$ ist bei jedem Auftreten gleich oder verschieden H, Deuterium, eine Alkylgruppe oder eine Arylgruppe;

$R^c$ ist bei jedem Auftreten unabhängig voneinander ausgewählt aus der Gruppe bestehend aus $CF_3$, $C(=O)R^1$, CN, einer mit einem oder mehreren $R^2$ substituierten Alkylgruppe, einer mit einem oder mehreren $R^2$ substituierten Arylgruppe, die optional mit einer oder mehreren unsubstituierten Alkylgruppe und/oder mit einer oder mehreren unsubstituierten oder Alkyl-substituierten Arylgruppe substituiert ist, einer unsubstituierten oder mit einem oder mehreren $R^2$ und/oder mit einer oder mehreren unsubstituierten Alkylgruppe und/oder mit einer oder mehreren unsubstituierten oder Alkyl-substituierten Arylgruppe substituierten Heteroarylgruppe und einer Gruppe der Unterformel T1 oder einer Gruppe der Unterformel T2:

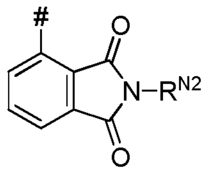

Unterformel T1

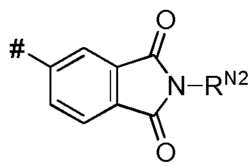

Unterformel T2;

$R^d$ ist bei jedem Auftreten gleich oder verschieden unabhängig voneinander ausgewählt aus der Gruppe H, Deuterium, $CF_3$, $C(=O)R^1$, CN, einer unsubstituierten oder mit einem oder mehreren $R^2$ substituierten Alkylgruppe, einer unsubstituierten oder mit einem oder mehreren $R^2$ und/oder mit einer oder mehreren unsubstituierten Alkylgruppe und/oder mit einer oder mehreren unsubstituierten oder Alkyl-substituierten Arylgruppe substituierten Arylgruppe, einer unsubstituierten oder mit $R^2$ und/oder mit einer oder mehreren unsubstituierten Alkylgruppe und/oder mit einer oder mehreren unsubstituierten oder Alkyl-substituierten Arylgruppe substituierten Heteroarylgruppe und einer Gruppe der Unterformel T1 oder einer Gruppe der Unterformel T2:

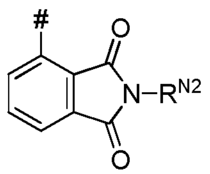

Unterformel T1

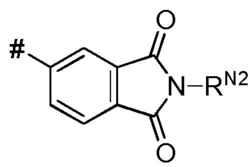

Unterformel T2

mit
$R^{N2}$ ist eine Alkylgruppe, eine unsubstituierte oder mit einem oder mehreren $R^{N3}$ substituierte Arylgruppe oder eine unsubstituierte oder mit einem oder mehreren $R^{N3}$ substituierte Heteroarylgruppe.

$R^{N3}$ ist eine Alkylgruppe, eine Arylgruppe oder eine Heteroarylgruppe.

In einer weiteren Ausführungsform ist $R^c$ bei jedem Auftreten unabhängig voneinander ausgewählt aus der Gruppe bestehend aus $CF_3$, $C(=O)R^1$, CN, einer mit einem oder mehreren $R^2$ substituierten Alkylgruppe, einer mit einem oder mehreren $R^2$ substituierten 6-Ring-Arylgruppe, die optional mit einer oder mehreren unsubstituierten Alkylgruppe und/oder mit einer oder mehreren unsubstituierten oder Alkyl-substituierten Arylgruppe substituiert ist, einer unsubstituierten oder mit einem oder mehreren $R^2$ und/oder mit einer oder mehreren unsubstituierten Alkylgruppe und/oder mit einer oder mehreren unsubstituierten oder Alkyl-substituierten Arylgruppe substituierten 6-Ring-Heteroarylgruppe und einer Gruppe der Unterformel T1 oder einer Gruppe der Unterformel T2:

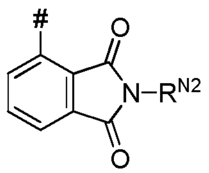

Unterformel T1

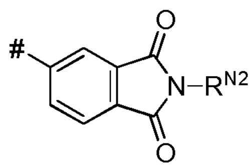

Unterformel T2

mit $R^{N2}$ ist eine Alkylgruppe, eine unsubstituierte oder mit einem oder mehreren $R^{N3}$ substituierte Arylgruppe oder eine unsubstituierte oder mit einem oder mehreren $R^{N3}$ substituierte Heteroarylgruppe.
$R^{N3}$ ist eine Alkylgruppe, eine Arylgruppe oder eine Heteroarylgruppe.

In einer weiteren Ausführungsform ist $R^c$ wie oben definiert mit der Maßgabe, dass $R^c$ nicht gleich Pyridin oder Pyrimidin ist.

In einer weiteren Ausführungsform ist $R^d$ bei jedem Auftreten gleich H.

In einer weiteren Ausführungsform weist das organische Molekül eine Struktur der Formel A5 auf oder hat eine Struktur der Formel A5.

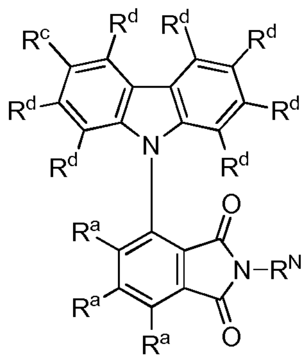

Formel A5

wobei gilt:

$R^N$ ist Methyl, Phenyl, Xylyl, Mesityl, Naphtyl, Biphenyl, Naphthylphenyl, Terphenyl oder 2,4,6-Triphenylphenyl;

$R^a$ ist bei jedem Auftreten gleich oder verschieden H, Deuterium, eine Alkylgruppe oder eine Arylgruppe;

$R^c$ ist bei jedem Auftreten gleich oder verschieden $CF_3$, $C(=O)R^1$, CN, eine mit einem oder mehreren $R^2$ substituierte Alkylgruppe, eine mit einem oder mehreren $R^2$ substituierte Arylgruppe, die optional mit einer oder mehreren unsubstituierten Alkylgruppe und/oder mit einer oder mehreren unsubstituierten oder Alkyl-substituierten Arylgruppe substituiert ist, eine unsubstituierte oder mit einem oder mehreren $R^2$ und/oder mit einer oder mehreren unsubstituierten Alkylgruppe und/oder mit einer oder mehreren unsubstituierten oder Alkyl-substituierten Arylgruppe substituierte Heteroarylgruppe oder eine Gruppe der Unterformel T1 oder eine Gruppe der Unterformel T2:

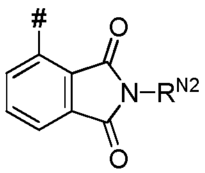

Unterformel T1

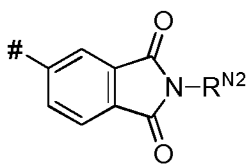

Unterformel T2

mit
$R^{N2}$ ist eine Alkylgruppe, eine unsubstituierte oder mit einem oder mehreren $R^{N3}$ substituierte Arylgruppe oder eine unsubstituierte oder mit einem oder mehreren $R^{N3}$ substituierte Heteroarylgruppe.

$R^{N3}$ ist eine Alkylgruppe, eine Arylgruppe oder eine Heteroarylgruppe;
$R^d$ ist bei jedem Auftreten gleich oder verschieden H, Deuterium, $CF_3$, $C(=O)R^1$, CN, eine unsubstituierte oder mit einem oder mehreren $R^2$ substituierte Alkylgruppe, eine unsubstituierte oder mit einem oder mehreren $R^2$ und/oder mit einer oder mehreren unsubstituierten Alkylgruppe und/oder mit einer oder mehreren unsubstituierten oder Alkyl-substituierten Arylgruppe substituierte Arylgruppe, eine unsubstituierte oder mit einem oder mehreren $R^2$ und/oder mit einer oder mehreren unsubstituierten Alkylgruppe und/oder mit einer oder mehreren unsubstituierten oder Alkyl-substituierten Arylgruppe substituierte Heteroarylgruppe oder eine Gruppe der Unterformel T1 oder eine Gruppe der Unterformel T2:

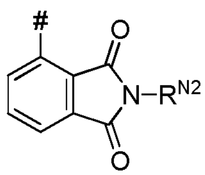

Unterformel T1

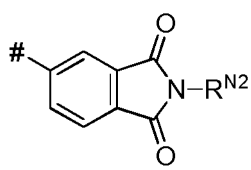

Unterformel T2

mit $R^{N2}$ ist eine Alkylgruppe, eine unsubstituierte oder mit einem oder mehreren $R^{N3}$ substituierte Arylgruppe oder eine

unsubstituierte oder mit einem oder mehreren $R^{N3}$ substituierte Heteroarylgruppe.

$R^{N3}$ ist eine Alkylgruppe, eine Arylgruppe oder eine Heteroarylgruppe.

In einer weiteren Ausführungsform ist $R^c$ bei jedem Auftreten unabhängig voneinander ausgewählt aus der Gruppe bestehend aus $CF_3$, C(=O)$R^1$, CN, einer mit einem oder mehreren $R^2$ substituierten Alkylgruppe, einer mit einem oder mehreren $R^2$ substituierten 6-Ring-Arylgruppe, die optional mit einer oder mehreren unsubstituierten Alkylgruppe und/oder mit einer oder mehreren unsubstituierten oder Alkyl-substituierten Arylgruppe substituiert ist, einer unsubstituierten oder mit einem oder mehreren $R^2$ und/oder mit einer oder mehreren unsubstituierten Alkylgruppe und/ oder mit einer oder mehreren unsubstituierten oder Alkyl-substituierten Arylgruppe substituierten 6-Ring-Heteroarylgruppe und einer Gruppe der Unterformel T1 oder einer Gruppe der Unterformel T2:

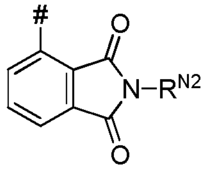

Unterformel T1

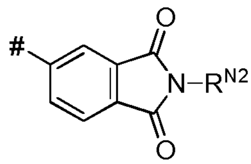

Unterformel T2

mit

$R^{N2}$ ist eine Alkylgruppe, eine unsubstituierte oder mit einem oder mehreren $R^{N3}$ substituierte Arylgruppe oder eine unsubstituierte oder mit einem oder mehreren $R^{N3}$ substituierte Heteroarylgruppe.

$R^{N3}$ ist eine Alkylgruppe, eine Arylgruppe oder eine Heteroarylgruppe.

In einer weiteren Ausführungsform ist $R^c$ wie oben definiert mit der Maßgabe, dass $R^c$ nicht gleich Pyridin oder Pyrimidin ist.

In einer weiteren Ausführungsform ist $R^d$ bei jedem Auftreten gleich H.

In einer Ausführungsform weist das organische Molekül eine Struktur der Formel A6 auf oder hat eine Struktur der Formel A6.

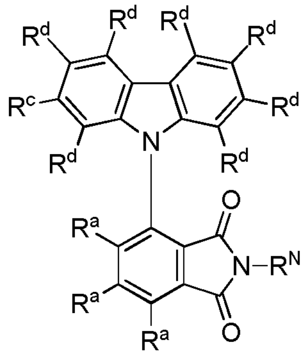

Formel A6

wobei gilt

$R^N$ ist Methyl, Phenyl, Xylyl, Mesityl, Naphtyl, Biphenyl, Naphthylphenyl, Terphenyl oder 2,4,6-Triphenylphenyl;

$R^a$ ist bei jedem Auftreten gleich oder verschieden H, Deuterium, eine Alkylgruppe oder eine Arylgruppe;

$R^c$ ist bei jedem Auftreten unabhängig voneinander ausgewählt aus der Gruppe bestehend aus $CF_3$, C(=O)$R^1$, CN, einer mit einem oder mehreren $R^2$ substituierten Alkylgruppe, einer mit einem oder mehreren $R^2$ substituierten Arylgruppe, die optional mit einer oder mehreren unsubstituierten Alkylgruppe und/oder mit einer oder mehreren unsubstituierten oder Alkyl-substituierten Arylgruppe substituiert ist, einer unsubstituierten oder mit einem oder mehreren $R^2$ und/oder mit einer oder mehreren unsubstituierten Alkylgruppe und/oder mit einer oder mehreren unsubstituierten oder Alkyl-substituierten Arylgruppe substituierten Heteroarylgruppe und einer Gruppe der Unterformel T1 oder einer Gruppe der Unterformel T2:

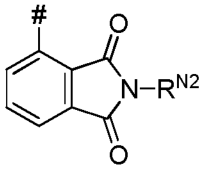
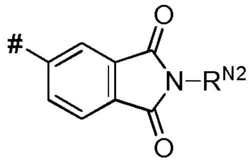

Unterformel T1 Unterformel T2

mit
$R^{N2}$ ist eine Alkylgruppe, eine unsubstituierte oder mit einem oder mehreren $R^{N3}$ substituierte Arylgruppe oder eine unsubstituierte oder mit einem oder mehreren $R^{N3}$ substituierte Heteroarylgruppe.

$R^{N3}$ ist eine Alkylgruppe, eine Arylgruppe oder eine Heteroarylgruppe;
$R^d$ ist bei jedem Auftreten unabhängig voneinander ausgewählt aus der Gruppe bestehend aus H, Deuterium, $CF_3$, C(=O)$R^1$, CN, einer unsubstituierten oder mit einem oder mehreren $R^2$ substituierten Alkylgruppe, einer unsubstituierten oder mit einem oder mehreren $R^2$ und/oder mit einer oder mehreren unsubstituierten Alkylgruppe und/ oder mit einer oder mehreren unsubstituierten oder Alkyl-substituierten Arylgruppe substituierten Arylgruppe, einer unsubstituierten oder mit $R^2$ und/oder mit einer oder mehreren unsubstituierten Alkylgruppe und/ oder mit einer oder mehreren unsubstituierten oder Alkyl-substituierten Arylgruppe substituierten Heteroarylgruppe und einer Gruppe der Unterformel T1 oder einer Gruppe der Unterformel T2:

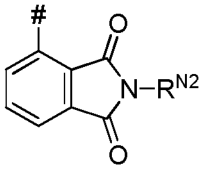
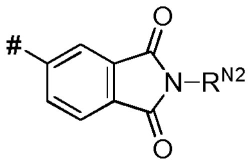

Unterformel T1 Unterformel T2

mit
$R^{N2}$ ist eine Alkylgruppe, eine unsubstituierte oder mit einem oder mehreren $R^{N3}$ substituierte Arylgruppe oder eine unsubstituierte oder mit einem oder mehreren $R^{N3}$ substituierte Heteroarylgruppe.
$R^{N3}$ ist eine Alkylgruppe, eine Arylgruppe oder eine Heteroarylgruppe.

In einer weiteren Ausführungsform ist $R^c$ bei jedem Auftreten unabhängig voneinander ausgewählt aus der Gruppe bestehend aus $CF_3$, C(=O)$R^1$, CN, einer mit einem oder mehreren $R^2$ substituierten Alkylgruppe, einer mit einem oder mehreren $R^2$ substituierten 6-Ring-Arylgruppe, die optional mit einer oder mehreren unsubstituierten Alkylgruppe und/oder mit einer oder mehreren unsubstituierten oder Alkyl-substituierten Arylgruppe substituiert ist, einer unsubstituierten oder mit einem oder mehreren $R^2$ und/oder mit einer oder mehreren unsubstituierten Alkylgruppe und/ oder mit einer oder mehreren unsubstituierten oder Alkyl-substituierten Arylgruppe substituierten 6-Ring-Heteroarylgruppe und einer Gruppe der Unterformel T1 oder einer Gruppe der Unterformel T2:

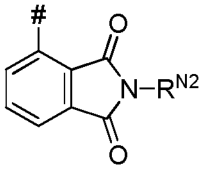
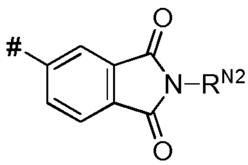

Unterformel T1 Unterformel T2

mit
$R^{N2}$ ist eine Alkylgruppe, eine unsubstituierte oder mit einem oder mehreren $R^{N3}$ substituierte Arylgruppe oder eine unsubstituierte oder mit einem oder mehreren $R^{N3}$ substituierte Heteroarylgruppe.
$R^{N3}$ ist eine Alkylgruppe, eine Arylgruppe oder eine Heteroarylgruppe.

In einer weiteren Ausführungsform ist $R^c$ wie oben definiert mit der Maßgabe, dass $R^c$ nicht gleich Pyridin oder Pyrimidin ist.

In einer weiteren Ausführungsform ist $R^d$ bei jedem Auftreten gleich H.

In einer weiteren Ausführungsform weist das organische Molekül eine Struktur der Formel A7 auf oder hat eine Struktur der Formel A7

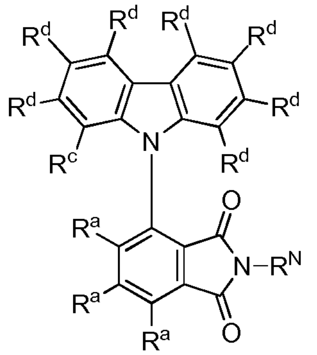

Formel A7

wobei gilt

$R^N$ ist Methyl, Phenyl, Xylyl, Mesityl, Naphtyl, Biphenyl, Naphthylphenyl, Terphenyl oder 2,4,6-Triphenylphenyl;

$R^a$ ist bei jedem Auftreten gleich oder verschieden H, Deuterium, eine Alkylgruppe oder eine Arylgruppe;

$R^c$ ist bei jedem Auftreten unabhängig voneinander ausgewählt aus der Gruppe bestehend aus $CF_3$, $C(=O)R^1$, CN, einer mit einem oder mehreren $R^2$ substituierten Alkylgruppe, einer mit einem oder mehreren $R^2$ substituierten Arylgruppe, die optional mit einer oder mehreren unsubstituierten Alkylgruppe und/oder mit einer oder mehreren unsubstituierten oder Alkyl-substituierten Arylgruppe substituiert ist, einer unsubstituierten oder mit einem oder mehreren $R^2$ und/oder mit einer oder mehreren unsubstituierten Alkylgruppe und/oder mit einer oder mehreren unsubstituierten oder Alkyl-substituierten Arylgruppe substituierten Heteroarylgruppe und einer Gruppe der Unterformel T1 oder einer Gruppe der Unterformel T2:

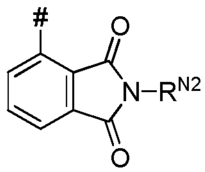

Unterformel T1

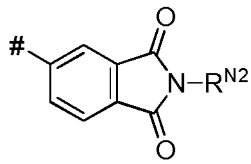

Unterformel T2

mit

$R^{N2}$ ist eine Alkylgruppe, eine unsubstituierte oder mit einem oder mehreren $R^{N3}$ substituierte Arylgruppe oder eine unsubstituierte oder mit einem oder mehreren $R^{N3}$ substituierte Heteroarylgruppe;

$R^{N3}$ ist eine Alkylgruppe, eine Arylgruppe oder eine Heteroarylgruppe;

$R^d$ ist bei jedem Auftreten gleich oder verschieden unabhängig voneinander ausgewählt aus der Gruppe H, Deuterium, $CF_3$, $C(=O)R^1$, CN, einer unsubstituierten oder mit einem oder mehreren $R^2$ substituierten Alkylgruppe, einer unsubstituierten oder mit einem oder mehreren $R^2$ und/oder mit einer oder mehreren unsubstituierten Alkylgruppe und/oder mit einer oder mehreren unsubstituierten oder Alkyl-substituierten Arylgruppe substituierten Arylgruppe, einer unsubstituierten oder mit $R^2$ und/oder mit einer oder mehreren unsubstituierten Alkylgruppe und/ oder mit einer oder mehreren unsubstituierten oder Alkyl-substituierten Arylgruppe substituierten Heteroarylgruppe und einer Gruppe der Unterformel T1 oder einer Gruppe der Unterformel T2:

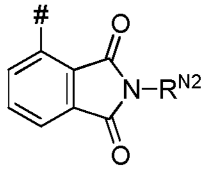
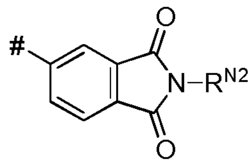

Unterformel T1 Unterformel T2

mit

$R^{N2}$ ist eine Alkylgruppe, eine unsubstituierte oder mit einem oder mehreren $R^{N3}$ substituierte Arylgruppe oder eine unsubstituierte oder mit einem oder mehreren $R^{N3}$ substituierte Heteroarylgruppe;

$R^{N3}$ ist eine Alkylgruppe, eine Arylgruppe oder eine Heteroarylgruppe.

In einer weiteren Ausführungsform ist $R^c$ bei jedem Auftreten unabhängig voneinander ausgewählt aus der Gruppe bestehend aus $CF_3$, C(=O)$R^1$, CN, einer mit einem oder mehreren $R^2$ substituierten Alkylgruppe, einer mit einem oder mehreren $R^2$ substituierten 6-Ring-Arylgruppe, die optional mit einer oder mehreren unsubstituierten Alkylgruppe und/oder mit einer oder mehreren unsubstituierten oder Alkyl-substituierten Arylgruppe substituiert ist, einer unsubstituierten oder mit einem oder mehreren $R^2$ und/oder mit einer oder mehreren unsubstituierten Alkylgruppe und/ oder mit einer oder mehreren unsubstituierten oder Alkyl-substituierten Arylgruppe substituierten 6-Ring-Heteroarylgruppe und einer Gruppe der Unterformel T1 oder einer Gruppe der Unterformel T2:

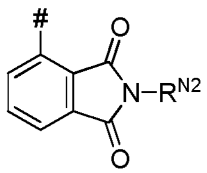
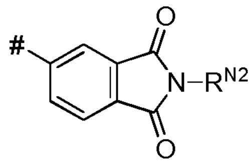

Unterformel T1 Unterformel T2

mit

$R^{N2}$ ist eine Alkylgruppe, eine unsubstituierte oder mit einem oder mehreren $R^{N3}$ substituierte Arylgruppe oder eine unsubstituierte oder mit einem oder mehreren $R^{N3}$ substituierte Heteroarylgruppe;
$R^{N3}$ ist eine Alkylgruppe, eine Arylgruppe oder eine Heteroarylgruppe.

In einer weiteren Ausführungsform ist $R^c$ wie oben definiert mit der Maßgabe, dass $R^c$ nicht gleich Pyridin oder Pyrimidin ist.
In einer weiteren Ausführungsform ist $R^d$ bei jedem Auftreten gleich H.
Eine Ausführungsform betrifft ein organisches Molekül der Formel A1, A2, A3, A4, A5, A6 oder A7, das mindestens eine CN-Gruppe aufweist.
Unter einer Arylgruppe, die jeweils mit den oben genannten Resten substituiert sein kann und die über beliebige Positionen am Aromaten verknüpft sein kann, werden im Sinne der Erfindung Gruppen verstanden, welche abgeleitet sind von: Benzol, Naphthalin, Anthracen und Phenanthren.
Beispielhafte Phenyl- oder Alkyl-substituierte 6-Ring-Arylgruppen sind insbesondere Toluol, Ethylbenzol, i-Propylbenzol, t-Butylbenzol, i-Butylbenzol, *o*-Xylol (1,2-Dimethylbenzol), *m*-Xylol (1,3-Dimethylbenzol), *p*-Xylol (1,4-Dimethylbenzol), 1,5-Dimethylbenzol, 1,2-Diethylbenzol, 1,3-Diethylbenzol, 1,4-Diethylbenzol, 1,5-Diethylbenzol, 1,2-Di-i-propylbenzol, 1,3-Di-i-propylbenzol, 1,4-Di-i-propylbenzol, 1,5-Di-i-propylbenzol, 1,2-Di-t-butylbenzol, 1,3-Di-t-butylbenzol, 1,4-Di-t-butylbenzol, 1,5-Di-t-butylbenzol, Mesitylen (1,3,5-Trimethylbenzol), 1,3,5-Triethylbenzol, 1,3,5-Tri-i-propylbenzol, 1,3,5-Tri-t-butylbenzol, Phenylbenzol, 1,2-Diphenylbenzol, 1,3-Diphenylbenzol, 1,4-Diphenylbenzol, und 1,3,5-Triphenylbenzol.
Unter einer Heteroarylgruppe, die jeweils mit den oben genannten Resten substituiert sein kann und die über beliebige Positionen am Heteroaromaten verknüpft sein kann, werden im Sinne der Erfindung Gruppen verstanden, welche abgeleitet sind von: Pyridin, Pyridazin (1,2-Diazin), Pyrimidin (1,3-Diazin), Pyrazin (1,4-Diazin), 1,3,5-Triazin, Acridin, Chinolin, Isochinolin, Chinoxalin und Naphthyridin.

Beispielhafte Phenyl- oder Alkyl-substituierte 6-Ring-Heteroarylgruppen sind insbesondere 2-Picolin (2-Methylpyridin), 3-Picolin (3-Methylpyridin), 4-Picolin (4-Methylpyridin), 2-Ethylpyridin, 3-Ethylpyridin, 4-Ethylpyridin, 2-i-Propylpyridin, 3-i-Propylpyridin, 4-i-Propylpyridin, 2-t-Butylpyridin, 3-t-Butylpyridin, 4-t-Butylpyridin, 2-i-Butylpyridin, 3-i-Butylpyridin, 4-i-Butylpyridin, 2,3-Dimethylpyridin, 2,4-Dimethylpyridin, 2,5-Dimethylpyridin, 2,6-Dimethylpyridin, 2,3-Diethylpyridin, 2,4-Diethylpyridin, 2,5-Diethylpyridin, 2,6-Diethylpyridin, 2,3-Di-i-propylpyridin, 2,4-Di-i-propylpyridin, 2,5-Di-i-propylpyridin, 2,6-Di-i-propylpyridin, 2,3-Di-t-butylpyridin, 2,4-Di-t-butylpyridin, 2,5-Di-t-butylpyridin, 2,6-Di-t-butylpyridin, 2,4,6-Trimethylpyridin, 2,4,6-Triethylpyridin, 2,4,6-Tri-i-propylpyridin, 2,4,6-Tri-t-butylpyridin, 2-Methylpyrimidin, 4-Methylpyrimidin, 5-Methylpyrimidin, 2,4-Dimethylpyrimidin, 2,5-Dimethylpyrimidin, 4,5-Dimethylpyrimidin, 4,6-Dimethylpyrimidin, 2,4-Diethylpyrimidin, 2,5-Diethylpyrimidin, 4,5-Diethylpyrimidin, 4,6-Diethylpyrimidin, 2,4-Di-i-propylpyrimidin, 2,5-Di-i-propylpyrimidin, 4,5-Di-i-propylpyrimidin, 4,6-Di-i-propylpyrimidin, 2,4-Di-t-butylpyrimidin, 2,5-Di-t-butylpyrimidin, 4,5-Di-t-butylpyrimidin, 4,6-Di-t-butylpyrimidin 2,4,5-Trimethylpyrimidin, 2,4,5-Triethylpyrimidin, 2,4,5-Tri-i-propylpyrimidin, 2,4,5-Tri-t-butylpyrimidin, 2,4,5-Trimethylpyrimidin, 2,4,6-Triethylpyrimidin, 2,4,6-Tri-i-propylpyrimidin, 2,4,6-Tri-t-butylpyrimidin, 4,5,6-Trimethylpyrimidin, 4,5,6-Triethylpyrimidin, 4,5,6-Tri-i-propylpyrimidin, 4,5,6-Tri-t-butylpyrimidin, 4,5,6-Trimethylpyrimidin, 2,4-Dimethyl-1,3,5-triazin und 2,4-Diphenyl-1,3,5-triazin.

Im Rahmen der vorliegenden Erfindung werden unter einer Alkylgruppe, in der optional einzelne H-Atome durch die oben genannten Gruppen substituiert sein können, beispielsweise die Reste Methyl, Ethyl, n-Propyl, i-Propyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl verstanden.

Die Anbindung der Reste $R^N$ kann über eine beliebige Position erfolgen. Im Sinne dieser Erfindung werden unter Biphenyl beispielsweise ortho-Biphenyl, para-Biphenyl, meta-Biphenyl, unter Terphenyl 1,2-Diphenylphenyl, 1,3-Diphenylphenyl und 1,4-Diphenylphenyl, und unter Naphthylphenyl beispielsweise ortho-Naphthylphenyl, meta-Naphthylphenyl, para-Naphthylphenyl verstanden. Beispielhafte, somit nicht limitierende Ausführungsformen sind:

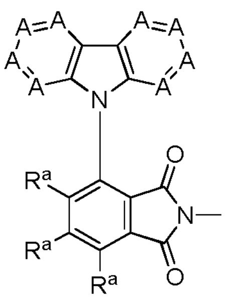
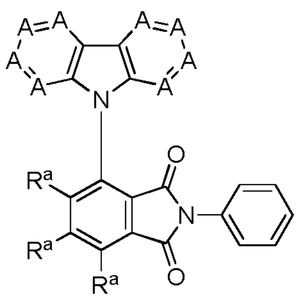
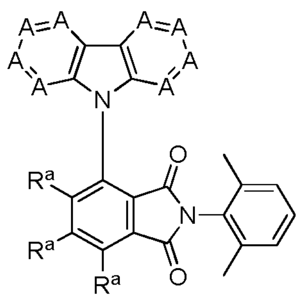
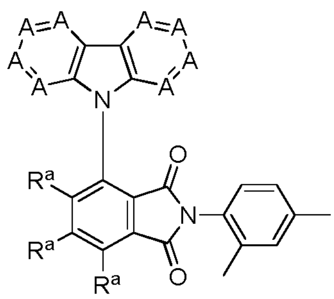

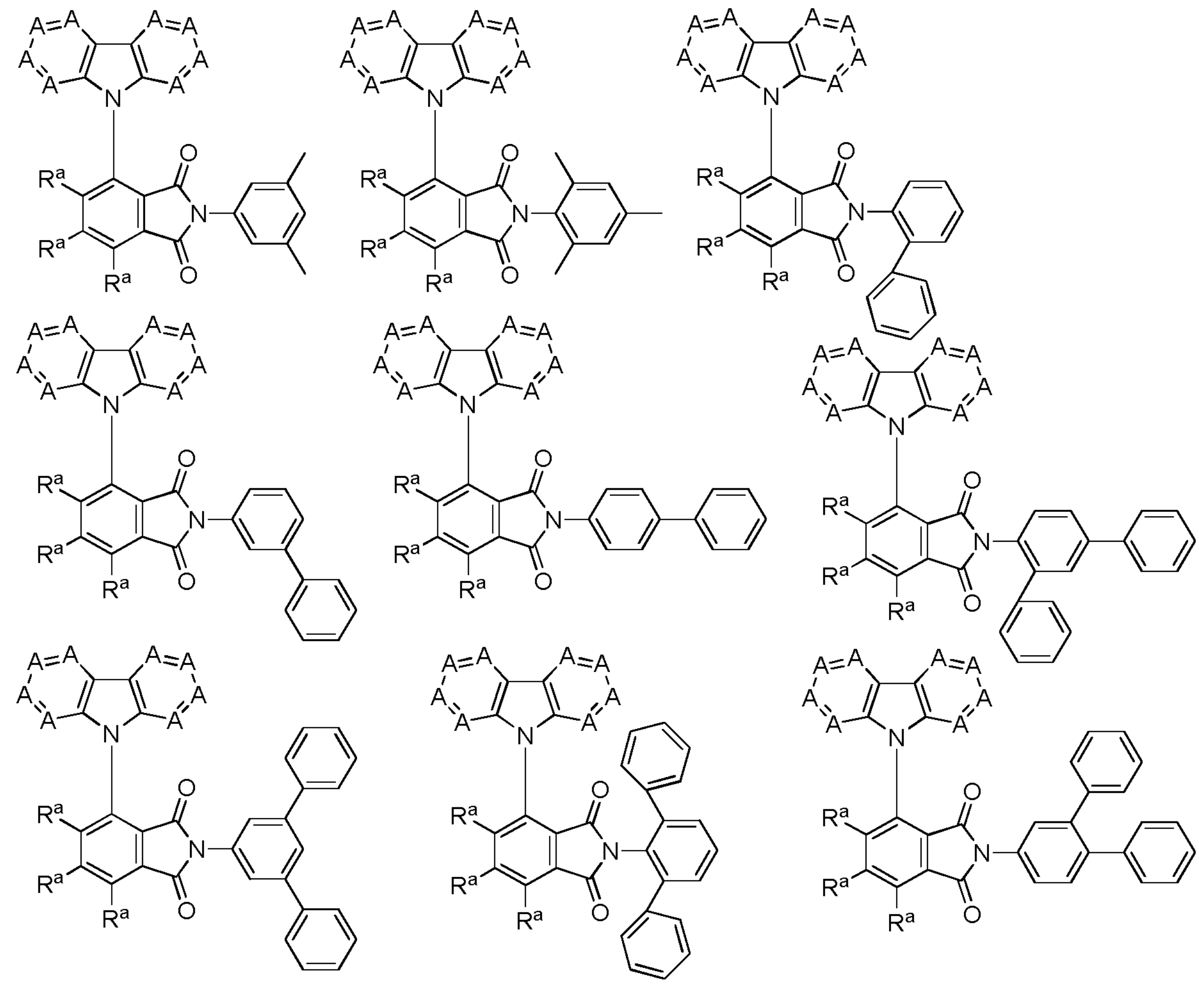

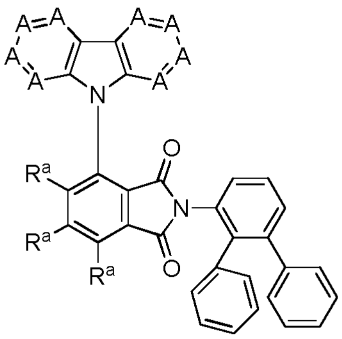

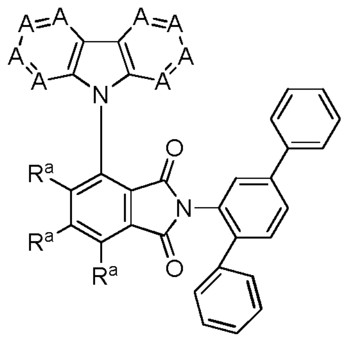

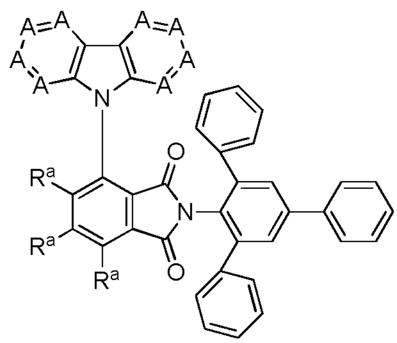

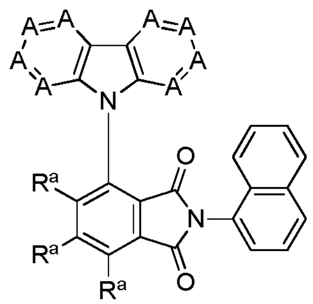

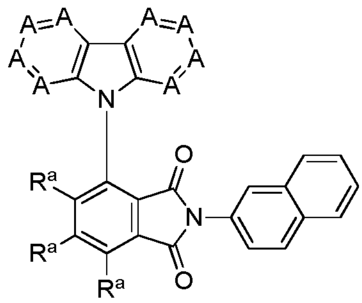

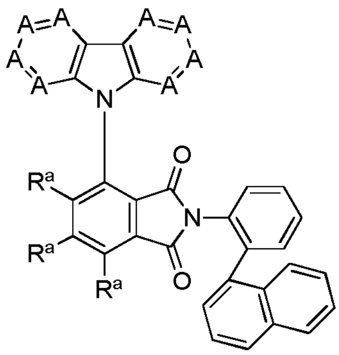

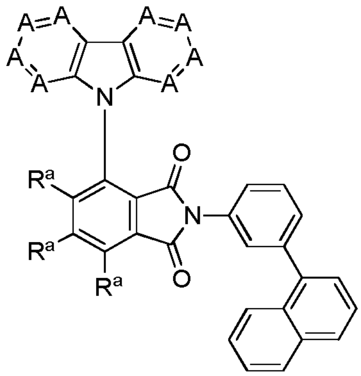

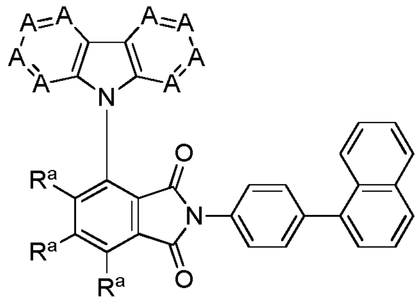

Die erfindungsgemäßen Moleküle weisen hohe Photolumineszenz Quantenausbeuten und geringe Exzitonenlebensdauern auf und stellen daher vorteilhafte Emittermaterialien für blaue OLEDs dar.

Eine Ausführungsform der Erfindung betrifft organische Moleküle, welche ein Emissionsmaximum bei zwischen 420 und 490 nm, bevorzugter zwischen 430 und 470 nm, noch bevorzugter zwischen 440 und 460 nm aufweisen.

Eine Ausführungsform der Erfindung betrifft organische Moleküle, welche eine Emissionslebensdauer von höchstens 150 $\mu$s, insbesondere von höchstens 100 $\mu$s, von höchsten 50 $\mu$s, oder von höchstens 10 $\mu$s aufweisen und/oder eine Hauptemissionsbande mit einer Halbwertsbreite kleiner als 120 nm, insbesondere kleiner als 100 nm, kleiner als 80 nm, oder kleiner als 60 nm aufweisen und/oder eine Photolumineszenz-Quantenausbeute (PLQY) von größer 30 %, insbesondere von größer 35 %, von größer 40 %, von größer 45 %, von größer 50 %, oder von größer 60 % aufweisen und/oder einen $\Delta E(S_1\text{-}T_1)$-Wert zwischen dem untersten angeregten Singulett ($S_1$)- und dem darunter liegenden Triplett ($T_1$)-Zustand von nicht höher als 5000 $cm^{-1}$, insbesondere nicht höher als 3000 $cm^{-1}$, oder nicht höher als 1500 $cm^{-1}$ oder 1000 $cm^{-1}$ aufweisen.

Insbesondere weisen die erfindungsgemäßen Moleküle einen "blue material index" (BMI), den Quotienten aus der PLQY (in %) und ihrer $CIE_{y}$-Farbkoordinate des von dem erfindungsgemäßen Molekül emittierten Lichts, von größer 150, insbesondere von größer 200, von größer 250 oder von größer 300 auf.

Die Bestimmung des $\Delta E(S_1\text{-}T_1)$-Wertes kann sowohl durch quantenmechanische Berechnungen mittels im Stand der Technik bekannten Computerprogrammen durchgeführt werden (z. B. mittels Turbomole-Programmen unter Ausführung von TD-DFT- und unter Berücksichtigung von CC2-Rechnungen) oder - wie weiter unten erläutert wird - experimentell bestimmt werden.

Die Energiedifferenz $\Delta E(S_1\text{-}T_1)$ lässt sich näherungsweise quantenmechanisch durch das mit dem Faktor 2 multiplizierte sogenannte Austauschintegral beschreiben. Dessen Wert hängt direkt ab von der Überlappung der Molekülorbitale. Diese Molekülorbitale sind über unterschiedliche Raumbereiche verteilt (teil-delokalisiert über $\pi$- bzw. $\pi^*$-Molekülorbitale). Das heißt, ein elektronischer Übergang zwischen den verschiedenen Molekülorbitalen repräsentiert einen sogenannten Charge-Transfer (CT)-Übergang. Je geringer die Überlappung der oben beschriebenen Molekülorbitale ist, desto ausgeprägter ist der elektronische Charge-Transfer Charakter. Das ist dann mit einer Abnahme des Austausch-Integrals und somit einer Abnahme der Energiedifferenz $\Delta E(S_1\text{-}T_1)$ verbunden.

Die Bestimmung des $\Delta E(S_1\text{-}T_1)$-Wertes kann experimentell folgendermaßen erfolgen:

Für ein vorgegebenes organisches Molekül lässt sich der Energieabstand $\Delta E(S_1\text{-}T_1) = \Delta E$ unter Verwendung der oben angegebenen Gleichung (1) einfach bestimmen. Eine Umformung ergibt:

$$\ln\{Int(S_1\rightarrow S_0)/Int(T_1\rightarrow S_0)\} = \ln\{k(S_1)/k(T_1)\} - (\Delta E/k_B)(1/T) \qquad (3)$$

Für die Messung der Intensitäten $Int(S_1\rightarrow S_0)$ und $Int(T_1\rightarrow S_0)$ kann jedes handelsübliche Spektralphotometer verwendet

werden. Eine graphische Auftragung der bei verschiedenen Temperaturen gemessenen (logarithmierten) Intensitätsverhältnisse ln{Int($S_1 \rightarrow S_0$)/Int($T_1 \rightarrow S_0$)} gegen den Kehrwert der absoluten Temperatur T ergibt in der Regel eine Gerade. Die Messung wird in einem Temperaturbereich von Raumtemperatur (300 K) bis 77 K oder bis 4,2 K durchgeführt, wobei die Temperatur mittels eines Kryostaten eingestellt wird. Die Intensitäten werden aus den (korrigierten) Spektren bestimmt, wobei Int($S_1 \rightarrow S_0$) bzw. Int($T_1 \rightarrow S_0$) die integrierten Fluoreszenz- bzw. Phosphoreszenz-Bandenintensitäten repräsentieren, welche sich mittels der zum Spektralphotometer gehörenden Programme bestimmen lassen. Die jeweiligen Übergänge (Bandenintensitäten) lassen sich leicht identifizieren, da die Triplett-Bande bei niedrigerer Energie liegt als die Singulett-Bande und mit sinkender Temperatur an Intensität gewinnt. Dabei werden die Messungen in sauerstofffreien verdünnten Lösungen (ca. $10^{-2}$ mol/L) oder an dünnen Filmen aus den entsprechenden Molekülen oder an mit den entsprechenden Molekülen dotierten Filmen durchgeführt. Verwendet man als Probe eine Lösung, so empfiehlt es sich, ein Lösemittel bzw. Lösemittelgemisch zu verwenden, das bei tiefen Temperaturen Gläser bildet, wie 2-Methyl-THF, THF (Tetrahydrofuran) oder aliphatische Kohlenwasserstoffe. Verwendet man als Probe einen Film, so eignet sich die Verwendung einer Matrix mit einer deutlich größeren Singulettsowie Triplett-Energie als die der organischen Emittermoleküle, z. B. PMMA (Polymethylmethacrylat). Dieser Film kann aus Lösung aufgebracht werden.

Die Geradensteigung beträgt $-\Delta E/k_B$. Mit $k_B$ = 1,380 $10^{-23}$ $JK^{-1}$ = 0,695 $cm^{-1}$ $K^{-1}$ lässt sich der Energieabstand direkt bestimmen.

Eine äquivalente Betrachtungsweise zeigt, dass mittels der Messung der Temperaturabhängigkeit der Emissionsabklingzeit der $\Delta E(S_1-T_1)$-Wert auch bestimmt werden kann.

Eine einfache, näherungsweise Abschätzung des $\Delta E(S_1-T_1)$-Wertes kann auch dadurch vorgenommen werden, dass bei tiefer Temperatur (z. B. 77 K oder 4,2 K unter Verwendung eines Kryostaten) die Fluoreszenz- und Phosphoreszenz-Spektren registriert werden. Der $\Delta E(S_1-T_1)$-Wert entspricht dann in Näherung der Energiedifferenz zwischen den hochenergetischen Anstiegsflanken der Fluoreszenz- bzw. Phosphoreszenz-Bande.

Je ausgeprägter der CT-Charakter eines organischen Moleküls ist, desto stärker verändern sich die elektronischen Übergangsenergien als Funktion der Lösungsmittelpolarität. So gibt bereits eine ausgeprägte Polaritätsabhängigkeit der Emissionsenergien einen Hinweis auf das Vorliegen kleiner $\Delta E(S_1-T_1)$-Werte.

In einem weiteren Aspekt betrifft die Erfindung die Verwendung der organischen Moleküle als lumineszierender Emitter oder als Hostmaterial in einer organischen optoelektronischen Vorrichtung, insbesondere wobei die organische optoelektronische Vorrichtung ausgewählt ist aus der Gruppe bestehend aus:

- organischen lichtemittierenden Dioden (OLEDs),
- lichtemittierenden elektrochemischen Zellen,
- OLED-Sensoren, insbesondere in nicht hermetisch nach außen abgeschirmten Gas- und Dampf-Sensoren,
- organischen Dioden,
- organischen Solarzellen,
- organischen Transistoren,
- organischen Feldeffekttransistoren,
- organischen Lasern und
- Down-Konversions-Elementen.

In einem weiteren Aspekt betrifft die Erfindung eine Zusammensetzung aufweisend oder bestehend aus:

(a) mindestens einem erfindungsgemäßen organischen Molekül, insbesondere als Emitter und/oder Host, und

(b) mindestens einem (d. h. einem, zwei oder mehreren) Emitter- und/oder Hostmaterialien, die von dem erfindungsgemäßen organischen Molekül verschiedenen ist bzw. sind und

(c) optional mindestens einem Farbstoff und/ oder mindestens einem organischen Lösungsmittel.

In einer Ausführungsform besteht die erfindungsgemäße Zusammensetzung aus einem erfindungsgemäßen organischen Molekül und einem oder mehreren Hostmaterialien. Das oder die Hostmaterialen weisen insbesondere Triplett ($T_1$)- und Singulett ($S_1$)- Energieniveaus auf, die energetisch höher liegen als die Triplett ($T_1$)- und Singulett ($S_1$)-Energieniveaus des erfindungsgemäßen organischen Moleküls. In einer Ausführungsform weist die Zusammensetzung neben dem erfindungsgemäßen organischen Molekül ein elektronendominantes und ein lochdominantes Hostmaterial auf. Das höchste besetzte Orbital (HOMO) und das niedrigste unbesetzte Orbital (LUMO) des lochdominanten Hostmaterials liegen energetisch insbesondere höher als das jeweilige des elektronendominanten Hostmaterials. Das HOMO des lochdominanten Hostmaterials liegt energetisch unter dem HOMO des erfindungsgemäßen organischen Moleküls, während das LUMO des elektronendominanten Hostmaterials energetisch über dem LUMO des erfindungsgemäßen organischen Moleküls liegt. Um Exciplex-Formation zwischen Emitter und Hostmaterial oder Hostmaterialien zu vermeiden, sollten die Materialien so gewählt sein, dass die Energieabstände zwischen den jeweiligen Orbitalen gering sind. Der Abstand zwischen dem LUMO des elektronendominanten Hostmaterials und dem LUMO des erfindungsgemäßen organischen Moleküls beträgt insbesondere weniger als 0,5 eV, bevorzugt weniger als 0,3 eV, noch bevorzugter weniger als 0,2 eV. Der Abstand zwischen dem HOMO des lochdominanten Hostmaterials und dem HOMO des erfindungsgemäßen organischen Moleküls beträgt insbesondere weniger als 0,5 eV, bevorzugt weniger als 0,3 eV, noch bevorzugter weniger als 0,2 eV.
In einem weiteren Aspekt betrifft die Erfindung eine organische optoelektronische Vorrichtung, die ein erfindungsgemäßes organisches Molekül oder eine erfindungsgemäße Zusammensetzung aufweist. Die organische optoelektronische Vorrichtung ist insbesondere ausgeformt als eine Vorrichtung ausgewählt aus der Gruppe bestehend aus organischer lichtemittierender Diode (OLED); lichtemittierender elektrochemischer Zelle; OLED-Sensor, insbesondere nicht hermetisch nach außen abgeschirmten Gas- und Dampf-Sensoren; organischer Diode; organischer Solarzelle; organischem Transistor; organischem Feldeffekttransistor; organischem Laser und Down-Konversion-Element.
Eine organische optoelektronische Vorrichtung aufweisend

- ein Substrat,
- eine Anode und
- eine Kathode, wobei die Anode oder die Kathode auf das Substrat aufgebracht sind, und
- mindestens eine lichtemittierende Schicht, die zwischen Anode und Kathode angeordnet ist und die ein erfindungsgemäßes organisches Molekül aufweist, stellt einen weitere Ausführungsform der Erfindung dar.

In einer Ausführungsform handelt es sich bei der optoelektronischen Vorrichtung um eine OLED. Eine typische OLED weist beispielsweise folgenden Schichtaufbau auf:

1. Substrat (Trägermaterial)
2. Anode
3. Lochinjektionsschicht (hole injection layer, HIL)
4. Lochtransportschicht (hole transport layer, HTL)
5. Elektronenblockierschicht (electron blocking layer, EBL)
6. Emitterschicht (emitting layer, EML)
7. Lochblockierschicht (hole blocking layer, HBL)
8. Elektronenleitschicht (electron transport layer, ETL)
9. Elektroneninjektionsschicht (electron injection layer, EIL)
10. Kathode.

Dabei sind einzelne Schichten lediglich in optionaler Weise vorhanden. Weiterhin können mehrere dieser Schichten zusammenfallen. Und es können einzelne Schichten mehrfach im Bauteil vorhanden sein.
Gemäß einer Ausführungsform ist mindestens eine Elektrode des organischen Bauelements transluzent ausgebildet. Hier wird mit "transluzent" eine Schicht bezeichnet, die durchlässig für sichtbares Licht ist. Dabei kann die transluzente Schicht klar durchscheinend, also transparent, oder zumindest teilweise Licht absorbierend und/oder teilweise Licht streuend sein, so dass die transluzente Schicht beispielsweise auch diffus oder milchig durchscheinend sein kann. Insbesondere ist eine hier als transluzent bezeichnete Schicht möglichst transparent ausgebildet, so dass insbesondere die Absorption von Licht so gering wie möglich ist.
Gemäß einer weiteren Ausführungsform weist das organische Bauelement, insbesondere eine OLED, einen invertierten Aufbau auf. Der invertierte Aufbau zeichnet sich dadurch aus, dass sich die Kathode auf dem Substrat befindet und die anderen Schichten entsprechend invertiert aufgebracht werden:

1. Substrat (Trägermaterial)

2. Kathode

3. Elektroneninjektionsschicht (electron injection layer, EIL)

4. Elektronenleitschicht (electron transport layer, ETL)

5. Lochblockierschicht (hole blocking layer, HBL)

6. Emissionsschicht bzw. Emitterschicht (emitting layer, EML)

7. Elektronenblockierschicht (electron blocking layer, EBL)

8. Lochtransportschicht (hole transport layer, HTL)

9. Lochinjektionsschicht (hole injection layer, HIL)

10. Anode

Dabei sind einzelne Schichten lediglich in optionaler Weise vorhanden. Weiterhin können mehrere dieser Schichten zusammenfallen. Und es können einzelne Schichten mehrfach im Bauteil vorhanden sein.
In einer Ausführungsform wird bei der invertierten OLED die Anodenschicht des typischen Aufbaus, z.B. eine ITO-Schicht (Indium-Zinn-Oxid), als Kathode geschaltet.
Gemäß einer weiteren Ausführungsform weist das organische Bauelement, insbesondere eine OLED, einen gestapelten Aufbau auf. Hierbei werden die einzelnen OLEDs übereinander und nicht wie üblich nebeneinander angeordnet. Durch einen gestapelten Aufbau kann die Erzeugung von Mischlicht ermöglicht werden. Beispielsweise kann dieser Aufbau bei der Erzeugung von weißem Licht eingesetzt werden, für dessen Erzeugung das gesamte sichtbare Spektrum typischerweise durch die Kombination des emittierten Lichts von blauen, grünen und roten Emittern abgebildet wird. Weiterhin können bei praktisch gleicher Effizienz und identischer Leuchtdichte signifikant längere Lebensdauern im Vergleich zu üblichen OLEDs erzielt werden. Für den gestapelten Aufbau wird optional eine sogenannte Ladungserzeugungsschicht (charge generation layer, CGL) zwischen zwei OLEDs eingesetzt. Diese besteht aus einer n-dotierten und einem p-dotierten Schicht, wobei die n-dotierte Schicht typischerweise näher an der Anode aufgebracht wird.
In einer Ausführungsform - einer sogenannten Tandem-OLED - treten zwei oder mehr Emissionsschichten zwischen Anode und Kathode auf. In einer Ausführungsform sind drei Emissionsschichten übereinander angeordnet, wobei eine Emissionsschicht rotes Licht emittiert, eine Emissionsschicht grünes Licht emittiert und eine Emissionsschicht blaues Licht emittiert und optional weitere Ladungserzeugungs-, Blockier- oder Transportschichten zwischen den einzelnen Emissionsschichten aufgebracht sind. In einer weiteren Ausführungsform werden die jeweiligen Emissionsschichten direkt angrenzend aufgebracht. In einer weiteren Ausführungsform befindet sich jeweils eine Ladungserzeugungsschicht zwischen den Emissionsschichten. Weiterhin können in einer OLED direkt angrenzende und durch Ladungserzeugungsschichten getrennte Emissionsschichten kombiniert werden.
Über den Elektroden und den organischen Schichten kann weiterhin noch eine Verkapselung angeordnet sein. Die Verkapselung kann beispielsweise in Form eines Glasdeckels oder in Form einer Dünnschichtverkapselung ausgeführt sein.
Als Trägermaterial der optoelektronischen Vorrichtung kann beispielsweise Glas, Quarz, Kunststoff, Metall, ein Siliziumwafer oder jedes andere geeignete feste oder flexible, optional durchsichtige Material dienen.
Das Trägermaterial kann beispielsweise ein oder mehrere Materialien in Form einer Schicht, einer Folie, einer Platte oder einem Laminat aufweisen.
Als Anode der optoelektronischen Vorrichtung können beispielsweise transparente leitende Metalloxide wie beispielsweise ITO (Indium-Zinn-Oxid), Zinkoxid, Zinnoxid, Cadmiumoxid, Titanoxid, Indiumoxid oder Aluminiumzinkoxid (AZO), $Zn_2SnO_4$, $CdSnO_3$, $ZnSnO_3$, $MgIn_2O_4$, $GaInO_3$, $Zn_2In_2O_5$ oder $In_4Sn_3O_{12}$ oder Mischungen unterschiedlicher transparenter leitender Oxide dienen.
Als Materialien einer HIL können beispielsweise PEDOT:PSS (Poly-3,4-ethylendioxythiophen:Polystyrolsulfonsäure), PEDOT (Poly-3,4-ethylendioxythiophen), m-MTDATA (4,4',4"-Tris[phenyl(m-tolyl)amino]triphenylamin), Spiro-TAD (2,2',7,7'-Tetrakis(N,N-diphenylamino)-9,9-spirobifluoren), DNTPD (4,4'-Bis[N-[4-{N,N-bis(3-methyl-phenyl)amino}phenyl]-N-phenylamino]biphenyl), NPB (N,N'-Bis-(1-naphthalenyl)-N,N'-bis-phenyl-(1,1'-biphenyl)-4,4'-diamin), NPNPB (N,N'-Diphenyl-N,N'-di-[4-(N,N-diphenyl-amino)phenyl]benzol), MeO-TPD (N,N,N',N'-Tetrakis(4-methoxyphenyl)benzol), HAT-CN (1,4,5,8,9,11-Hexaazatriphenylen-hexacarbonitril) oder Spiro-NPD (N,N'-diphenyl-N,N'-Bis-(1-naphthyl)-9,9'-spirobifluorene-2,7-diamin) dienen. Beispielhaft ist die Schichtdicke 10-80 nm. Des Weiteren können kleine Moleküle können verwendet werden (z. B. Kupfer-Phthalocyanin (CuPc z. B. 10 nm dick)) oder Metalloxide wie beispielhaft $MoO_3$, $V_2O_5$.
Als Materialien einer HTL können tertiäre Amine, Carbazolderivate, mit Polystyrolsulfonsäure dotiertes Polyethylendioxythiophen, mit Camphersulfonsäure dotiertes Polyanilin poly-TPD (Poly(4-butylphenyl-diphenyl-amin)), [alpha]-NPD (Poly(4-butylphenyl-diphenyl-amin)), TAPC (4,4'-Cyclohexyliden-bis[*N*,*N*-bis(4-methylphenyl)benzenamin]), TCTA (Tris(4-carbazoyl-9-ylphenyl)amin), 2-TNATA (4,4',4"-Tris[2-naphthyl(phenyl)amino]triphenylamin), Spiro-TAD, DNTPD, NPB, NPNPB, MeO-TPD, HAT-CN oder TrisPcz (9,9'-Diphenyl-6-(9-phenyl-9H-carbazol-3-yl)-9H,9'H-3,3'-bicarbazol) dienen. Beispielhaft ist die Schichtdicke 10-100 nm.
Die HTL kann eine p-dotierte Schicht aufweisen, die einen anorganischen oder organischen Dotierstoff in einer organischen löcherleitenden Matrix aufweist. Als anorganischer Dotierstoff können beispielsweise Übergangsmetalloxide wie etwa Vanadiumoxid, Molybdänoxid oder Wolframoxid genutzt werden. Als organische Dotierstoffe können beispielsweise Tetrafluorotetracyanoquinodimethan (F4-TCNQ), Kupfer-Pentafluorobenzoat (Cu(I)pFBz) oder Übergangsmetallkomplexe verwendet werden. Beispielhaft ist die Schichtdicke 10 nm bis 100 nm.

Als Materialien einer Elektronenblockierschicht können beispielsweise mCP (1,3-Bis(carbazol-9-yl)benzol), TCTA, 2-TNATA, mCBP (3,3-Di(9H-carbazol-9-yl)biphenyl), tris-Pcz (9,9'-Diphenyl-6-(9-phenyl-9H-carbazol-3-yl)-9H,9'H-3,3'-bicarbazol), CzSi (9-(4-tert-Butylphenyl)-3,6-bis(triphenylsilyl)-9H-carbazol) oder DCB (N,N'-Dicarbazolyl-1,4-dimethylbenzol) dienen. Beispielhaft ist die Schichtdicke 10 nm bis 50 nm.

Die Emitter-Schicht EML oder Emissionsschicht besteht aus oder enthält Emittermaterial oder eine Mischung aufweisend mindestens zwei Emittermaterialien und optional ein oder mehreren Hostmaterialien. Geeignete Hostmaterialien sind beispielsweise mCP, TCTA, 2-TNATA, mCBP, CBP (4,4'-Bis-(N-carbazolyl)-biphenyl), Sif87 (Dibenzo[b,d]thiophen-2-yltriphenylsilan), Sif88 (Dibenzo[b,d]thiophen-2-yl)diphenylsilan) oder DPEPO (Bis[2-((oxo)diphenylphosphino)phenyl]ether). Für im Grünen oder im Roten emittierendes Emittermaterial oder einer Mischung aufweisend mindestens zwei Emittermaterialien eignen sich die gängigen Matrixmaterialien wie CBP. Für im Blauen emittierendes Emittermaterial oder einer Mischung aufweisend mindestens zwei Emittermaterialien können UHG-Matrixmaterialien (Ultra-High energy Gap Materialien) (siehe z. B. M.E. Thompson et al., Chem. Mater. 2004, 16, 4743) oder andere sogenannten Wide-Gap-Matrixmaterialien eingesetzt werden. Beispielhaft ist die Schichtdicke 10 nm bis 250 nm.

Die Lochblockierschicht HBL kann beispielsweise BCP (2,9-Dimethyl-4,7-diphenyl-1,10-phenanthrolin = Bathocuproin), Bis-(2-methyl-8-hydroxychinolinato)-(4-phenylphenolato)-aluminium(III) (BAlq), Nbphen (2,9-Bis(naphthalen-2-yl)-4,7-diphenyl-1,10-phenanthrolin), Alq3 (Aluminium-tris(8-hydroxychinolin)), TSPO1 (Diphenyl-4-triphenylsilylphenyl-phosphinoxid) oder TCB/TCP (1,3,5-Tris(N-carbazolyl)benzol/ 1,3,5-tris(carbazol)-9-yl) benzol) aufweisen. Beispielhaft ist die Schichtdicke 10 nm bis 50 nm.

Die Elektronentransportschicht ETL kann beispielsweise Materialien auf Basis von $AlQ_3$, TSPO1, BPyTP2 (2,7-Di(2,2'-bipyridin-5-yl)triphenyl), Sif87, Sif88, BmPyPhB (1,3-Bis[3,5-di(pyridin-3-yl)phenyl]benzol) oder BTB (4,4'-Bis-[2-(4,6-diphenyl-1,3,5-triazinyl)]-1,1'-biphenyl) aufweisen. Beispielhaft ist die Schichtdicke 10 nm bis 200 nm.

Als Materialien einer dünnen Elektroneninjektionsschicht EIL können beispielsweise CsF, LiF, 8-Hydroxyquinolinolatolithium (Liq), $Li_2O$, $BaF_2$, MgO oder NaF verwendet werden.

Als Materialien der Kathodenschicht können Metalle oder Legierungen dienen, beispielsweise Al, Al > AlF, Ag, Pt, Au, Mg, Ag:Mg. Typische Schichtdicken betragen 100 nm bis 200 nm. Insbesondere werden ein oder mehrere Metalle verwendet, die stabil an der Luft sind und/oder die selbstpassivierend, beispielsweise durch Ausbildung einer dünnen schützenden Oxidschicht, sind.

Als Materialien zur Verkapselung sind beispielsweise Aluminiumoxid, Vanadiumoxid, Zinkoxid, Zirkoniumoxid, Titanoxid, Hafniumoxid, Lanthanoxid, Tantaloxid geeignet.

In einer Ausführungsform der erfindungsgemäßen organischen optoelektronischen Vorrichtung ist das erfindungsgemäße organische Molekül als Emissionsmaterial in einer lichtemittierenden Schicht EML eingesetzt, wobei es entweder als Reinschicht oder in Kombination mit einem oder mehreren Hostmaterialien eingesetzt ist.

Eine Ausführungsform der Erfindung betrifft organische optoelektronische Vorrichtungen, welche eine externe Quanteneffizienz (EQE) bei 1000 cd/$m^2$ von größer 5 %, insbesondere von größer 8 %, insbesondere von größer 10 %, oder von größer 13 %, oder von größer 16 % und insbesondere von größer 20 % und/oder ein Emissionsmaximum bei einer Wellenlänge zwischen 420 nm und 500 nm, insbesondere zwischen 430 nm und 490 nm, oder zwischen 440 nm und 480 nm und insbesondere zwischen 450 nm und 470 nm und/oder einen LT80 Wert bei 500 cd/$m^2$ von größer 30 h, insbesondere von größer 70 h, oder von größer 100 h, oder von größer 150 h und insbesondere von größer 200 h aufweisen.

Der Massenanteil des erfindungsgemäßen organischen Moleküls an der Emitter-Schicht EML beträgt in einer weiteren Ausführungsform in einer lichtemittierenden Schicht in optischen Licht emittierenden Vorrichtungen, insbesondere in OLEDs, zwischen 1 % und 80 %. In einer Ausführungsform der erfindungsgemäßen organischen optoelektronischen Vorrichtung ist die lichtemittierende Schicht auf ein Substrat aufgebracht, wobei bevorzugt eine Anode und eine Kathode auf das Substrat aufgebracht sind und die lichtemittierende Schicht zwischen Anode und Kathode aufgebracht ist.

Die lichtemittierende Schicht weist in einer Ausführungsform ausschließlich ein erfindungsgemäßes organisches Molekül in 100 % Konzentration auf, wobei die Anode und die Kathode auf das Substrat aufgebracht sind, und die lichtemittierende Schicht zwischen Anode und Kathode aufgebracht ist.

In einer Ausführungsform der erfindungsgemäßen organischen optoelektronischen Vorrichtung sind eine löcher- und elektroneninjizierende Schicht zwischen Anode und Kathode, und eine löcher- und elektronentransportierende Schicht zwischen löcher- und elektroneninjizierende Schicht, und die lichtemittierende Schicht zwischen löcher- und elektronentransportierender Schicht aufgebracht.

Die organische optoelektronische Vorrichtung weist in einer weiteren Ausführungsform der Erfindung auf: ein Substrat, eine Anode, eine Kathode und mindestens je eine löcher- und elektroneninjizierende Schicht, und mindestens je eine

löcher- und elektronentransportierende Schicht, und mindestens eine lichtemittierende Schicht, die erfindungsgemäßes organisches Molekül und ein oder mehrere Hostmaterialen aufweist, deren Triplett ($T_1$)- und Singulett ($S_1$)-Energieniveaus energetisch höher liegen als die Triplett ($T_1$)- und Singulett ($S_1$)-Energieniveaus des organischen Moleküls, wobei die Anode und die Kathode auf das Substrat aufgebracht ist, und die löcher- und elektroneninjizierende Schicht zwischen Anode und Kathode aufgebracht ist, und die löcher- und elektronentransportierende Schicht zwischen löcher- und elektroneninjizierende Schicht aufgebracht ist, und die lichtemittierende Schicht zwischen löcher- und elektronentransportierende Schicht aufgebracht ist.

In einem weiteren Aspekt betrifft die Erfindung ein Verfahren zur Herstellung eines optoelektronischen Bauelements. Dabei wird ein erfindungsgemäßes organisches Molekül verwendet.

In einer Ausführungsform umfasst das Herstellungsverfahren die Verarbeitung des erfindungsgemäßen organischen Moleküls mittels eines Vakuumverdampfungsverfahrens oder aus einer Lösung.

Erfindungsgemäß ist auch ein Verfahren zur Herstellung einer erfindungsgemäßen optoelektronischen Vorrichtung, bei dem mindestens eine Schicht der optoelektronischen Vorrichtung

- mit einem Sublimationsverfahren beschichtet wird,
- mit einem OVPD (Organic Vapor Phase Deposition) Verfahren beschichtet wird,
- mit einer Trägergassublimation beschichtet wird, und/oder
- aus Lösung oder mit einem Druckverfahren hergestellt wird.

Bei der Herstellung der erfindungsgemäßen optoelektronischen Vorrichtung werden bekannte Verfahren eingesetzt. Generell werden die Schichten einzeln auf ein geeignetes Substrat in aufeinanderfolgenden Abscheideverfahrensschritten aufgebracht. Bei der Gasphasenabscheidung können die gebräuchlichen Methoden, wie thermische Verdampfung, chemische Gasphasenabscheidung (CVD), physikalische Gasphasenabscheidung (PVD) angewendet werden. Für active matrix OLED Displays erfolgt die Abscheidung auf eine AMOLED Backplane als Substrat.

Alternativ können Schichten aus Lösungen oder Dispersionen in geeigneten Lösungsmitteln aufgebracht werden. Beispielhafte geeignete Beschichtungsverfahren sind Rotationsbeschichtung (spin-coating), Tauchbeschichtung (dip-coating) und Strahldruckmethoden. Die einzelnen Schichten können erfindungsgemäß sowohl über dasselbe als auch über jeweils verschiedene Beschichtungsmethoden hergestellt werden.

**Beispiele**

**Allgemeine Vorschriften**

AAV1:

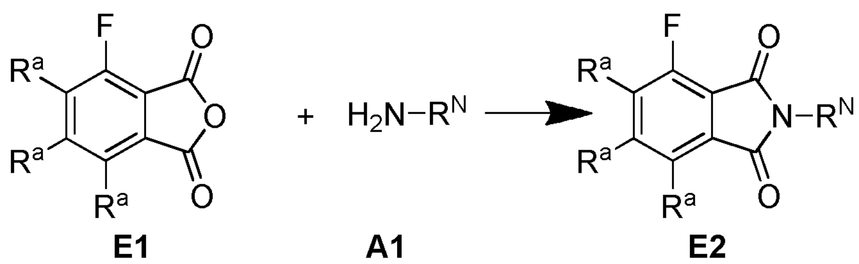

In einem Rundkolben mit Rückflusskühler wird **E1** (1 Äquivalent) in Eisessig suspendiert. Nach Zugabe von **A1** (1,1 Äquivalente) wird 3 Stunden bei 100 °C gerührt. Nach abkühlen wird die Reaktionslösung am Rotationsverdampfer soweit möglich eingeengt. Der Rückstand wird in $CH_2Cl_2$ aufgenommen und zweimal mit gesättigter $Na_2CO_3$ gewaschen. Die vereinigten organischen Phasen werden über $MgSO_4$ getrocknet. Das Lösungsmittel wird am Rotationsverdampfer entfernt. Nach Trocknen am Hochvakuum wird **E2** als Produkt erhalten, welches dann in der Regel ohne weitere Aufreinigung eingesetzt werden kann. Bei Bedarf kann das Produkt **E2** durch Umkristallisation weiter aufgereinigt werden.

**Beispielhafte A1 und E2 Kombinationen**

| A1 | E2 |
|---|---|
| 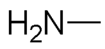 | 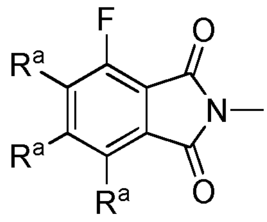 |
| 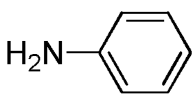 | 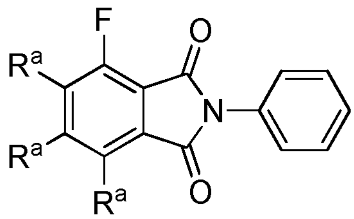 |
| 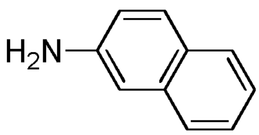 | 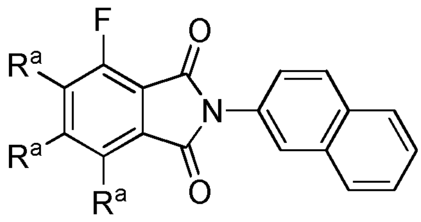 |
| 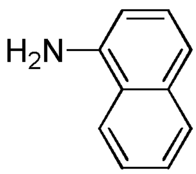 | 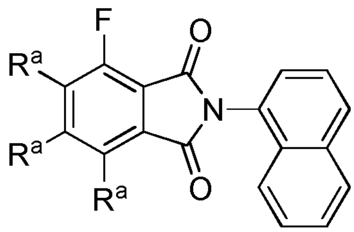 |
| 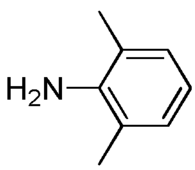 | 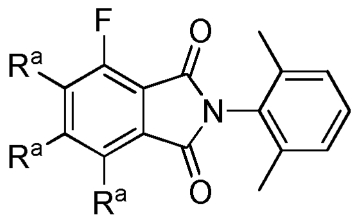 |
| 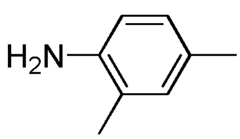 | 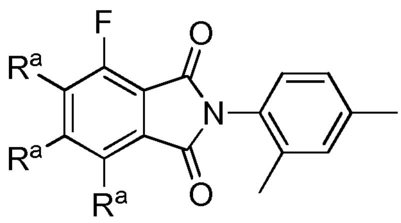 |
| 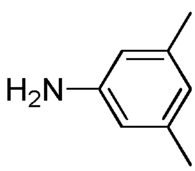 | 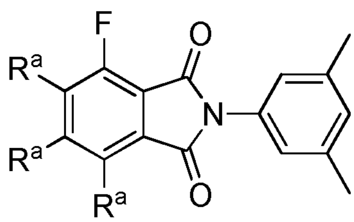 |

(fortgesetzt)

| A1 | E2 |
|---|---|
| 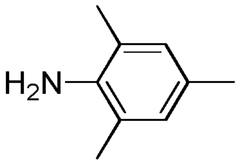 | 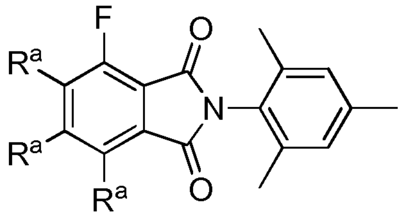 |
| 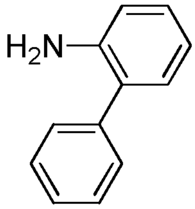 | 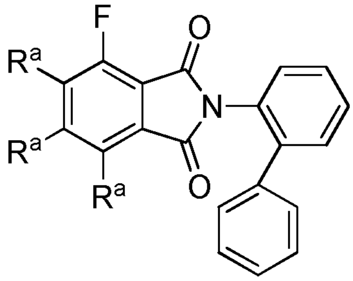 |
| 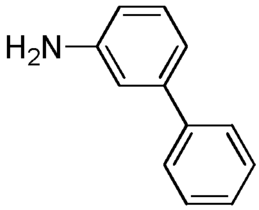 | 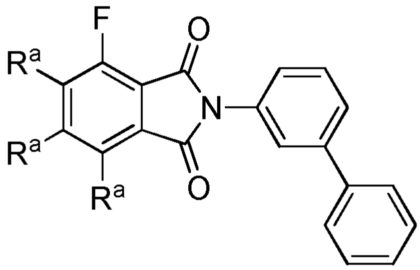 |
| 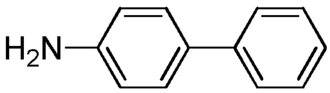 | 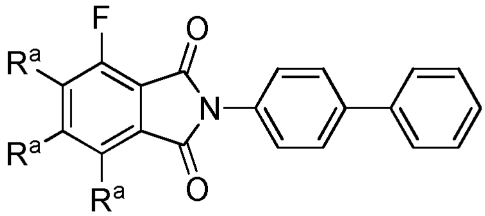 |
| 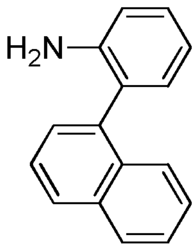 | 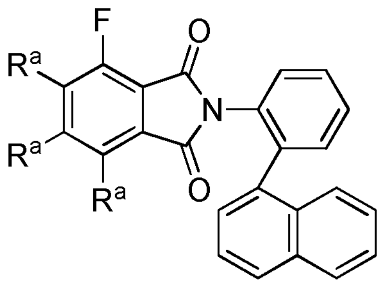 |
| 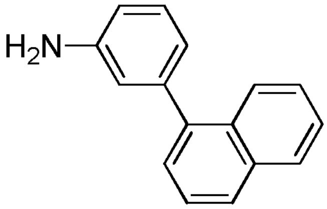 | 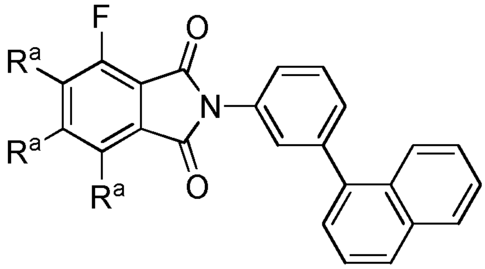 |

(fortgesetzt)

| A1 | E2 |
|---|---|
| 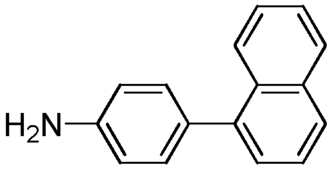 | 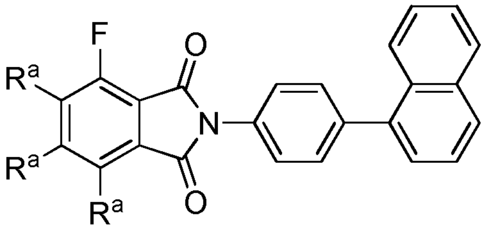 |
| 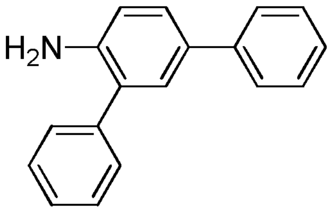 | 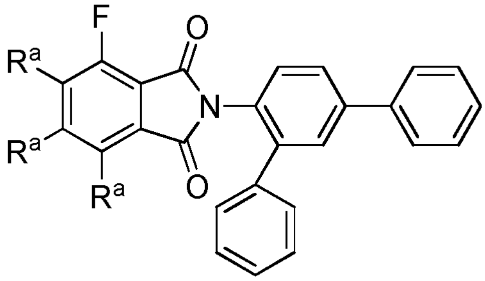 |
| 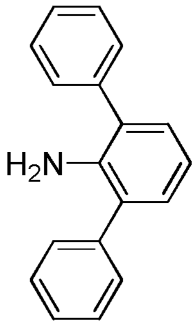 | 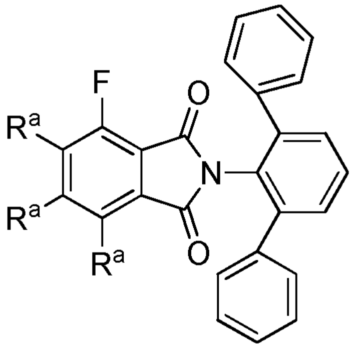 |
| 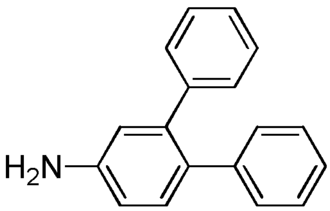 | 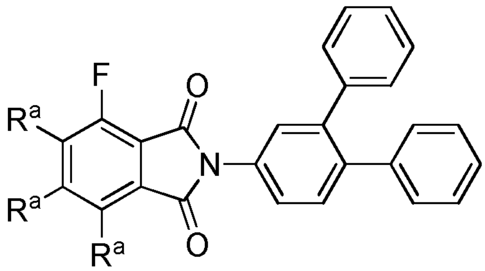 |
| 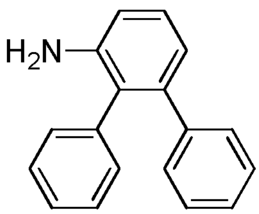 | 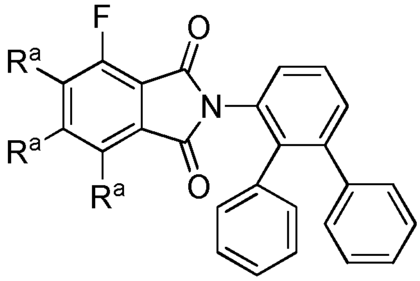 |

(fortgesetzt)

| A1 | E2 |
| --- | --- |
| 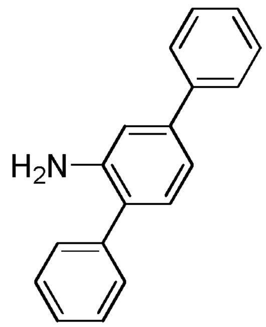 | 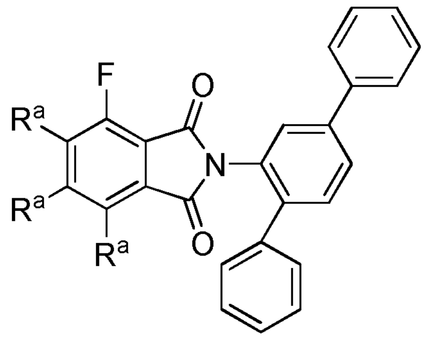 |
| 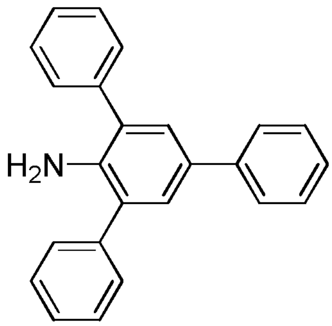 | 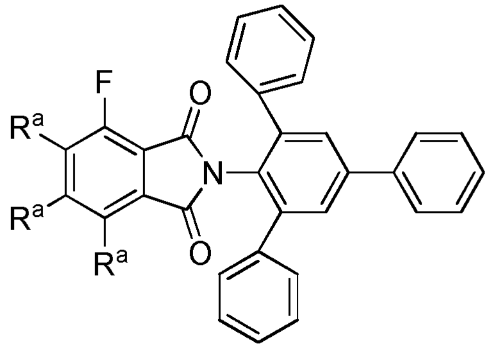 |

**AAV2:**

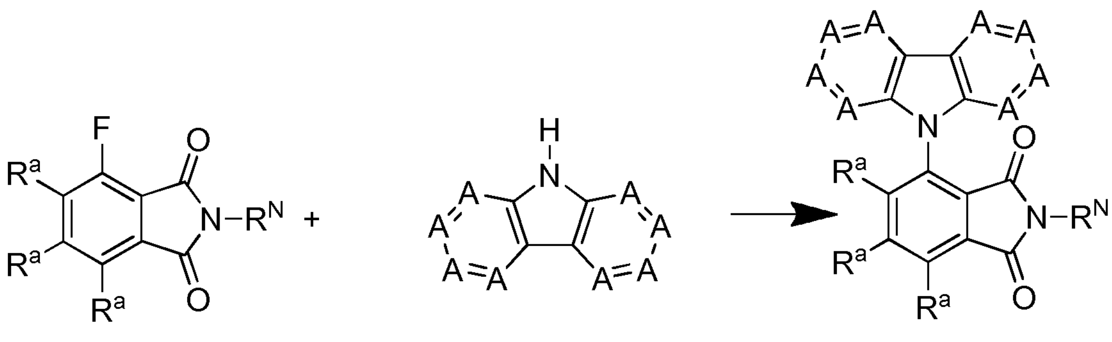

E2 E3

In einem Rundkolben werden Phthalimid **E2** (1 Äquivalent), ein Carbazol-Derivat **E3** (1 Äquivalent) und $K_3PO_4$ (2 Äquivalente) vorgelegt und für 5 min evakuiert. Unter Inertgasatmosphäre wird DMSO (trocken) zugegeben und die Reaktionslösung für 16 h bei 100 °C gerührt. Nach abkühlen wird die Reaktionslösung auf Wasser geschüttet und mit $CH_2Cl_2$ extrahiert. Nach erneuter Extraktion mit $CH_2Cl_2$ werden die vereinigten organischen Phasen 2x mit Wasser und mit gesättigter NaCl-Lösung gewaschen. Anschließend wird über $MgSO_4$ getrocknet und das Lösemittel am Rotationsverdampfer entfernt. Das jeweilige Produkt kann durch Umkristallisation gereinigt werden.

**AAV3:**

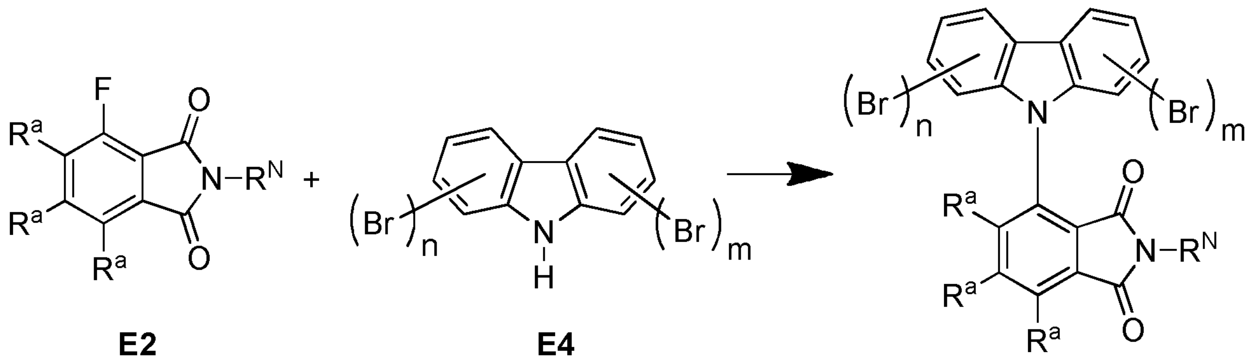

E2 E4

E5

n = 1 bis 4, m = 0 bis 4

In einem Rundkolben werden **E2** (1,2 Äquivalente), ein Brom-substituiertes Carbazol **E4** (1,0 Äquivalente) und $K_3PO_4$ (2 Äquivalente) vorgelegt und für 5 min evakuiert. Unter Inertgasatmosphäre wird DMSO (trocken) zugegeben und die Reaktionslösung für 16 h bei 100 °C gerührt. Nach abkühlen wird die Reaktionslösung auf Wasser geschüttet und mit $CH_2Cl_2$ extrahiert. Nach erneuter Extraktion mit $CH_2Cl_2$ werden die vereinigten organischen Phasen 2x mit Wasser und mit gesättigter NaCl-Lösung gewaschen. Anschließend wird über $MgSO_4$ getrocknet und das Lösemittel am Rotationsverdampfer entfernt. Das jeweilige Produkt kann durch Umkristallisation gereinigt werden.

Erfindungsgemäß kann statt Brom-substituiertem Carbazol auch Chlor- oder Iodsubstituiertes Carbazol verwendet werden.

**Beispielhafte E4 und E5 Kombinationen**

| E4 | E5 |
|---|---|
| 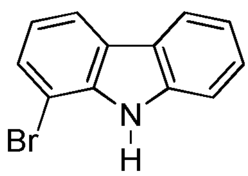 | 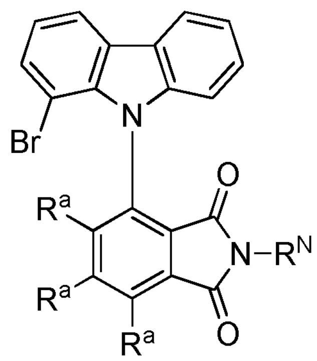 |
| 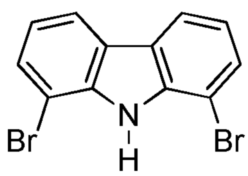 | 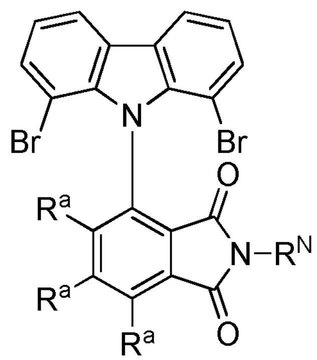 |

(fortgesetzt)

| E4 | E5 |
|---|---|
| 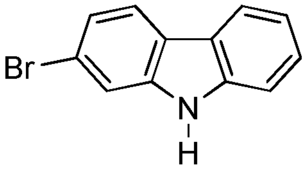 | 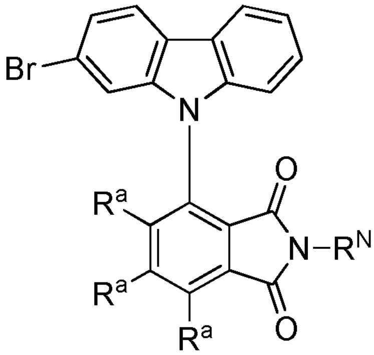 |
| 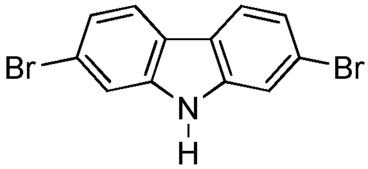 | 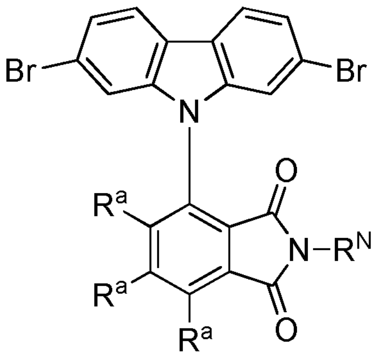 |
| 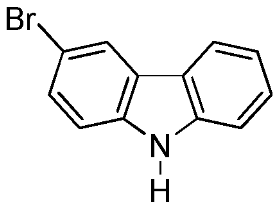 | 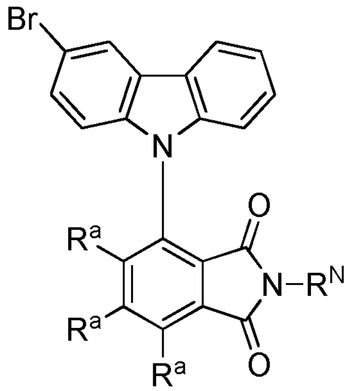 |
| 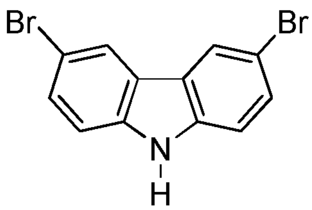 | 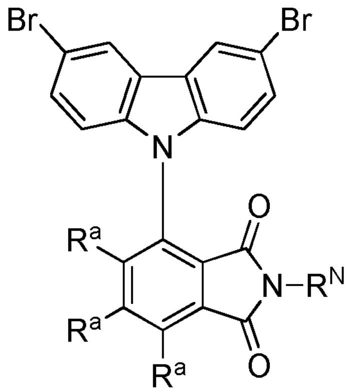 |

(fortgesetzt)

| E4 | E5 |
|---|---|
| 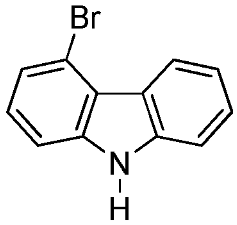 | 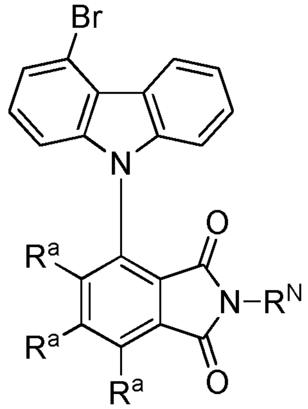 |
| 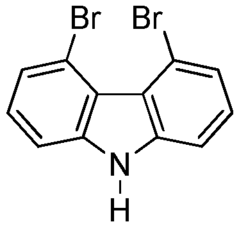 | 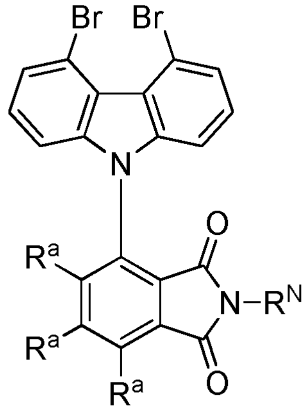 |

**AAV4:**

**Stufe 1**

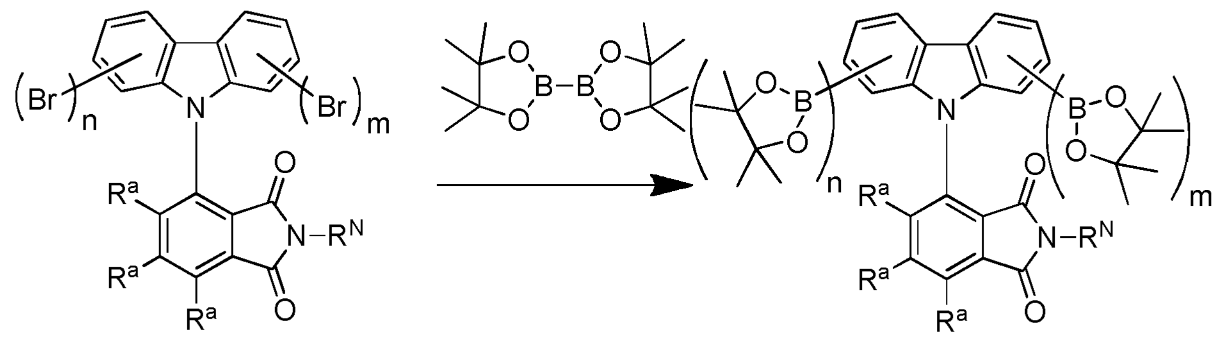

**E5** **E6**

**E5** (1,00 Äquivalente), Bis(pinacolato)diboron (1,5 mal (n+m) Äquivalente), Tris(dibenzylideneacetone)dipalladium (0,01 Äquivalente), 2-(Dicyclohexylphosphino)-2',4',6'-triisopropylbiphenyl (0,04 Äquivalente) und tribasisches Kaliumphosphat (3n + 3m Äquivalente) werden unter Stickstoff in Dioxan für 12 h bis 24 h bei 110 °C gerührt. Das erhaltene Rohprodukt kann durch Umkristallisation gereinigt werden.

**Stufe 2:**

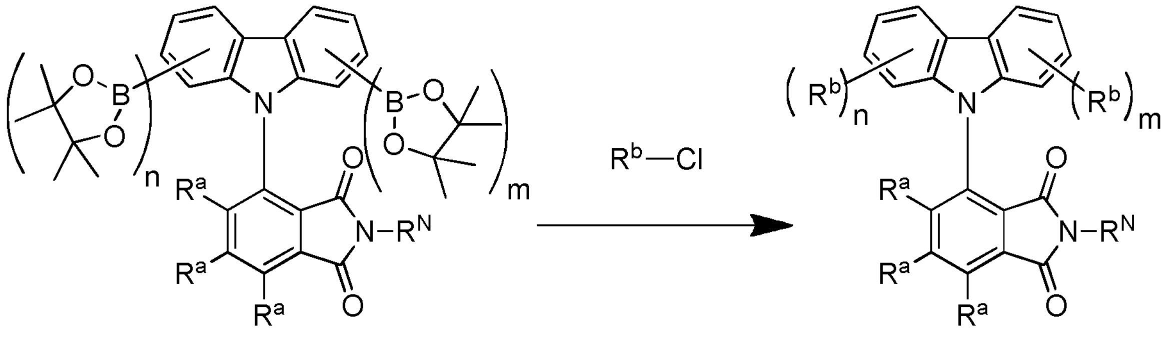


**E6**

**E6** (1,00 Äquivalente), $R^b$-Cl (1,3n + 1,3m Äquivalente), Tris(dibenzylideneacetone)dipalladium (0,01 Äquivalente), 2-(Dicyclohexylphosphino)-2',4',6'-triisopropylbiphenyl (0,04 Äquivalente) und tribasisches Kaliumphosphat (2,5n + 2,5m Äquivalente) werden unter Stickstoff in einem Toluol:/Wasser (10:1) Gemisch für 12 - 24 h bei 100 °C gerührt. Das erhaltene Rohprodukt wird durch Flash-Chromatographie oder durch Umkristallisation gereinigt.

Erfindungsgemäß kann statt $R^b$-Cl auch $R^b$-Br oder $R^b$-I verwendet werden.

**AAV5:**

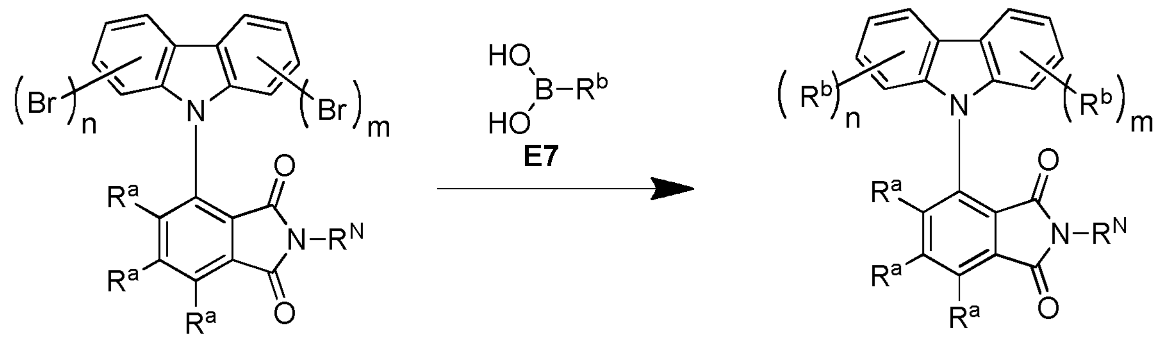


**E5**

**E5** (1,00 Äquivalente), die entsprechende Boronsäure des Restes $R^b$ **E7** (1,3n + 1,3m Äquivalente), Tris(dibenzylideneacetone)dipalladium (0,01 Äquivalente), 2-(Dicyclohexylphosphino)-2',4',6'-triisopropylbiphenyl (0,04 Äquivalente) und tribasisches Kaliumphosphat (3n + 3m Äquivalente) werden unter Stickstoff in Dioxan für 12 - 24 h bei 110 °C gerührt. Das erhaltene Rohprodukt wird durch Flash-Chromatographie oder durch Umkristallisation gereinigt.

Erfindungsgemäß kann statt einer Boronsäure auch ein entsprechender Boronsäureester verwendet werden.

Die erfindungsgemäßen Moleküle können jeweils gemäß der AAV2 oder einer Kombination von AAV3 und AAV4 oder AAV3 und AAV5 erhalten werden. Die Produkte der Synthesewege unterscheiden sich jeweils lediglich durch die erhaltenen Ausbeuten bzw. Reinheit vor der Aufreinigung. Nach entsprechender Aufreinigung sind die Produkte von äquivalenter Qualität.

**Berechnungen nach der Dichtefunktionaltheorie**

Für die Optimierung der Molekülstrukturen wurde das BP86-Funktional (Becke, A. D. Phys. Rev. A1988, 38, 3098-3100; Perdew, J. P. Phys. Rev. B1986, 33, 8822-8827) verwendet, wobei die resolution-of-identity-Näherung (RI) (Sierka, M.; Hogekamp, A.; Ahlrichs, R. J. Chem. Phys. 2003, 118, 9136-9148; Becke, A.D., J.Chem.Phys. 98 (1993) 5648-5652; Lee, C; Yang, W; Parr, R.G. Phys. Rev. B 37 (1988) 785-789) zum Einsatz kam. Anregungsenergien wurden

bei der mit BP86 optimierten Struktur mit der Time-Dependent DFT-Methode (TD-DFT) unter Verwendung des B3LYP-Funktionals (Becke, A.D., J.Chem.Phys. 98 (1993) 5648-5652; Lee, C; Yang, W; Parr, R.G. Phys. Rev. B 37 (1988) 785-789; Vosko, S. H.; Wilk, L.; Nusair, M. Can. J. Phys. 58 (1980) 1200-1211; Stephens, P. J.; Devlin, F. J.; Chabalowski, C. F.; Frisch, M. J. J.Phys.Chem. 98 (1994) 11623-11627) berechnet. In allen Rechnungen wurden def2-SV(P)-Basissätze (Weigend, F.; Ahlrichs, R. Phys. Chem. Chem. Phys. 2005, 7, 3297-3305; Rappoport, D.; Furche, F. J. Chem. Phys. 2010, 133, 134105/1-134105/11) und ein m4 -Grid zur numerischen Integration verwendet. Alle DFT-Rechnungen wurden mit dem Turbomole-Programmpaket (Version 6.5) (TURBOMOLE V6.4 2012, eine Entwicklung der Universität Karlsruhe und Forschungszentrum Karlsruhe GmbH, 1989-2007, TURBOMOLE GmbH, seit 2007; http://www.turbomole.com) durchgeführt.

**Photophysikalische Messungen**

*Vorbehandlung von optischen Gläsern*

Alle Gläser (Küvetten und Substrate aus Quarzglas, Durchmesser: 1 cm) wurden nach jeder Benutzung gereinigt: Je dreimaliges Spülen mit Dichlormethan, Aceton, Ethanol, demineralisiertem Wasser, Einlegen in 5 % Hellmanex-Lösung für 24 h, gründliches Ausspülen mit demineralisiertem Wasser. Zum Trocknen wurden die optischen Gläser mit Stickstoff abgeblasen.

***Probenvorbereitung: Lösungen***

1-2 mg der Probe wurden in 100 ml des jeweiligen Lösemittels gelöst, Konzentration $10^{-5}$ mol/L. Die Küvette wurde luftdicht verschlossen und 10 min. entgast.

***Probenvorbereitung, Film: Spin-Coating*** (Gerät: Spin150, SPS euro.)

Die Probenkonzentration entsprach 10mg/ml, angesetzt in Toluol oder Chlorbenzol. Programm: 1) 3 s bei 400 U/min; 2) 20 Sek. bei 1000 U/min bei 1000 Upm/ s. 3) 10 s bei 4000 U/min bei 1000 Upm/ s. Die Filme wurden nach dem Beschichten für 1 min bei 70 °C an Luft auf einer Präzisionsheizplatte von LHG getrocknet.

***Absorptionsspektroskopie***

Lösungen: UV-VIS-Spektren wurden auf einem Gerät der Firma Thermo Scientific, Modell Evolution 201 aufgenommen. (Siehe Probenvorbereitung: Lösungen)
Film: UV-VIS-Spektren wurden auf einem Gerät der Firma Thermo Scientific, Modell Evolution 201 aufgenommen. (Siehe Probenvorbereitung, Film: Spin-Coating)

***Photolumineszenzspektroskopie und TCSPC***

Steady-state Emissionsspektroskopie wurde mit einem Fluoreszenzspektrometer der Firma Horiba Scientific, Modell FluoroMax-4 durchgeführt, ausgestattet mit einer 150 W Xenon-Arc Lampe, Anregungs- und Emissionsmonochromatoren und einer Hamamatsu R928 Photomultiplier-Röhre, sowie einer TCSPC-Option. Emissions- und Anregungsspektren wurden korrigiert durch Standardkorrekturkurven.

Die Emissionsabklingzeiten wurden ebenfalls auf diesem System gemessen unter Verwendung der TCSPC-Methode mit dem FM-2013 Zubehör und einem TCSPC-Hub von Horiba Yvon Jobin. Anregungsquellen: NanoLED 370 (Wellenlänge: 371 nm, Pulsdauer: 1.1 ns), NanoLED 290 (Wellenlänge: 294 nm, Pulsdauer: <1 ns), SpectraLED 310 (Wellenlänge: 314nm), SpectraLED 355 (Wellenlänge: 355nm).

Die Auswertung (exponentielles Fitten) erfolgte mit dem Softwarepaket DataStation und der DAS 6 Auswertungssoftware. Der Fit wurde über die Chi-Quadrat-Methode angegeben

$$c^2 = \sum_{k=1}^{i} \frac{(e_i - o_i)^2}{e_i}$$

mit $e_i$: Durch den Fit vorhergesagte Größe und $o_i$: Gemessenen Größe.

***Quanteneffizienzbestimmung***

Die Messung der Photolumineszenzquantenausbeute (PLQY) erfolgte mittels eines *Absolute PL Quantum Yield Measurement C9920-03G*-Systems der Firma *Hamamatsu Photonics.* Dieses besteht aus einer 150 W Xenon-Gasentladungslampe, automatisch justierbaren Czerny-Turner Monochromatoren (250 nm bis 950 nm) und einer Ulbricht-Kugel mit hochreflektierender Spektralon-Beschichtung (einem Teflon-Derivat), die über ein Glasfaserkabel mit einem PMA-12 Vielkanaldetektor mit BT- *(back thinned-)* CCD-Chip mit 1024 x 122 Pixeln (Größe 24 x 24 µm) verbunden ist. Die Auswertung der Quanteneffizienz und der CIE-Koordinaten erfolgte mit Hilfe der Software U6039-05 Version 3.6.0 Für G9920-OXG (PMA-12). Das Emissionsmaximum wird in nm, die Quantenausbeute $\Phi$ in % und die CIE-Farbkoordinaten als x,y-Werte angegeben.

Die PLQY wurde für Polymerfilme, Lösungen und Pulverproben nach folgendem Protokoll bestimmt:

1) Durchführung der Qualitätssicherung: Als Referenzmaterial dient Anthracene in Ethanol mit bekannter Konzentration.

2) Ermitteln der Anregungswellenlänge: Es wurde zuerst das Absorptionsmaximum des organischen Moleküls bestimmt und mit diesem angeregt.

3) Durchführung der Probenmessung: Es wurde von entgasten Lösungen und Filmen unter Stickstoff-Atmosphäre die absolute Quantenausbeute bestimmt. Die Berechnung erfolgte systemintern nach folgender Gleichung:

$$\Phi_{PL} = \frac{n_{photon}, emittiert}{n_{photon}, absorbiert} = \frac{\int \frac{\lambda}{hc}\left[Int_{emittiert}^{Probe}(\lambda) - Int_{absorbiert}^{Probe}(\lambda)\right]d\lambda}{\int \frac{\lambda}{hc}\left[Int_{emittiert}^{Referenz}(\lambda) - Int_{absorbiert}^{Referenz}(\lambda)\right]d\lambda}$$

mit der Photonenzahl $n_{photon}$ und der Intensität Int.

**Herstellung und Charakterisierung von organischen Elektrolumineszenzvorrichtungen aus der Gasphase**

Mit den erfindungsgemäßen organischen Molekülen können OLED-Devices mittels Vakuum-Sublimationstechnik erstellt werden. Enthält eine Schicht mehrere Komponenten, so ist das Verhältnis dieser in Massenprozent angegeben.

Diese noch nicht optimierten OLEDs können standardmäßig charakterisiert werden; hierfür werden die Elektrolumineszenzspektren, die externe Quanteneffizienz (gemessen in %) in Abhängigkeit von der Helligkeit, berechnet aus dem von der Fotodiode detektiertem Licht, und dem Strom aufgenommen. Aus dem zeitlichen Verlauf der Elektrolumineszenzspektren kann die Lebensdauer der OLEDs bestimmt werden. Der angegebene LT50-Wert entspricht hierbei der Zeit, bei der die Leuchtdichte auf 50 % des Startwertes abgefallen ist. Analog entspricht der LT70-Wert der Zeit, bei der die Leuchtdichte auf 70 % des Startwertes abgefallen ist.

**Beispiel 1**

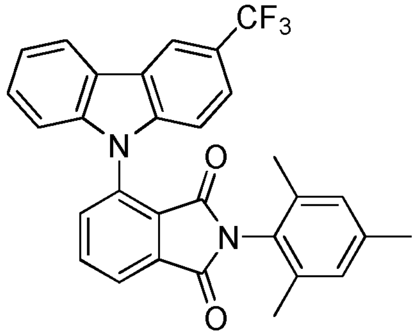

Beispiel 1 wurde nach AAV2 aus 3-Trifluormethyl-9*H*-carbazol und *N*-Mesityl-3-fluorphthalimid in einer Ausbeute von 49% hergestellt.

$^1$H NMR (500 MHz, Chloroform-d) δ 8.40 (m, 1H), 8.17 (dt, J = 7.7, 1.0 Hz, 1H), 8.15 (dd, J = 7.4, 1.0 Hz, 1H), 8.05 (t, J = 7.7 Hz, 1H), 7.97 (dd, J = 8.0, 1.0 Hz, 1H), 7.63 (dd, J = 8.8, 1.8 Hz, 1H), 7.46 (ddd, J = 8.3, 7.2, 1.2 Hz, 1H), 7.37

(ddd, J = 8.0, 7.3, 1.0 Hz, 1H), 7.28 - 7.24 (m, 3H), 6.95-6.92 (m, 2H), 2.27 (s, 3H), 2.11 (s, 3H), 2.09 (s, 3H). $^{19}F$ NMR (471 MHz, CDCl3) δ -60.38.

Figur 1 zeigt das Emissionsspektrum von Beispiel **1** (10 % in PMMA). Das Emissionsmaximum liegt bei 469 nm. Die Photolumineszenzquantenausbeute (PLQY) beträgt 39 % und die Halbwertsbreite (FWHM) 90 nm (0,49 eV). Es ergibt sich eine $CIE_y$ von 0,24 und ein BMI von 163. Die Emissionslebensdauer beträgt 7,5 µs.

**Beispiel 2**

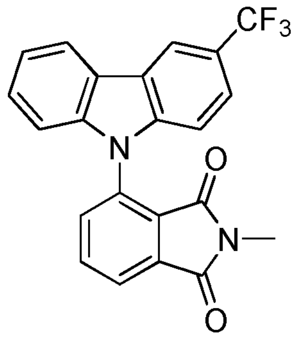

Beispiel 2 wurde nach AAV2 aus 3-Trifluormethyl-9*H*-carbazol und N-Methyl-3-fluorphthalimid in einer Ausbeute von 67% hergestellt.

Figur 2 zeigt das Emissionsspektrum von Beispiel **2** (10 % in PMMA). Das Emissionsmaximum liegt bei 458 nm. Die Photolumineszenzquantenausbeute (PLQY) beträgt 34 % und die Halbwertsbreite (FWHM) 88 nm (0,50 eV). Es ergibt sich eine $CIE_y$ von 0,19 und ein BMI von 179. Die Emissionslebensdauer beträgt 10,5 µs.

**Beispiel 3**

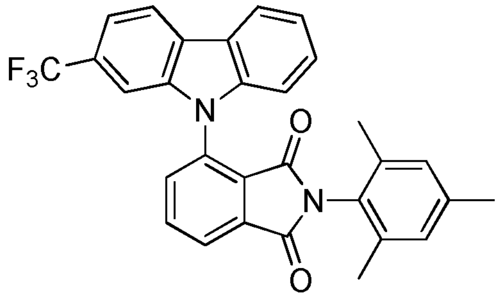

Beispiel 3 wurde nach AAV2 aus 2-Trifluormethyl-9*H*-carbazol und *N*-Mesityl-3-fluorphthalimid in einer Ausbeute von 57% hergestellt.
$^{1}H$ NMR (500 MHz, Chloroform-d) δ 8.22 (d, J = 8.1 Hz, 1H), 8.18 (dt, J = 7.8, 0.9 Hz, 1H), 8.15 (dd, J = 7.4, 1.0 Hz, 1H), 8.06 (t, J = 7.8 Hz, 1H), 7.97 (dd, J = 8.0, 0.9 Hz, 1H), 7.56 (dd, J = 8.2, 1.4 Hz, 1H), 7.48 (ddd, J = 8.3, 7.2, 1.2 Hz, 1H), 7.44 (s, 1H), 7.37 (td, J = 7.6, 0.9 Hz, 1H), 7.31 (d, J = 8.3 Hz, 1H), 6.95 (s, 1H), 6.93 (s, 1H), 2.27 (s, 3H), 2.12 (s, 3H), 2.09 (s, 3H). Figur 3 zeigt das Emissionsspektrum von Beispiel **3** (10 % in PMMA). Das Emissionsmaximum liegt bei 467 nm. Die Photolumineszenzquantenausbeute (PLQY) beträgt 46 % und die Halbwertsbreite (FWHM) 86 nm (0,47 eV). Es ergibt sich eine $CIE_y$ von 0,22 und ein BMI von 209. Die Emissionslebensdauer beträgt 8,5 µs.

**Beispiel 4**

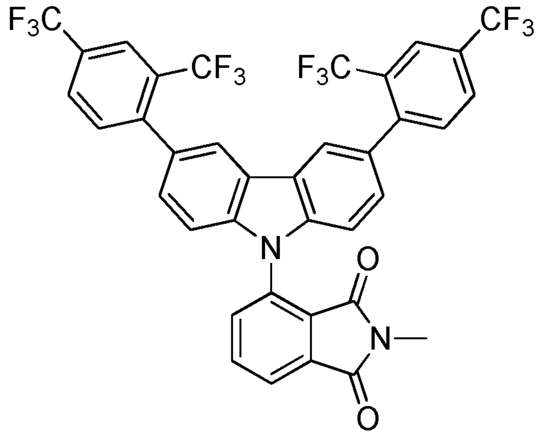

Beispiel 4 wurde nach AAV5 aus 3-(3,6-Dibromcarbazolyl)-*N*-methylphthalimid und 2,4-Trifluormethylphenyl-1-boronsäure in einer Ausbeute von 44% hergestellt.

$^1$H NMR (500 MHz, Chloroform-d) δ 8.09 (m, 2H), 8.06 (dd, J = 7.2, 1.2 Hz, 1H), 8.05 - 8.03 (m, 2H), 7.99 (t, J = 7.6 Hz, 1H), 7.95 (dd, J = 8.0, 1.2 Hz, 1H), 7.88 - 7.83 (m, 2H), 7.63 (d, J = 7.9 Hz, 2H), 7.39 (dd, J = 8.4, 1.6 Hz, 2H), 7.23 (d, J = 8.4 Hz, 2H), 3.16 (s, 3H).

$^{19}$F NMR (471 MHz, CDCl3) δ -57.00, -62.66.

Figur 4 zeigt das Emissionsspektrum von Beispiel **4** (10 % in PMMA). Das Emissionsmaximum liegt bei 465 nm. Die Photolumineszenzquantenausbeute (PLQY) beträgt 50 % und die Halbwertsbreite (FWHM) 89 nm (0,49 eV). Es ergibt sich eine $CIE_y$ von 0,21 und ein BMI von 238.

**Beispiel 5**

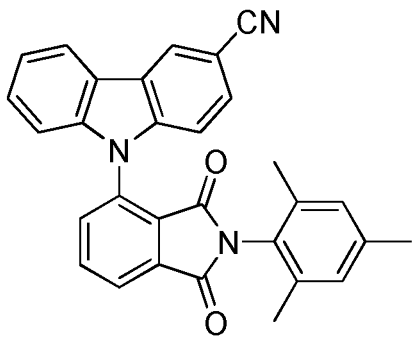

Beispiel 5 wurde nach AAV2 aus 3-Cyano-9*H*-carbazol und *N*-Mesityl-3-fluorphthalimid in einer Ausbeute von 62% hergestellt.

$^1$H NMR (500 MHz, Chloroform-d) δ 8.47- 8.43 (m, 1H), 8.18-8.14 (m, 2H), 8.07 (t, J = 7.7 Hz, 1H), 7.96 (dd, J = 8.0, 1.0 Hz, 1H), 7.64 (dd, J = 8.5, 1.6 Hz, 1H), 7.49 (ddd, J = 8.4, 7.3, 1.2 Hz, 1H), 7.42-7.37 (m, 1H), 7.28-7.22 (m, 2H), 6.93 (m, 2H), 2.27 (s, 3H), 2.10 (s, 3H), 2.07 (s, 3H).

Figur 5 zeigt das Emissionsspektrum von Beispiel **5** (10 % in PMMA). Das Emissionsmaximum liegt bei 462 nm. Die Photolumineszenzquantenausbeute (PLQY) beträgt 36 % und die Halbwertsbreite (FWHM) 87 nm (0,49 eV). Es ergibt sich eine $CIE_y$ von 0,20 und ein BMI von 180. Die Emissionslebensdauer beträgt 6,6 μs.

**Beispiel 6**

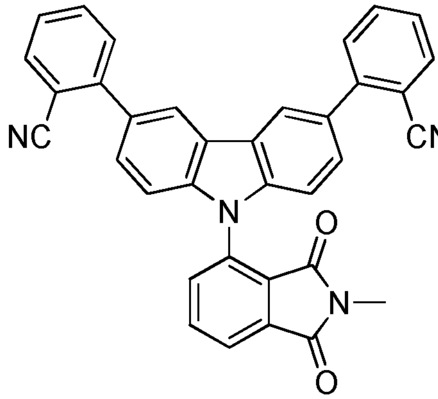

Beispiel 6 wurde nach AAV 5 aus 3-(3,6-Dibromcarbazolyl)-*N*-methylphthalimid und 2-Cyanophenylboronsäure in einer Ausbeute von 61% hergestellt.
$^1$H NMR (500 MHz, Chloroform-d) $\delta$ 8.38 (d, J = 1.7 Hz, 2H), 8.06 (dd, J = 7.3, 1.1 Hz, 1H), 7.99 (t, J = 7.7 Hz, 1H), 7.93 (dd, J = 7.9, 1.1 Hz, 1H), 7.83 - 7.78 (m, 2H), 7.71 - 7.63 (m, 6H), 7.45 (ddd, J = 7.8, 6.4, 2.3 Hz, 2H), 7.28 (d, J = 8.5 Hz, 2H), 3.15 (d, J = 1.1 Hz, 3H).

Figur 6 zeigt das Emissionsspektrum von Beispiel **6** (10 % in PMMA). Das Emissionsmaximum liegt bei 477 nm. Die Photolumineszenzquantenausbeute (PLQY) beträgt 56 % und die Halbwertsbreite (FWHM) 93 nm (0,48 eV). Es ergibt sich eine $CIE_y$ von 0,30 und ein BMI von 187. Die Emissionslebensdauer beträgt 59 $\mu$s.

**Beispiel 7**

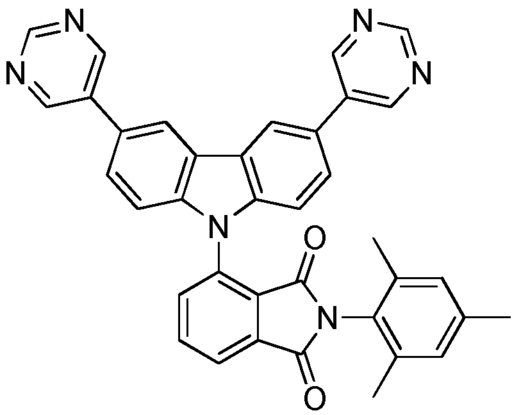

Beispiel 7 wurde nach AAV 5 aus 3-(3,6-Dibromcarbazolyl)-*N*-mesitylphthalimid und Pyrimidin-5-Boronsäure in einer Ausbeute von 22% hergestellt.
$^1$H NMR (500 MHz, Chloroform-d) $\delta$ 9.22 (s, 2H), 9.07 (s, 4H), 8.40 (d, J = 1.7 Hz, 2H), 8.21-8.17 (m, 1H), 8.11 (t, J = 7.7 Hz, 1H), 8.06 - 8.01 (m, 1H), 7.65 (dd, J = 8.5, 1.8 Hz, 2H), 7.39 (d, J = 8.5 Hz, 2H), 6.93 (s, 2H), 2.26 (s, 3H), 2.11 (s, 6H).

Figur 7 zeigt das Emissionsspektrum von Beispiel **7** (10 % in PMMA). Das Emissionsmaximum liegt bei 490 nm. Die Photolumineszenzquantenausbeute (PLQY) beträgt 58 % und die Halbwertsbreite (FWHM) 97 nm (0,48 eV). Es ergibt sich eine $CIE_y$ von 0,38 und ein BMI von 153. Die Emissionslebensdauer beträgt 9,8 $\mu$s.

**Beispiel 8**

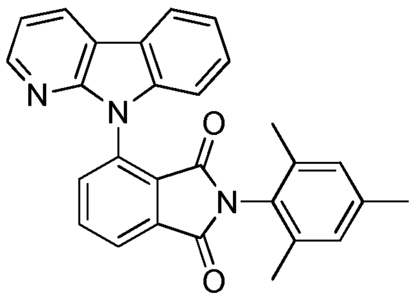

Beispiel 8 wurde nach AAV 2 aus *N*-Mesityl-3-fluorphthalimid und 9H-Pyrido[2,3-b]indole in einer Ausbeute von 26% hergestellt.

$^1$H NMR (500 MHz, Chloroform-d) δ 8.41 - 8.35 (m, 2H), 8.14-8.11 (m, 1H), 8.09 (dd, J = 6.4, 2.0 Hz, 1H), 8.06 - 8.00 (m, 2H), 7.47 (td, J = 7.6, 7.1, 1.2 Hz, 1H), 7.38 - 7.33 (m, 2H), 7.25 (dd, J = 7.7, 4.9 Hz, 1H), 6.93 (s, 1H), 6.91 (s, 1H), 2.26 (s, 3H), 2.14 (s, 3H), 2.08 (s, 3H).

Figur 8 zeigt das Emissionsspektrum von Beispiel **8** (10 % in PMMA). Das Emissionsmaximum liegt bei 448 nm. Die Photolumineszenzquantenausbeute (PLQY) beträgt 32 % und die Halbwertsbreite (FWHM) 86 nm (0,51 eV). Es ergibt sich eine $CIE_y$ von 0,15 und ein BMI von 213.

**Beispiel 9**

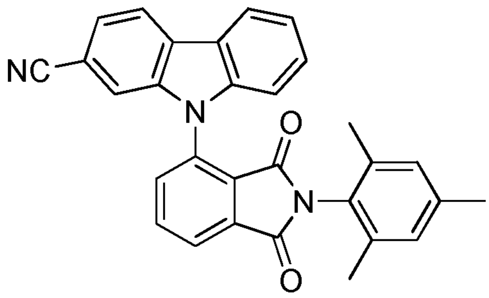

Beispiel 9 wurde nach der folgenden Vorschrift synthetisiert:

In einem Schlenkkolben wurden unter Stickstoffatmosphäre 3-(2-bromcarbazolyl)-*N*-mesitylphthalimid (1 Äquivalent) und CuCN (1.5 Äquivalente) in trockenem DMF (2 mL pro mmol Arylbromid) 24 h auf 150 °C erhitzt. Nach dem Abkühlen auf Raumtemperatur wurde der gebildete braune Niederschlag abfiltriert und mit DMF nachgewaschen. Durch Zugabe des doppelten Volumens an Wasser zum Filtrat wurde das Rohprodukt als gelbgrüner Feststoff ausgefällt. Der Niederschlag wurde abfiltriert, mit Wasser gewaschen und in Ethylacetat aufgenommen. Die resultierende Lösung wurde über MgSO4 getrocknet und unter vermindertem Druck vom Lösungsmittel befreit. Der Rückstand wurde per MPLC gereinigt (Eluent: $CH_2Cl_2$/Cyclohexan 50:50 - 100:0). Ausbeute: 12%.

$^1$H NMR (500 MHz, Chloroform-d) δ 8.20 (dd, J = 8.0, 0.7 Hz, 1H), 8.19-8.15 (m, 2H), 8.08 (t, J = 7.7 Hz, 1H), 7.97 (dd, J = 8.0, 1.0 Hz, 1H), 7.56 (dd, J = 8.1, 1.3 Hz, 1H), 7.53 - 7.47 (m, 2H), 7.38 (ddd, J = 8.0, 7.2, 0.9 Hz, 1H), 7.30 - 7.27 (m, 1H), 6.95 (s, 1H), 6.93 (s, 1H), 2.28 (s, 3H), 2.14 (s, 3H), 2.08 (s, 3H).

Figur 9 zeigt das Emissionsspektrum von Beispiel **9** (10 % in PMMA). Das Emissionsmaximum liegt bei 463 nm. Die Photolumineszenzquantenausbeute (PLQY) beträgt 36 % und die Halbwertsbreite (FWHM) 87 nm (0,48 eV). Es ergibt sich eine $CIE_y$ von 0,21 und ein BMI von 171.

**Beispiel 10**

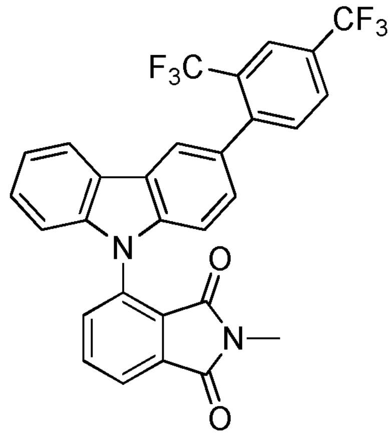

Beispiel 10 wurde nach AAV 5 aus 3-(3-Bromcarbazolyl)-*N*-methylphthalimid und 2,4-Bis(trifluormethyl)phenylboronsäure in einer Ausbeute von 46% hergestellt.

$^1$H NMR (500 MHz, Chloroform-d) δ 8.14 (dt, J = 7.7, 0.9 Hz, 1H), 8.10 (d, J = 1.6 Hz, 1H), 8.06 - 8.04 (m, 1H), 8.03 (dd, J = 7.3, 1.1 Hz, 1H), 7.98 - 7.93 (m, 1H), 7.90 (dd, J = 8.0, 1.1 Hz, 1H), 7.88 - 7.84 (m, 1H), 7.63 (d, J = 8.0 Hz, 1H), 7.43 (ddd, J = 8.4, 7.2, 1.2 Hz, 1H), 7.37 - 7.31 (m, 2H), 7.20 (d, J = 8.4 Hz, 1H), 7.18-7.16 (m, 1H), 3.14 (s, 3H).

$^{19}$F NMR (471 MHz, CDCl3) δ -57.02, -62.64.

Figur 10 zeigt das Emissionsspektrum von Beispiel **10** (10 % in PMMA). Das Emissionsmaximum liegt bei 474 nm. Die Photolumineszenzquantenausbeute (PLQY) beträgt 61 % und die Halbwertsbreite (FWHM) 92 nm. (0,49 eV). Es ergibt sich eine $CIE_y$ von 0,28 und ein BMI von 218. Die Emissionslebensdauer beträgt 7,0 μs.

**Beispiel 11**

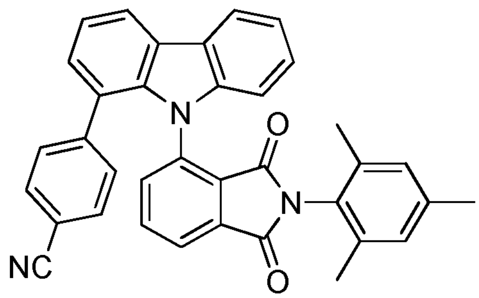

Beispiel 11 wurde nach AAV 5 aus 3-(1-Bromcarbazolyl)-*N*-mesitylphthalimid und 4-Cyanophenylboronsäure in einer Ausbeute von 22% hergestellt.

$^1$H NMR (500 MHz, Chloroform-d) δ 8.22 (dd, J = 7.8, 1.2 Hz, 1H), 8.19-8.16 (m, 1H), 7.80 (dd, J = 7.4, 0.9 Hz, 1H), 7.56 (t, J = 7.7 Hz, 1H), 7.44 - 7.37 (m, 3H), 7.37 - 7.24 (m, 7H), 7.05 (dt, J = 8.2, 0.9 Hz, 1H), 6.96 (s, 1H), 6.90 (s, 1H), 2.27 (s, 3H), 2.15 (s, 3H), 1.99 (s, 3H).

Figur 11 zeigt das Emissionsspektrum von Beispiel **11** (10% in PMMA). Das Emissionsmaximum liegt bei 482 nm. Die Photolumineszenzquantenausbeute (PLQY) beträgt 61 % und die Halbwertsbreite (FWHM) 94 nm (0,48 eV). Es ergibt sich eine $CIE_y$ von 0,33 und ein BMI von 185.

**Beispiel 12**

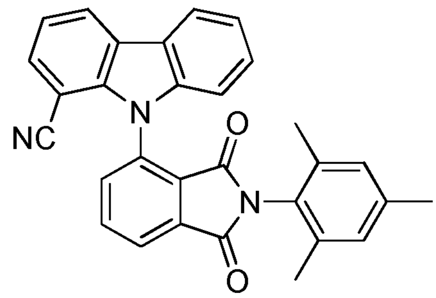

Beispiel 12 wurde gemäß der folgenden Vorschrift hergestellt:
In einem Zweihalskolben mit Rückflusskühler wurden unter Stickstoffatmosphäre 3-(1-Bromcarbazolyl)-*N*-mesitylphthalimid (1 Äquivalent) und CuCN (1.5 Äquivalente) in trockenem DMF während 15 h auf 250 °C erhitzt. Nach dem Abkühlen auf RT wurde aus der Reaktionslösung durch Zugabe des gleichen Volumens an Wasser das Rohprodukt ausgefällt. Der Niederschlag wurde abfiltriert und mit $CH_2Cl_2$ behandelt. Die resultierende Lösung wurde mit Wasser gewaschen, über MgSO4 getrocknet und unter vermindertem Druck vom Lösungsmittel befreit. Der Rückstand wurde mit heißem Ethanol gewaschen. Die Waschlösung wurde verworfen und der Rückstand aus Toluol umkristallisiert. Ausbeute: 69%.
$^1H$ NMR (500 MHz, Chloroform-d): $\delta$ = 8.35 (dd, *J* = 7.9, 1.2 Hz, 1H), 8.19 (dd, *J* = 7.4, 1.0 Hz, 1H), 8.16 (dt, *J* = 7.8, 0.9 Hz, 1H), 8.05 (t, *J* = 7.7 Hz, 1H), 7.98 (dd, *J* = 7.9, 0.9 Hz, 1H), 7.66 (dd, J = 7.6, 1.2 Hz, 1H), 7.48 (ddd, J = 8.3, 7.2, 1.2 Hz, 1H), 7.39 (td, J = 7.6, 0.9 Hz, 1H), 7.33 (t, *J* = 7.7 Hz, 1H), 7.24 (dt, *J* = 8.3, 0.8 Hz, 1H), 6.94 (bs, 1H), 6.87 (bs, 1H), 2.25 (s, 3H), 2.20 (s, 3H), 2.00 (s, 3H) ppm.

Figur 12 zeigt das Emissionsspektrum von Beispiel **12** (10 % in PMMA). Das Emissionsmaximum liegt bei 453 nm. Die Photolumineszenzquantenausbeute (PLQY) beträgt 44 % und die Halbwertsbreite (FWHM) 84 nm (0,49 eV). Es ergibt sich eine $CIE_y$ von 0,16 und ein BMI von 275.

**Beispiel 13**

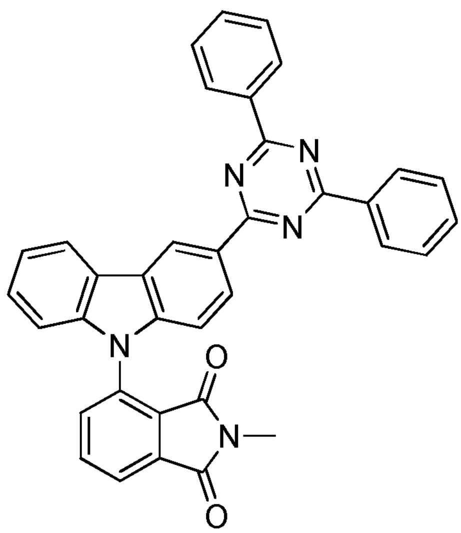

Beispiel 13 wurde nach AAV 4 ausgehend von 3-(3-Bromcarbazolyl)-*N*-methylphthalimid in einer Ausbeute von 57% hergestellt.
$^1H$ NMR (500 MHz, Chloroform-d) $\delta$ 9.62-9.61 (m, 1H), 8.89 (dd, J = 8.7, 1.7 Hz, 1H), 8.86-8.81 (m, 4H), 8.41-8.38 (m, 1H), 8.07 (dd, J = 7.3, 1.0 Hz, 1H), 7.99 (t, J = 7.6 Hz, 1H), 7.93 (dd, J = 7.9, 1.0 Hz, 1H), 7.66 - 7.58 (m, 6H), 7.48-7.44 (m, 1H), 7.42 (td, J = 7.4, 1.2 Hz, 1H), 7.29 - 7.26 (m, 1H), 7.21 -7.19 (m, 1H), 3.13 (s, 3H).

Figur 13 zeigt das Emissionsspektrum von Beispiel **13** (10 % in PMMA). Das Emissionsmaximum liegt bei 478 nm. Die Photolumineszenzquantenausbeute (PLQY) beträgt 58 % und die Halbwertsbreite (FWHM) 92 nm. (0,48 eV). Es ergibt sich eine $CIE_y$ von 0,30 und ein BMI von 193.

**Beispiel 14**

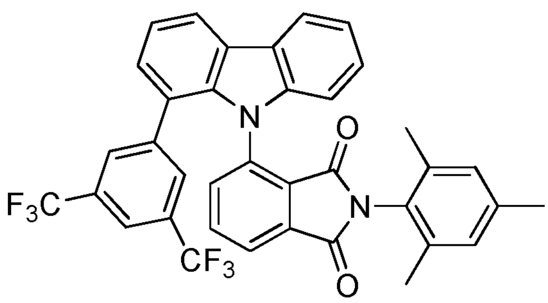

Beispiel 14 wurde nach AAV 5 aus 3-(1-Bromcarbazolyl)-*N*-mesitylphthalimid und 3,5-Bis(trifluormethyl)benzolboronsäure in einer Ausbeute von 49% hergestellt.

$^1$H NMR (500 MHz, Chloroform-d) δ 8.26 (dd, J = 7.8, 1.2 Hz, 1H), 8.20 - 8.16 (m, 1H), 7.76 (dd, J = 7.4, 0.9 Hz, 1H), 7.79 - 7.47 (breites Signal, 2H), 7.61 (s, 1H), 7.45 (t, J = 7.6 Hz, 1H), 7.41 (t, J =7.41 Hz, 1H), 7.39 - 7.32 (m, 3H), 7.25 (dd, J = 8.0, 0.9 Hz, 1H), 7.01 - 6.97 (m, 1H), 6.95 - 6.92 (m, 1H), 6.91-6.89 (m, 1H), 2.27 (s, 3H), 2.10 (s, 3H), 2.03 (s, 3H).

$^{19}$F NMR (471 MHz, CDCl3) δ -62.5.

Figur 14 zeigt das Emissionsspektrum von Beispiel **14** (10 % in PMMA). Das Emissionsmaximum liegt bei 477 nm. Die Photolumineszenzquantenausbeute (PLQY) beträgt 65 % und die Halbwertsbreite (FWHM) 93 nm (0,48 eV). Es ergibt sich eine $CIE_y$ von 0,31 und ein BMI von 210.

**Beispiel 15**

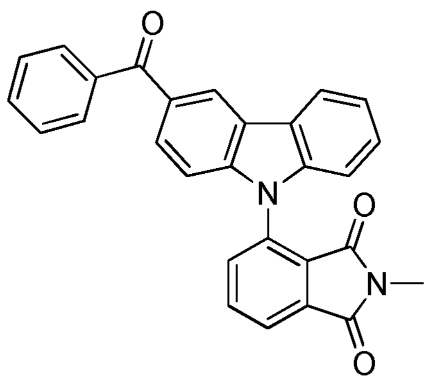

$^1$H NMR (500 MHz, Chloroform-d) δ 8.65 (dd, J = 1.7, 0.6 Hz, 1H), 8.17 (dt, J = 7.8, 1.0 Hz, 1H), 8.06 (dd, J = 7.4, 1.0 Hz, 1H), 7.98 (t, J = 7.8 Hz, 1H), 7.96 (dd, J = 8.6, 1.6 Hz, 1H), 7.89-7.85 (m, 3H), 7.64 - 7.59 (m, 1H), 7.55-7.50 (m, 2H), 7.44 (ddd, J = 8.3, 7.2, 1.2 Hz, 1H), 7.36 (ddd, J = 8.1, 7.2, 1.0 Hz, 1H), 7.19 - 7.15 (m, 2H), 3.12 (s, 3H).

Figur 15 zeigt das Emissionsspektrum von Beispiel **15** (10 % in PMMA). Das Emissionsmaximum liegt bei 467 nm. Die Photolumineszenzquantenausbeute (PLQY) beträgt 48 % und die Halbwertsbreite (FWHM) 92 nm (0,50 eV). Es ergibt sich eine $CIE_y$ von 0,24 und ein BMI von 200.

**Beispiel 16**

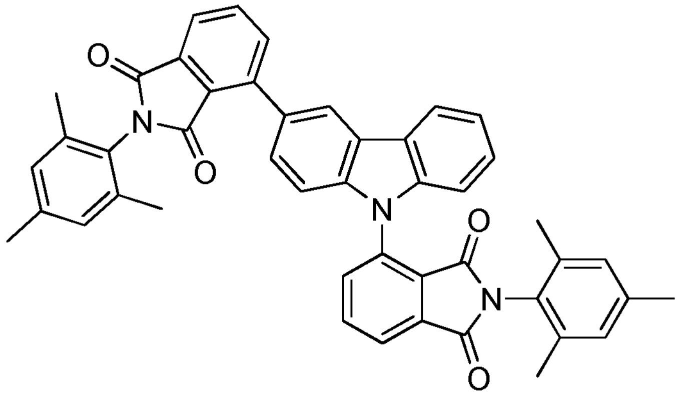

Beispiel 16 wurde nach AAV 4 durch die Überführung von 3-(3-Bromcarbazolyl)-*N*-mesitylphthalimid in den entsprechenden Boronsäurepinacolester und die anschließende Umsetzung mit *N*-Mesityl-3-chlorphthalimid in einer Ausbeute von 87% hergestellt.

$^1$H NMR (500 MHz, Chloroform-d) δ 8.14 (d, J = 7.7 Hz, 1H), 8.10 (dd, J = 6.9, 1.4 Hz, 1H), 8.03 - 7.99 (m, 1H), 7.99 - 7.94 (m, 2H), 7.89 - 7.82 (m, 2H), 7.68 (dd, J = 8.5, 1.8 Hz, 1H), 7.40 (ddd, J = 8.3, 7.2, 1.2 Hz, 1H), 7.35-7.23 (m, 4H), 6.97 (s, 2H), 6.93 (m, 2H), 2.30 (s, 3H), 2.27 (s, 3H), 2.14 (s, 3H), 2.13 (s, 3H), 2.12 (s, 3H), 2.11 (s, 3H).

Figur 16 zeigt das Emissionsspektrum von Beispiel **16** (10 % in PMMA). Das Emissionsmaximum liegt bei 489 nm. Die Photolumineszenzquantenausbeute (PLQY) beträgt 59 % und die Halbwertsbreite (FWHM) 97 nm (0,48 eV). Es ergibt sich eine $CIE_y$ von 0,39 und ein BMI von 151.

**Beispiel 17**

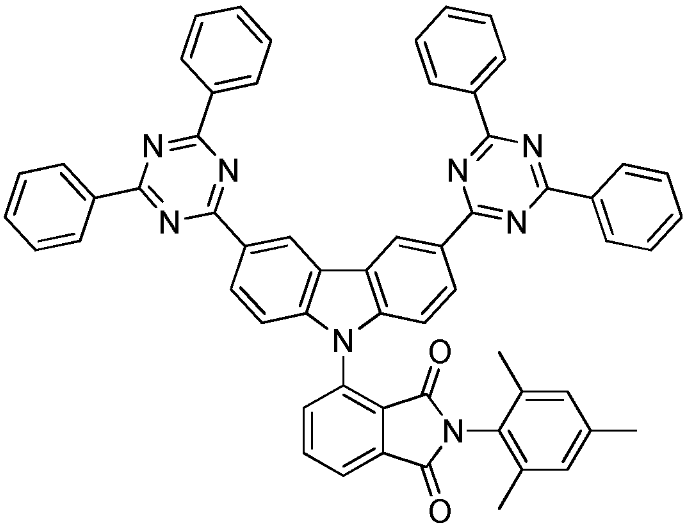

Beispiel 17 wurde nach AAV 4 durch die Überführung von 3-(3,6-Dibromcarbazolyl)-*N*-mesitylphthalimid in den entsprechenden Bis(boronsäurepinacolester) und die anschließende Umsetzung mit Chlordiphenyltriazin in einer Ausbeute von 79% hergestellt.

$^1$H NMR (500 MHz, Chloroform-d): δ = 9.77 (dd, *J* = 1.7, 0.6 Hz, 2H), 8.93 (dd, *J* = 8.7, 1.7 Hz, 2H), 8.88 - 8.86 (m, 8H), 8.23 (dd, *J* = 7.2, 1.2 Hz, 1H), 8.15 - 8.09 (m, 2H), 7.68 - 7.62 (m, 12H), 7.40 (dd, *J* = 8.6, 0.6 Hz, 2H), 6.94 (s, 2H), 2.26 (s, 3H), 2.16 (s, 6H) ppm.

Figur 17 zeigt das Emissionsspektrum von Beispiel **17** (10 % in PMMA). Das Emissionsmaximum liegt bei 488 nm. Die Photolumineszenzquantenausbeute (PLQY) beträgt 55 % und die Halbwertsbreite (FWHM) 94 nm (0,47 eV). Es ergibt sich eine $CIE_y$ von 0,37 und ein BMI von 149.

**Beispiel 18**

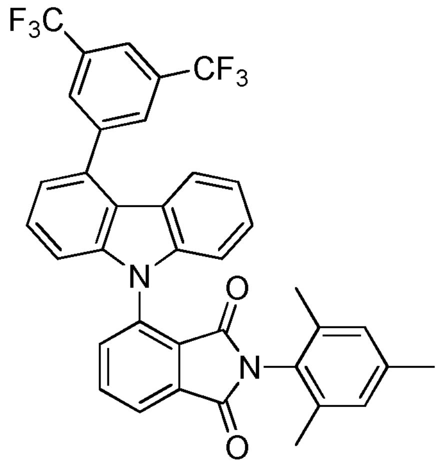

Beispiel 18 wurde nach AAV 5 aus 3-(4-Bromcarbazolyl)-*N*-mesitylphthalimid und 3,5-Bis(trifluormethyl)benzolboronsäure in einer Ausbeute von 63 % hergestellt.

$^1$H NMR (500 MHz, Chloroform-d): δ = 8.16 (d, *J* = 1.0 Hz, 1H), 8.15 (d, *J* = 1.0 Hz, 2H), 8.07 (t, *J* = 7.7 Hz, 1H), 8.01 (s, 1H), 7.99 (dd, *J* = 7.9, 1.0 Hz, 1H), 7.46 (dd, *J* = 8.3, 7.3 Hz, 1H), 7.37 (ddd, *J* = 8.3, 7.1, 1.2 Hz, 1H), 7.33 (dt, *J* = 8.1, 0.9 Hz, 1H), 7.28 (dd, *J* = 8.3, 0.9 Hz, 1H), 7.24 (d, *J* = 8.1 Hz, 1H), 7.17 (dd, *J* = 7.4, 0.9 Hz, 1H), 7.08 (ddd, *J* = 8.1, 7.1, 1.0 Hz, 1H), 6.93 (s, 2H), 2.27 (s, 3H), 2.12 (s, 3H), 2.09 (s, 3H) ppm.

Figur 18 zeigt das Emissionsspektrum von Beispiel **18** (10 % in PMMA). Das Emissionsmaximum liegt bei 480 nm. Die Photolumineszenzquantenausbeute (PLQY) beträgt 58 % und die Halbwertsbreite (FWHM) 92 nm (0,48 eV). Es ergibt sich eine $CIE_y$ von 0,31 und ein BMI von 187.

**Beispiel 19**

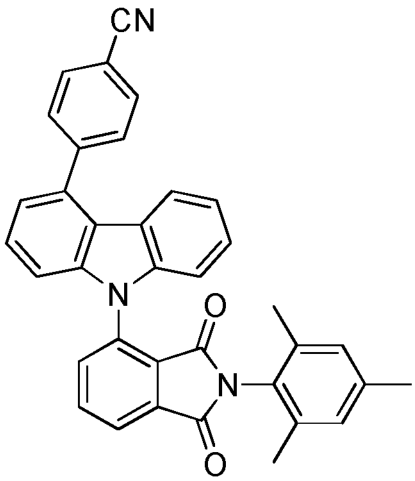

Beispiel 19 wurde nach AAV 5 aus 3-(4-Bromcarbazolyl)-*N*-mesitylphthalimid und 4-Cyanophenylboronsäure in einer Ausbeute von 42% hergestellt.

$^1$H NMR (500 MHz, Chloroform-d): δ = 8.14 (dd, *J* = 7.4, 1.0 Hz, 1H), 8.05 (t, *J* = 7.7 Hz, 1H), 7.98 (dd, *J* = 8.0, 1.0 Hz, 1H), 7.85 - 7.76 (m, 2H), 7.78 - 7.76 (m, 2H), 7.44 (dd, *J* = 8.3, 7.3 Hz, 1H), 7.40 (dt, *J* = 8.0, 0.9 Hz, 1H), 7.35 - 7.33 (m, 1H), 7.24 (d, 1H), 7.22 (dt, *J* = 8.3, 0.9 Hz, 1H), 7.12 (dd, *J* = 7.3, 0.9 Hz, 1H), 7.07 (ddd, *J* = 8.1, 7.0, 1.0 Hz, 1H), 6.93 (s, 2H), 2.27 (s, 3H), 2.12 (s, 3H), 2.09 (s, 3H) ppm.

Figur 19 zeigt das Emissionsspektrum von Beispiel **19** (10 % in PMMA). Das Emissionsmaximum liegt bei 486

nm. Die Photolumineszenzquantenausbeute (PLQY) beträgt 56 % und die Halbwertsbreite (FWHM) 94 nm (0,47 eV). Es ergibt sich eine $CIE_y$ von 0,36 und ein BMI von 156.

**Beispiel 20**

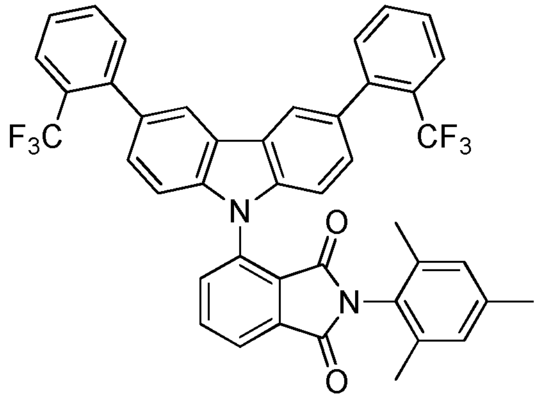

Beispiel 20 wurde nach AAV 5 aus 3-(3,6-Dibromcarbazolyl)-*N*-mesitylphthalimid und 2-Trifluormethylphenylboronsäure in einer Ausbeute von 40% hergestellt.
$^1$H NMR (500 MHz, Chloroform-d): δ = 8.12 (dd, *J* = 7.1, 1.3 Hz, 1H), 8.09 - 8.03 (m, 4H), 7.77 (d, *J* = 7.7 Hz, 2H), 7.58 (t, *J* = 7.6 Hz, 2H), 7.48 (d, *J* = 7.9 Hz, 2H), 7.44 (d, *J* = 7.8 Hz, 2H), 7.38 (d, *J* = 7.4 Hz, 2H), 7.28 (d, *J* = 8.5 Hz, 2H), 6.96 (s, 2H), 2.29 (s, 3H), 2.14 (s, 6H) ppm.

Das Emissionsspektrum von Beispiel **20** (10 % in PMMA) wurde gemessen. Das Emissionsmaximum liegt bei 484 nm. Die Photolumineszenzquantenausbeute (PLQY) beträgt 68 % und die Halbwertsbreite (FWHM) 94 nm (0,48 eV). Es ergibt sich eine $CIE_y$ von 0,35 und ein BMI von 194.

**Beispiel 21**

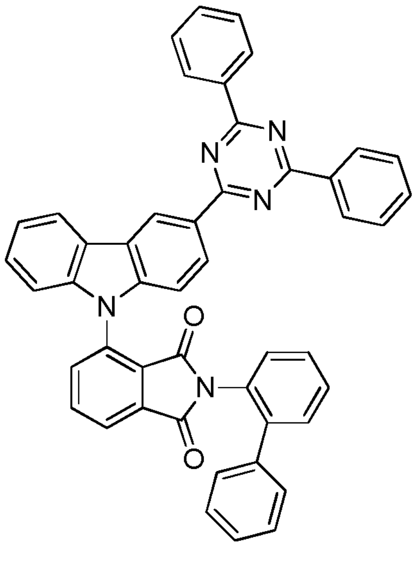

Beispiel 21 wurde nach AAV 4 ausgehend von 3-(3-Bromcarbazolyl)-*N*-(*o*-biphenyl)phthalimid in einer Ausbeute von 66% hergestellt.
$^1$H NMR (500 MHz, Chloroform-d): δ (ppm) = 9.54 (dd, *J* = 12.2, 1.6 Hz), 8.87-8.85 (m), 8.83-8.81 (m), 8.73 (dd, *J* = 8.7, 1.7 Hz), 8.33-8.31 (m), 8.05 (td, *J* = 7.5, 1.0 Hz), 7.98 (td, *J* = 7.7, 3.1 Hz, 1H), 7.92 (dd, *J* = 7.9, 1.1 Hz), 7.91 (dd, *J* = 7.9, 1.1 Hz), 7.65-7.27 (m), 6.60-6.57 (m). Das Produkt besteht aus zwei Rotameren, deren NMR-Signale sich überlagern.

Figur 21 zeigt das Emissionsspektrum von Beispiel **21** (10 % in PMMA). Das Emissionsmaximum liegt bei 481 nm. Die Photolumineszenzquantenausbeute (PLQY) beträgt 68 % und die Halbwertsbreite (FWHM) 92 nm (0,47 eV). Es ergibt sich eine $CIE_y$ von 0,33 und ein BMI von 206. Die Emissionslebensdauer beträgt 5,7 µs.

**Beispiel 22**

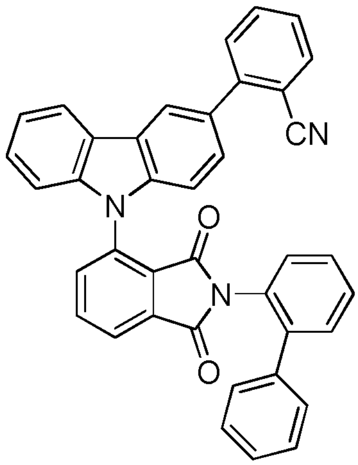

Beispiel 22 wurde nach AAV 4 ausgehend von 3-(3-Bromcarbazolyl)-*N*-(*o*-biphenyl)phthalimid in einer Ausbeute von 42% hergestellt.

Das Emissionsspektrum von Beispiel **22** (10 % in PMMA) wurde aufgenommen. Das Emissionsmaximum liegt bei 489 nm. Die Photolumineszenzquantenausbeute (PLQY) beträgt 62 % und die Halbwertsbreite (FWHM) 97 nm (0,48 eV). Es ergibt sich eine $CIE_y$ von 0,37 und ein BMI von 168. Die Emissionslebensdauer beträgt 5,9 µs.

Beispiel 23

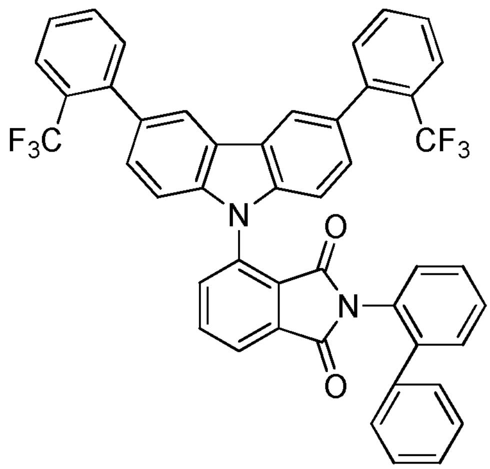

Beispiel 23 wurde nach AAV 5 aus 3-(3,6-Dibromcarbazolyl)-*N*-(*o*-biphenyl)phthalimid und 2-Trifluormethylphenylboronsäure in einer Ausbeute von 80% hergestellt.

Das Emissionsspektrum von Beispiel **23** (10 % in PMMA) wurde gemessen. Das Emissionsmaximum liegt bei 483 nm. Die Photolumineszenzquantenausbeute (PLQY) beträgt 68 % und die Halbwertsbreite (FWHM) 93 nm (0,47 eV). Es ergibt sich eine $CIE_y$ von 0,34 und ein BMI von 200.

**Beispiel 24**

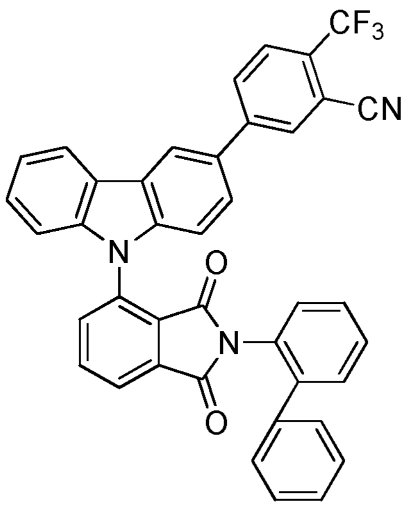

Beispiel 24 wurde nach AAV 4 durch die Überführung von 3-(3-Bromcarbazolyl)-*N*-(*o*-biphenyl)phthalimid in den entsprechenden Bis(boronsäurepinacolester) und die anschließende Umsetzung mit 3-Brom-6-trifluormethyl-benzonitril in einer Ausbeute von 64% hergestellt

Das Emissionsspektrum von Beispiel **24** (10% in PMMA) wurde gemessen. Das Emissionsmaximum liegt bei 491 nm. Die Photolumineszenzquantenausbeute (PLQY) beträgt 65 % und die Halbwertsbreite (FWHM) 96 nm (0,48 eV). Es ergibt sich eine $CIE_y$ von 0,37 und ein BMI von 176.

**Beispiel 25**

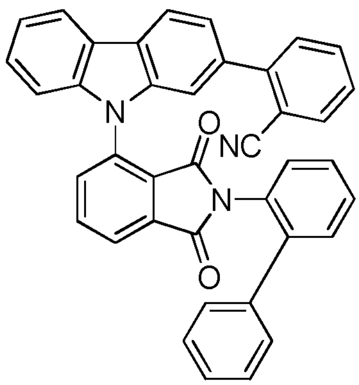

Beispiel 25 wurde nach AAV 4 durch die Überführung von 3-(2-Bromcarbazolyl)-*N*-(*o*-biphenyl)phthalimid in den entsprechenden Bis(boronsäurepinacolester) und die anschließende Umsetzung mit 2-Brombenzonitril in einer Ausbeute von 97 % hergestellt.

Das Emissionsspektrum von Beispiel **25** (10 % in PMMA) wurde aufgenommen. Das Emissionsmaximum liegt bei 476 nm. Die Photolumineszenzquantenausbeute (PLQY) beträgt 61 % und die Halbwertsbreite (FWHM) 91 nm (0,48 eV). Es ergibt sich eine $CIE_y$ von 0,29 und ein BMI von 210.

**Beispiel 26**

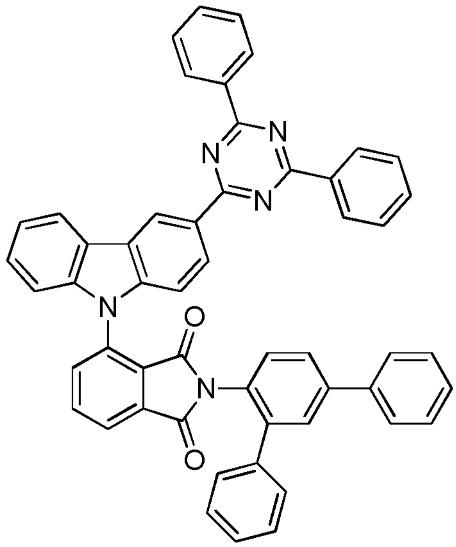

Beispiel 26 wurde nach AAV 4 durch die Überführung von 3-(2-Bromcarbazolyl)-*N*-(*o*-(meta-terphenyl))phthalimid in den entsprechenden Bis(boronsäurepinacolester) und die anschließende Umsetzung mit Chlordiphenyltriazin in einer Ausbeute von 44% hergestellt. Das Emissionsspektrum von Beispiel **26** (10 % in PMMA) wurde aufgenommen. Das Emissionsmaximum liegt bei 486 nm. Die Photolumineszenzquantenausbeute (PLQY) beträgt 65 % und die Halbwertsbreite (FWHM) 93 nm (0,47 eV). Es ergibt sich eine $CIE_y$ von 0,36 und ein BMI von 181. Die Emissionslebensdauer beträgt 5,6 μs.

**Beispiel 27**

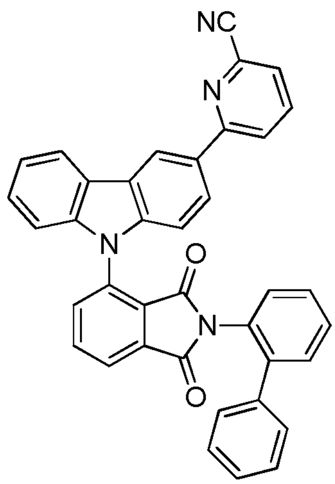

Beispiel 27 wurde nach AAV 4 durch die Überführung von 3-(2-Bromcarbazolyl)-*N*-(*o*-biphenyl)phthalimid in den entsprechenden Bis(boronsäurepinacolester) und die anschließende Umsetzung mit 2-Brom-6-cyanopyridin in einer Ausbeute von 13% hergestellt. Das Emissionsspektrum von Beispiel **27** (10 % in PMMA) wurde aufgenommen. Das Emissionsmaximum liegt bei 489 nm. Die Photolumineszenzquantenausbeute (PLQY) beträgt 61 % und die Halbwertsbreite (FWHM) 97 nm (0,48 eV). Es ergibt sich eine $CIE_y$ von 0,38 und ein BMI von 160.

**Beispiel 28**

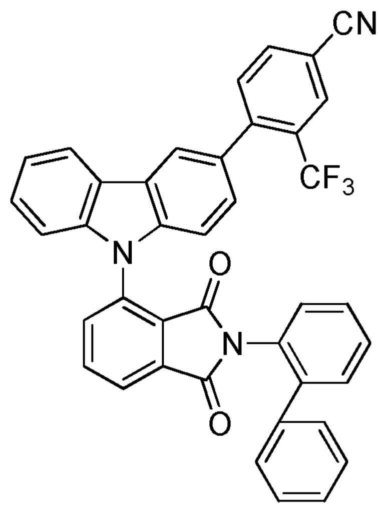

Beispiel 28 wurde nach AAV 4 durch die Überführung von 3-(3-Bromcarbazolyl)-*N*-(*o*-biphenyl)phthalimid in den entsprechenden Bis(boronsäurepinacolester) und die anschließende Umsetzung mit 4-Brom-3-trifluormethyl-benzonitril in einer Ausbeute von 94% hergestellt.

Das Emissionsspektrum von Beispiel **28** (10 % in PMMA) wurde gemessen. Das Emissionsmaximum liegt bei 479 nm. Die Photolumineszenzquantenausbeute (PLQY) beträgt 63 % und die Halbwertsbreite (FWHM) 92 nm (0,46 eV). Es ergibt sich eine $CIE_y$ von 0,31 und ein BMI von 203.

**Beispiel 29**

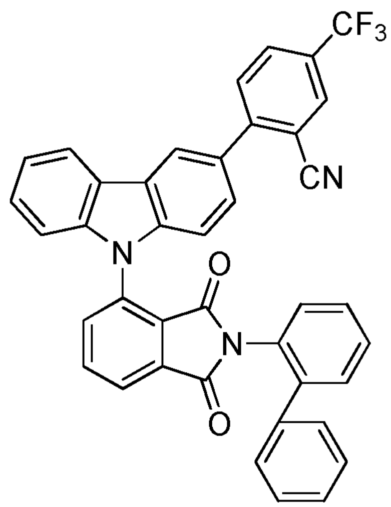

Beispiel 29 wurde nach AAV 4 durch die Überführung von 3-(3-Bromcarbazolyl)-*N*-(*o*-biphenyl)phthalimid in den entsprechenden Bis(boronsäurepinacolester) und die anschließende Umsetzung mit 2-Brom-5-trifluormethyl-benzonitril in einer Ausbeute von 36% hergestellt.

Das Emissionsspektrum von Beispiel **29** (10 % in PMMA) wurde gemessen. Das Emissionsmaximum liegt bei 475 nm. Die Photolumineszenzquantenausbeute (PLQY) beträgt 67 % und die Halbwertsbreite (FWHM) 91 nm (0,48 eV). Es ergibt sich eine $CIE_y$ von 0,29 und ein BMI von 231.

**Beispiel 30**

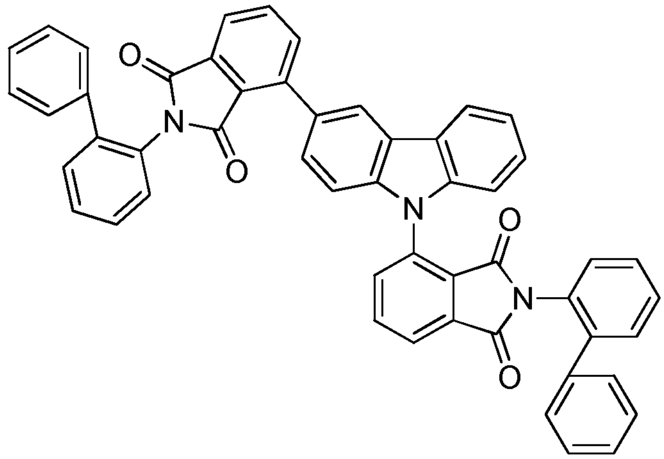

Beispiel 30 wurde nach AAV 4 durch die Überführung von 3-(3-Bromcarbazolyl)-*N*-(*o*-biphenyl)phthalimid in den entsprechenden Boronsäurepinacolester und die anschließende Umsetzung mit *N*-(*o*-biphenyl)phthalimid-3-chlorphthalimid in einer Ausbeute von 45% hergestellt.

Das Emissionsspektrum von Beispiel **30** (10 % in PMMA) wurde gemessen. Das Emissionsmaximum liegt bei 490 nm. Die Photolumineszenzquantenausbeute (PLQY) beträgt 61 % und die Halbwertsbreite (FWHM) 96 nm (0,48 eV). Es ergibt sich eine $CIE_y$ von 0,38 und ein BMI von 161.

**Beispiel 31**

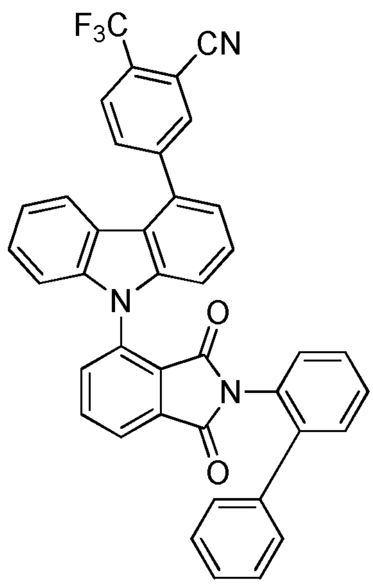

Beispiel 31 wurde nach AAV 4 durch die Überführung von 3-(4-Bromcarbazolyl)-*N*-(*o*-biphenyl)phthalimid in den entsprechenden Bis(boronsäurepinacolester) und die anschließende Umsetzung mit 3-Brom-6-trifluormethyl-benzonitril in einer Ausbeute von 40% hergestellt.

Das Emissionsspektrum von Beispiel **31** (10 % in PMMA) wurde gemessen. Das Emissionsmaximum liegt bei 474 nm. Die Photolumineszenzquantenausbeute (PLQY) beträgt 61 % und die Halbwertsbreite (FWHM) 93 nm (0,48 eV). Es ergibt sich eine $CIE_y$ von 0,33 und ein BMI von 203.

**Beispiel 32**

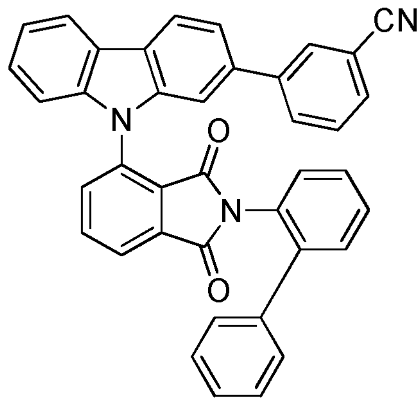

Beispiel 32 wurde nach AAV 5 aus 3-(2-Bromcarbazolyl)-*N*-(*o*-biphenyl)phthalimid und 3-Cyanophenylboronsäure in einer Ausbeute von 31% hergestellt.

Das Emissionsspektrum von Beispiel **32** (10 % in PMMA) wurde gemessen. Das Emissionsmaximum liegt bei 474 nm. Die Photolumineszenzquantenausbeute (PLQY) beträgt 54 % und die Halbwertsbreite (FWHM) 93 nm (0,48 eV). Es ergibt sich eine $CIE_y$ von 0,32 und ein BMI von 169.

**Beispiele D1 und D2:**

Beispiel **3** und Beispiel **5** wurden in OLEDs mit folgendem Aufbau getestet:

| Schicht | Dicke | D1 | D2 |
|---|---|---|---|
| **10** | 100 nm | Al | Al |
| **9** | 2 nm | Liq | Liq |
| **8** | 30 nm | TPBi | TPBi |
| **7** | 10 nm | DPEPO | DPEPO |
| **6** | 20 nm | **3**:DPEPO 20:80 | **5**:DPEPO 30:70 |
| **5** | 10 nm | CzSi | CzSi |
| **4** | 20 nm | TCTA | TCTA |
| **3** | 50 nm | NPB | NPB |
| **2** | 20 nm | m-MTDATA | m-MTDATA |
| **1** | 120 nm | ITO | ITO |

**Performance-Daten**

| OLED-Bauteil | Maximale Leistungseffizienz (Power Efficiency) (lm/W) | Maximale Stromausbeute (Current Efficiency) (cd/A) | Maximale EQE (%) | $\lambda_{max}$ (nm) |
|---|---|---|---|---|
| **D1** | 12,8 ± 0,6 | 26,0 ± 0,5 | 12,7 ± 0,3 | 476 |
| **D2** | 12,6 ± 0,6 | 25,1 ± 0,5 | 12,3 ± 0,2 | 474 |

**Beispiel D3**

Beispiel **21** wurde in einem OLED-Bauteil ("Bauteil D3") mit folgendem Aufbau getestet (Anteil des erfindungsgemäßen Moleküls an der Emissionsschicht ist in Massenprozent angegeben):

| Schicht | Dicke | Material |
|---|---|---|
| **9** | 100 nm | Al |
| **8** | 2 nm | Liq |
| **7** | 40 nm | NBPhen |

(fortgesetzt)

| Schicht | Dicke | Material |
|---|---|---|
| **6** | 20 nm | **Beispiel 21** (10%):H1 |
| **5** | 10 nm | TCTA |
| **4** | 110 nm | NPB |
| **3** | 5 nm | HAT-CN |
| **2** | 50 nm | PEDOT |
| **1** | 130 nm | ITO |
| **Substrat** | | Glas |

**Performance-Daten**

| | |
|---|---|
| $EQE_{max}$ (maximale Externe Quanteneffizienz) | 11,7 ± 0,3% |
| EQE bei 500 cd/$m^2$ | 9.3 ± 0,2 % |
| LT 80 bei 500 cd/$m^2$ | 131 h |

Das Emissionsmaximum liegt bei 478 nm.

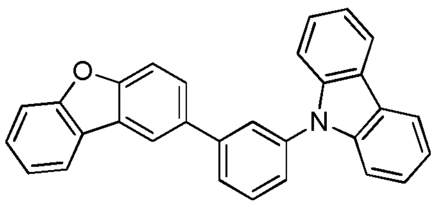

H1

**Beispiel D4**

Beispiel **21** wurde in einem OLED-Bauteil ("Bauteil D4") mit folgendem Aufbau getestet (Anteil des erfindungsgemäßen Moleküls an der Emissionsschicht ist in Massenprozent angegeben):

| Schicht | Dicke | Material |
|---|---|---|
| **7** | 100 nm | Al |
| **6** | 2 nm | Liq |
| **5** | 40 nm | NBPhen |
| **4** | 20 nm | **Beispiel 21** (30%):mCBP |
| **3** | 10 nm | TCTA |
| **2** | 80 nm | NPB |
| **1** | 130 nm | ITO |
| **Substrat** | | Glas |

**Performance-Daten**

| | |
|---|---|
| $EQE_{max}$ (maximale Externe Quanteneffitienz) | 8.91 ± 0,03% |
| EQE bei 1000 cd/$m^2$ | 8.74 ± 0,02 % |

(fortgesetzt)

| | |
|---|---|
| LT 80 bei 1000 cd/m$^2$ | 72 h |

Das Emissionsmaximum liegt bei 486 nm, CIEx wurde mit 0.24 und die CIEy: 0.39 bei 4.5 V bestimmt.

**Weitere Beispiele:**

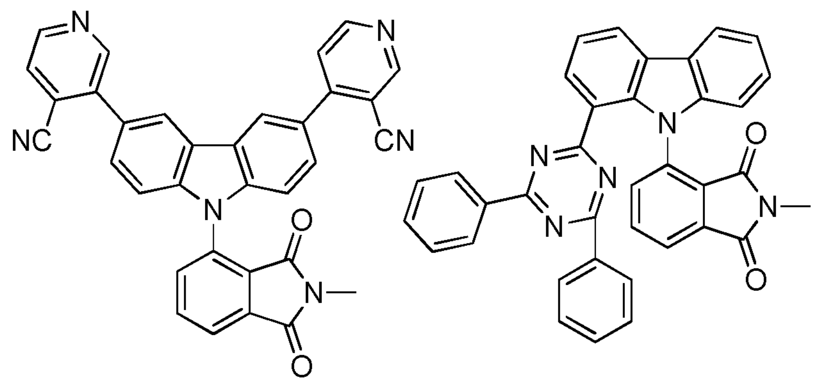
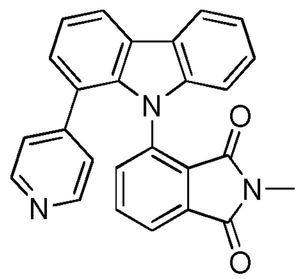

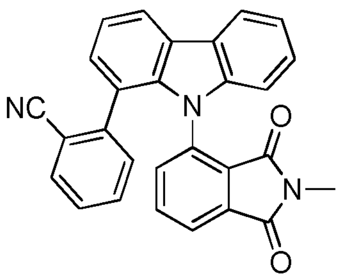
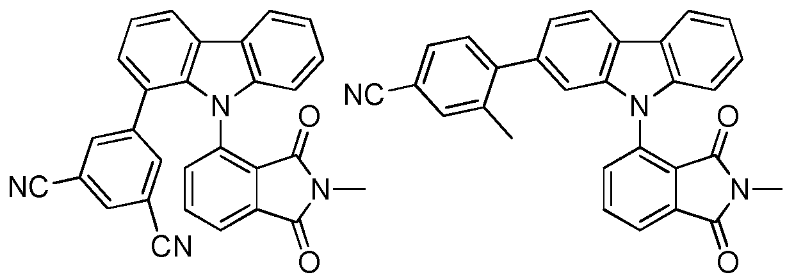

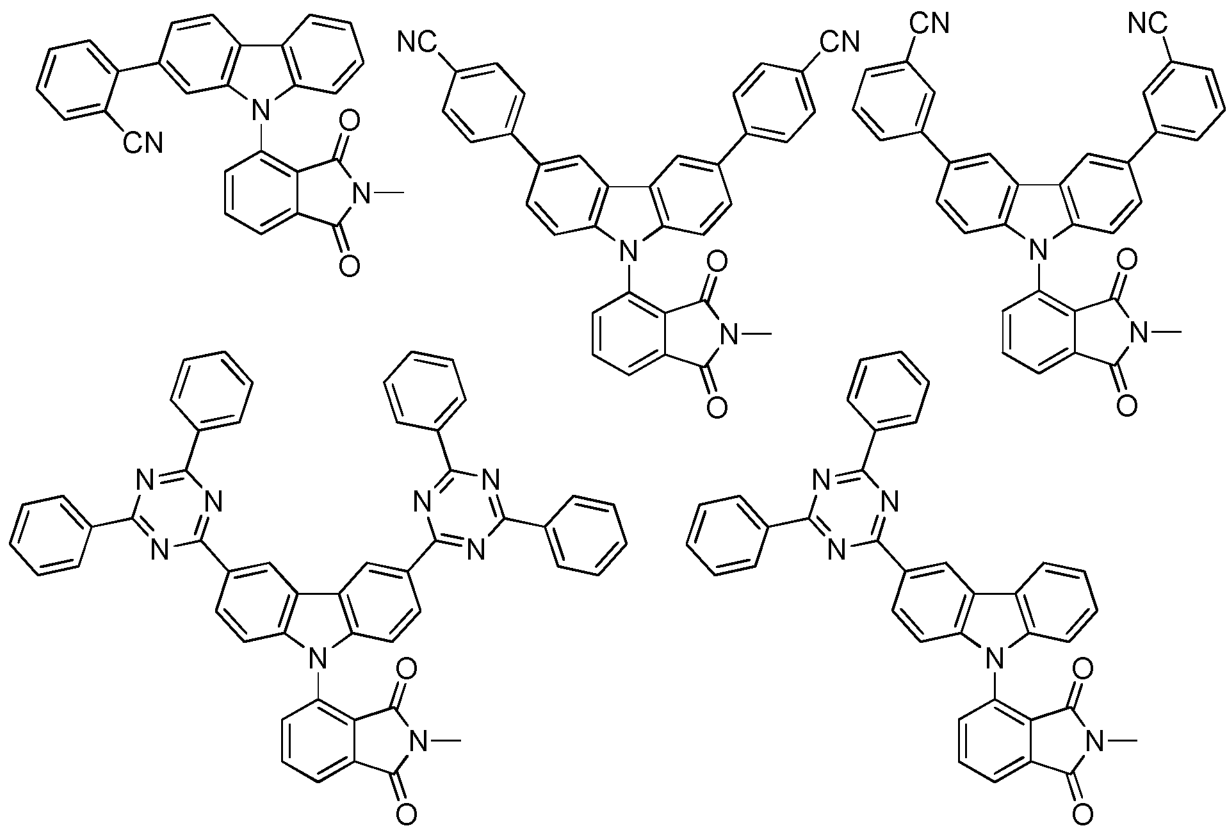

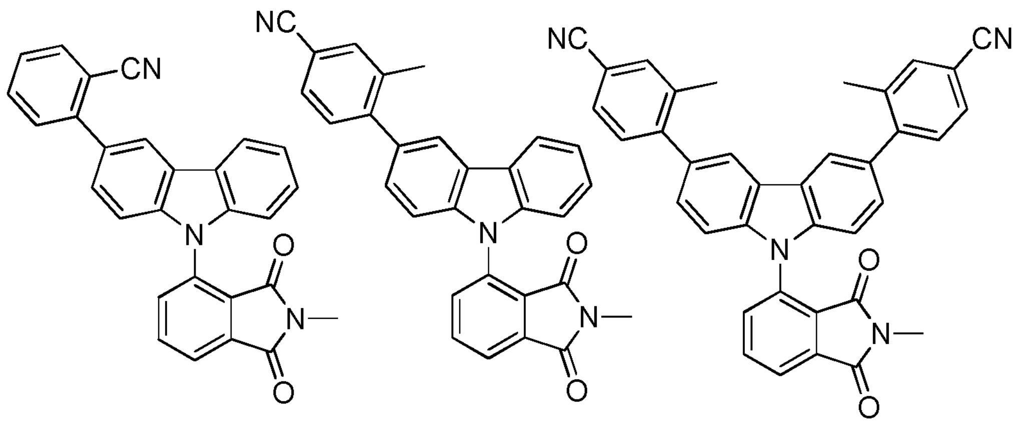

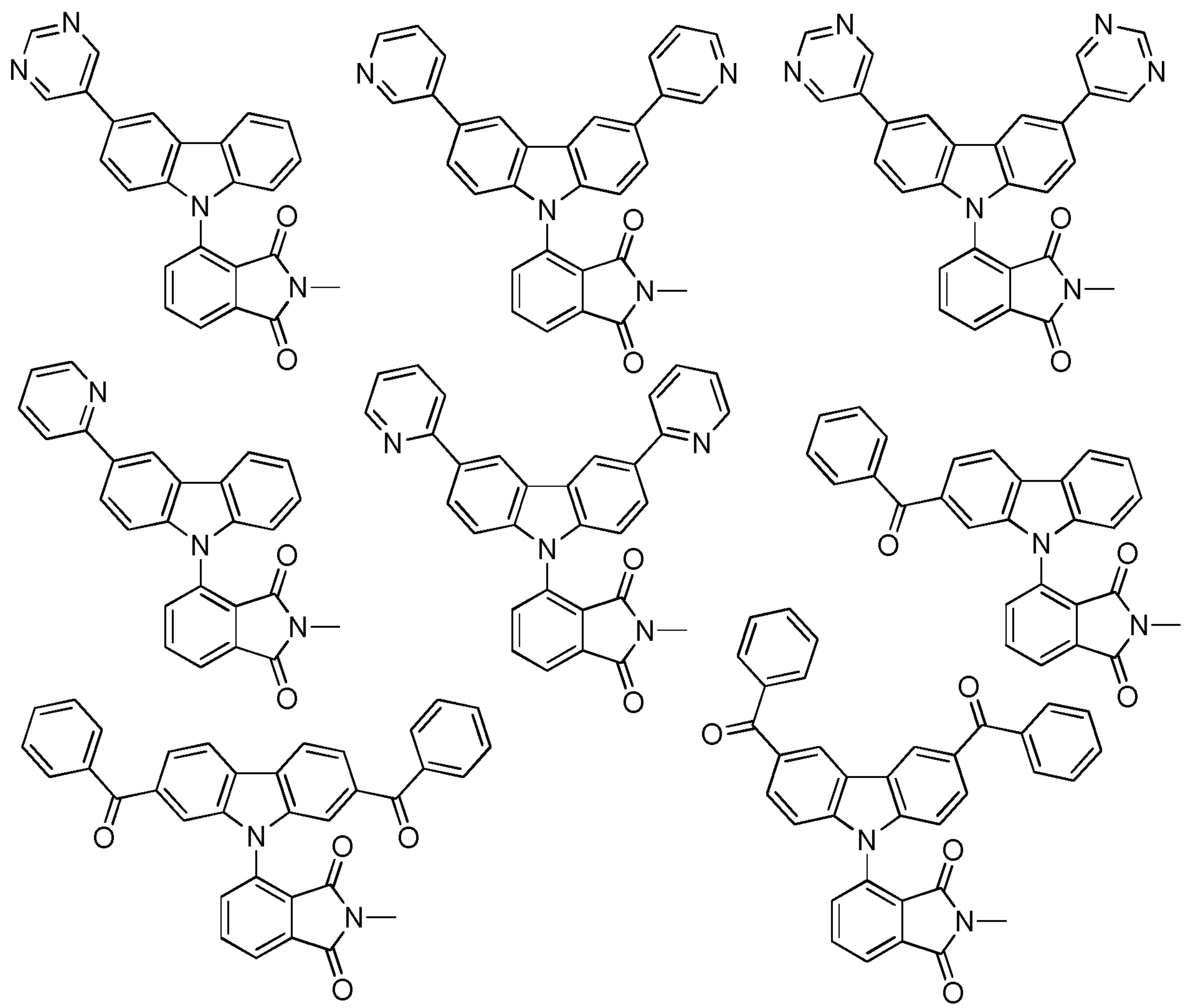

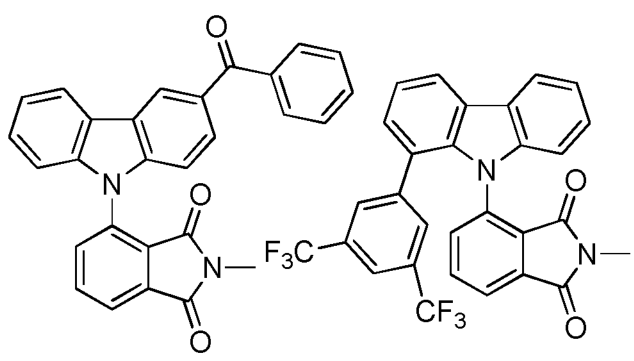

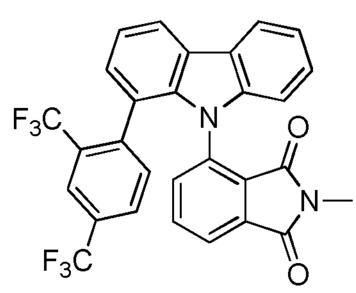

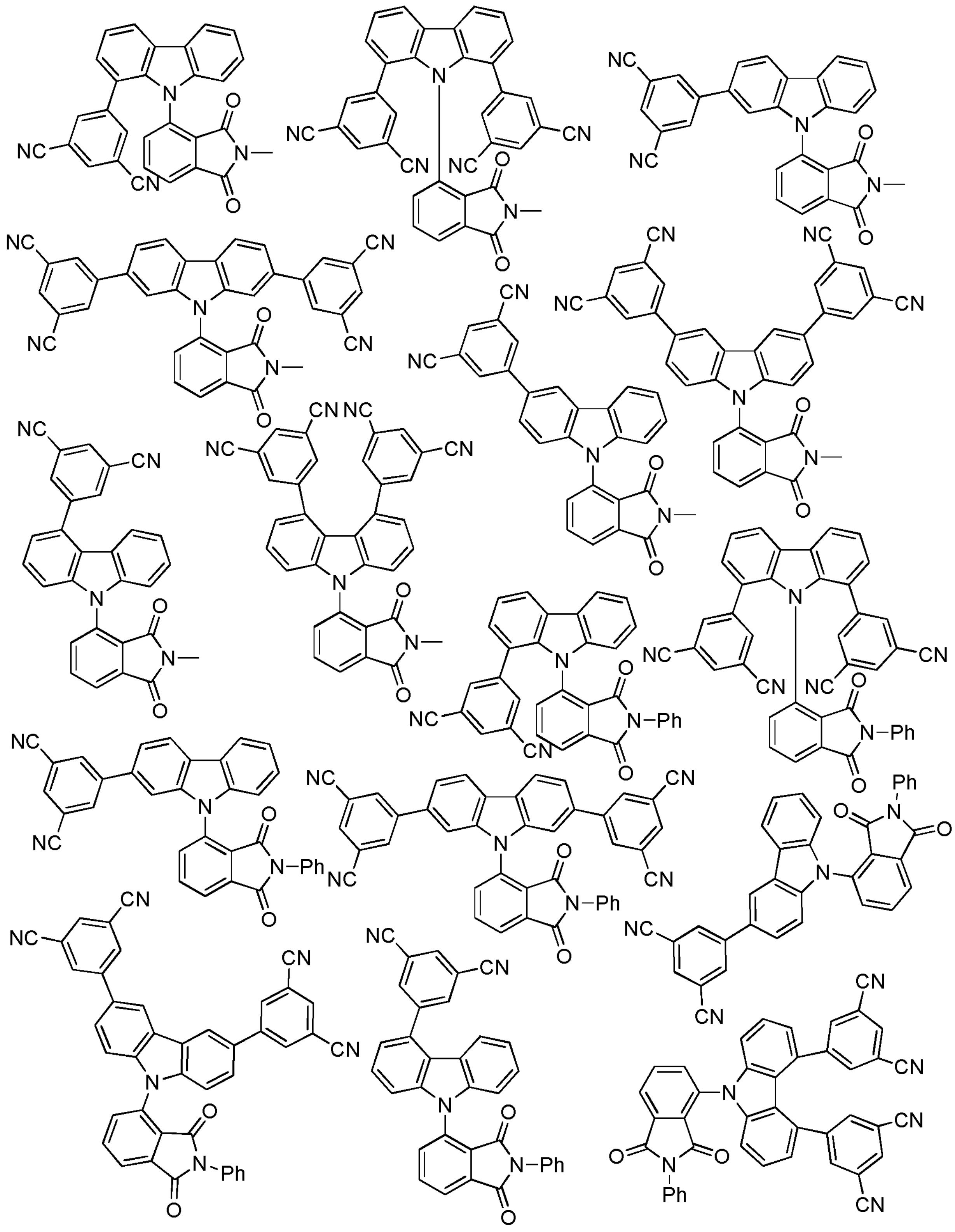

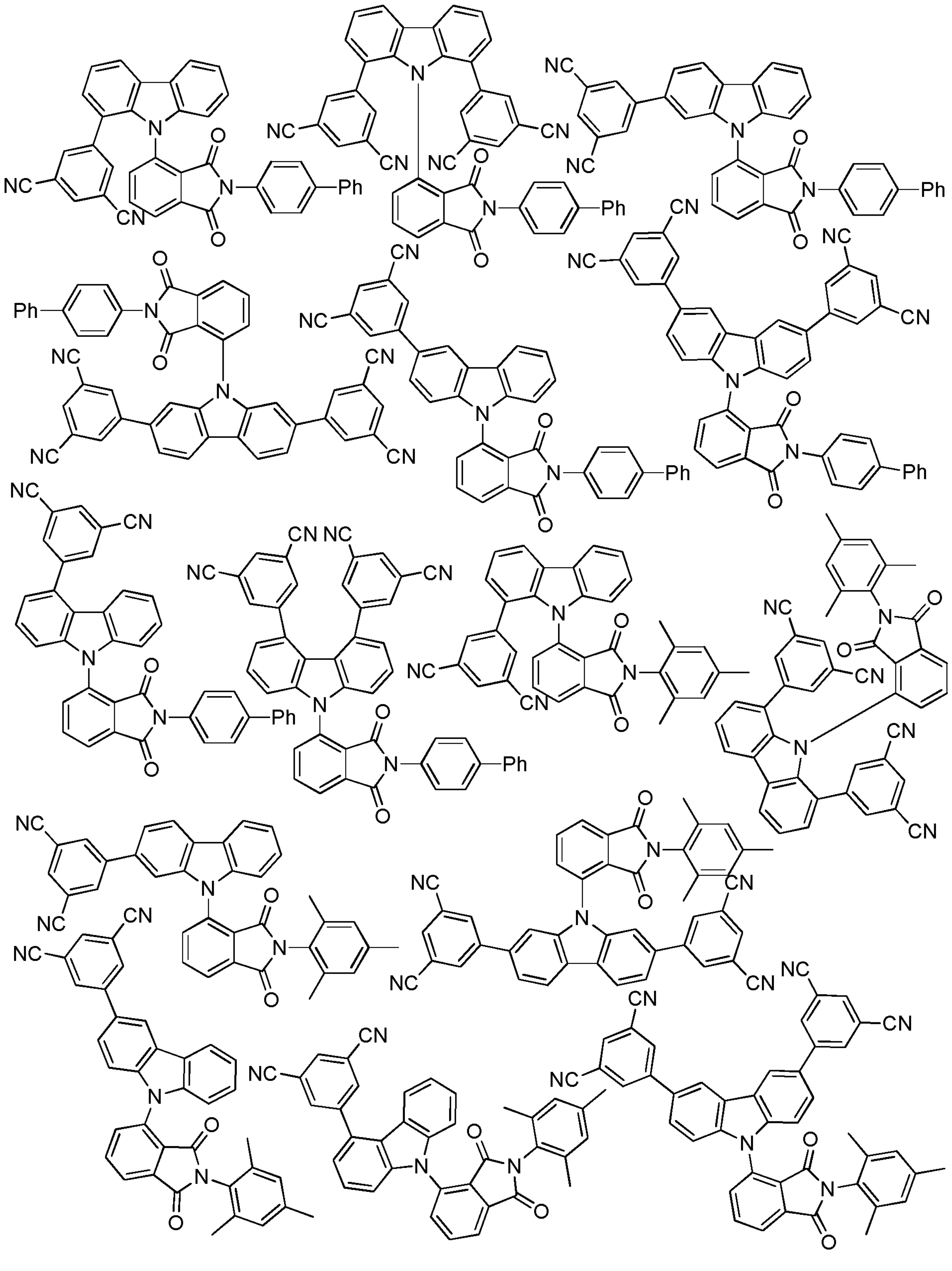

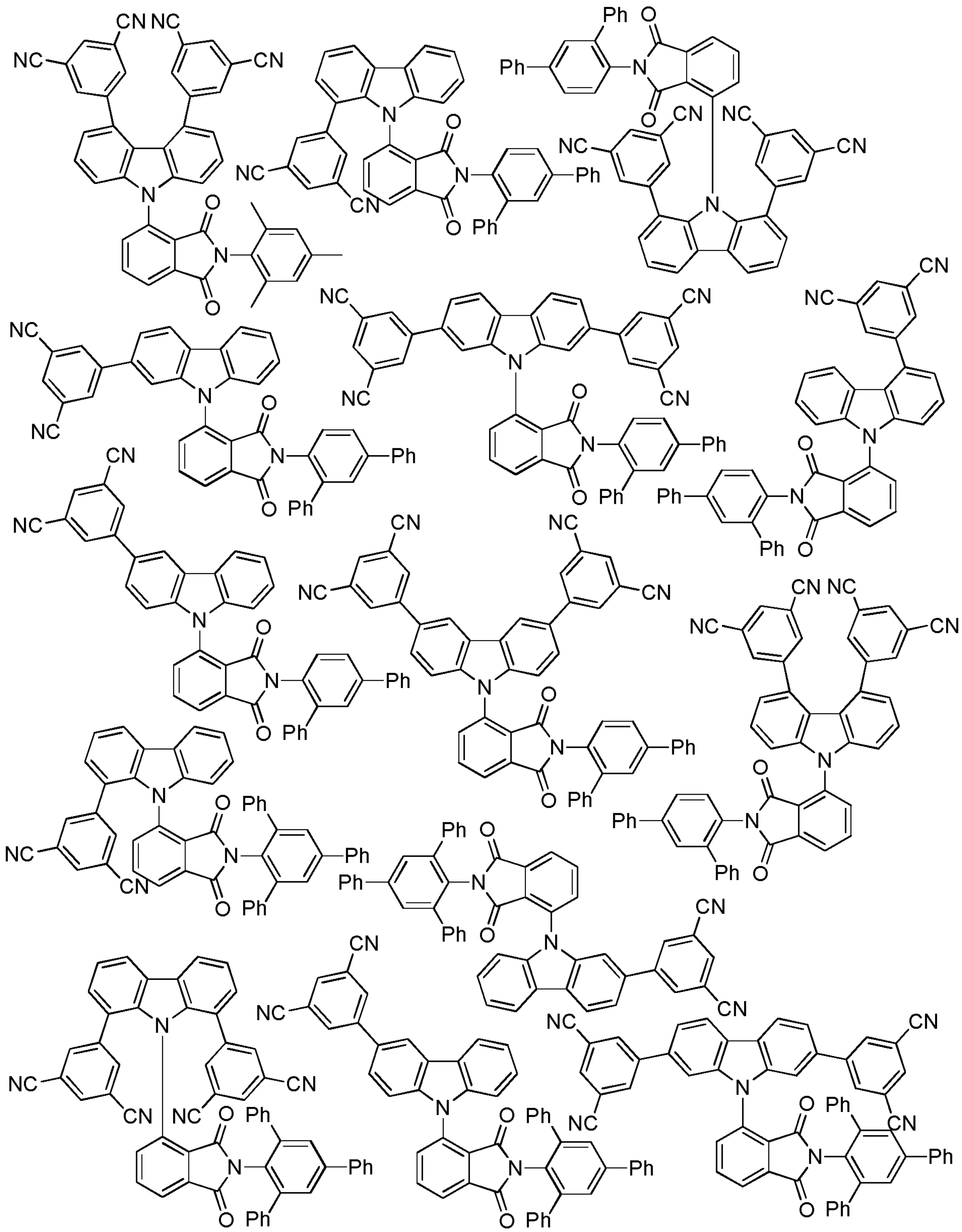

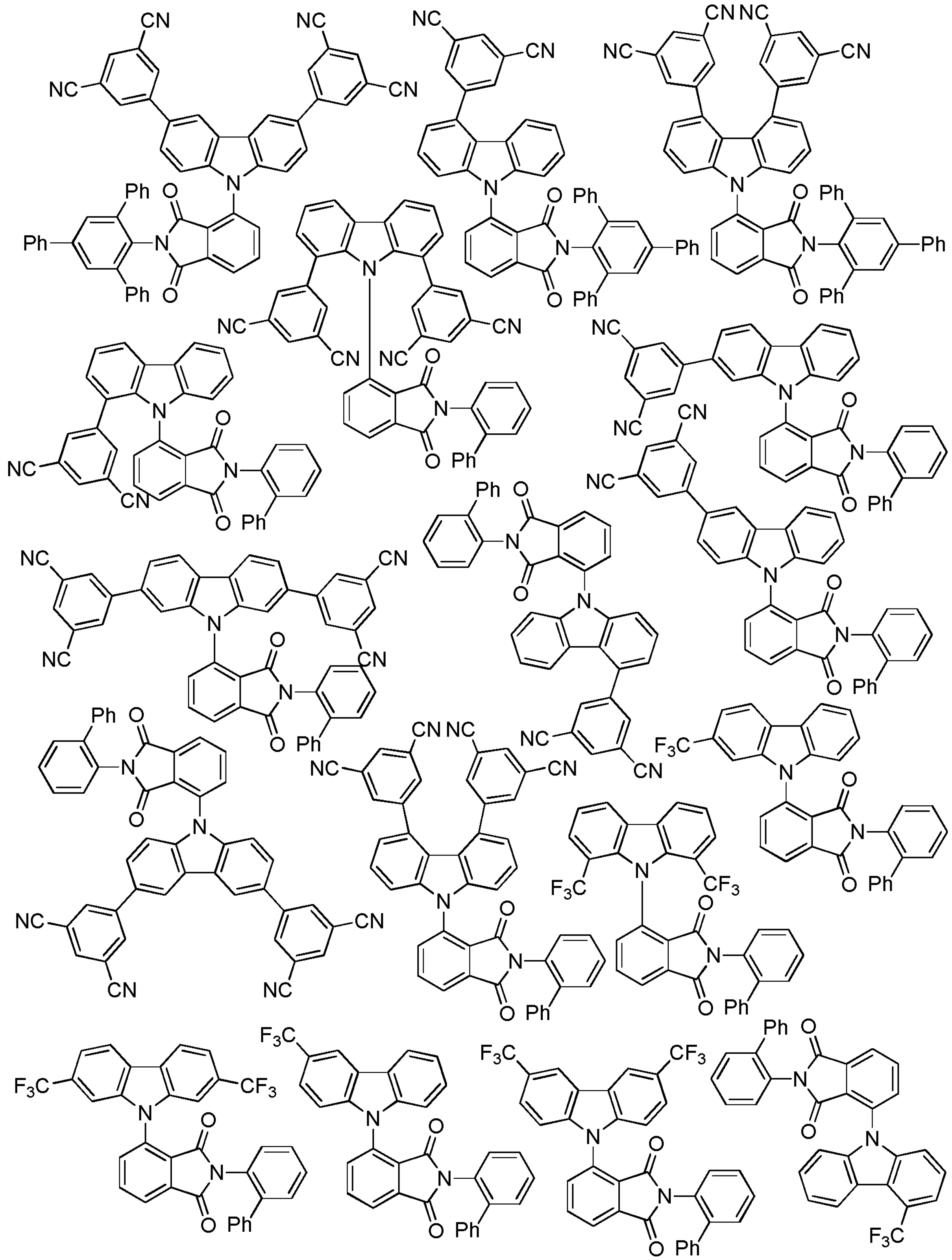

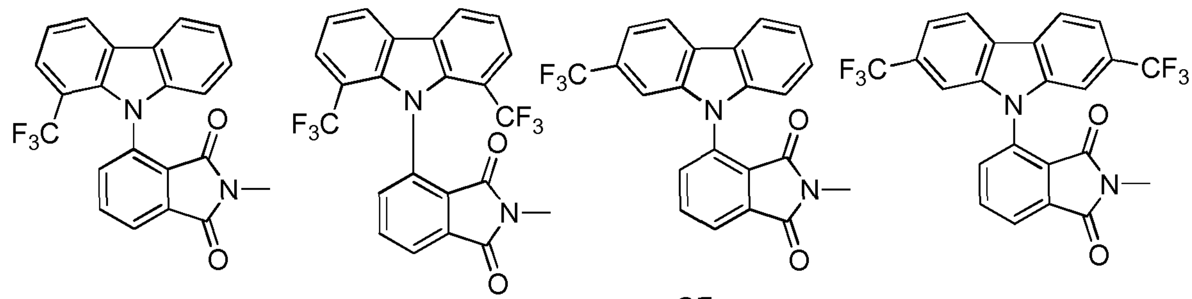

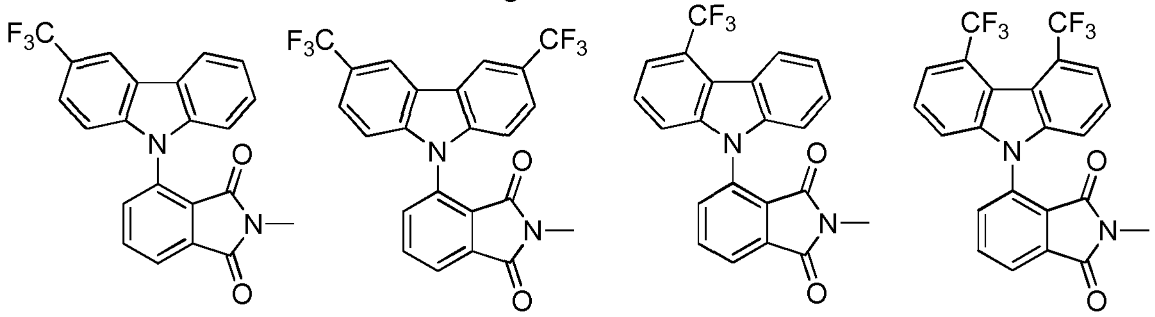

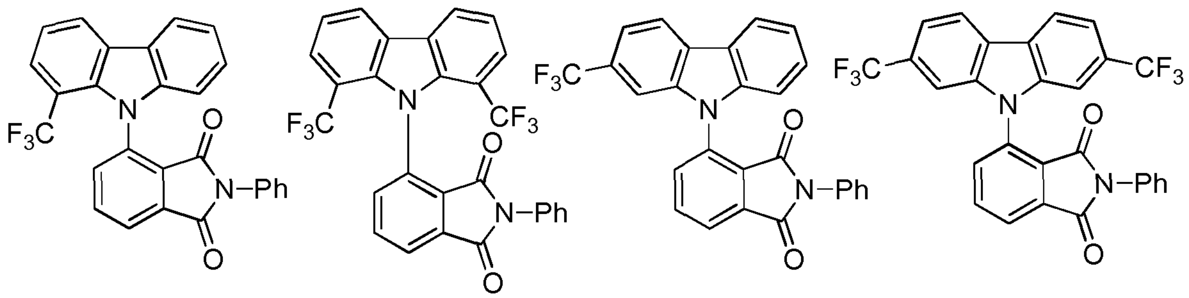

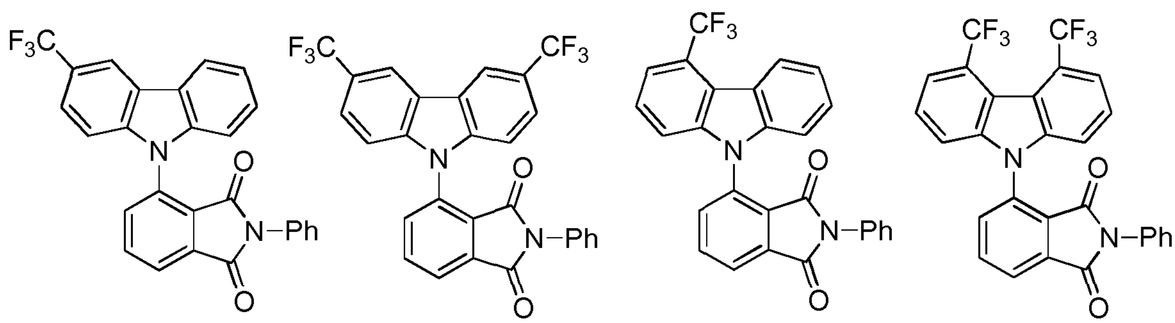

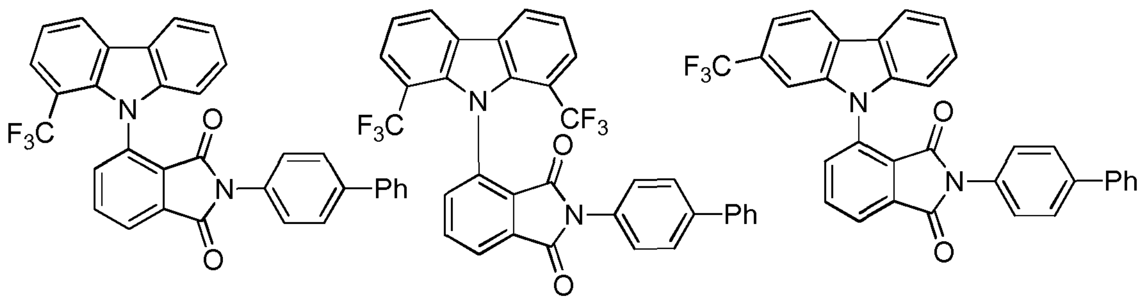

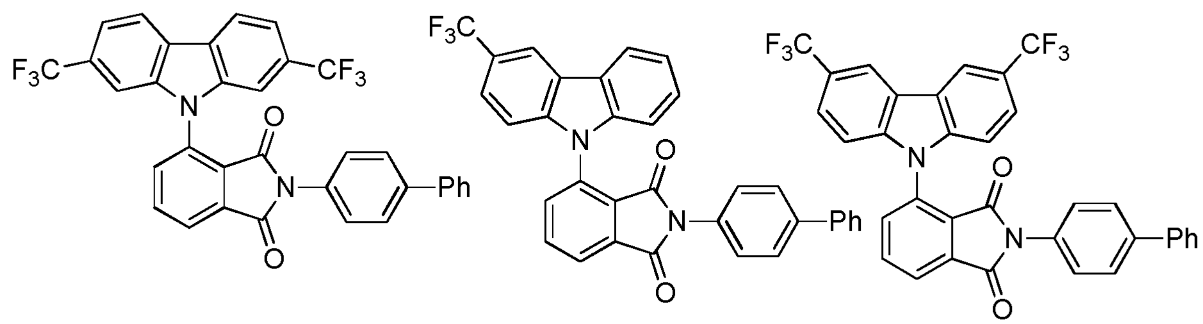

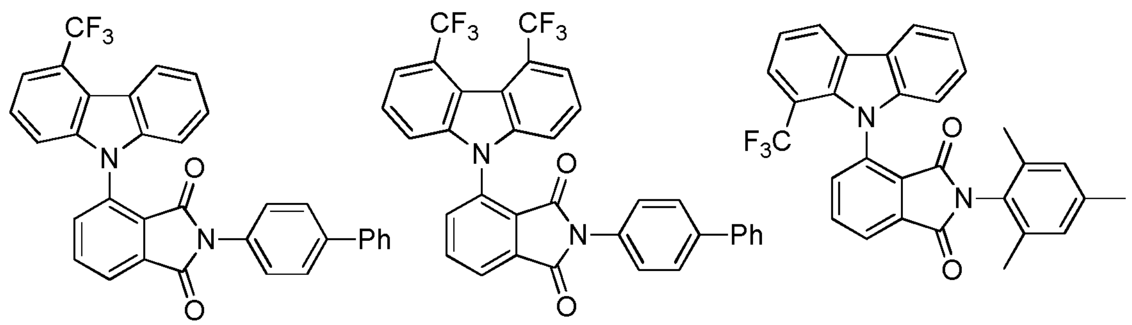

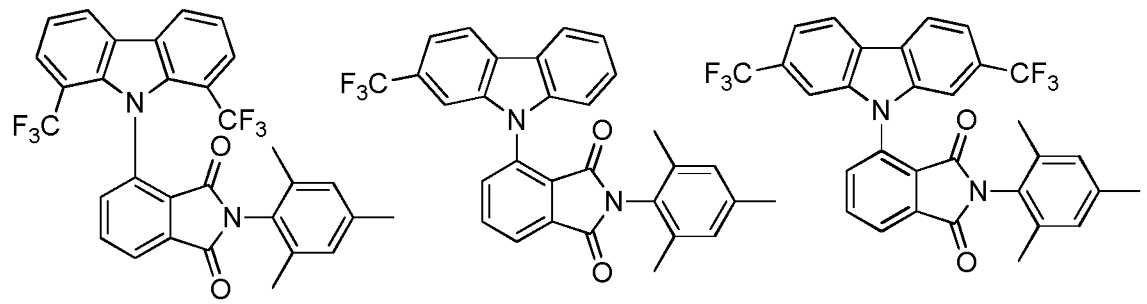

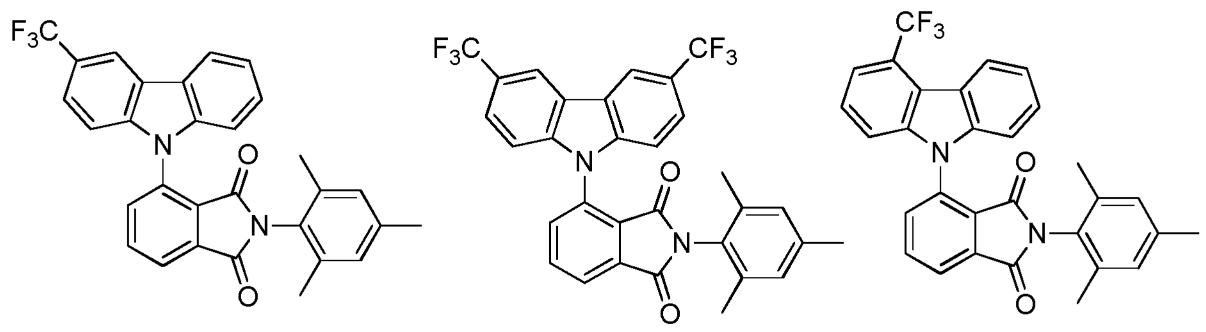

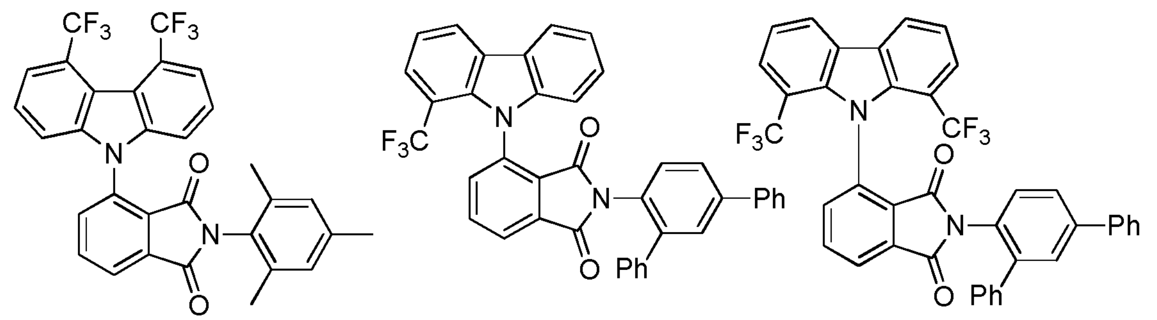

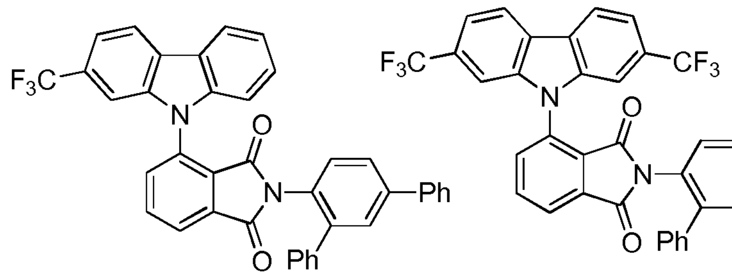

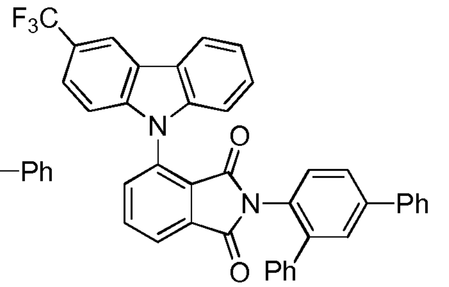

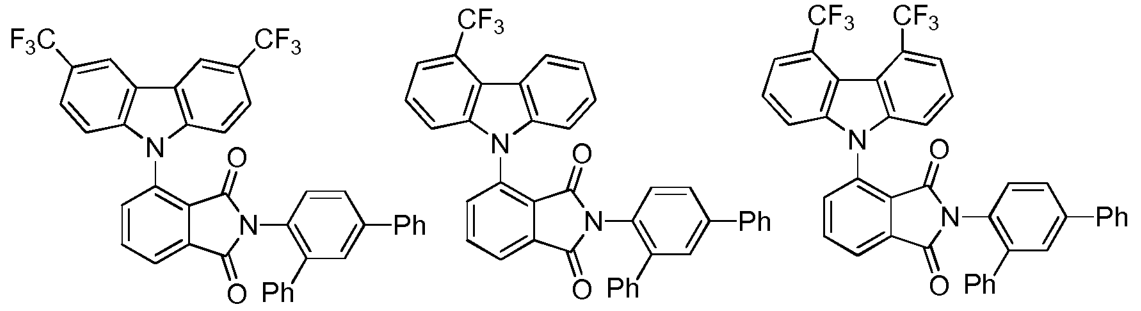

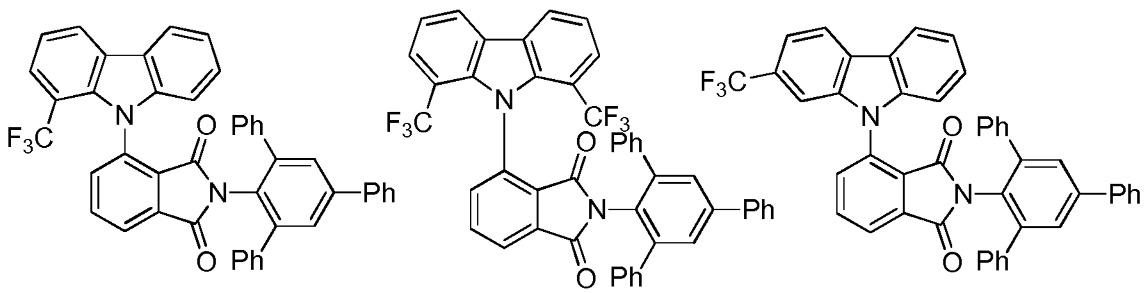

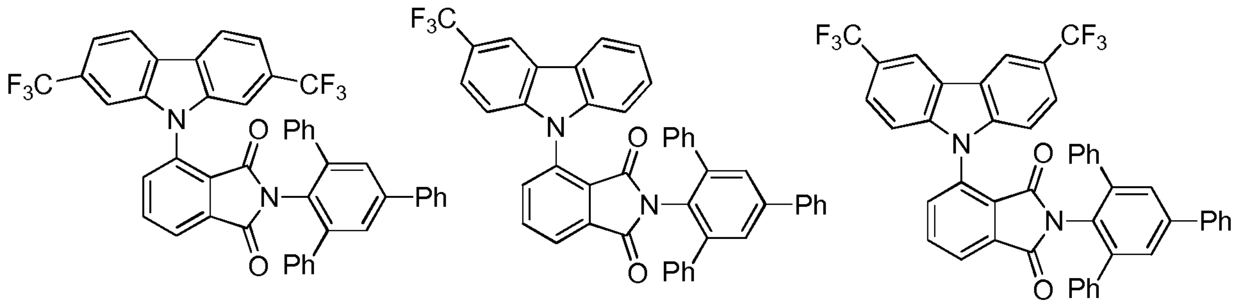

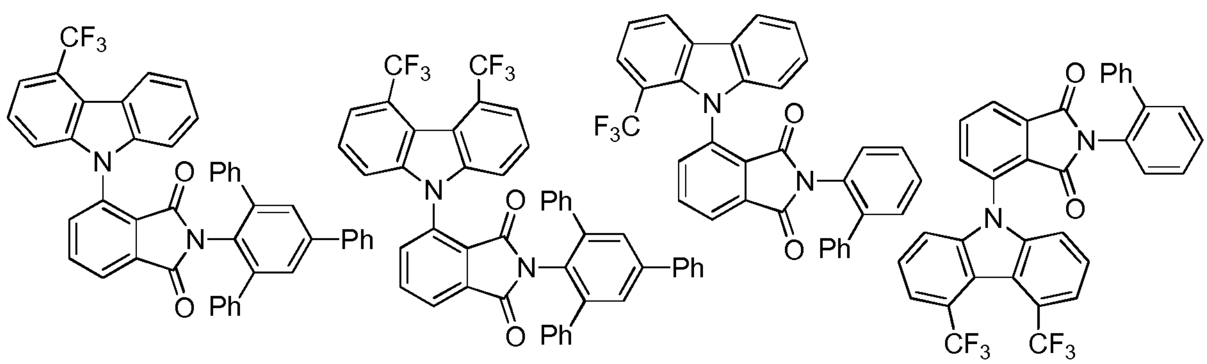

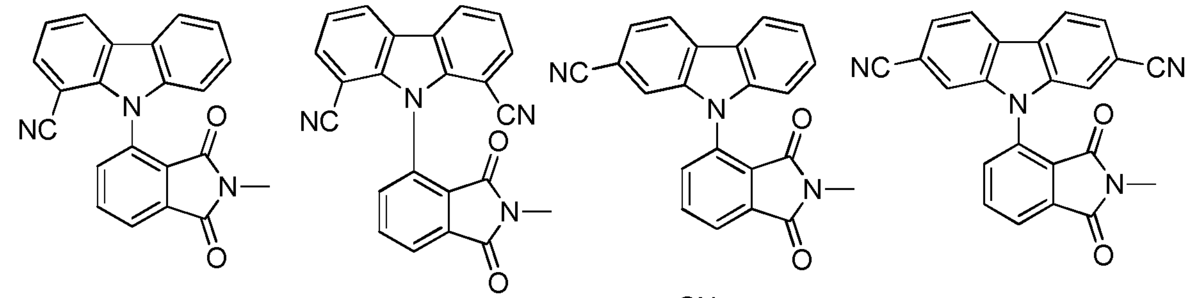

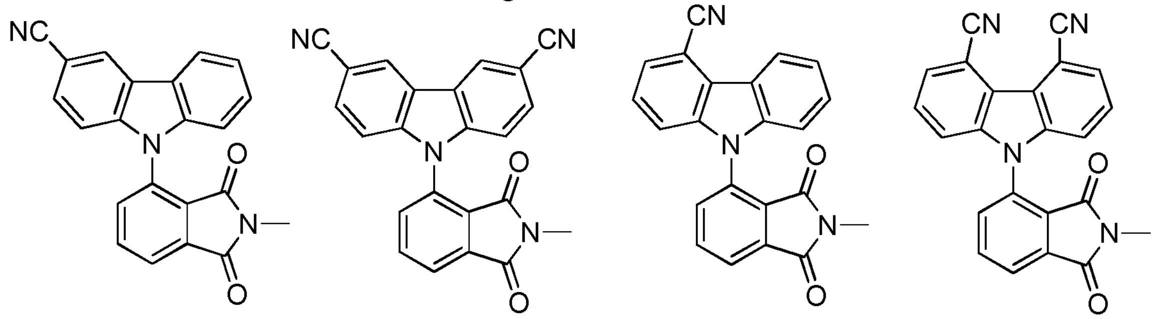

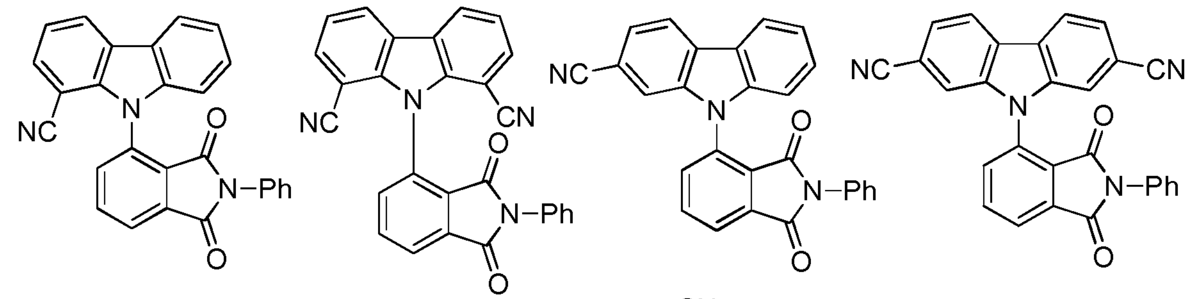

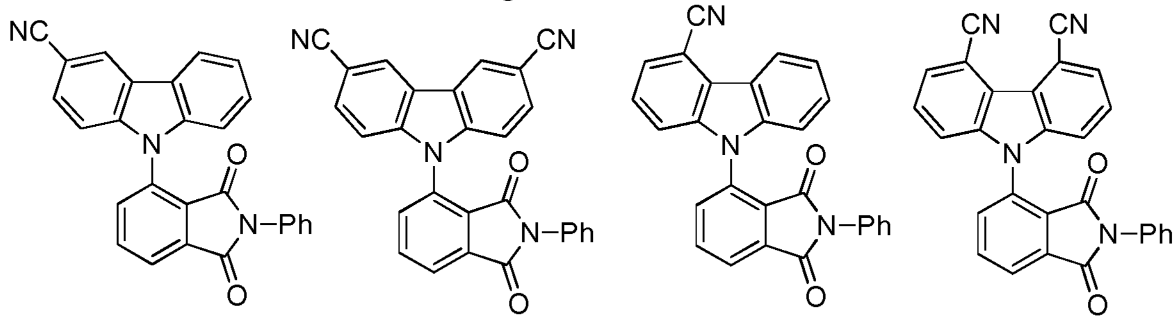

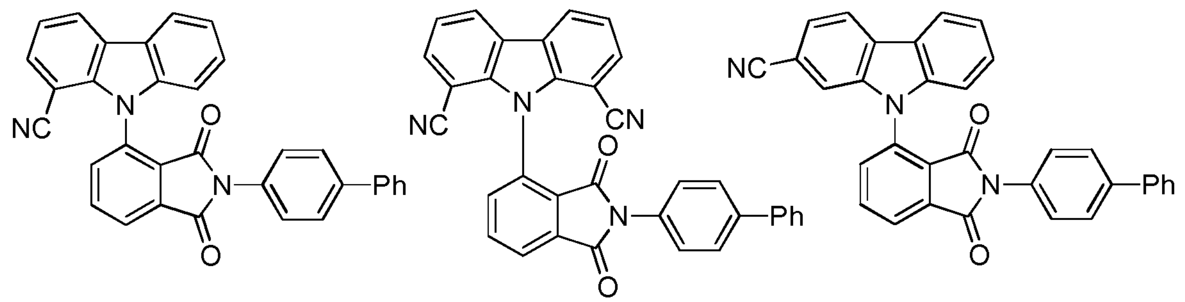

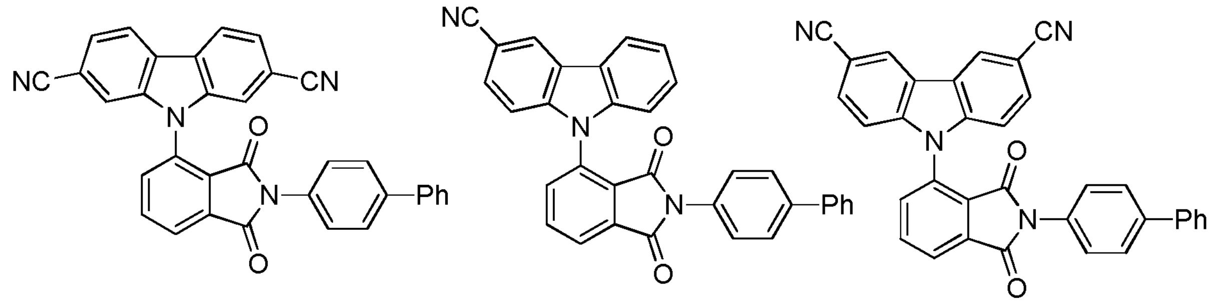

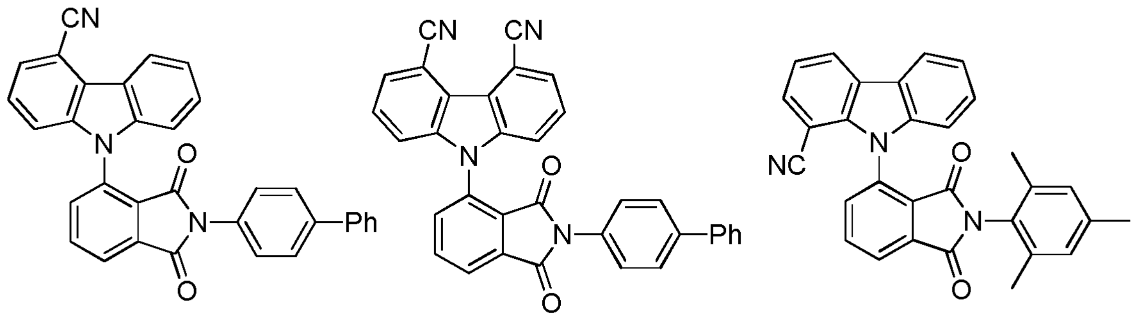

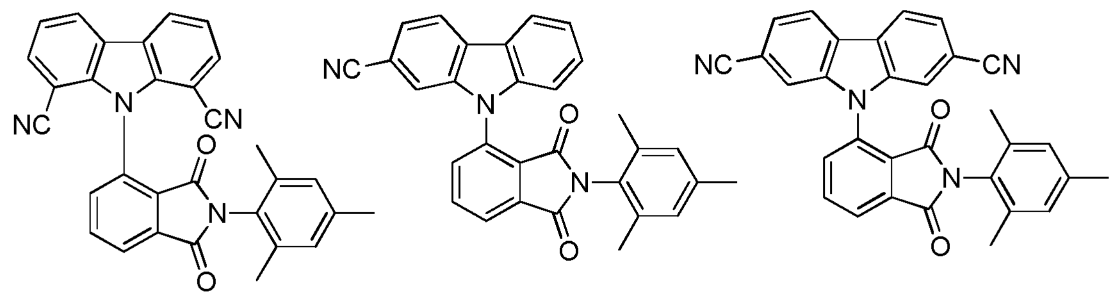

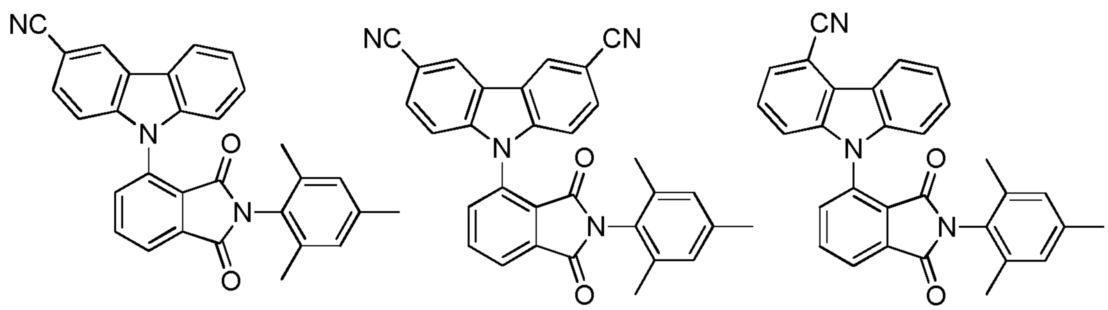

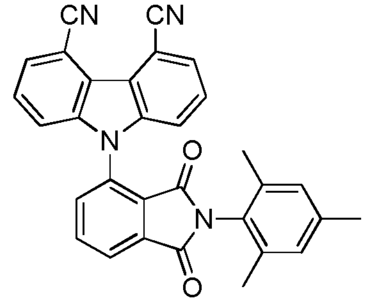

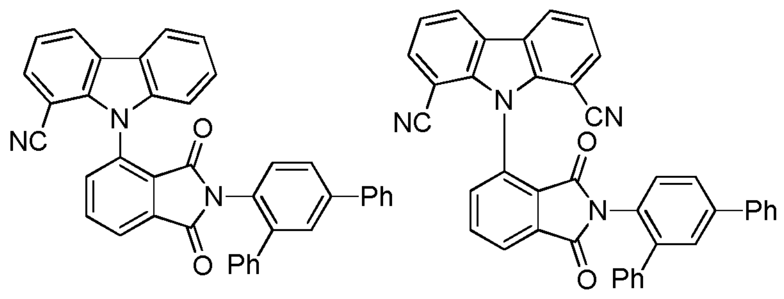

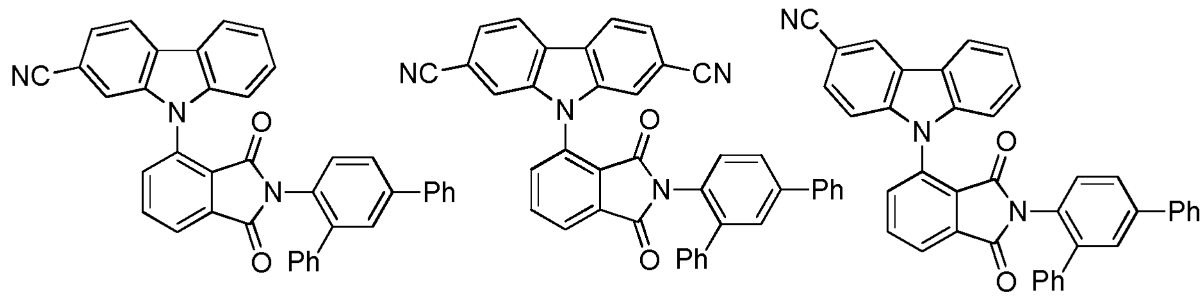

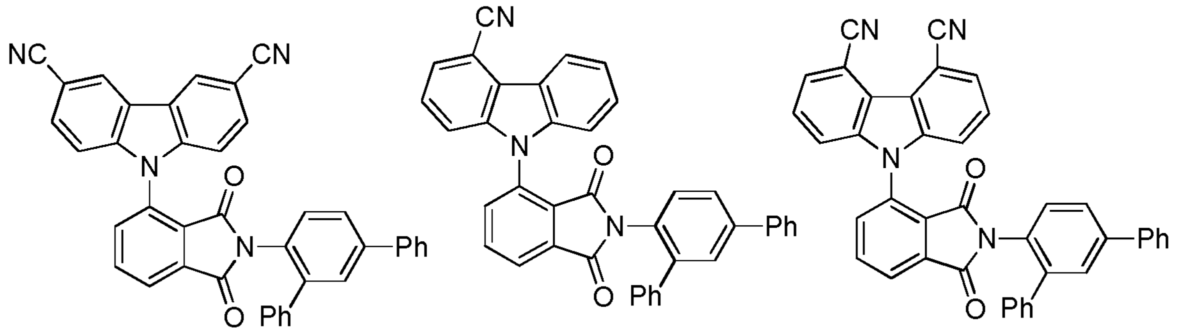

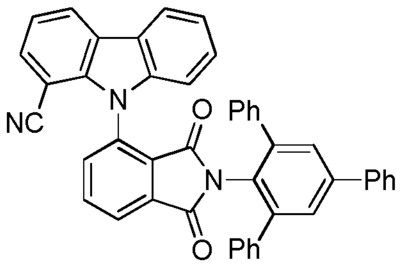

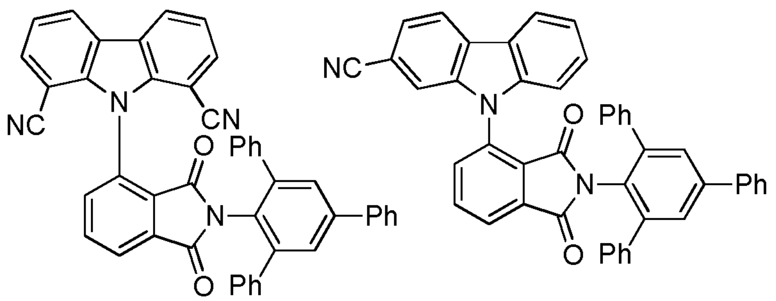

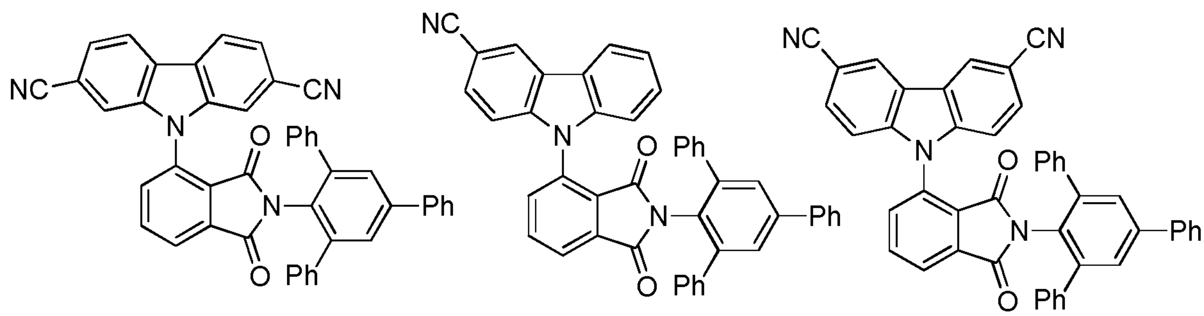

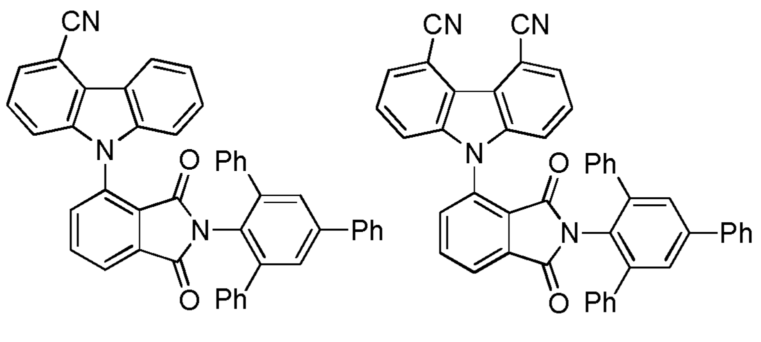

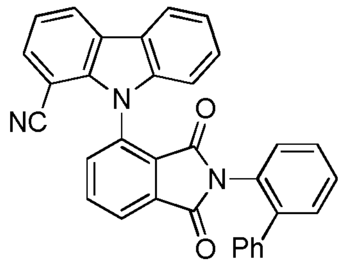

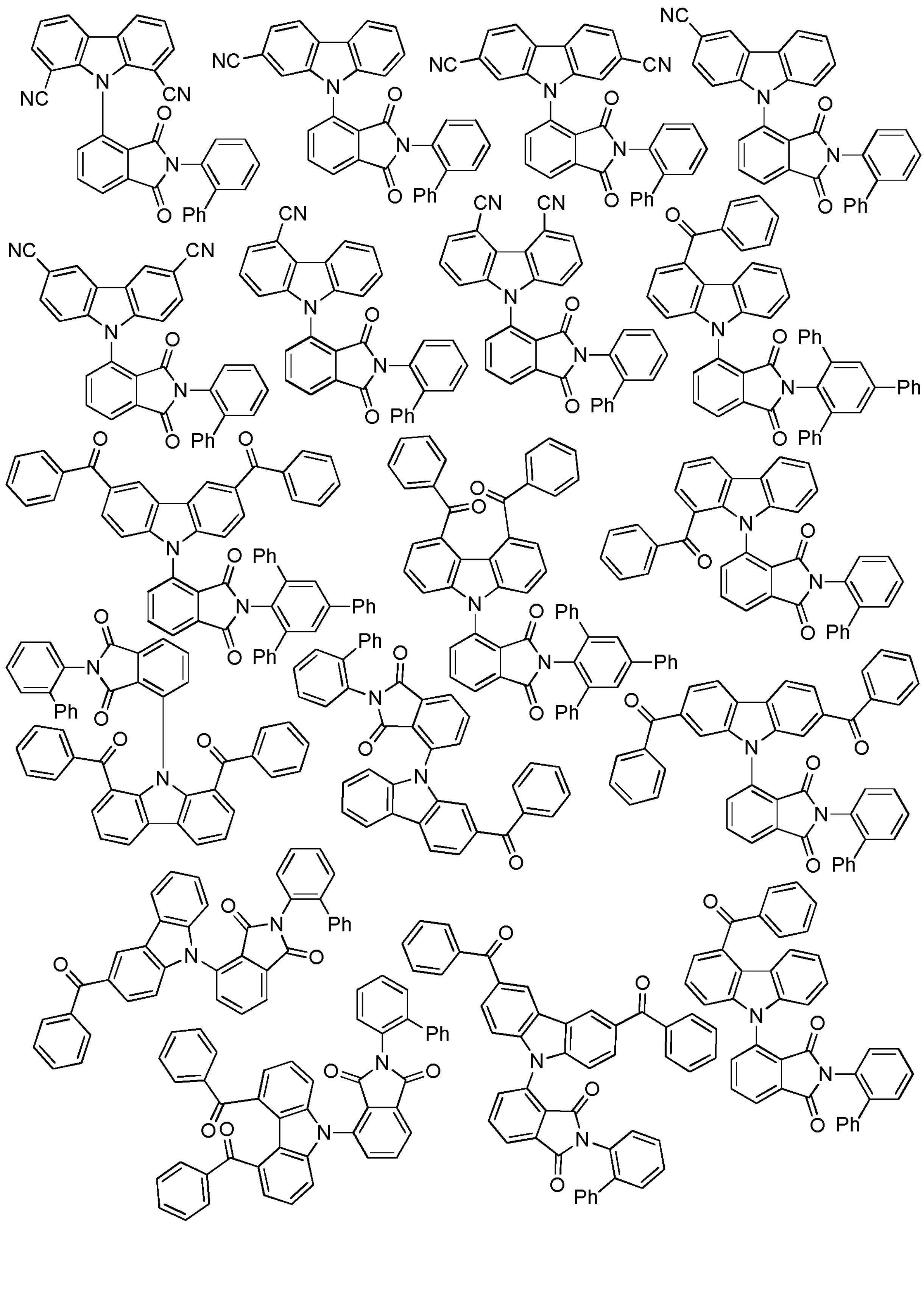

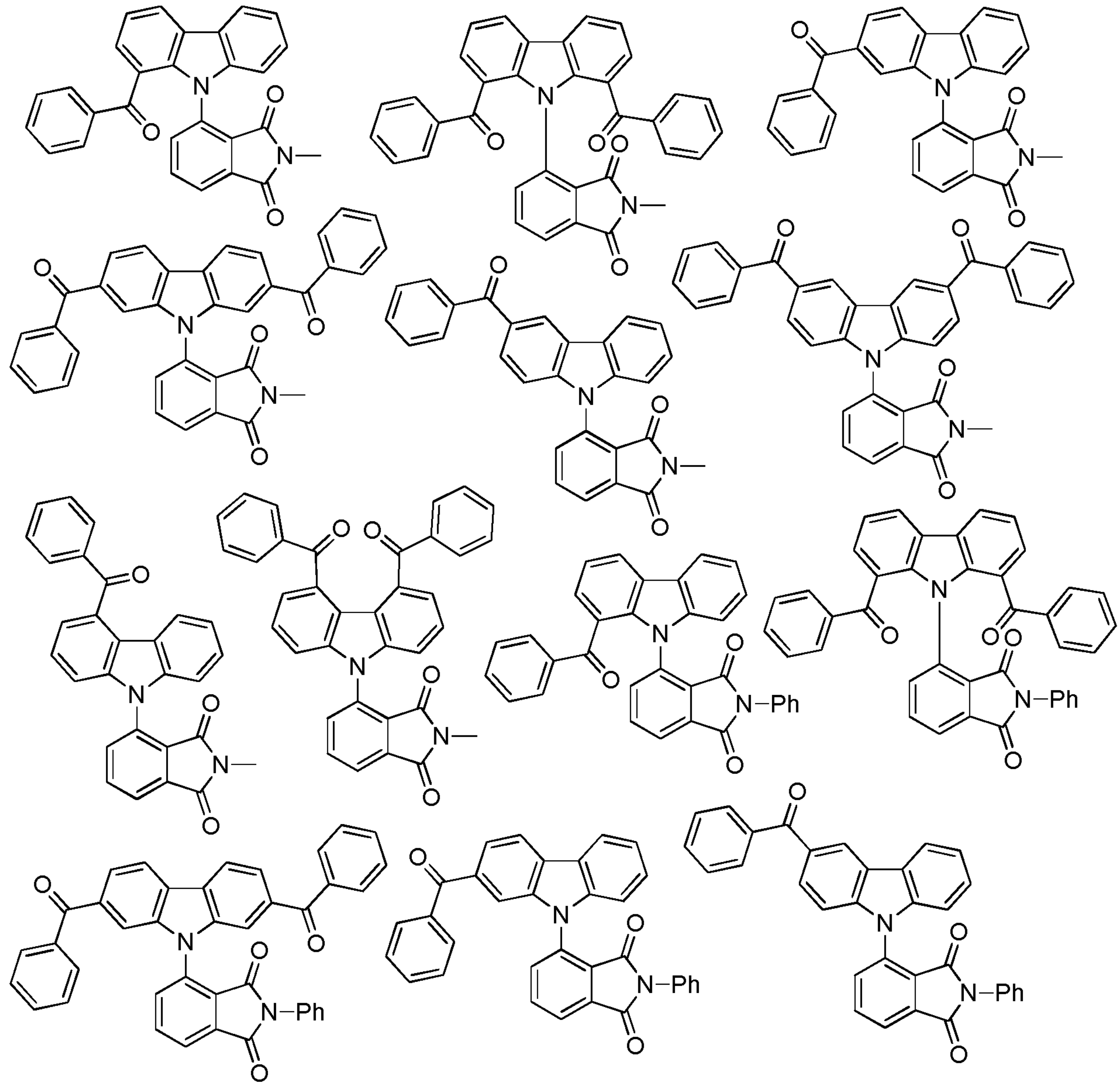

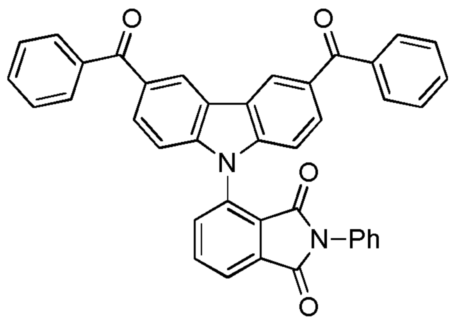

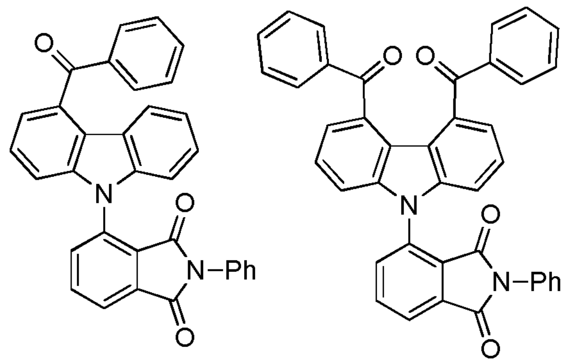

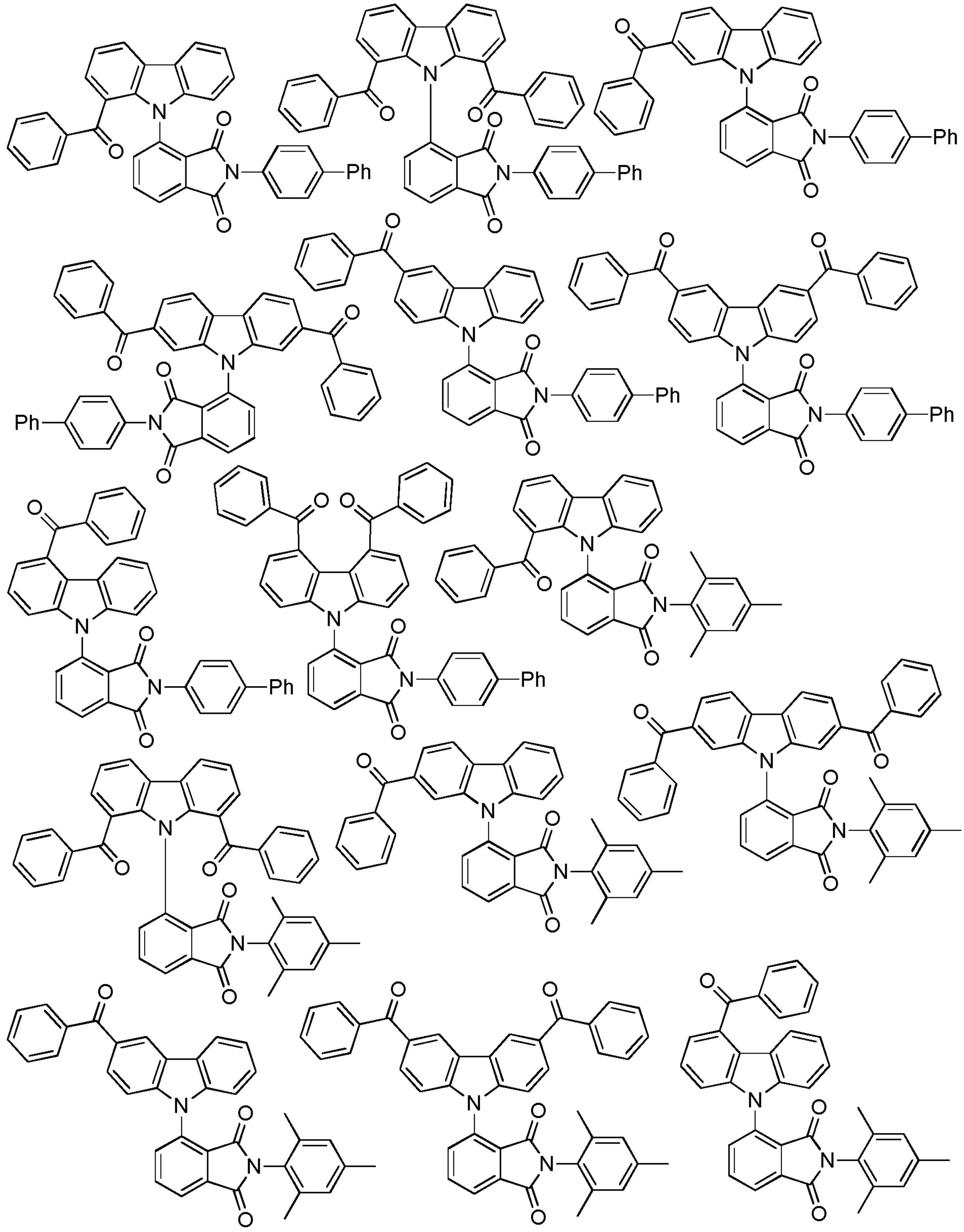

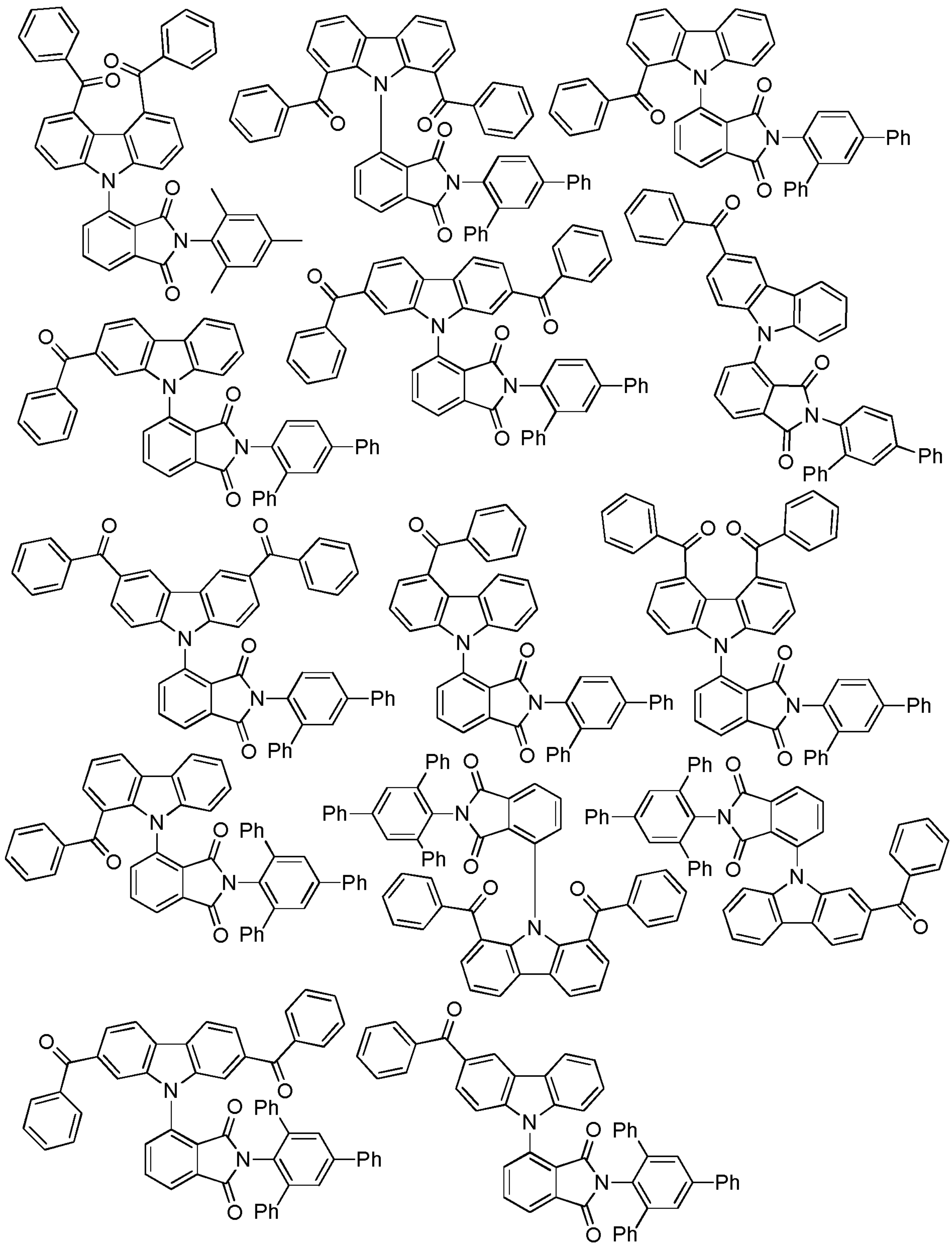

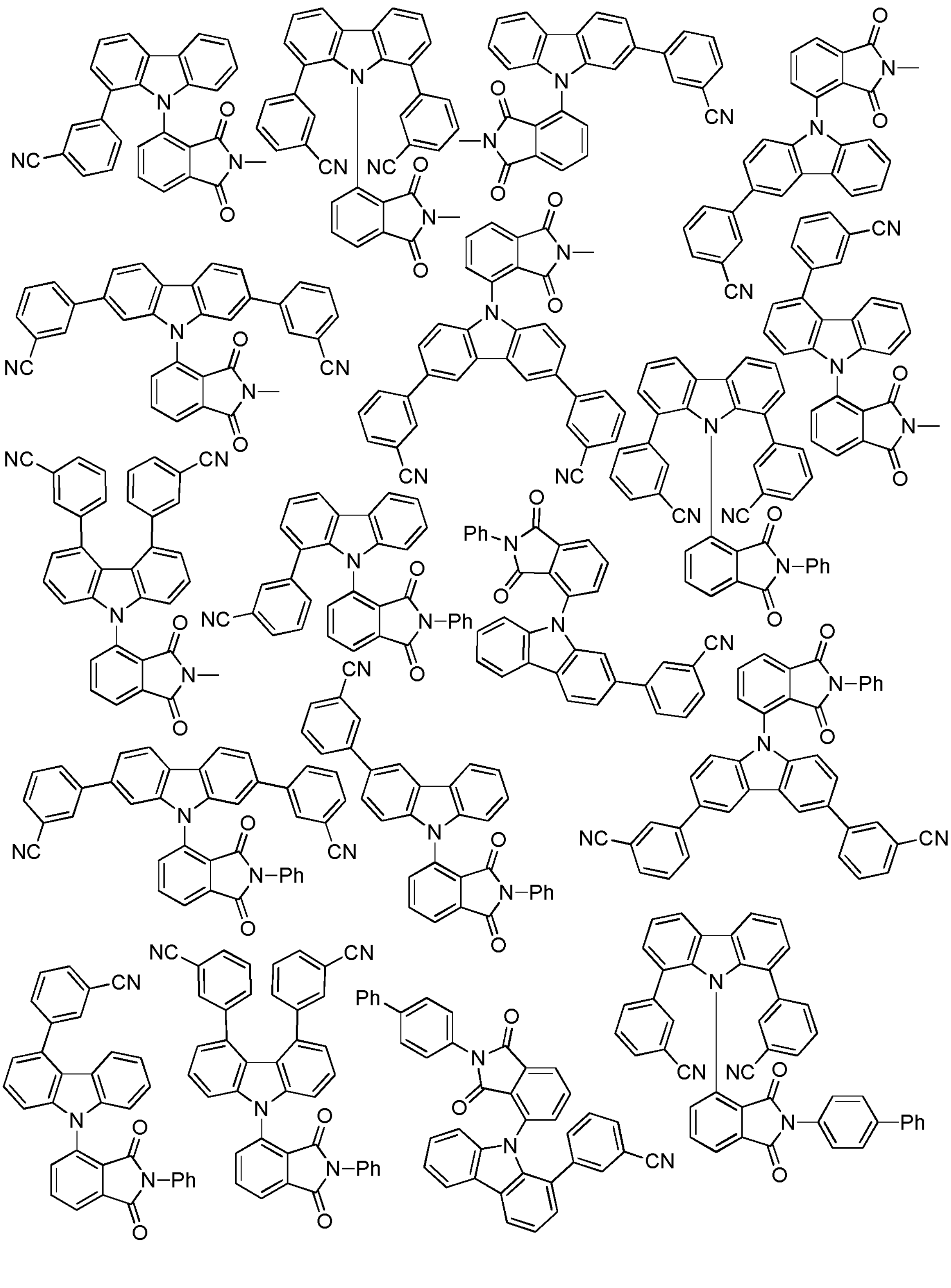

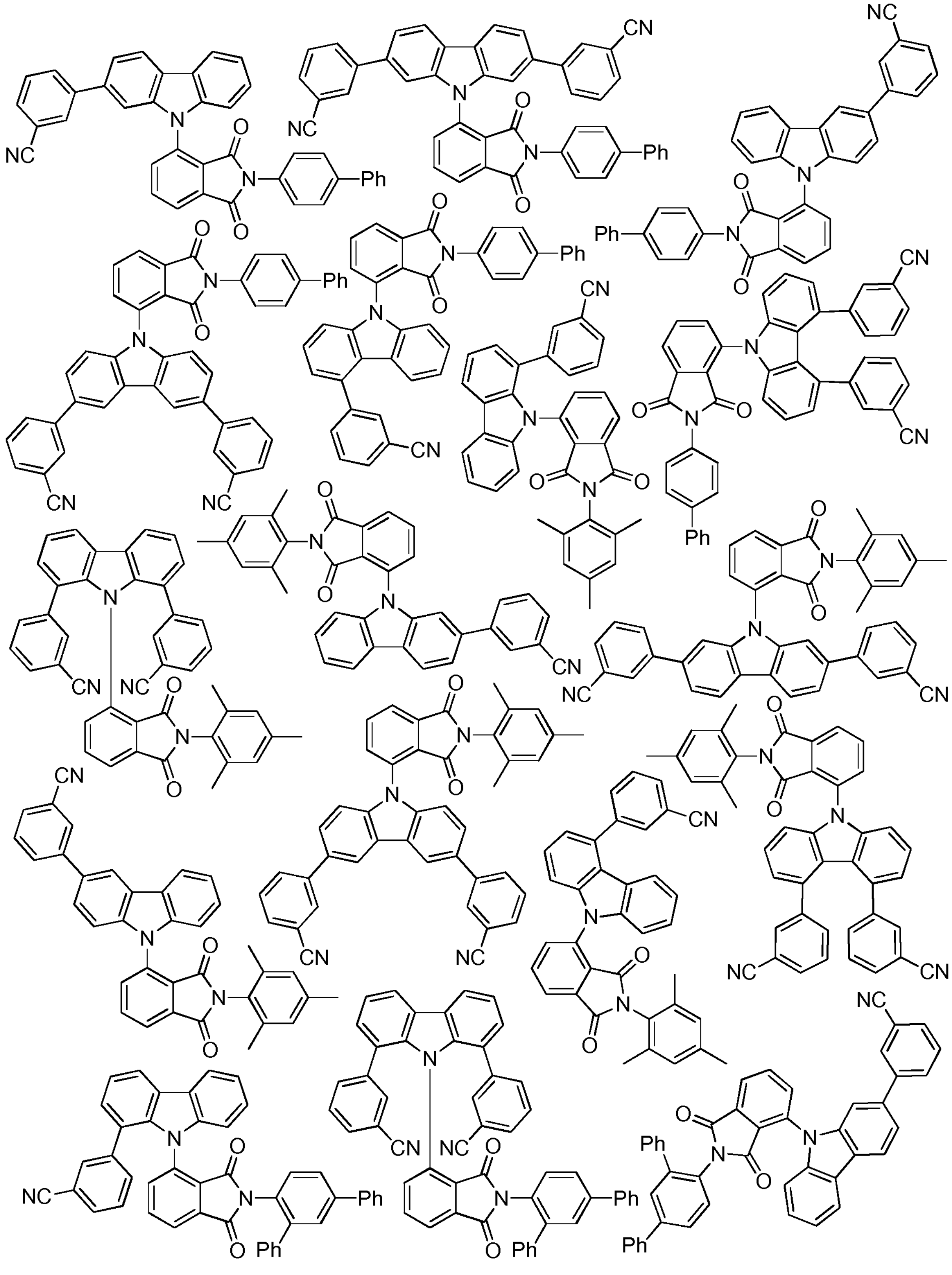

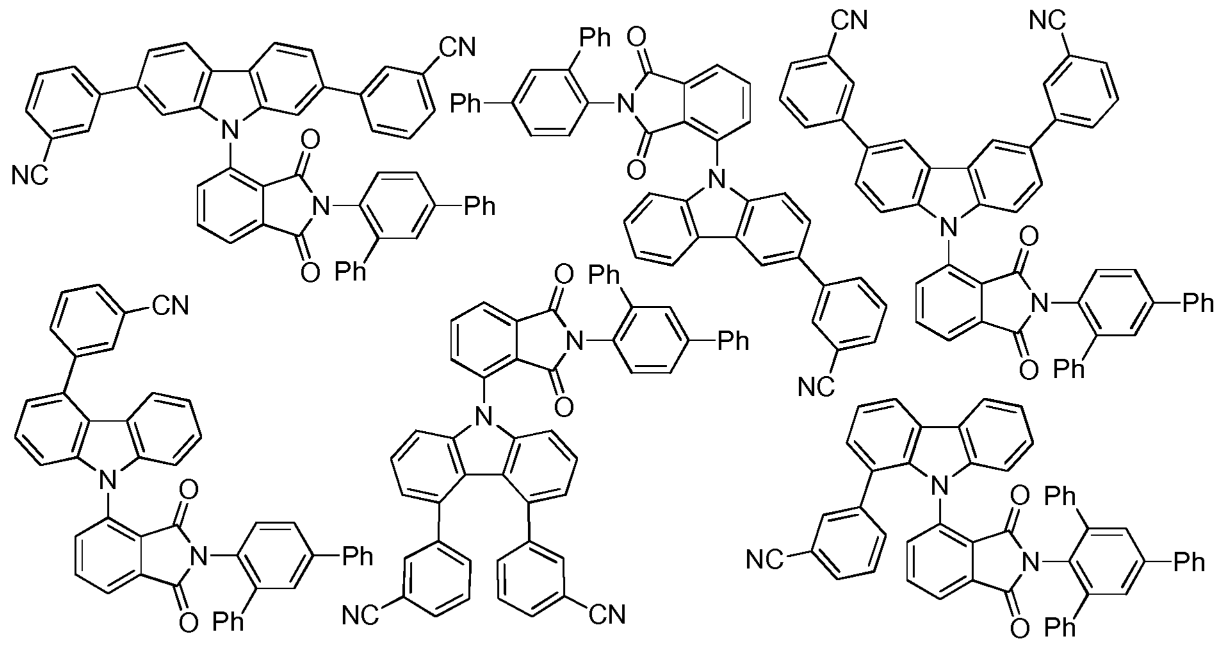

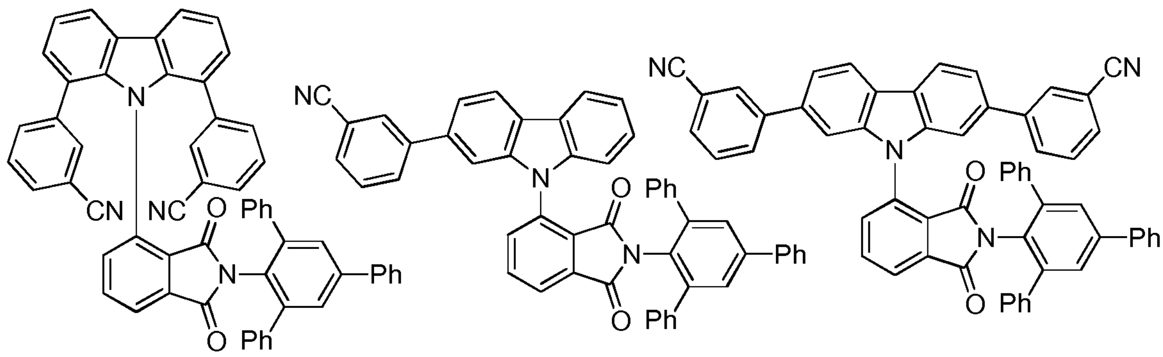

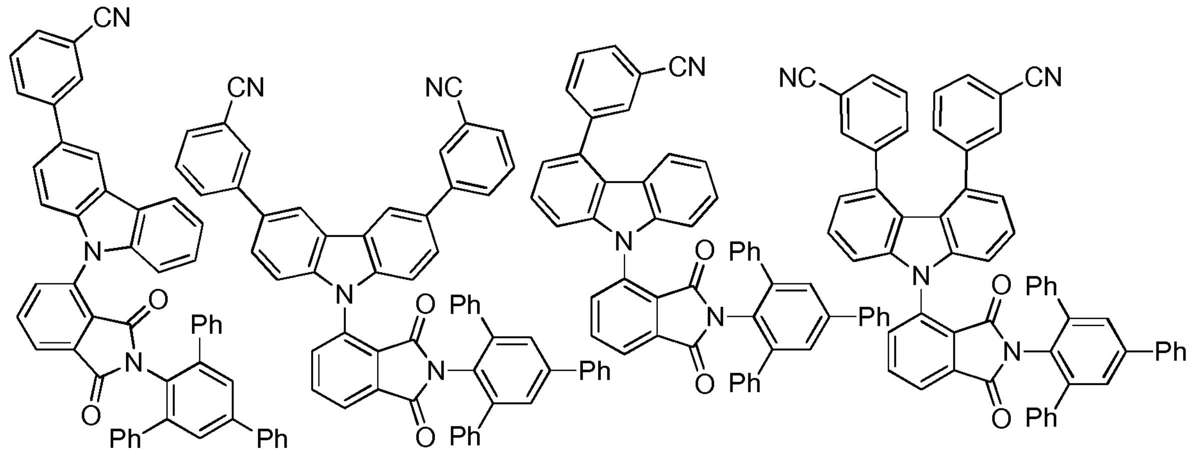

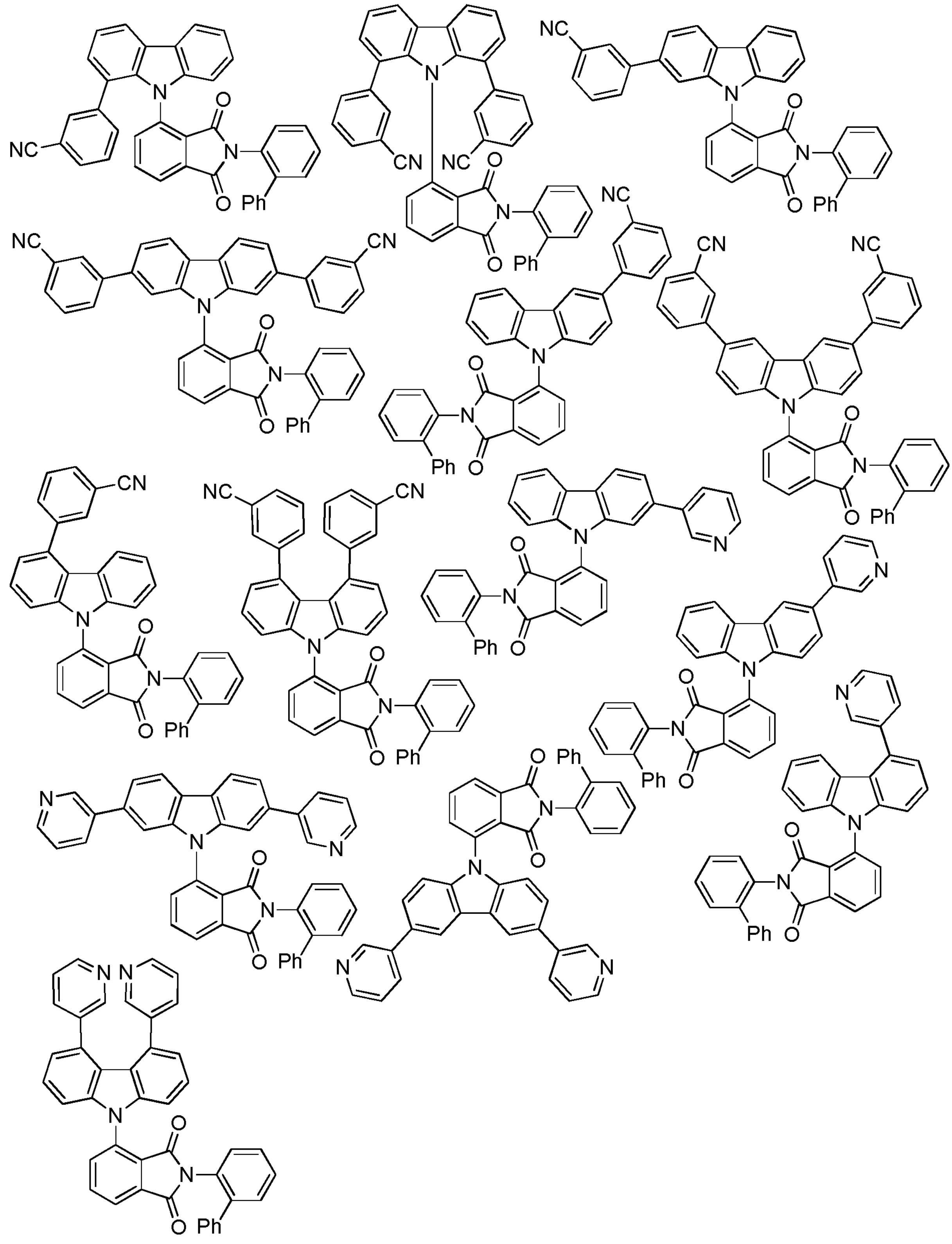

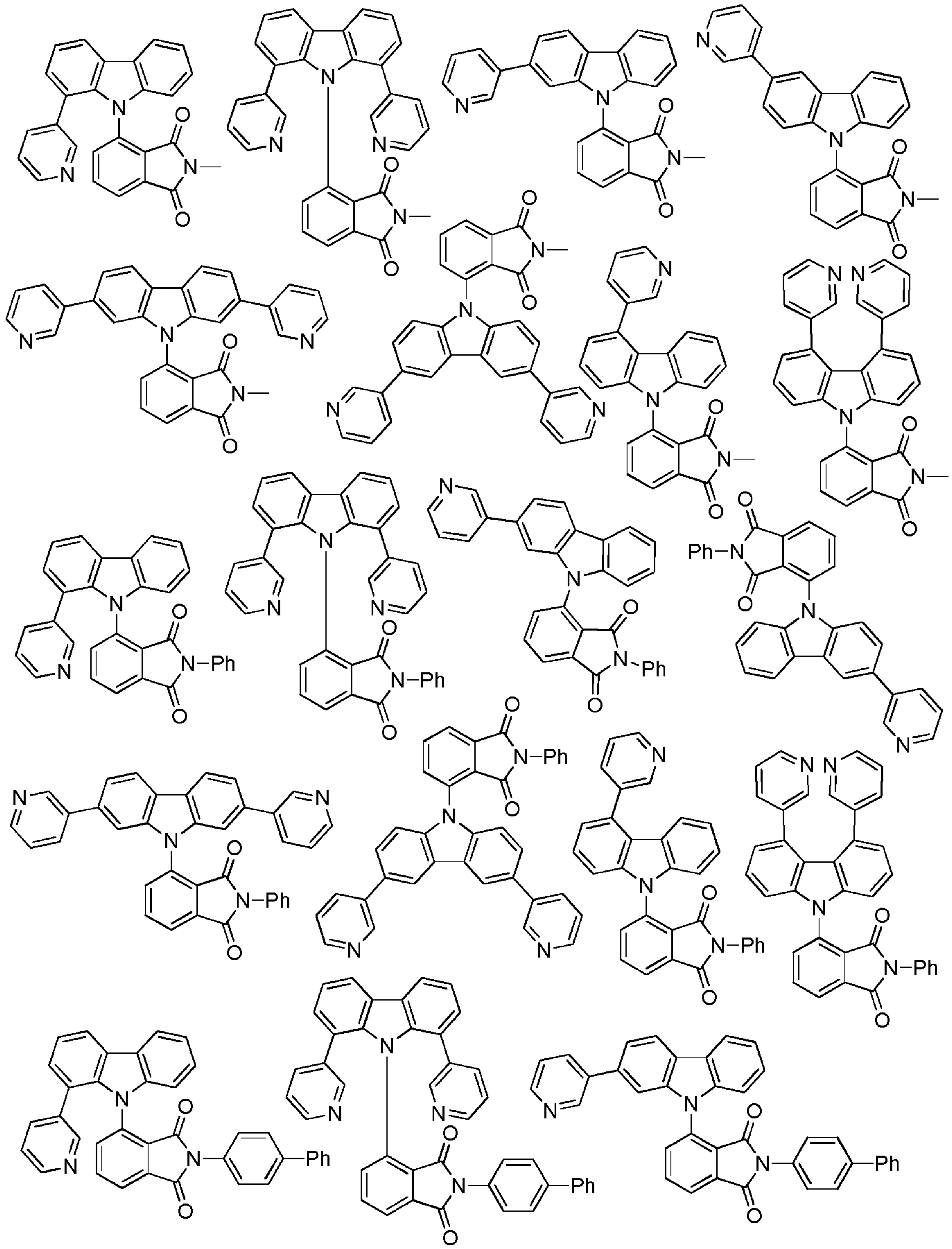

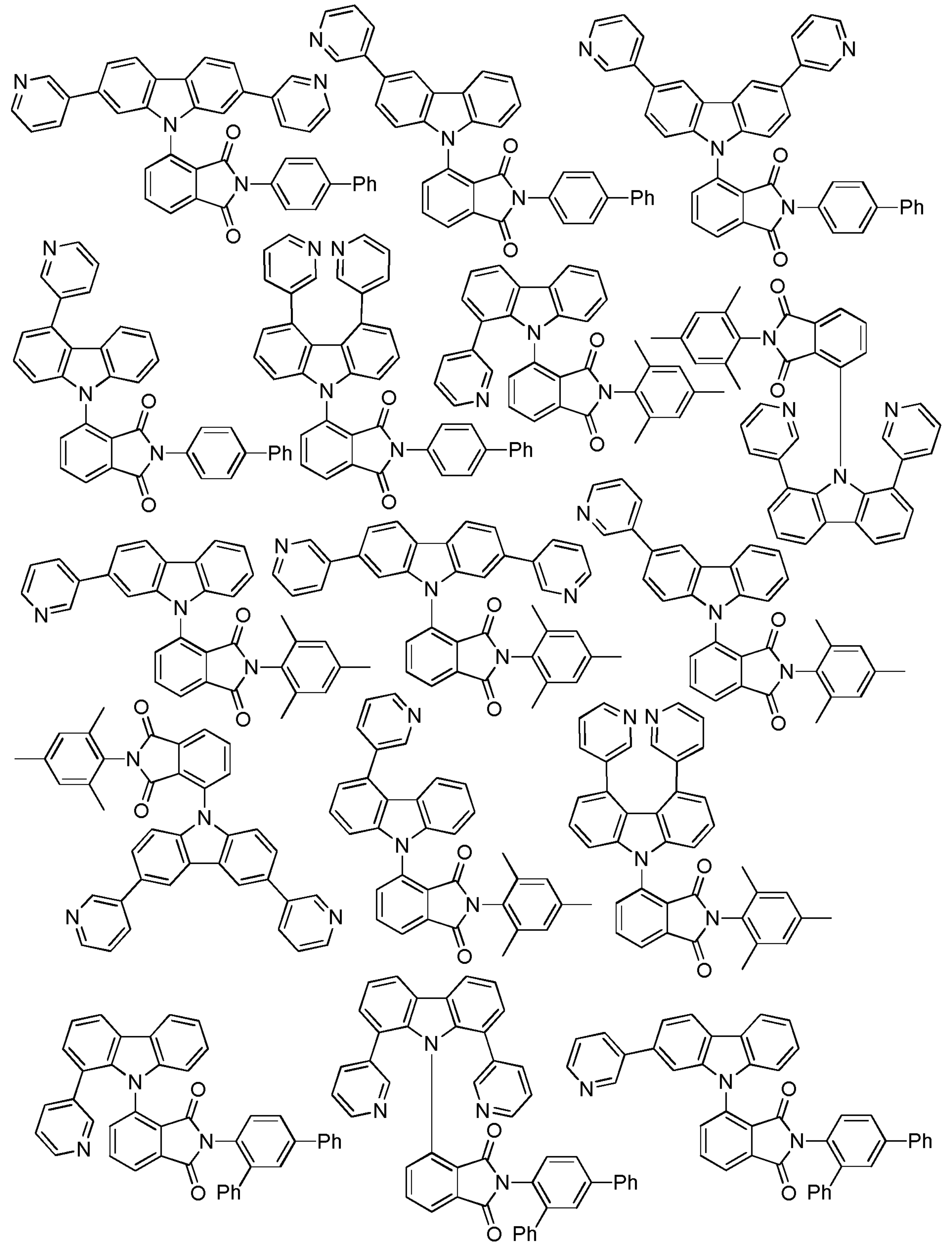

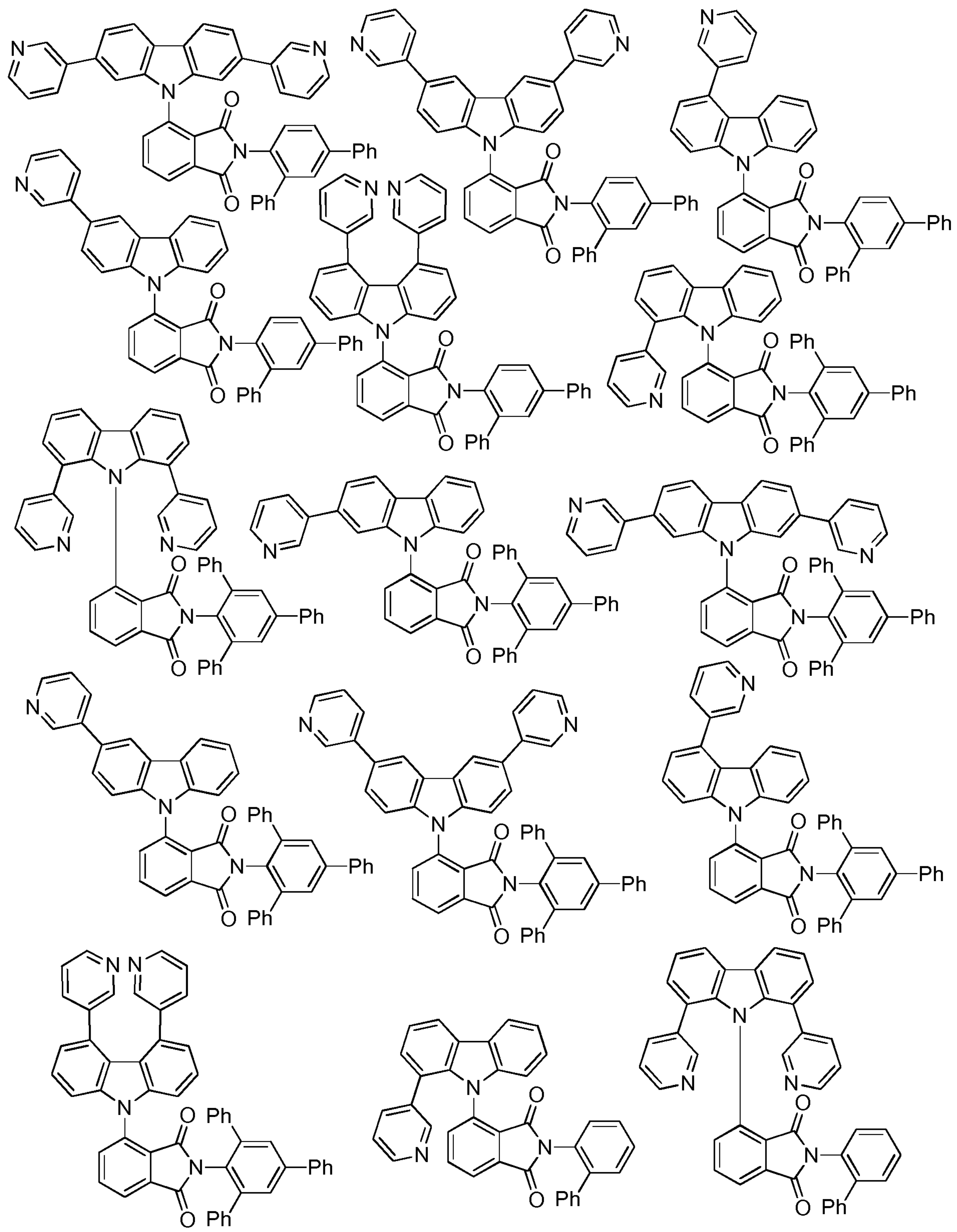

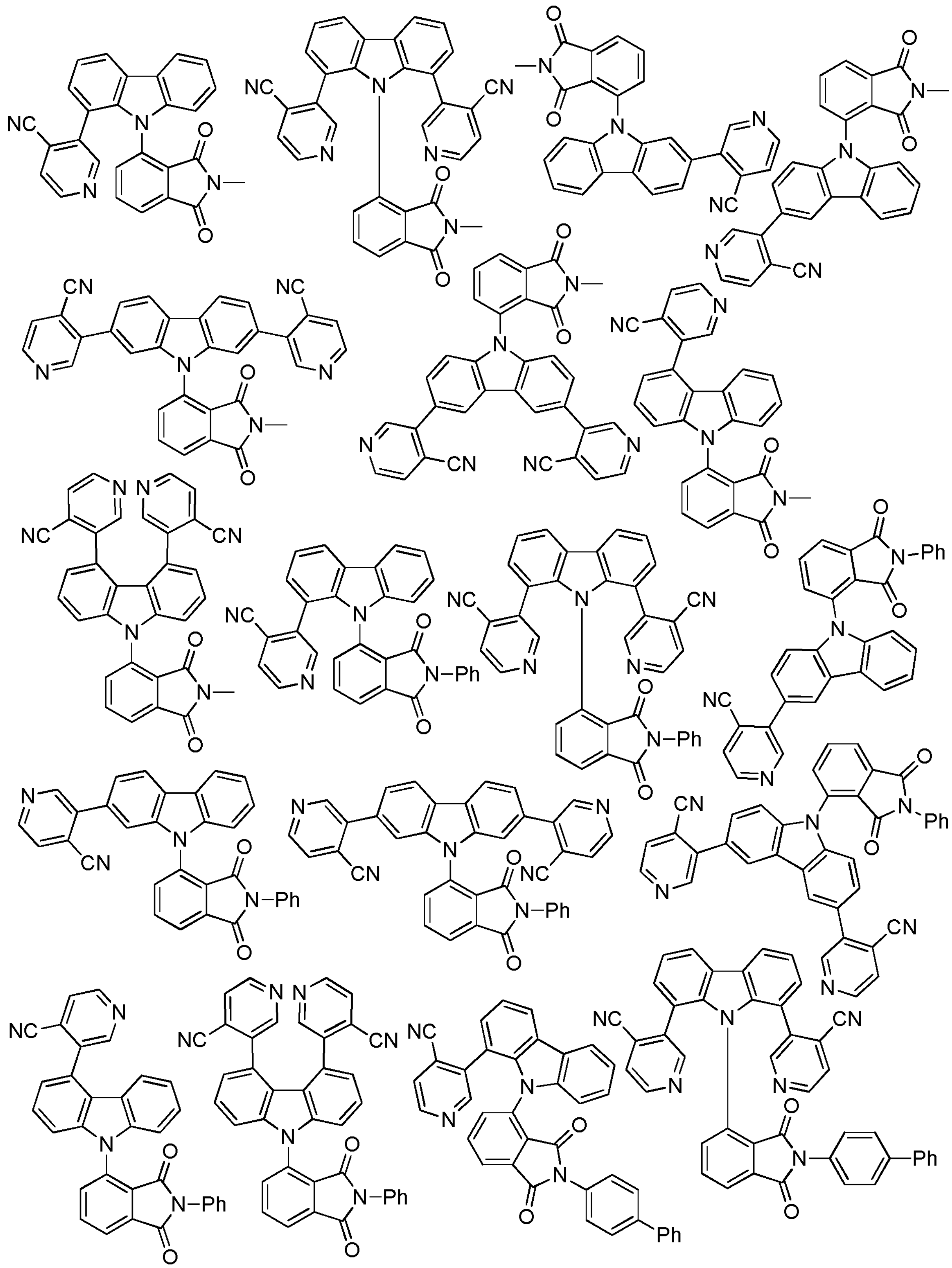

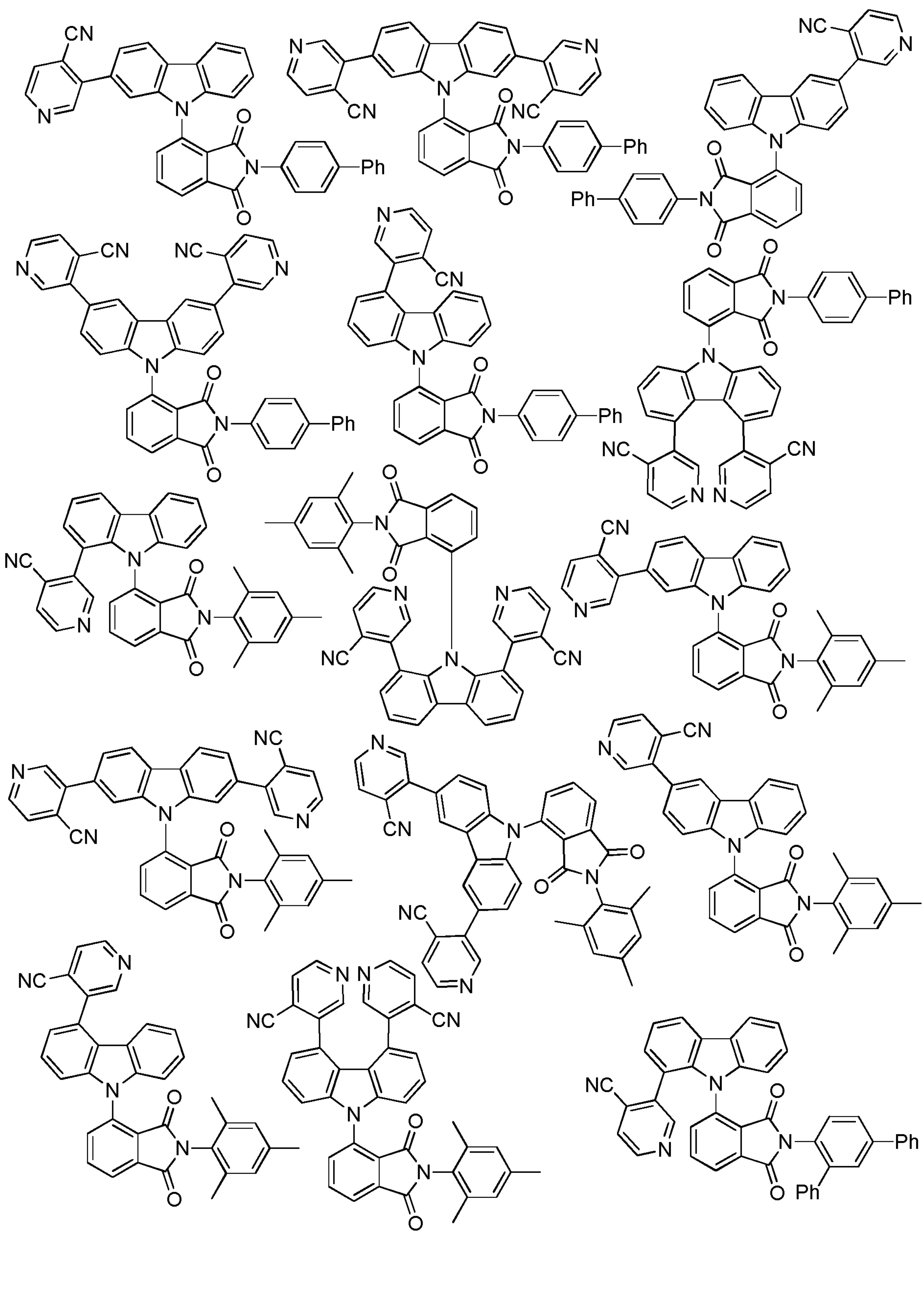

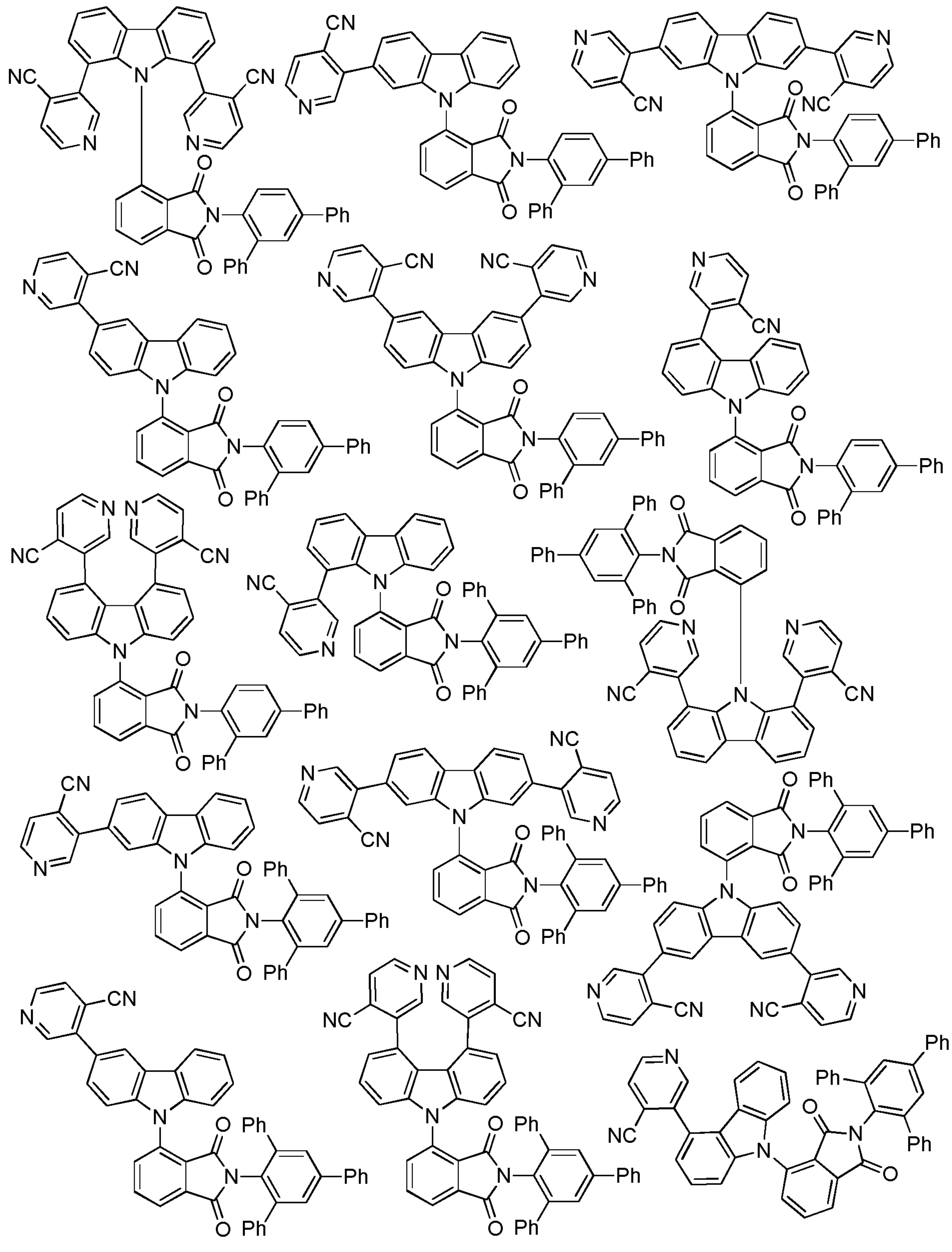

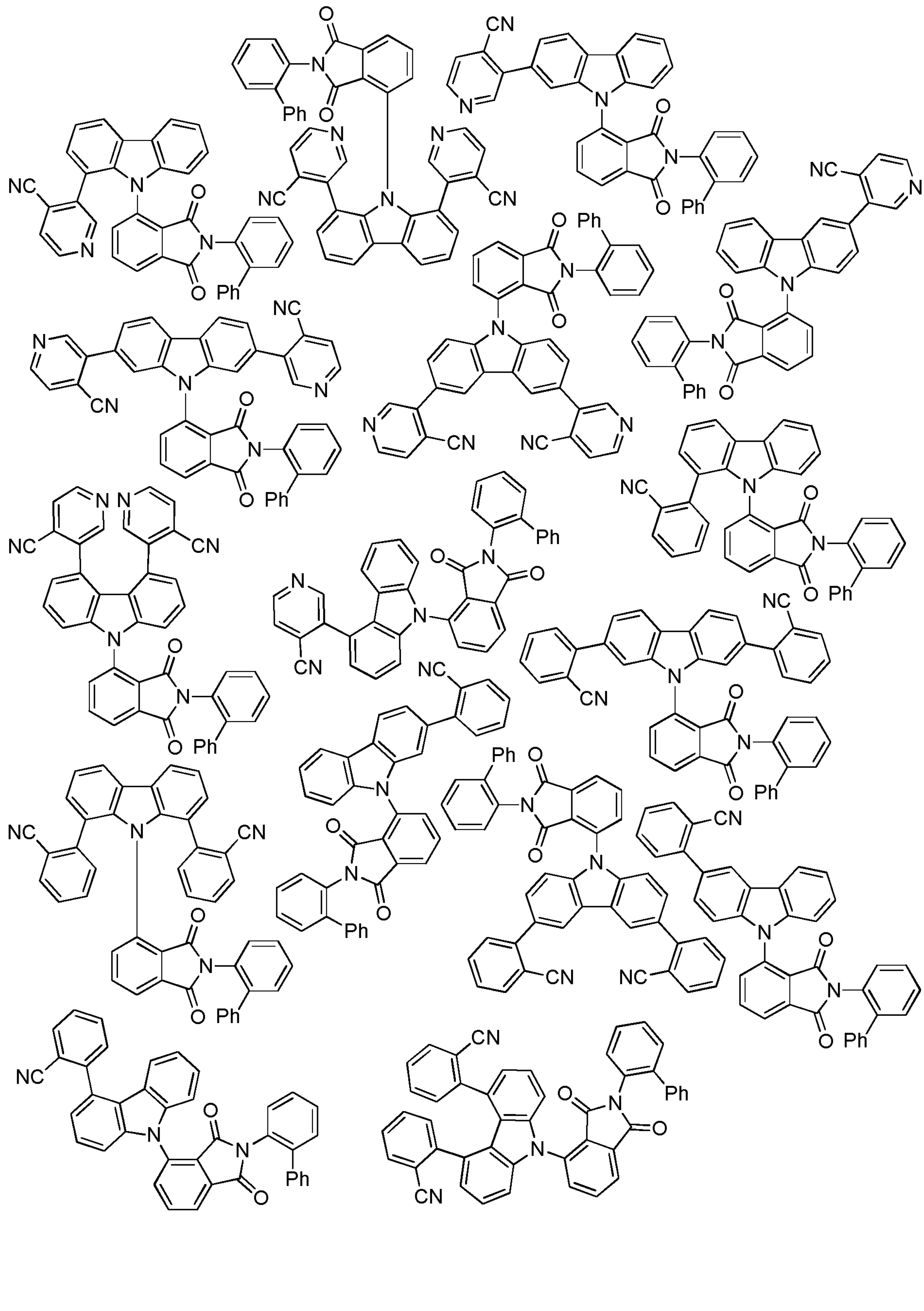

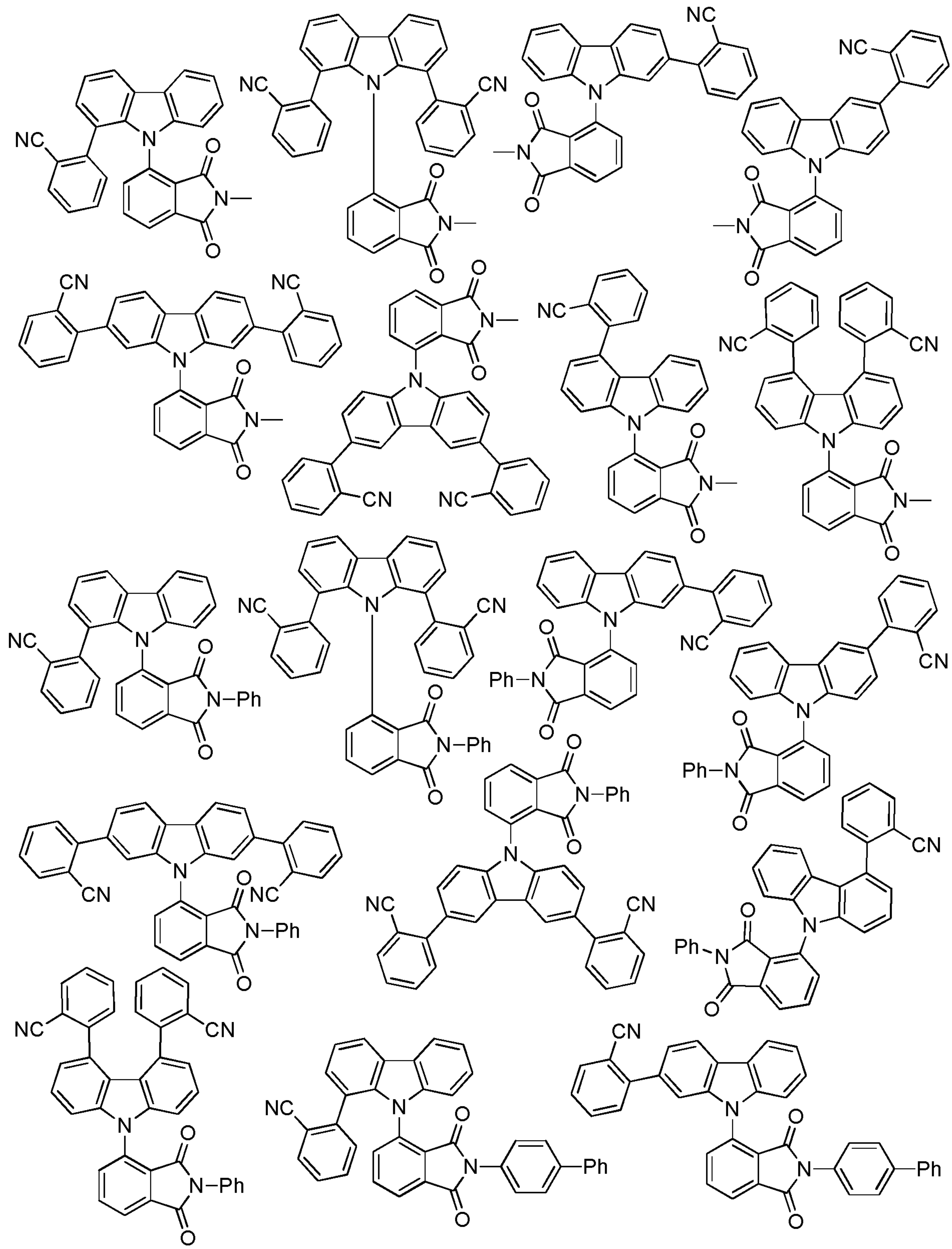

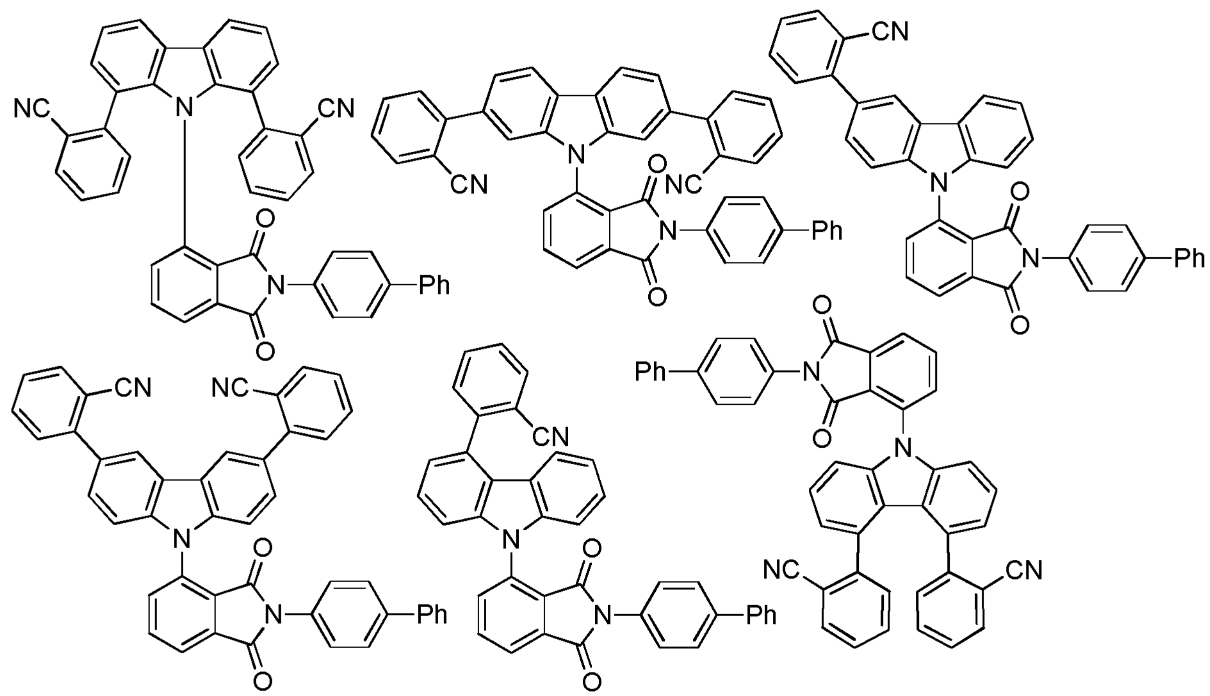

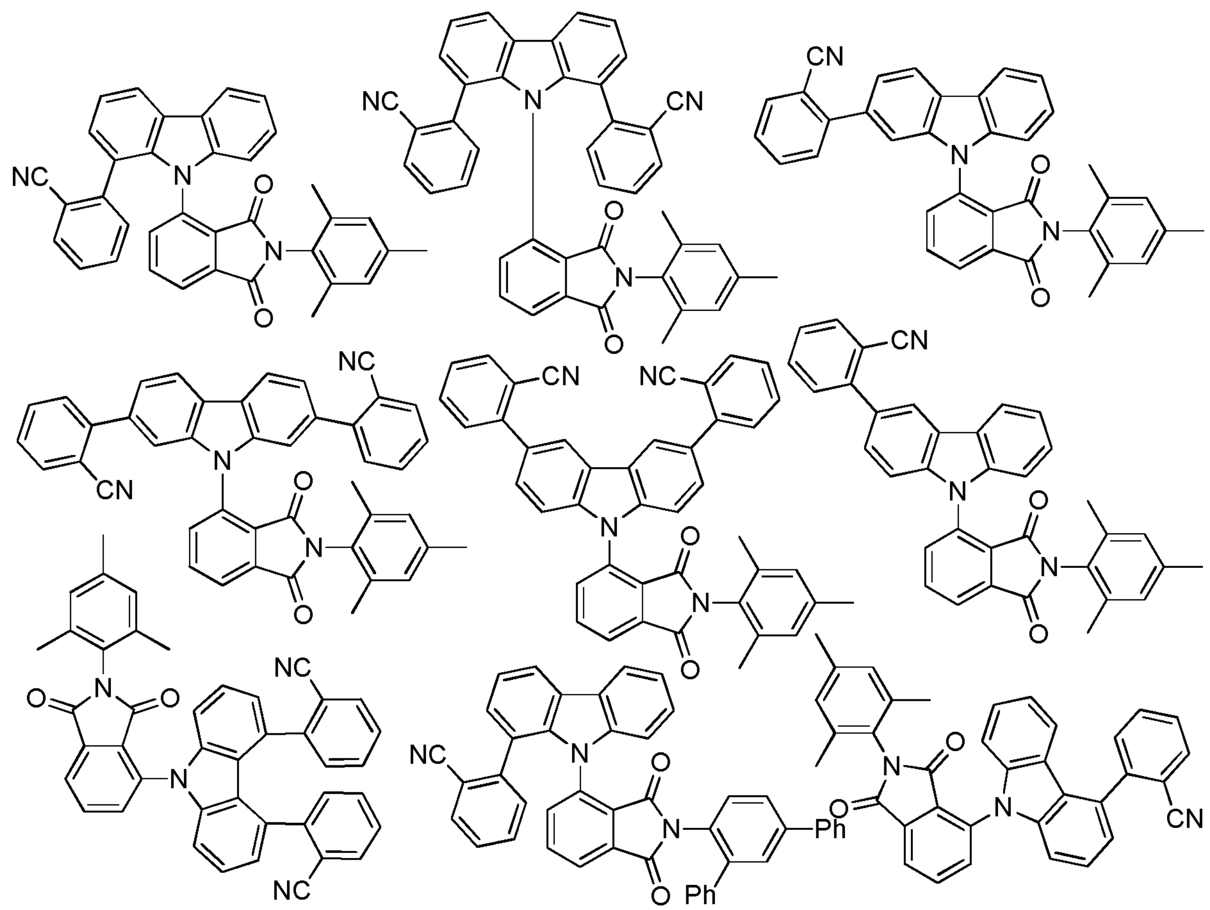

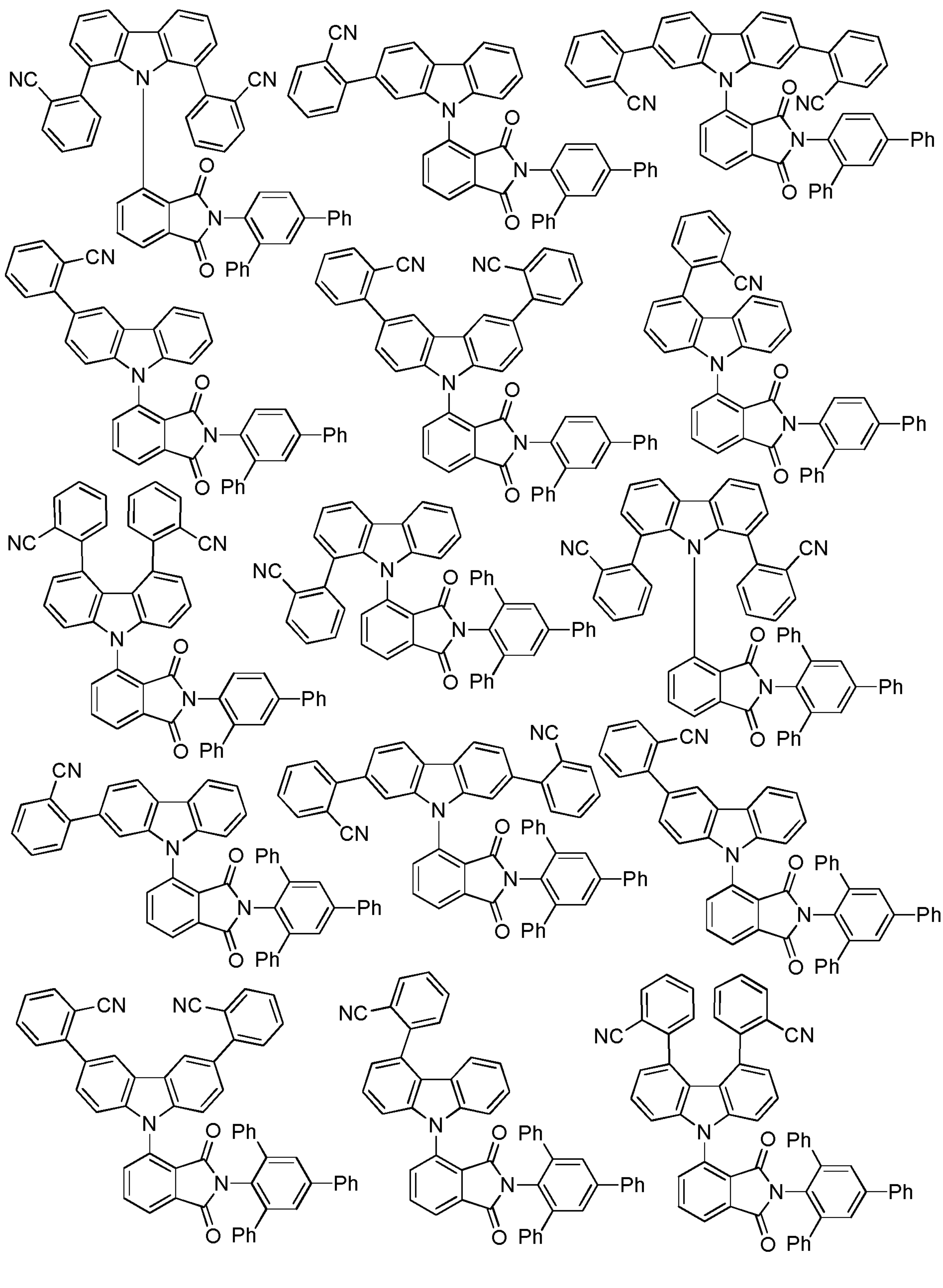

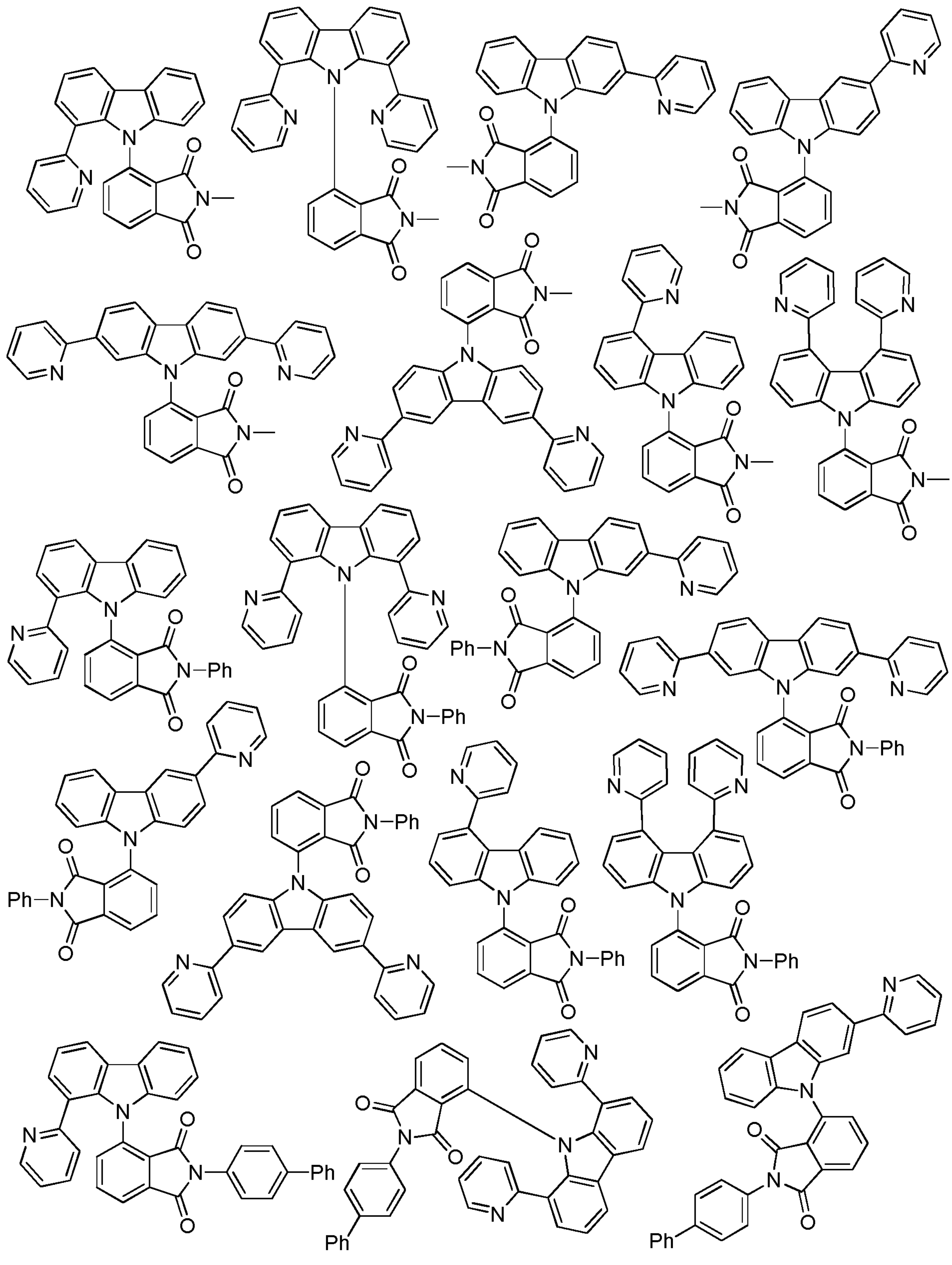

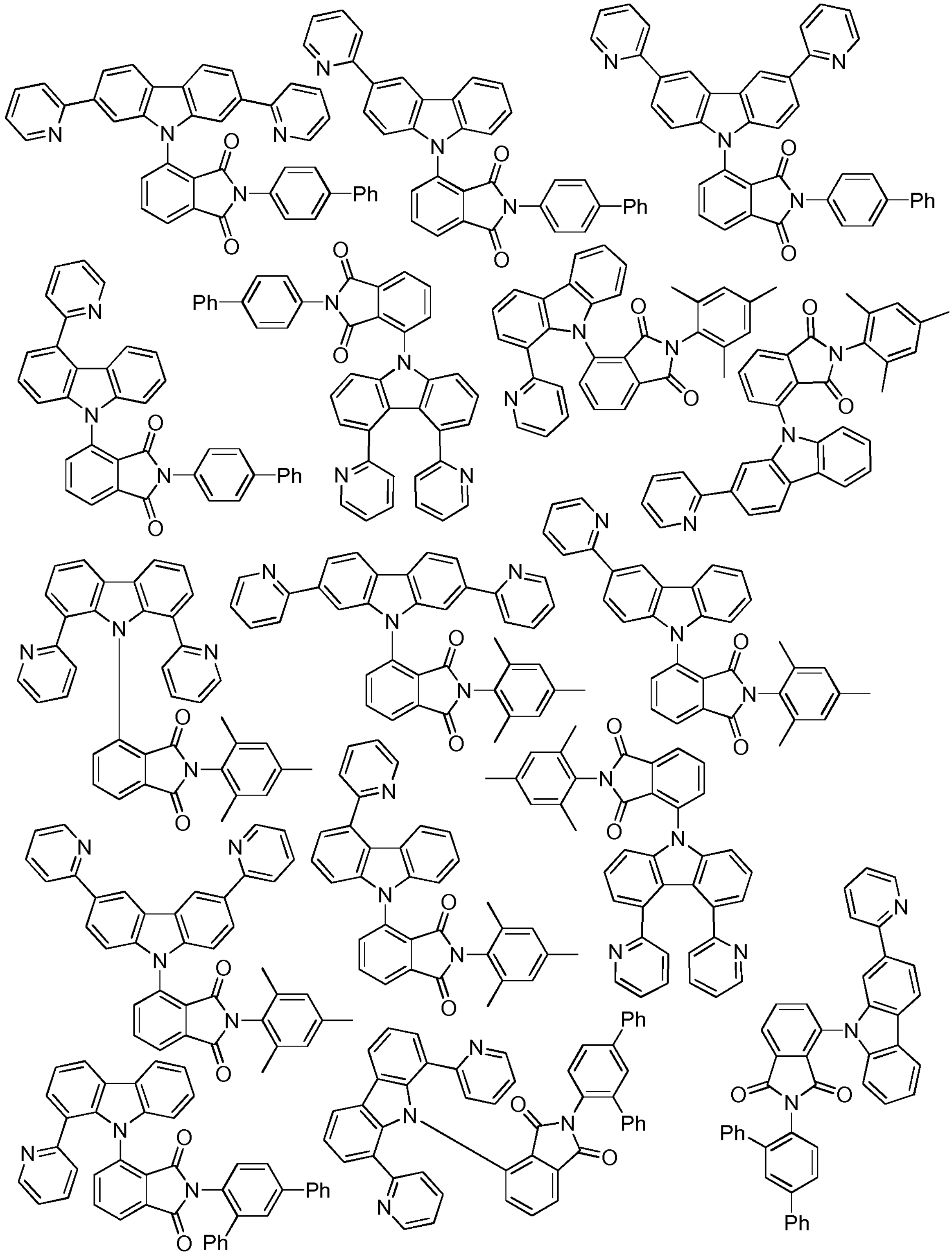

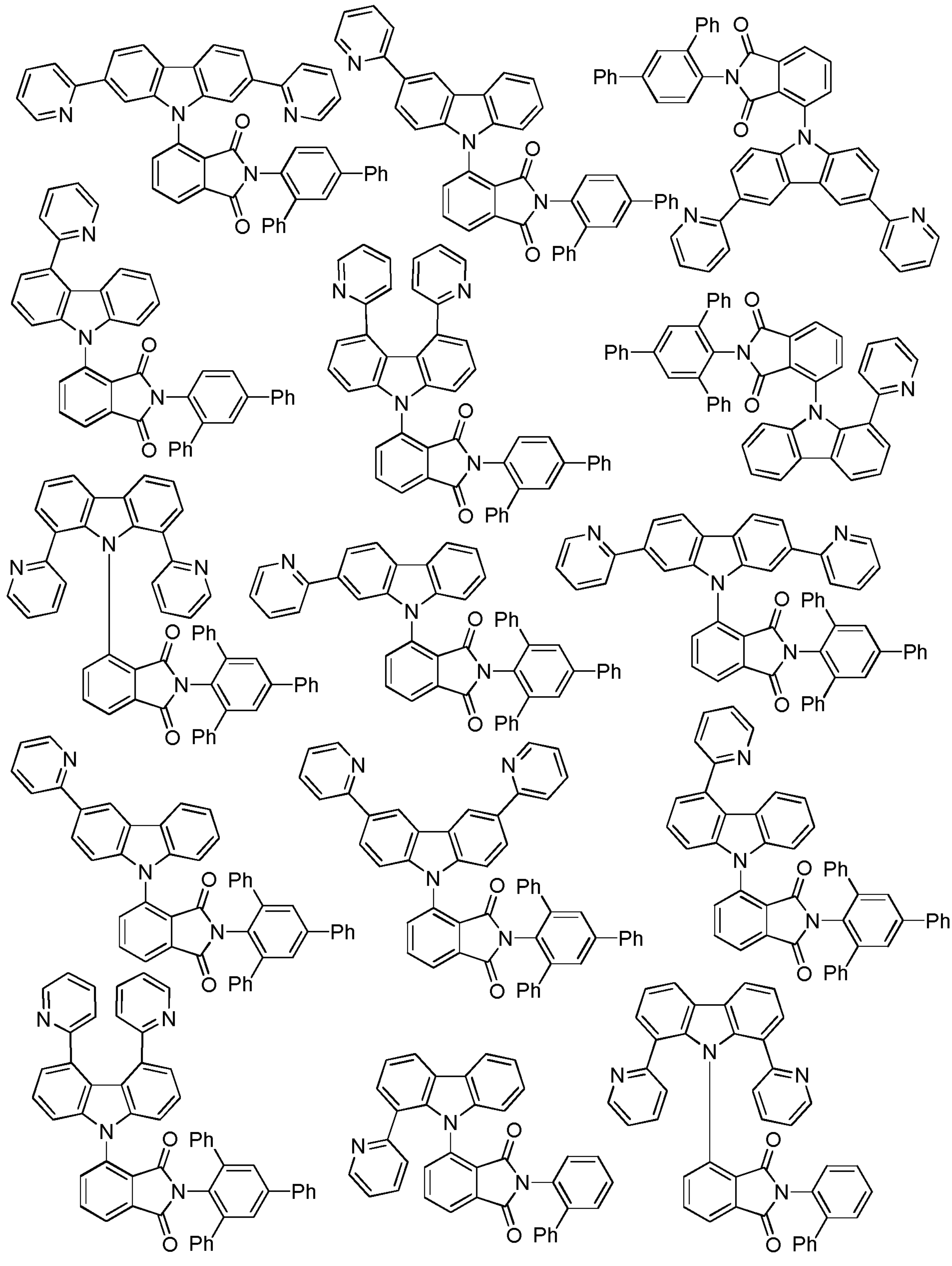

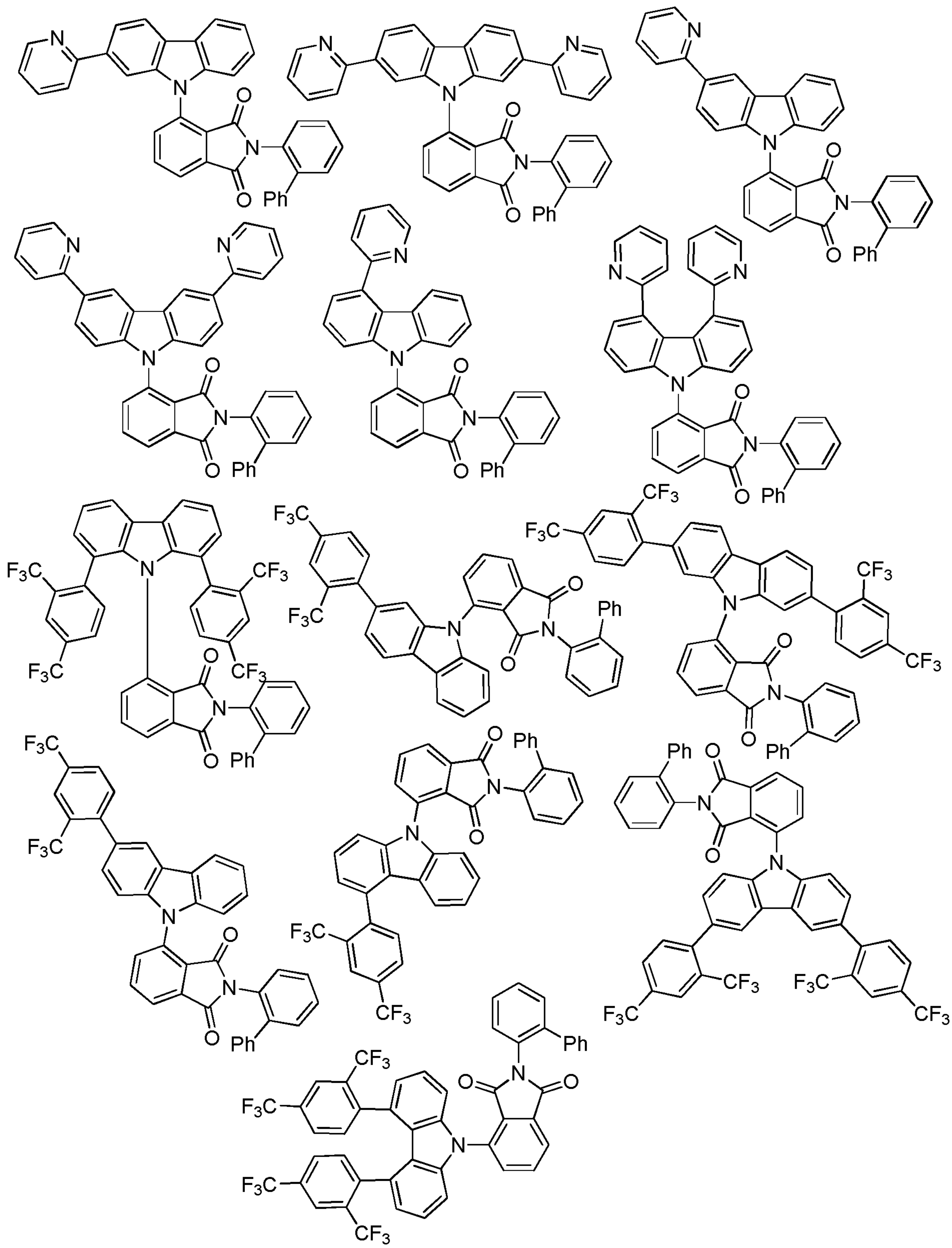

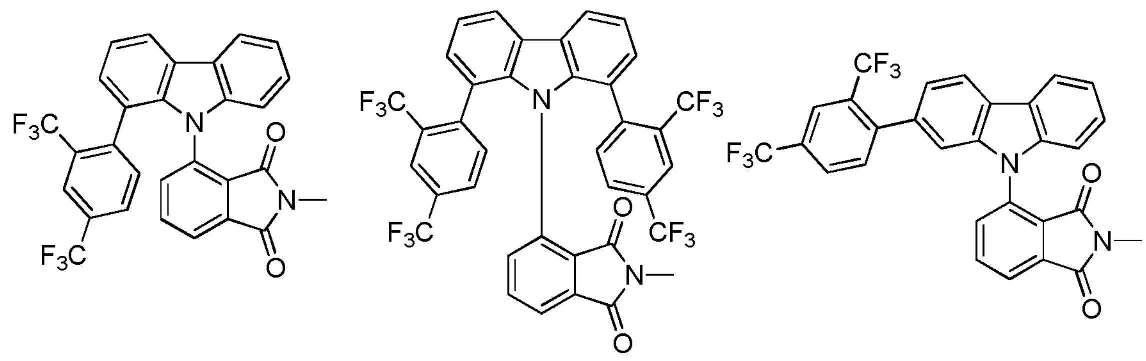

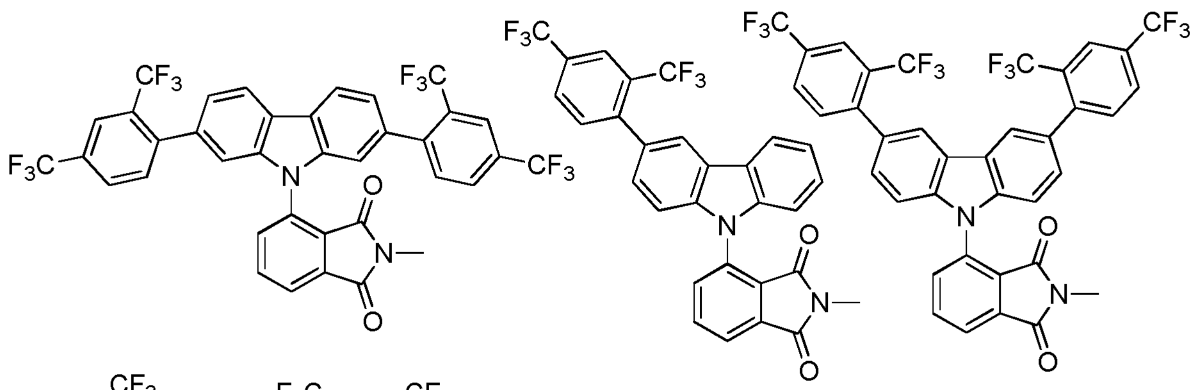

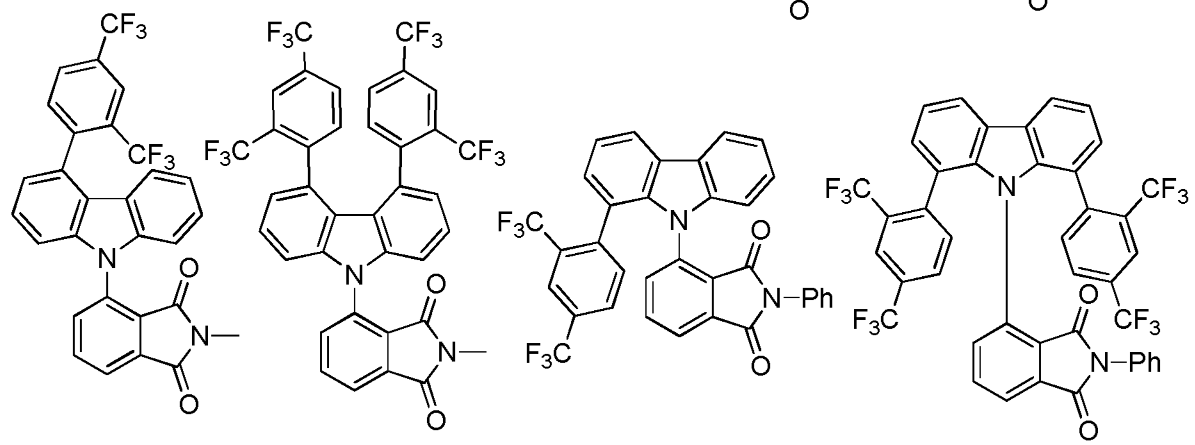

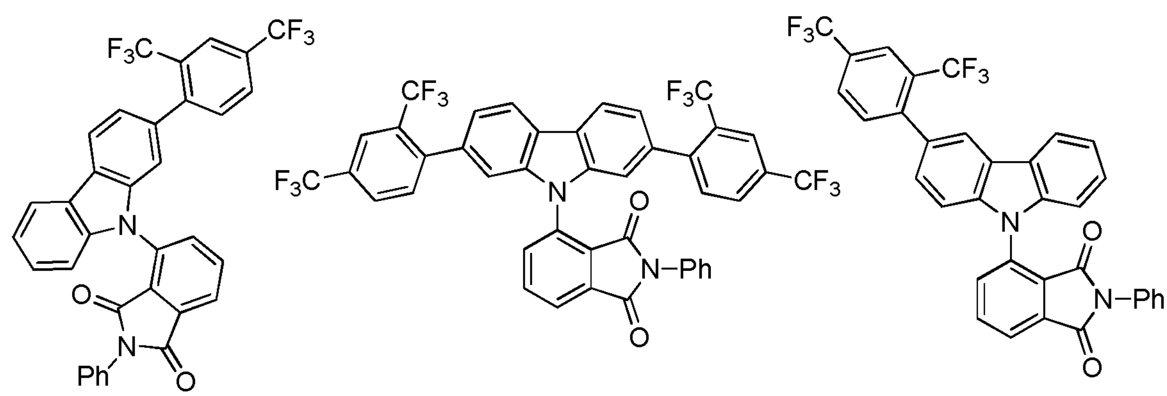

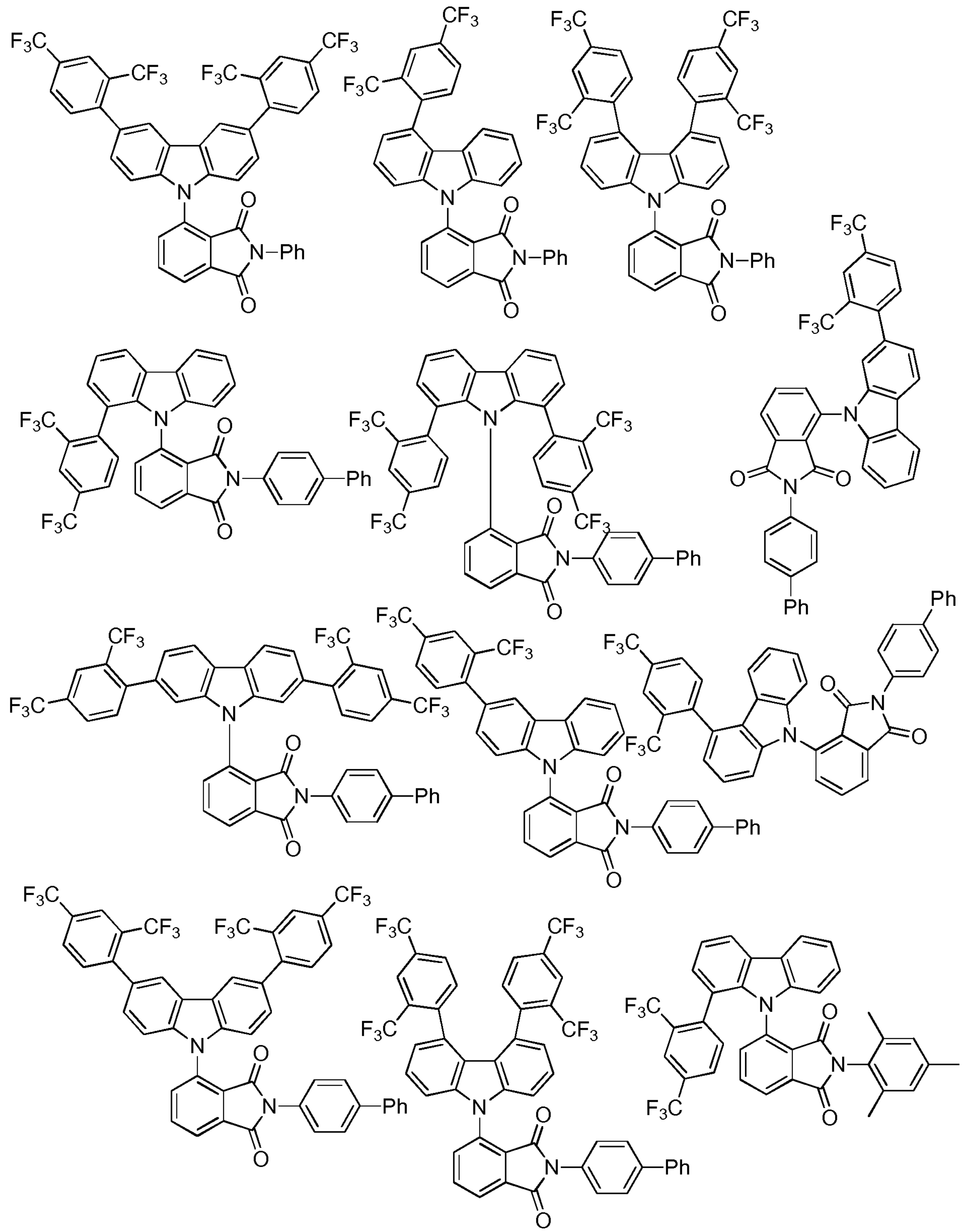

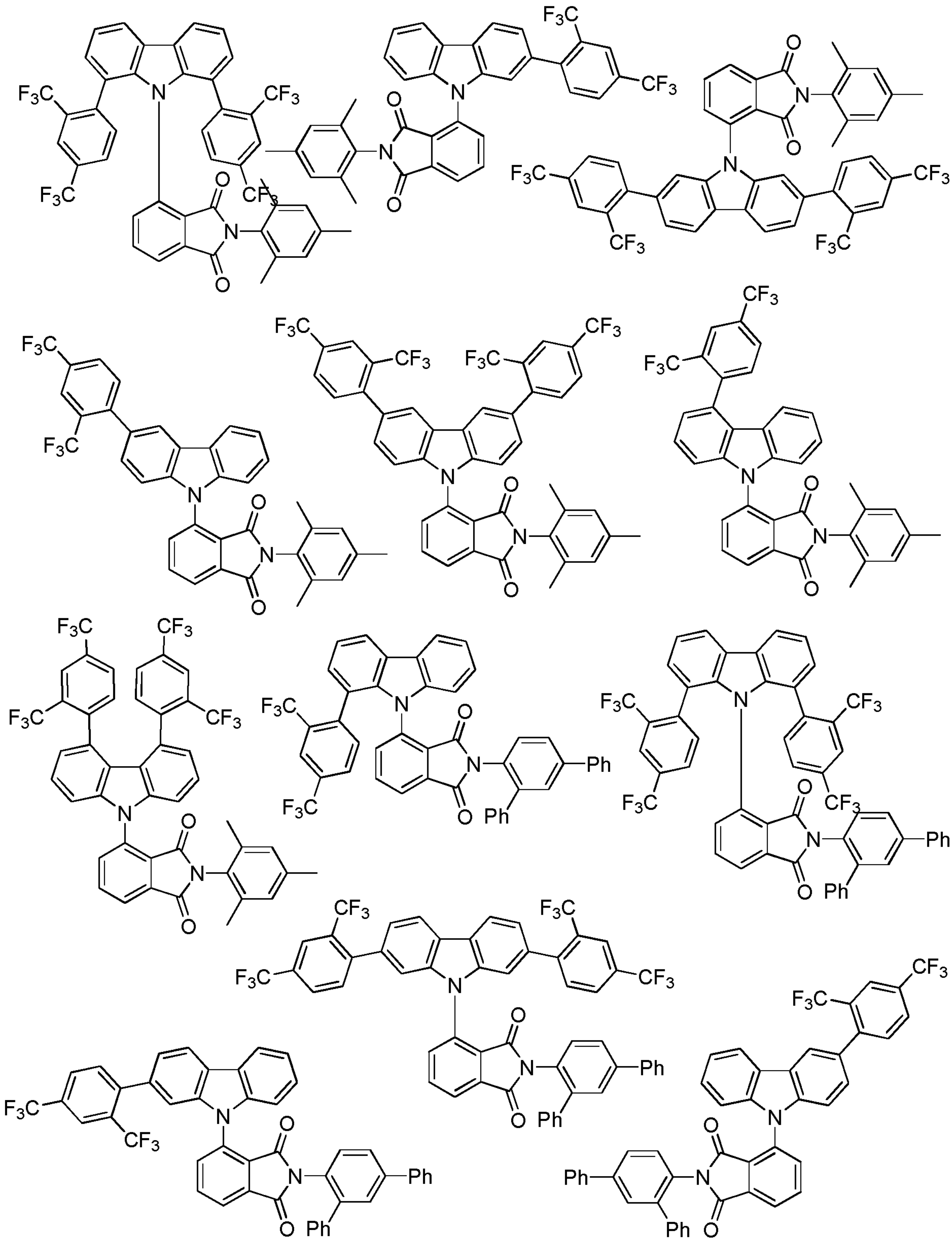

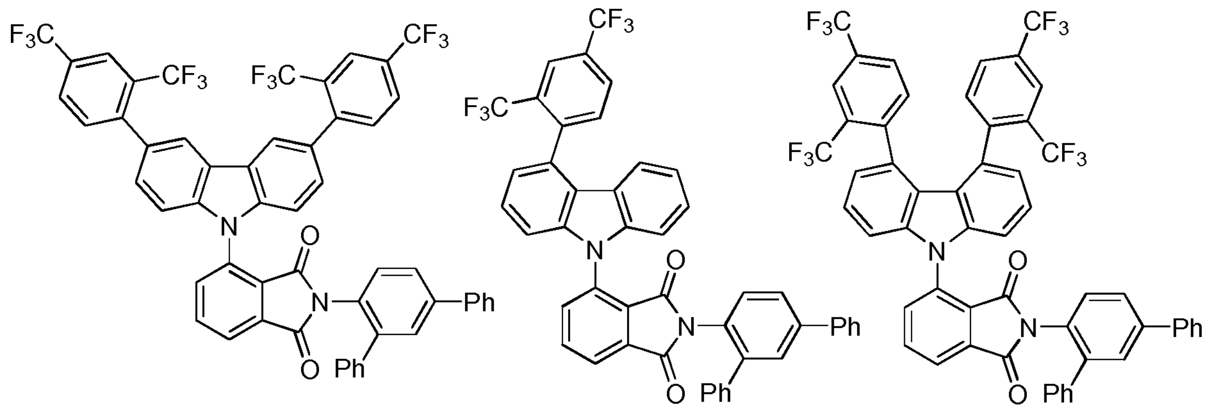

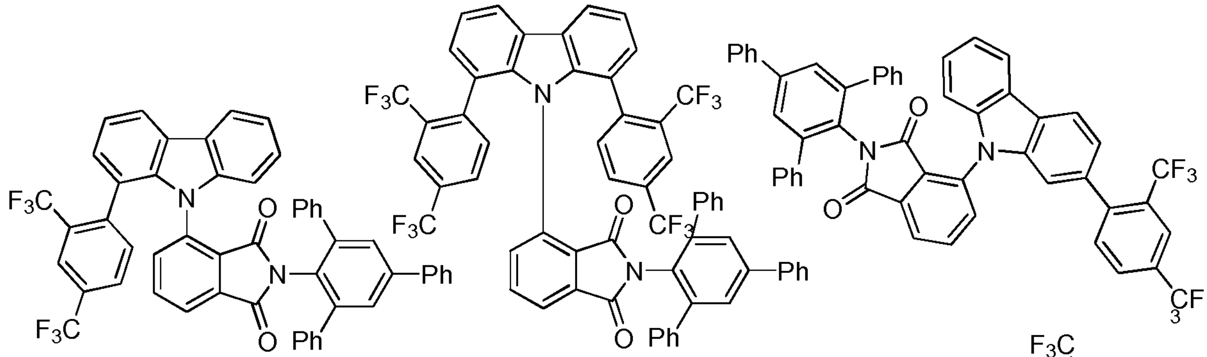

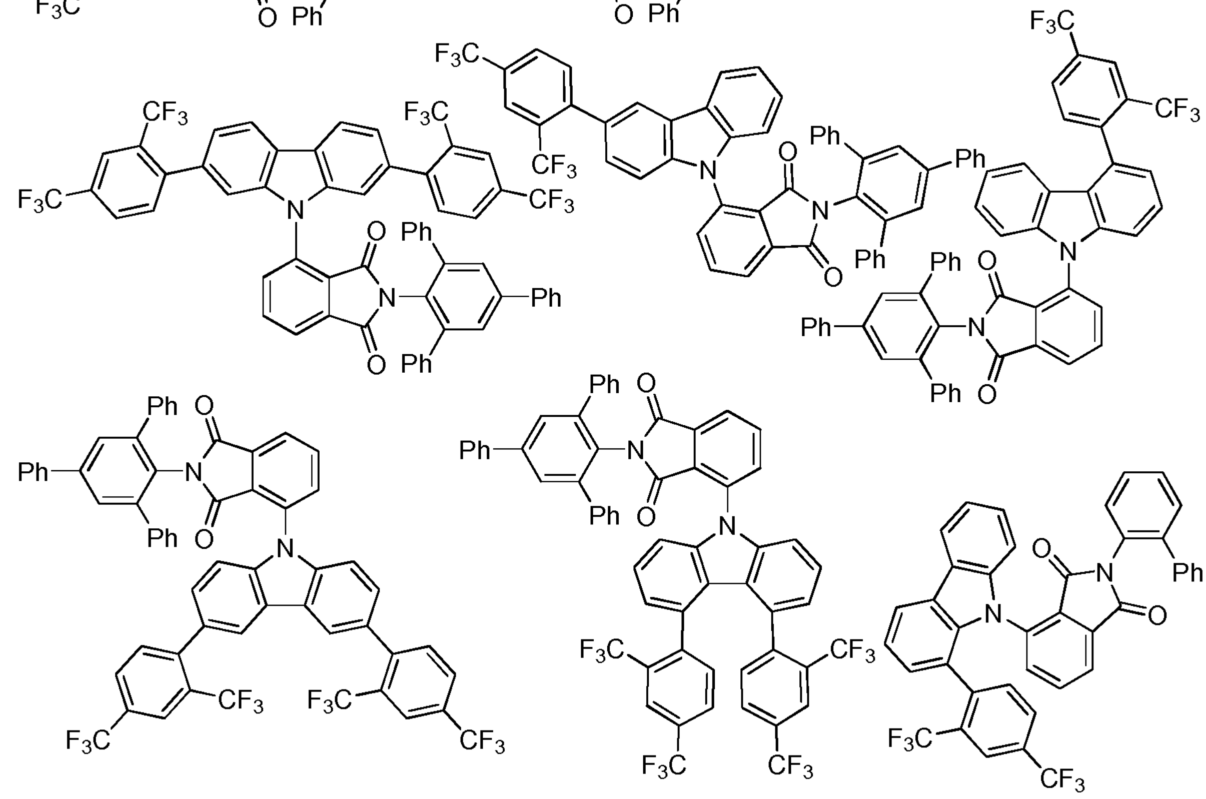

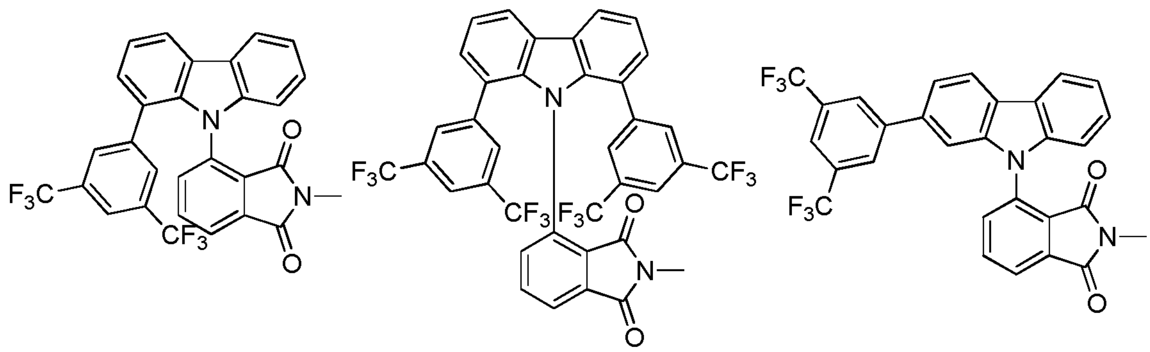

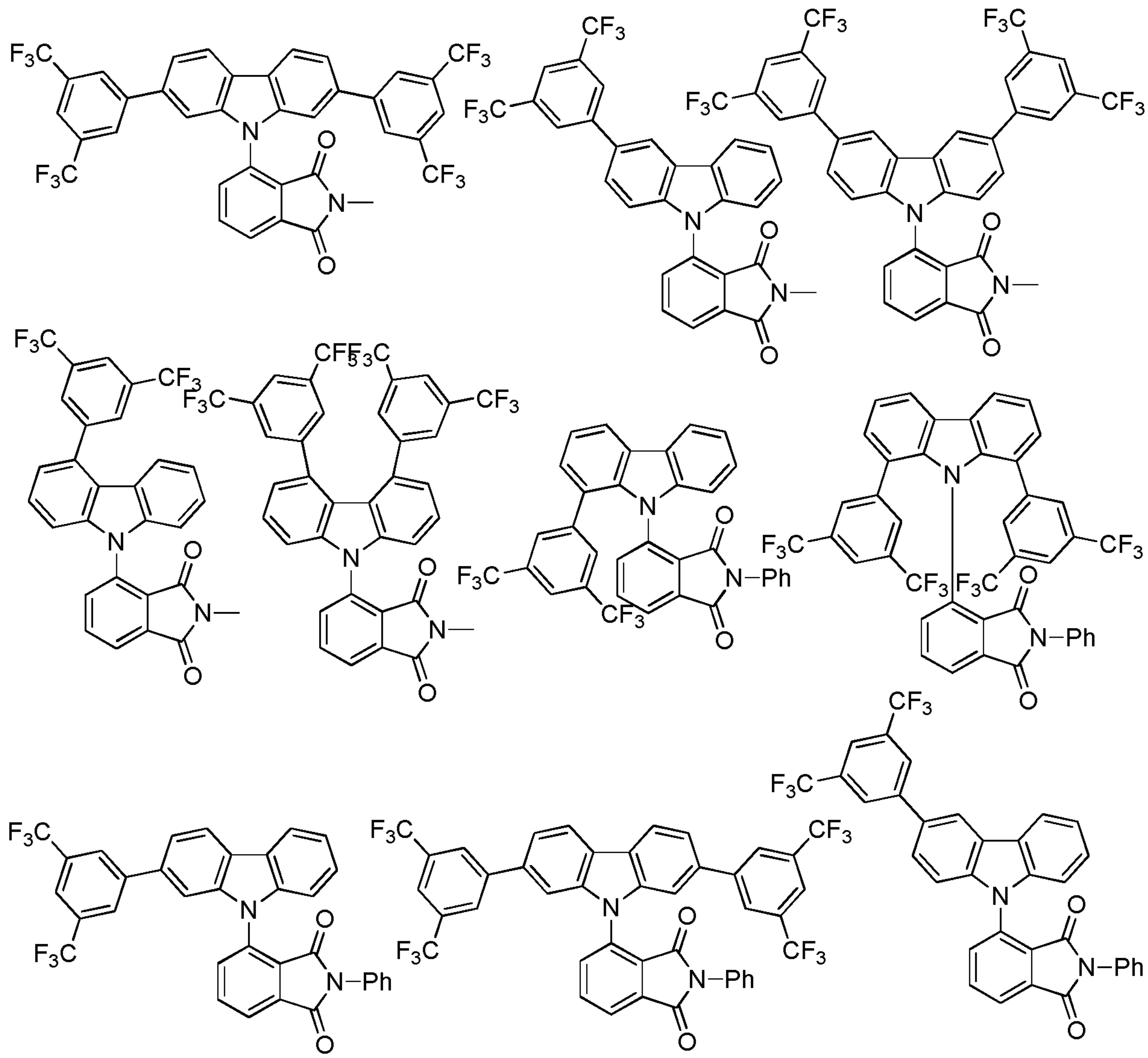

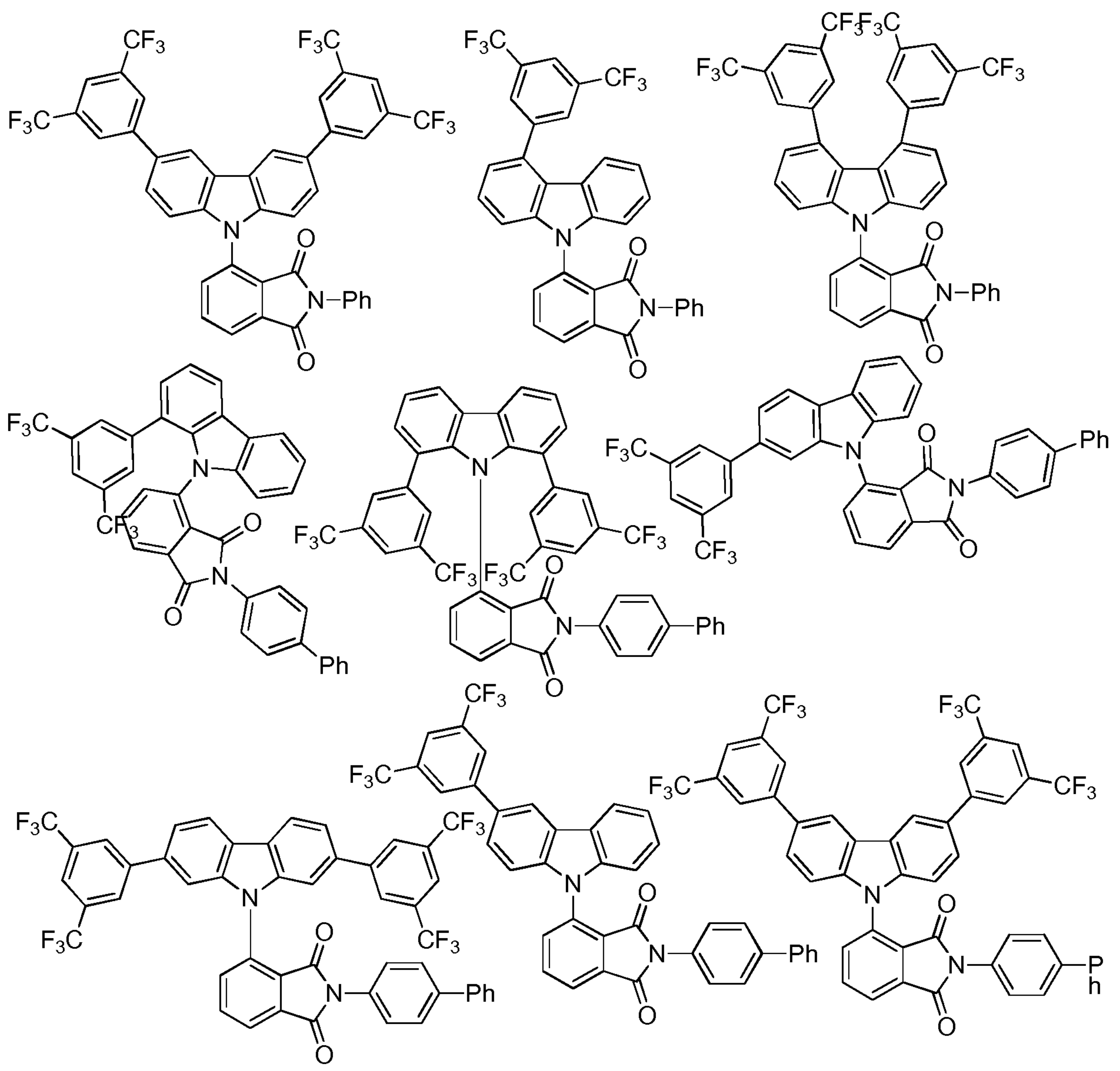

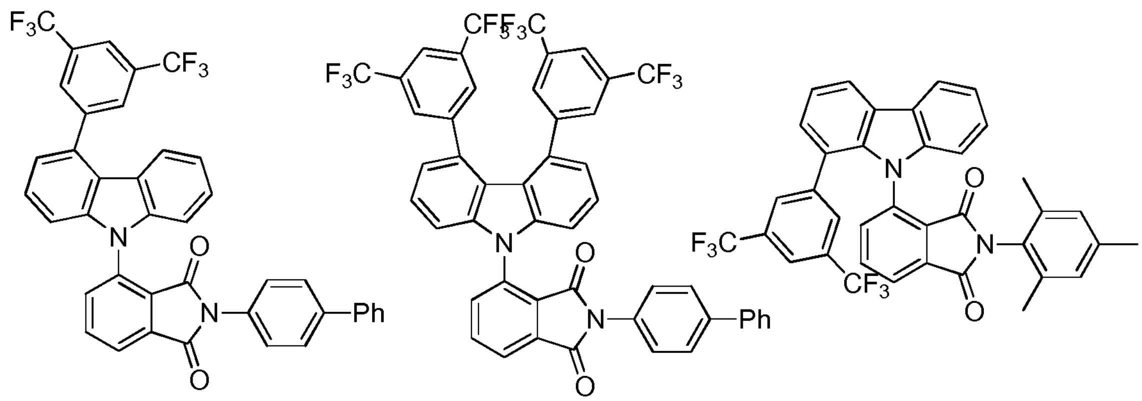

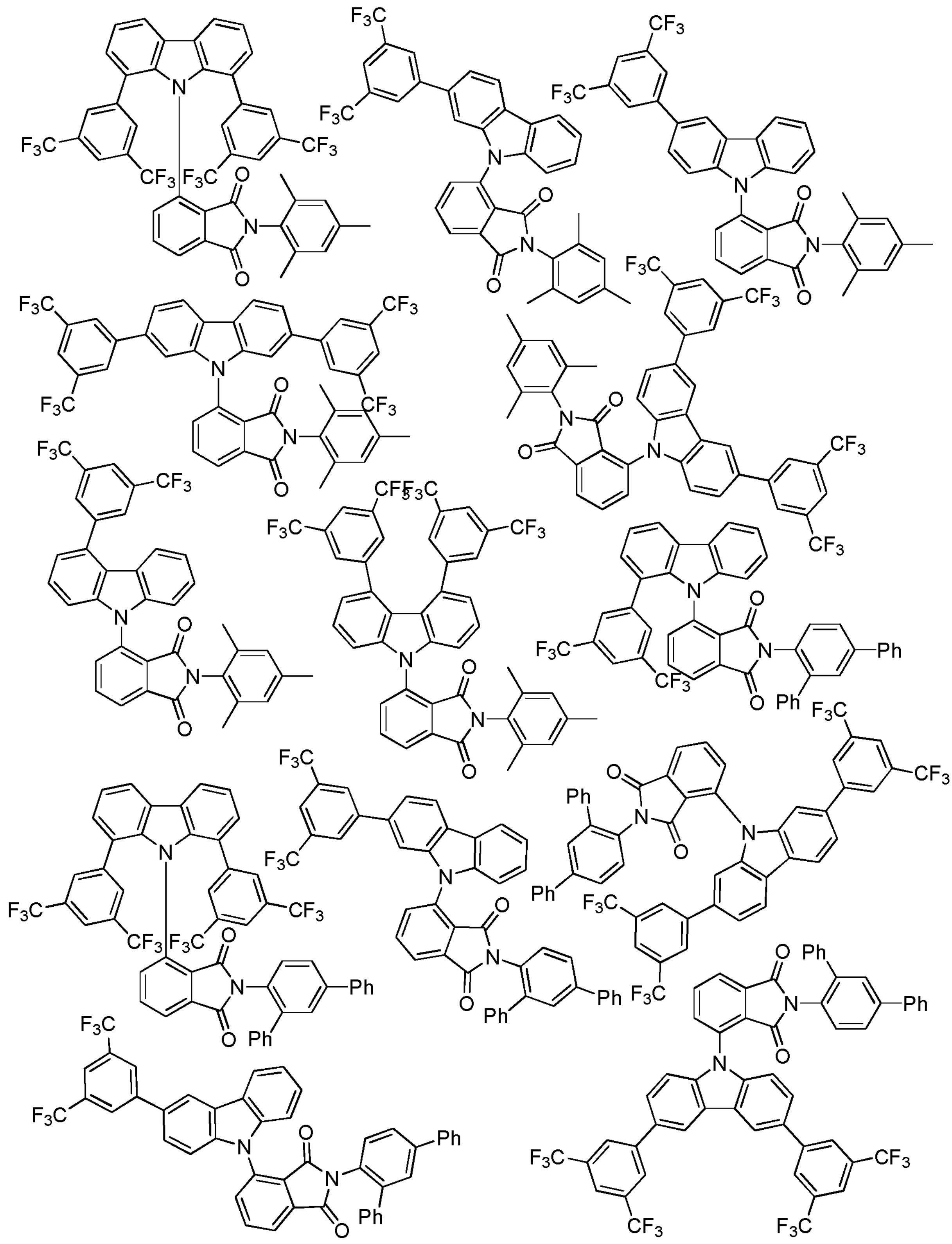

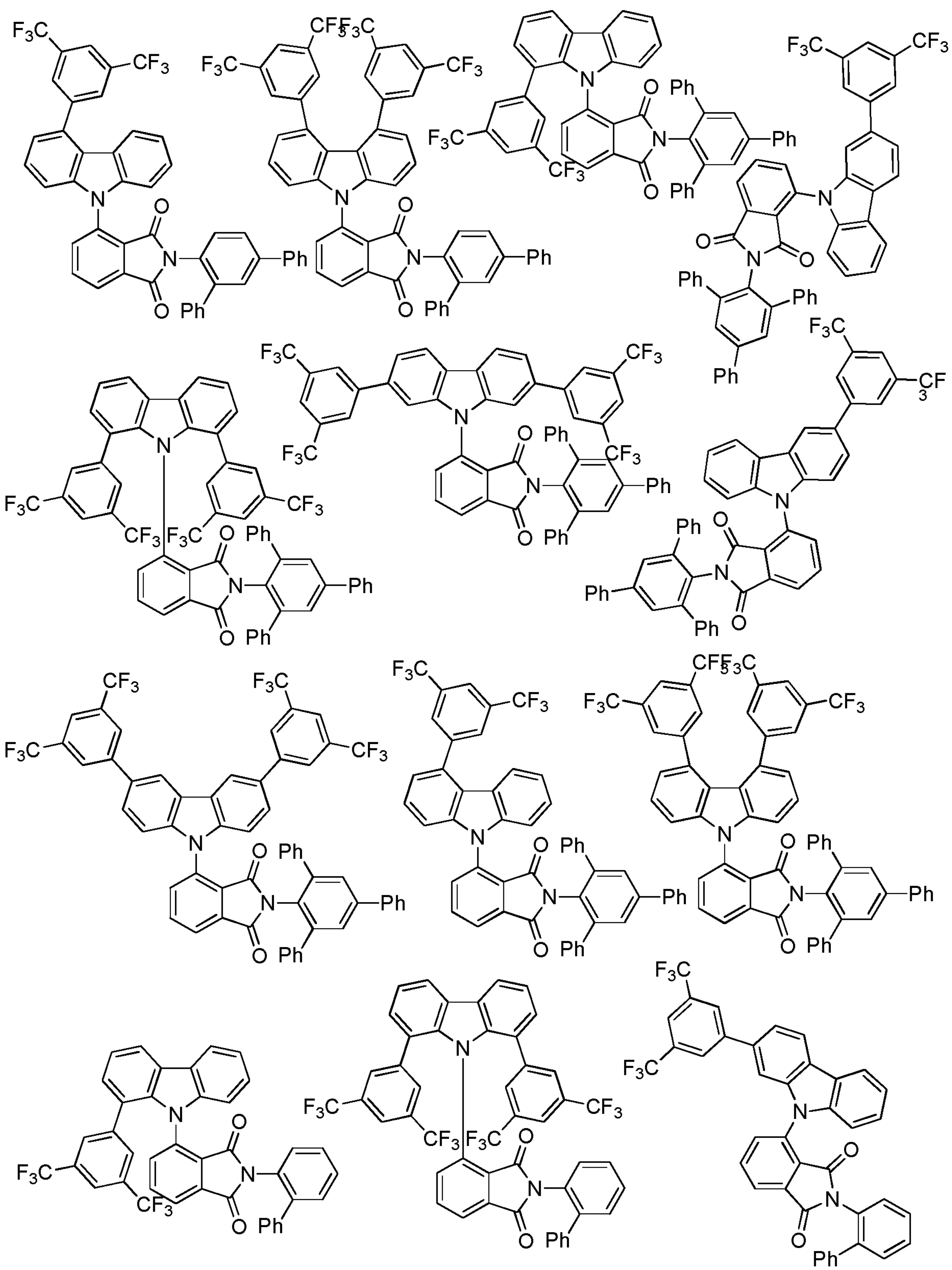

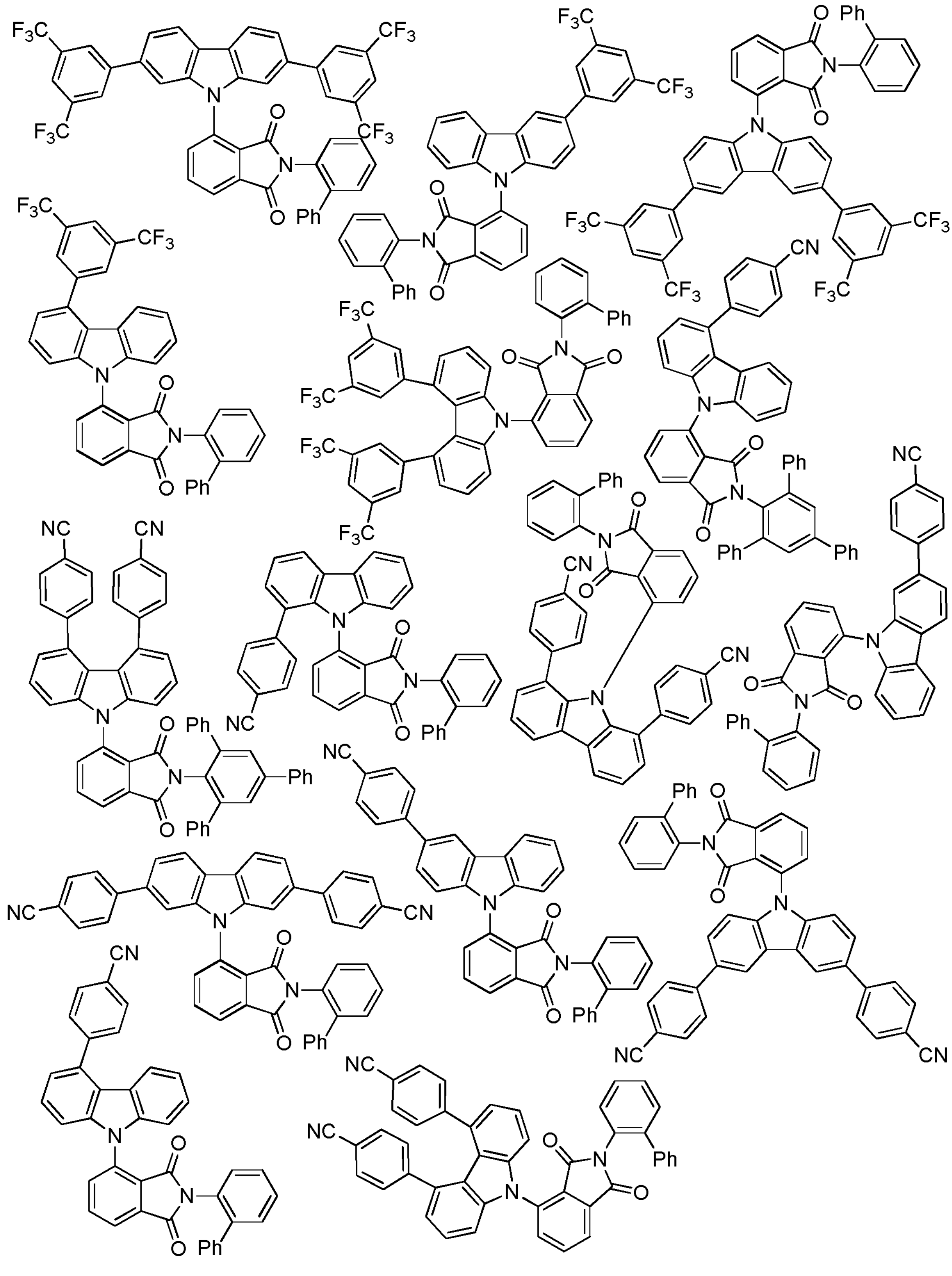

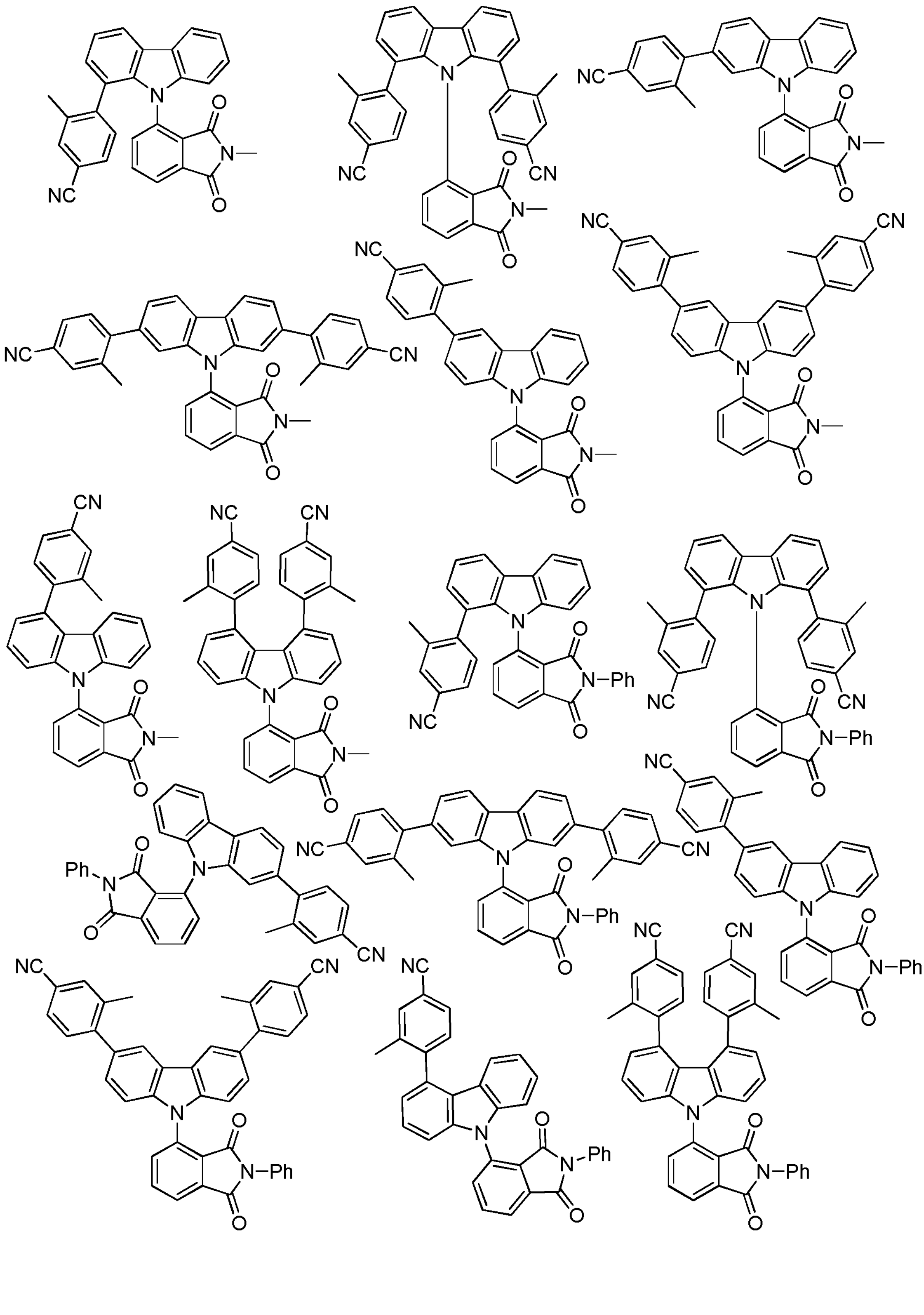

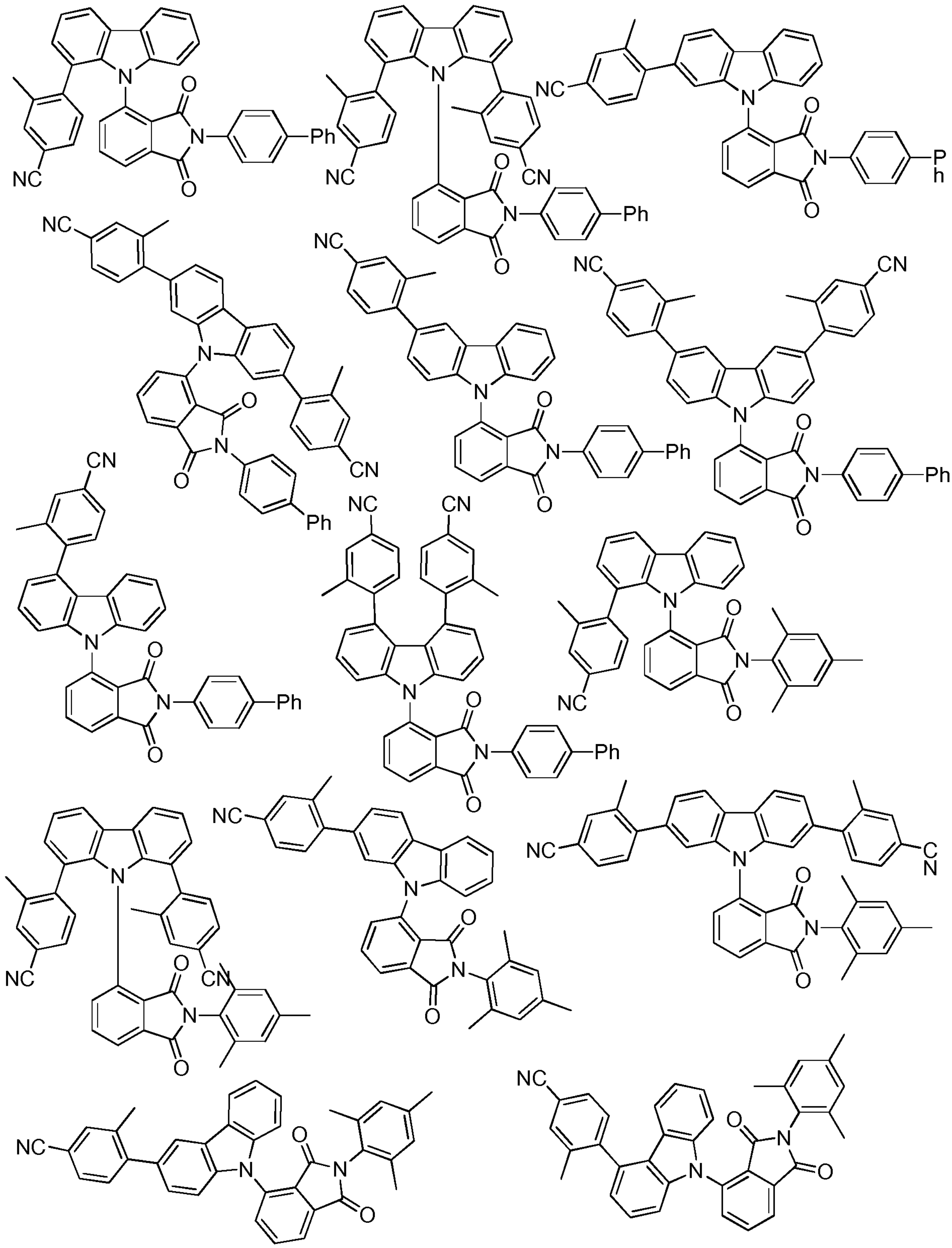

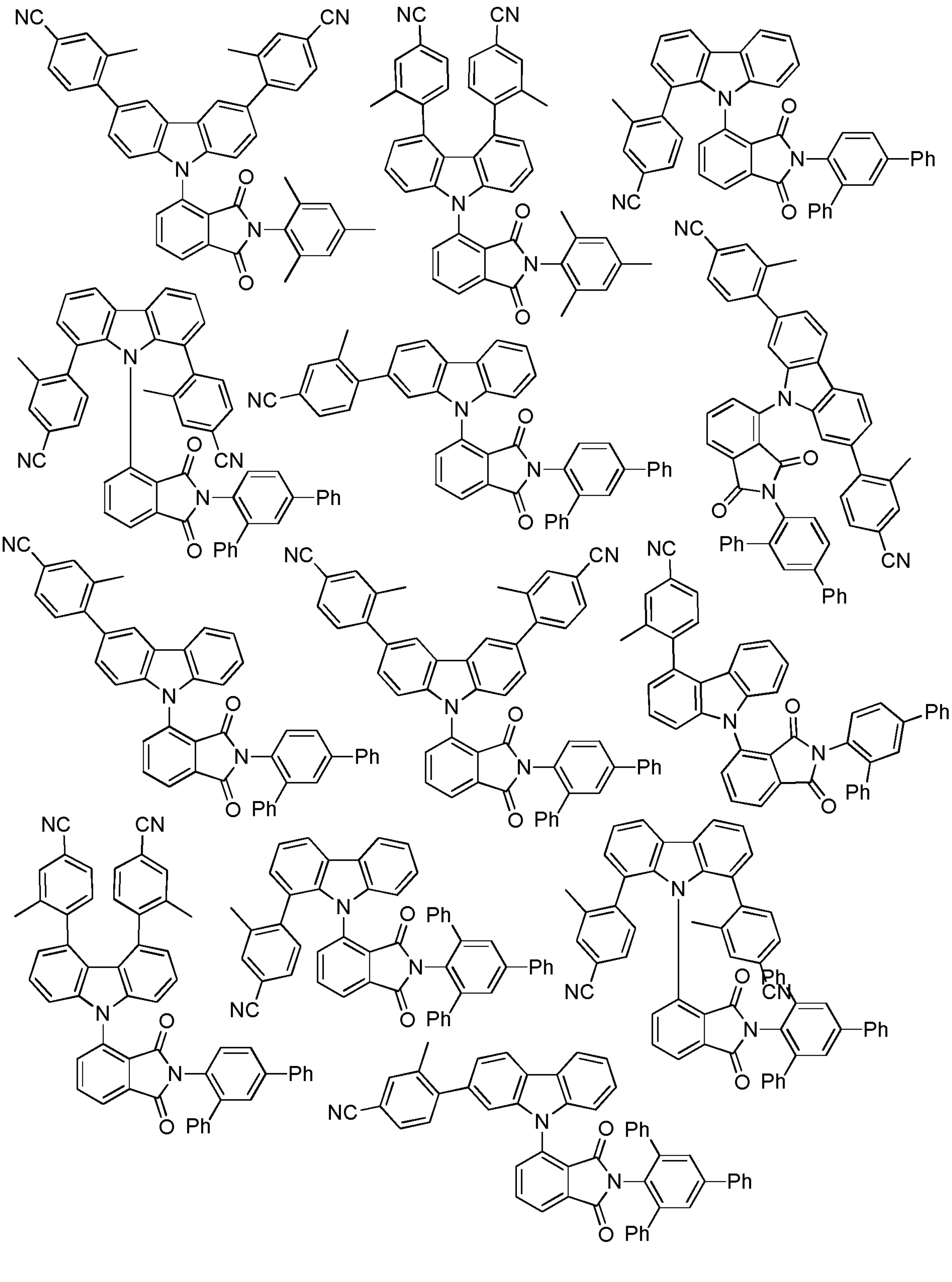

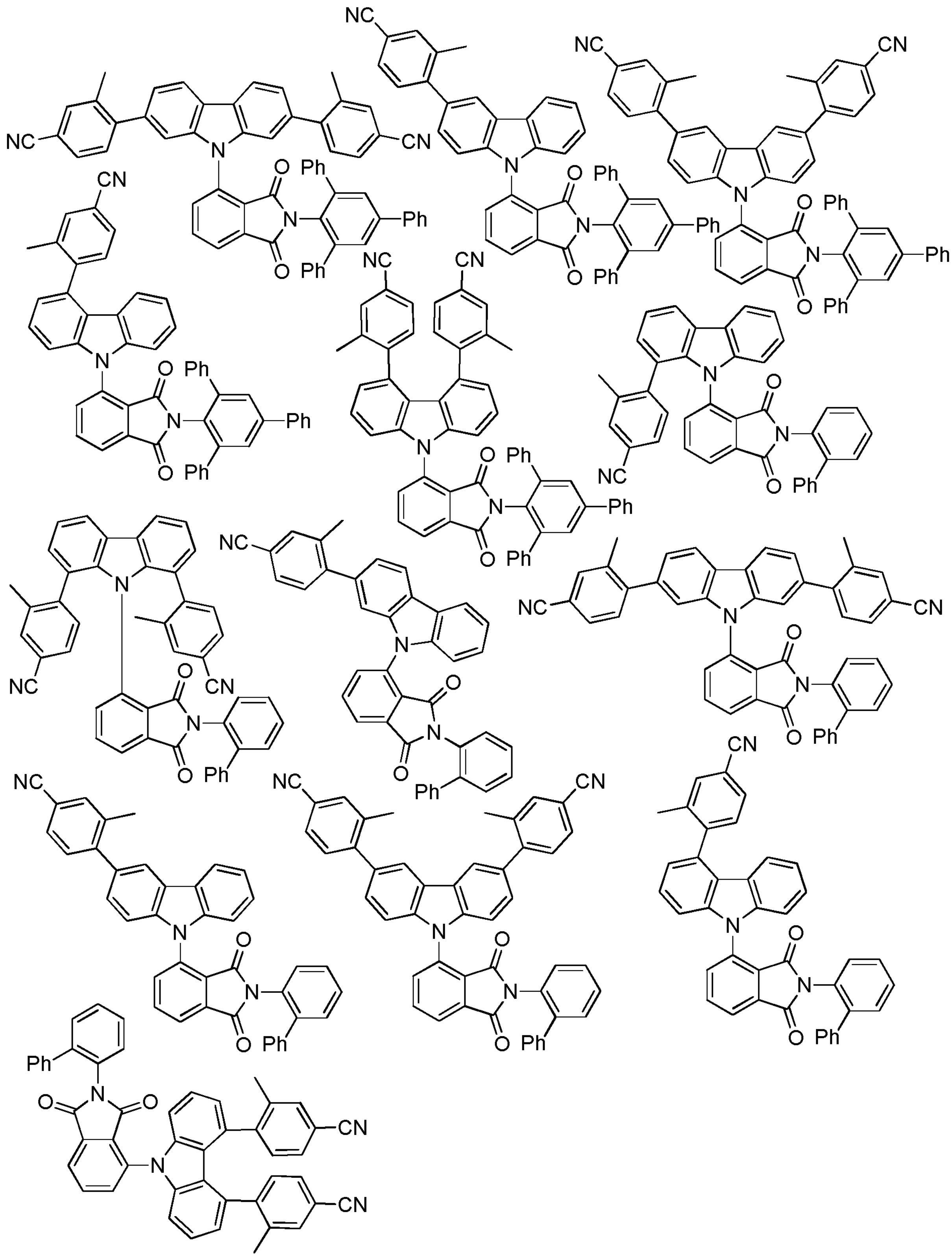

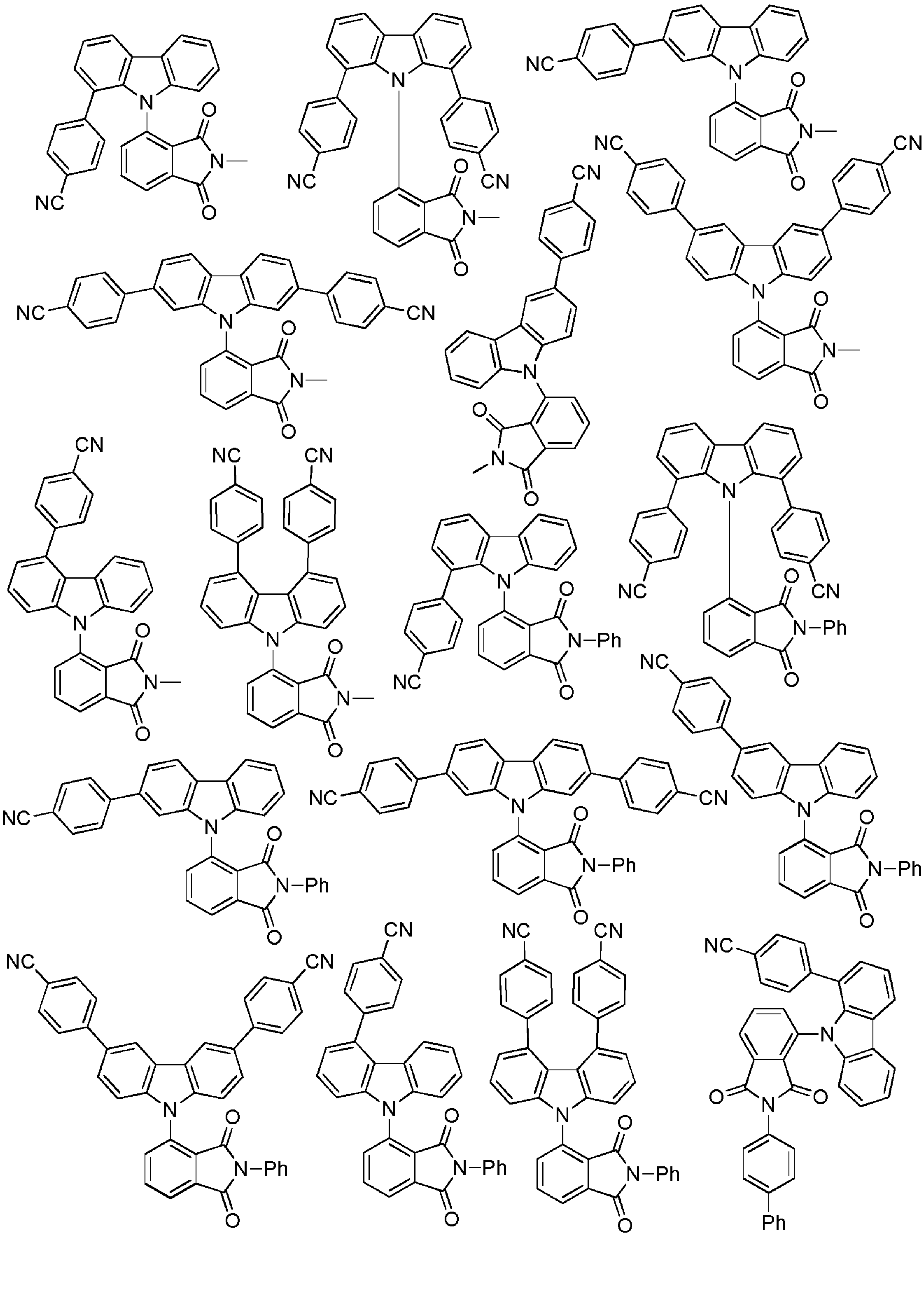

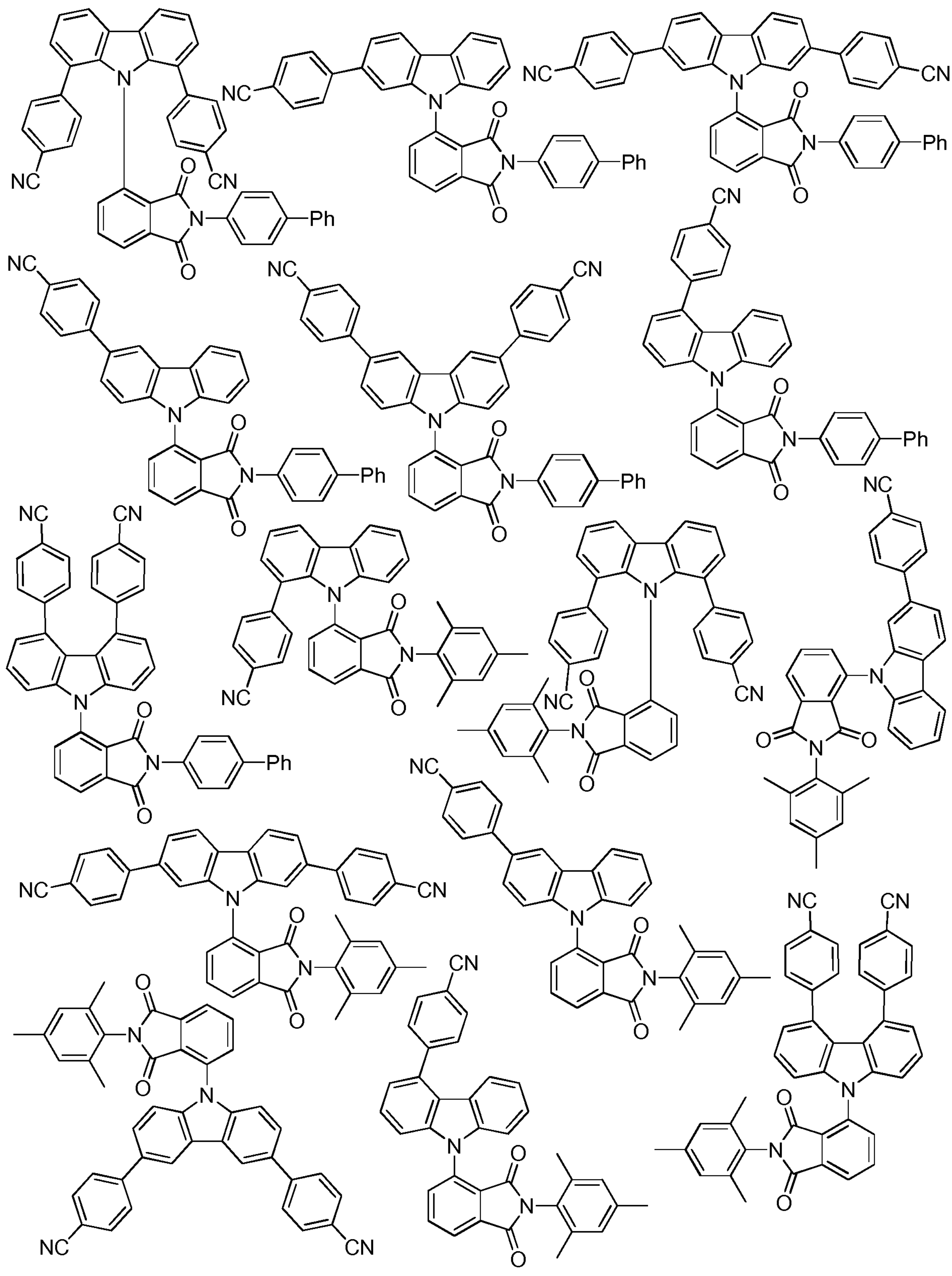

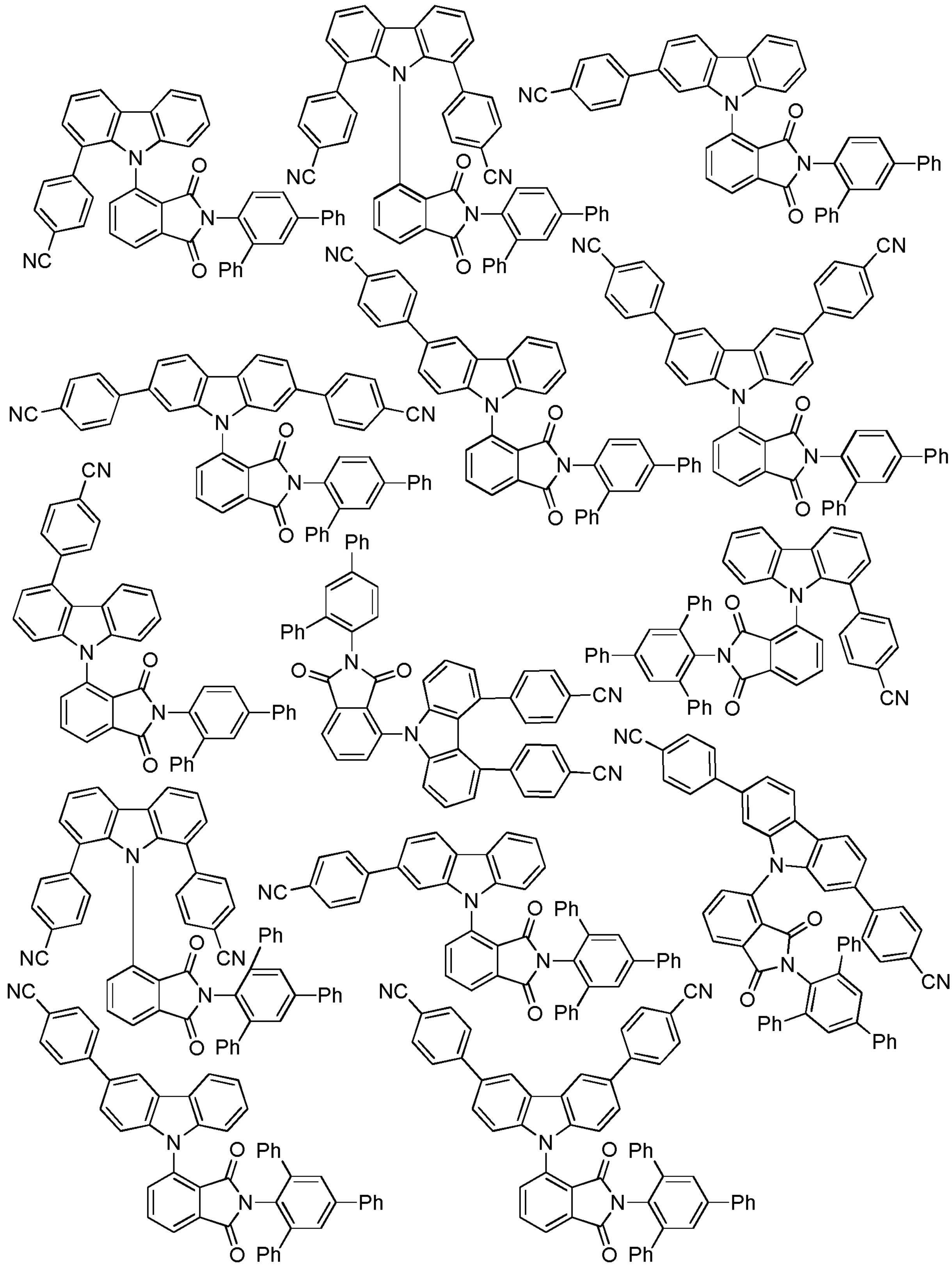

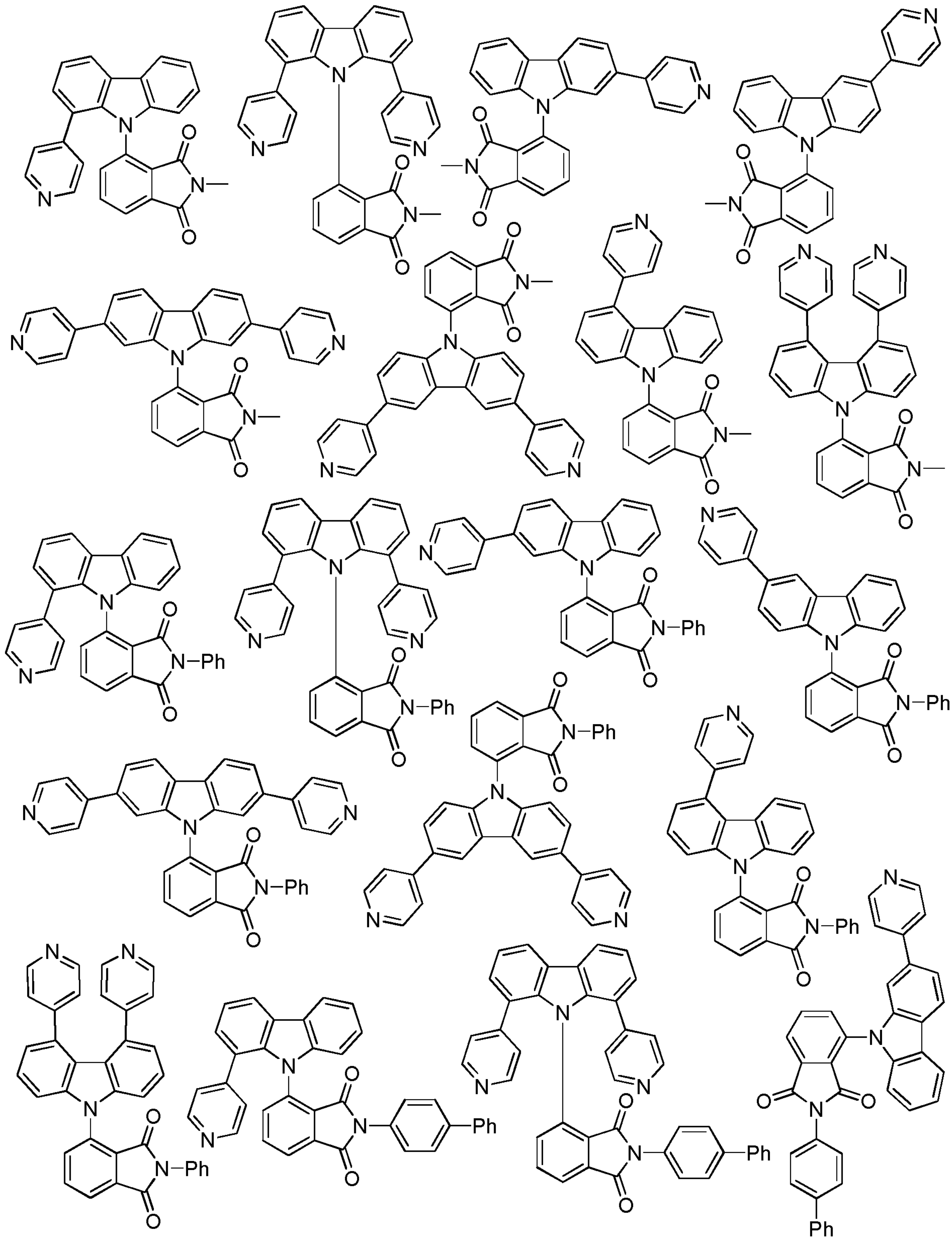

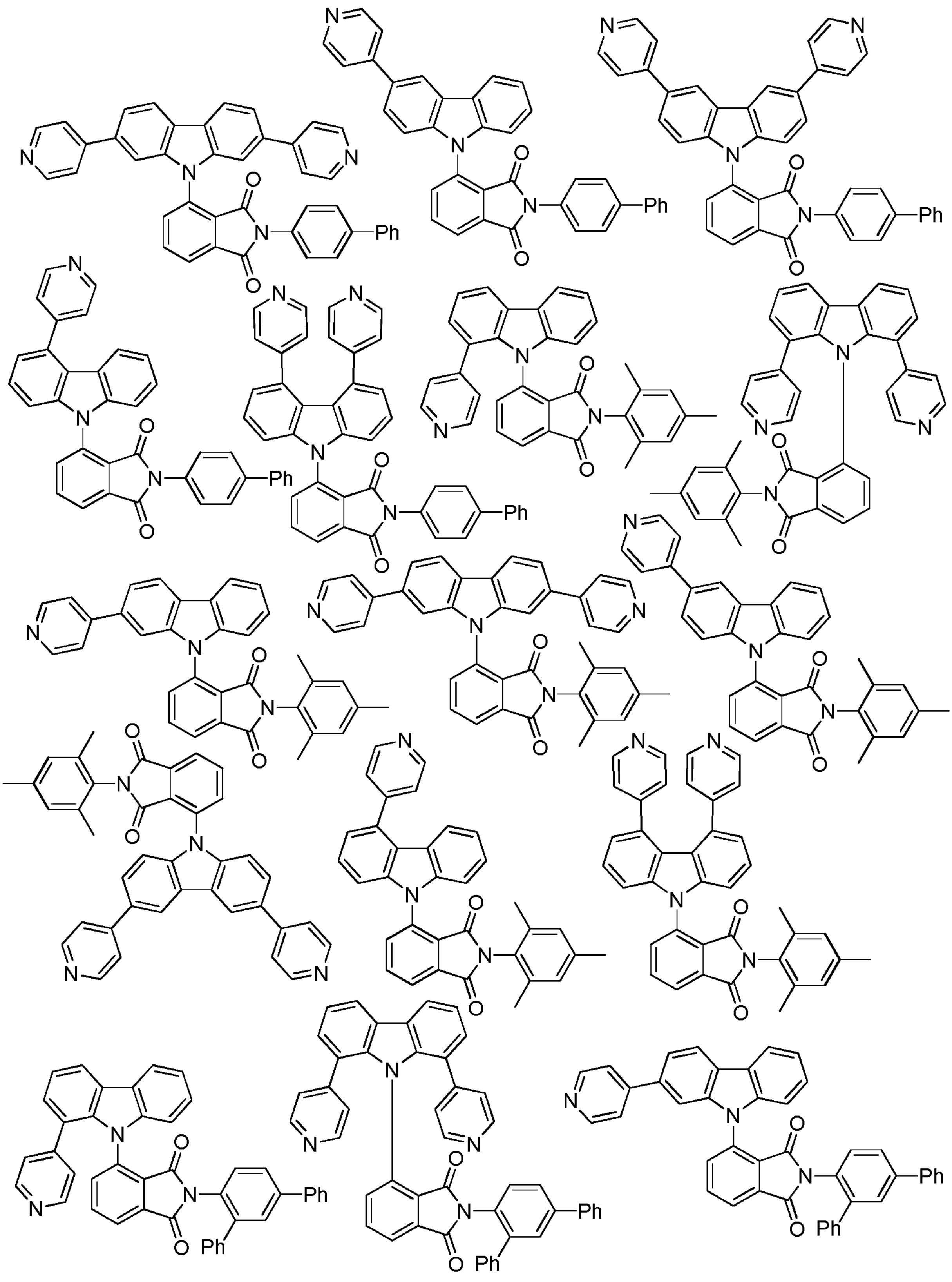

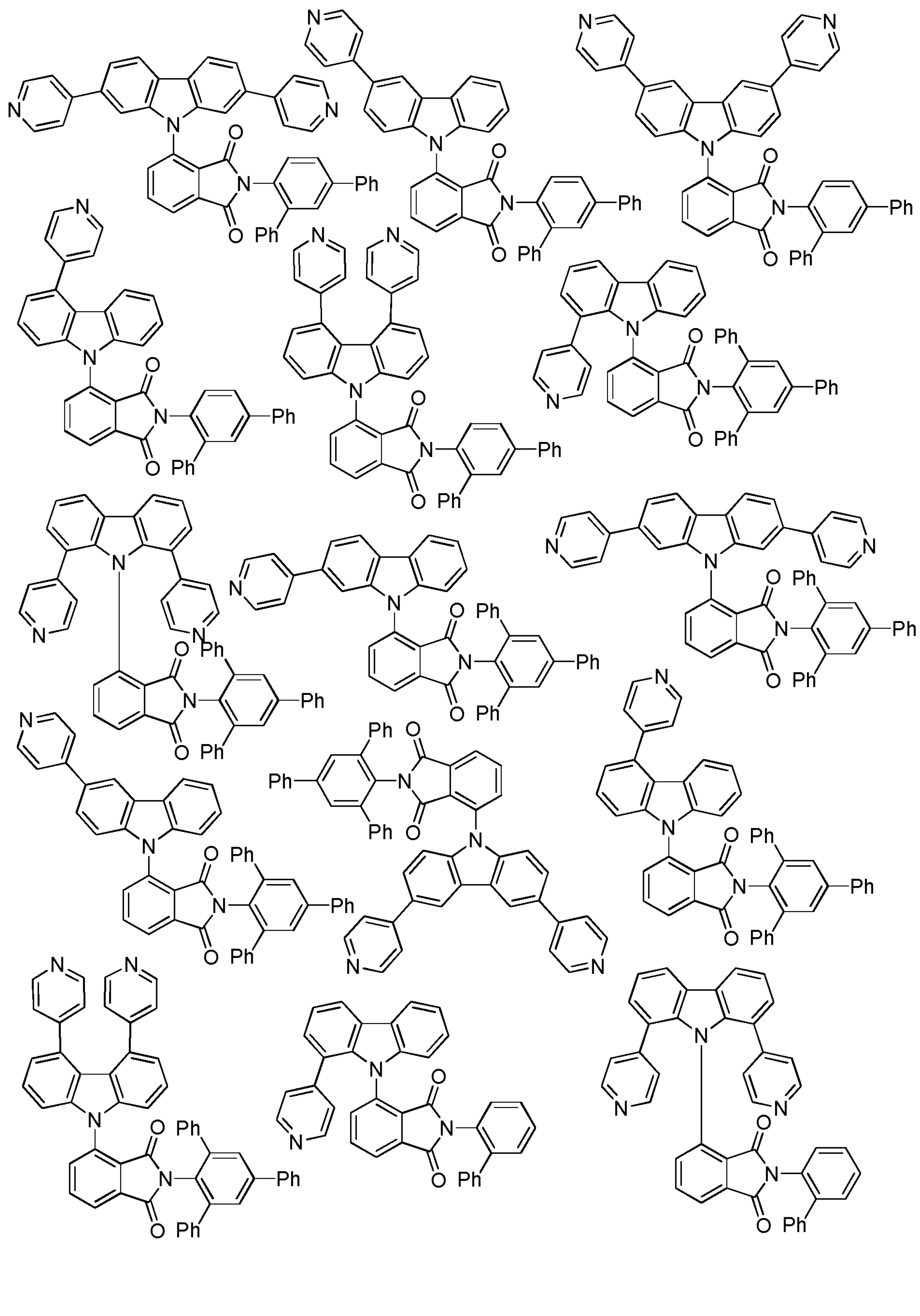

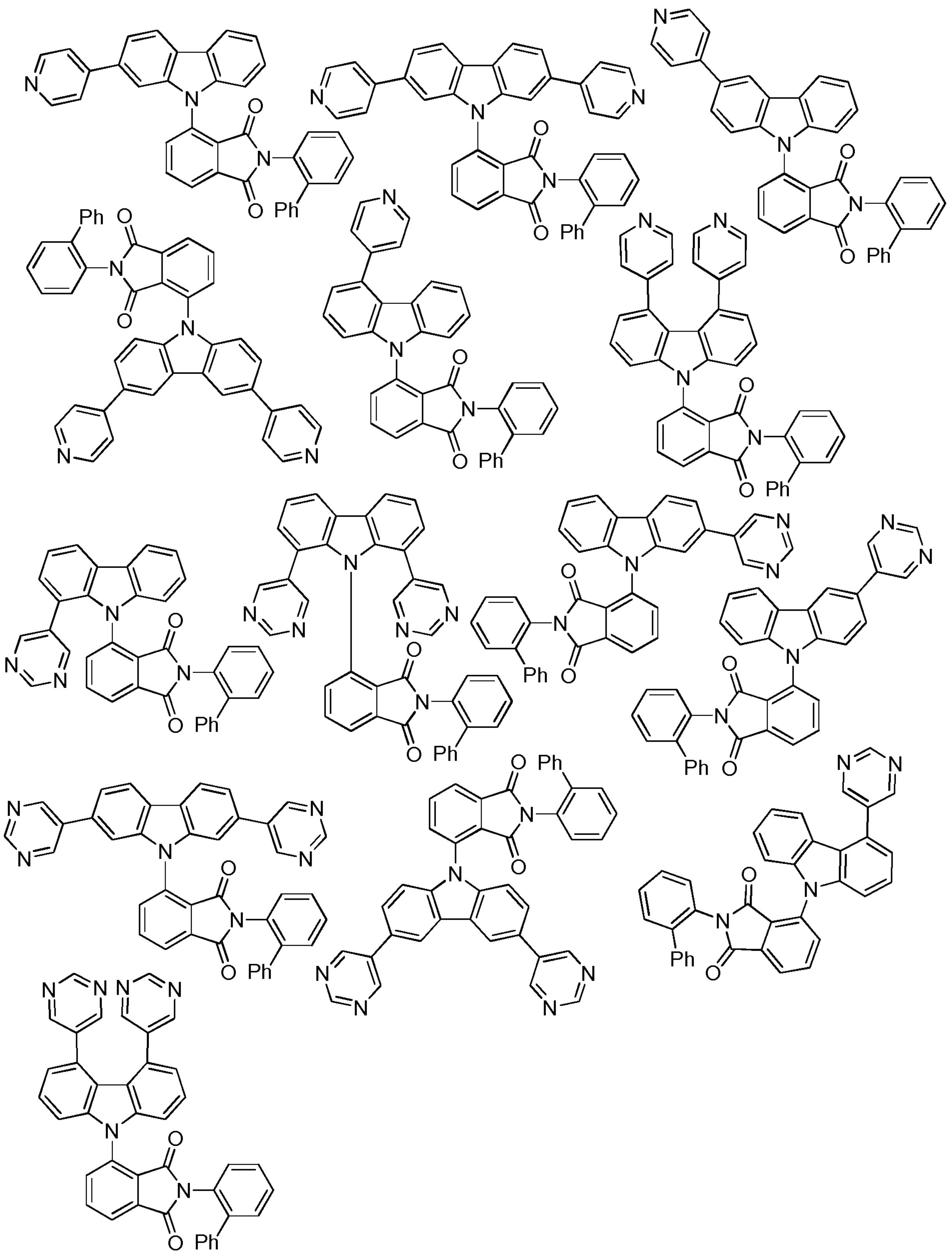

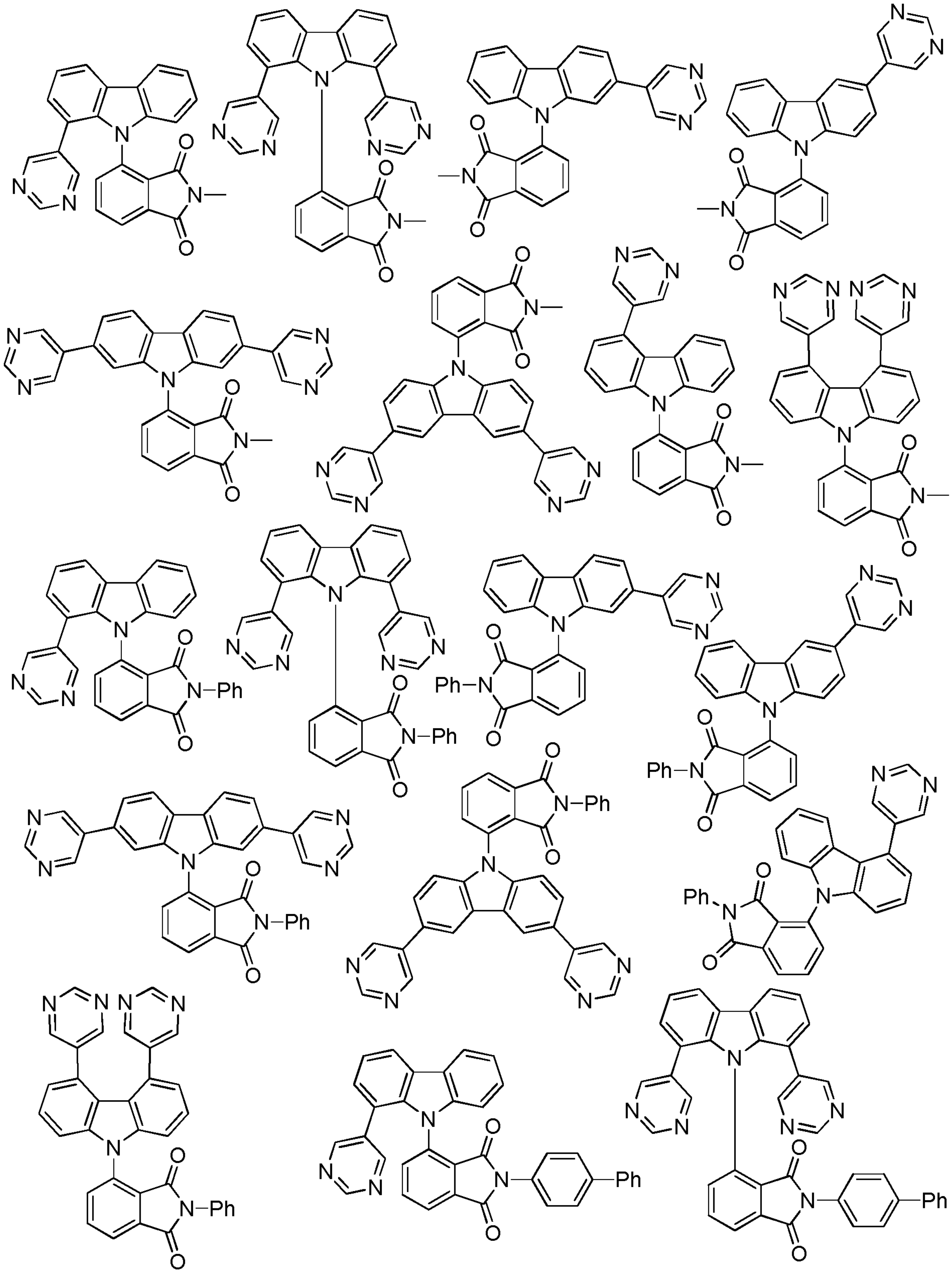

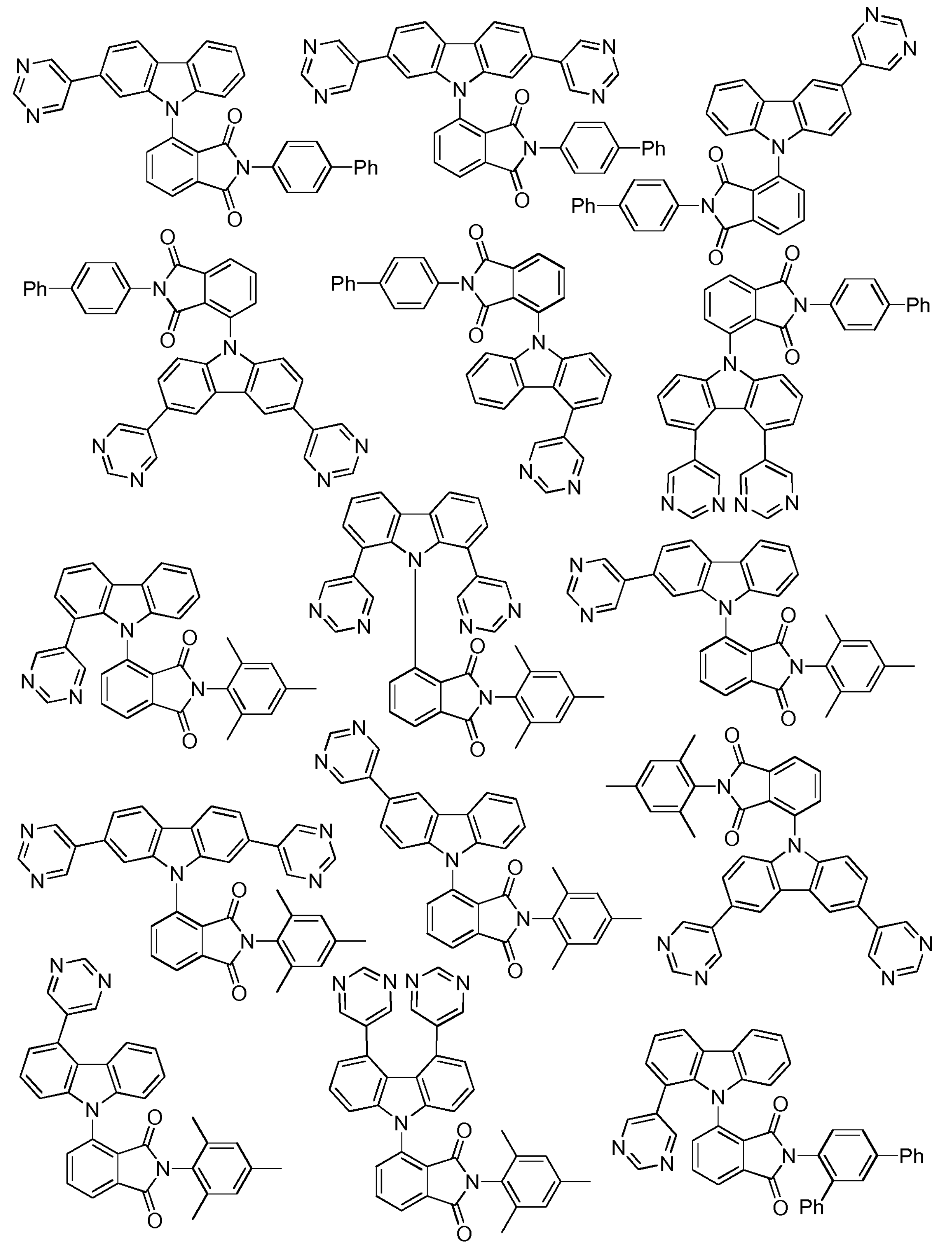

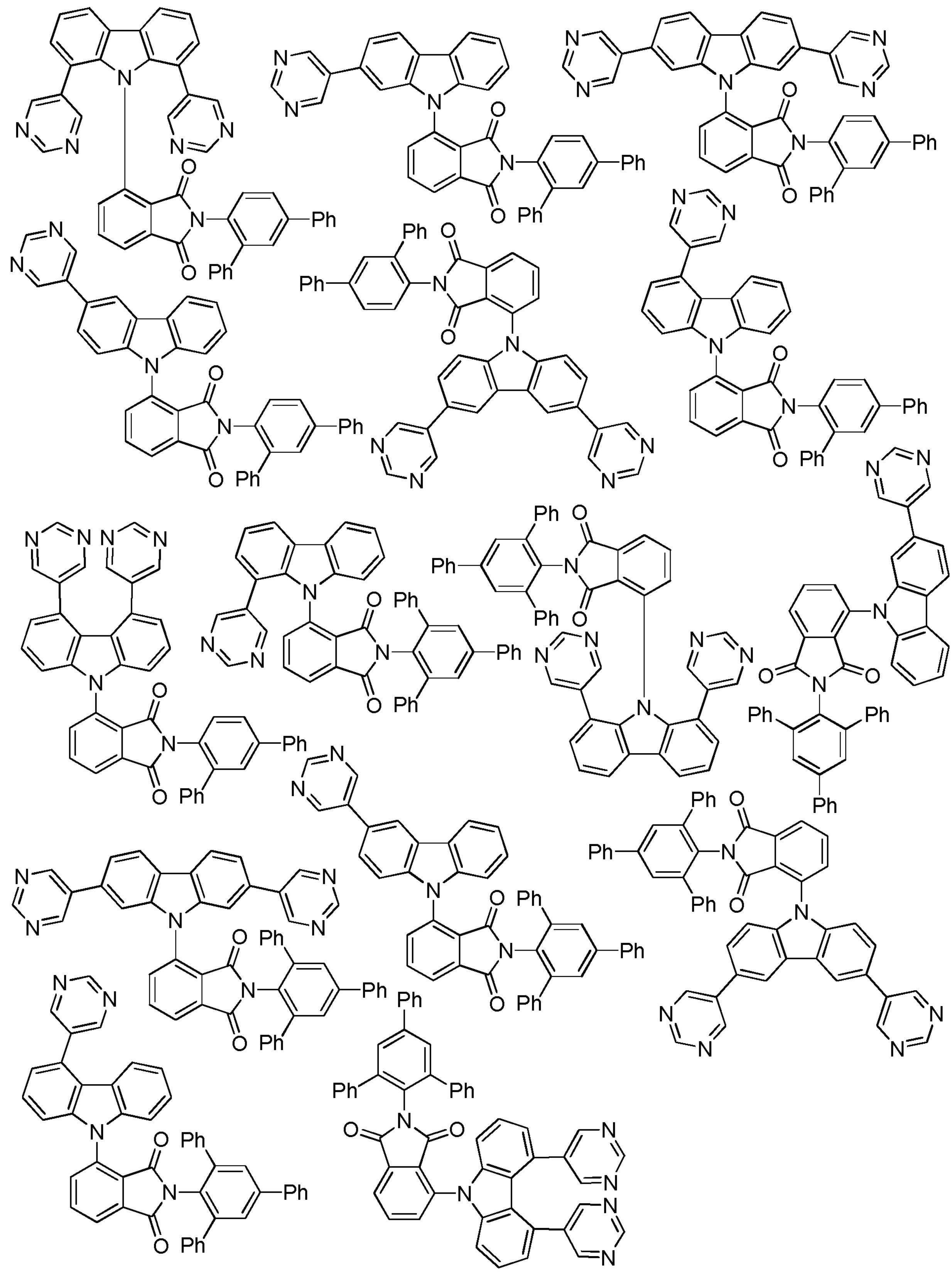

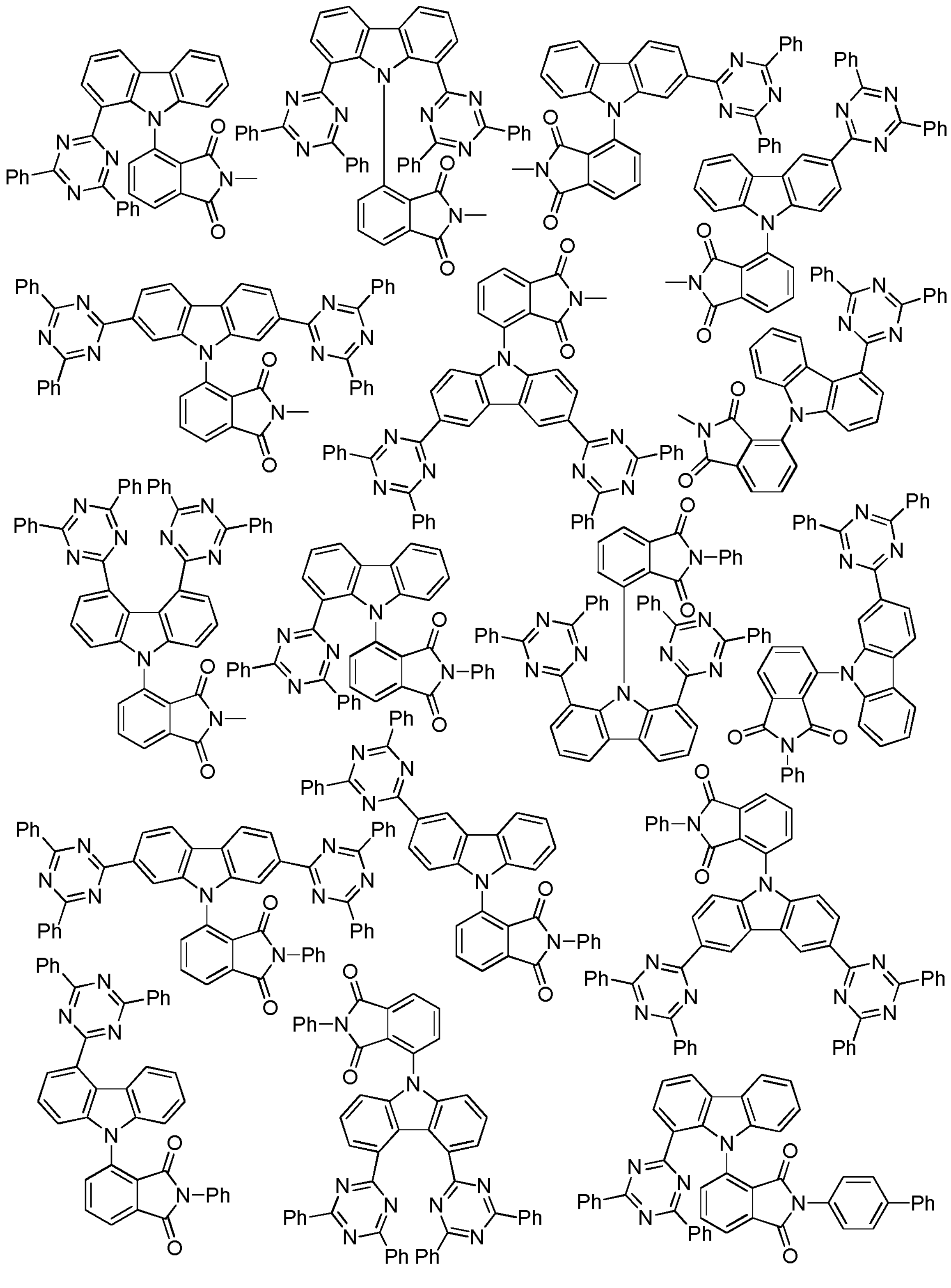

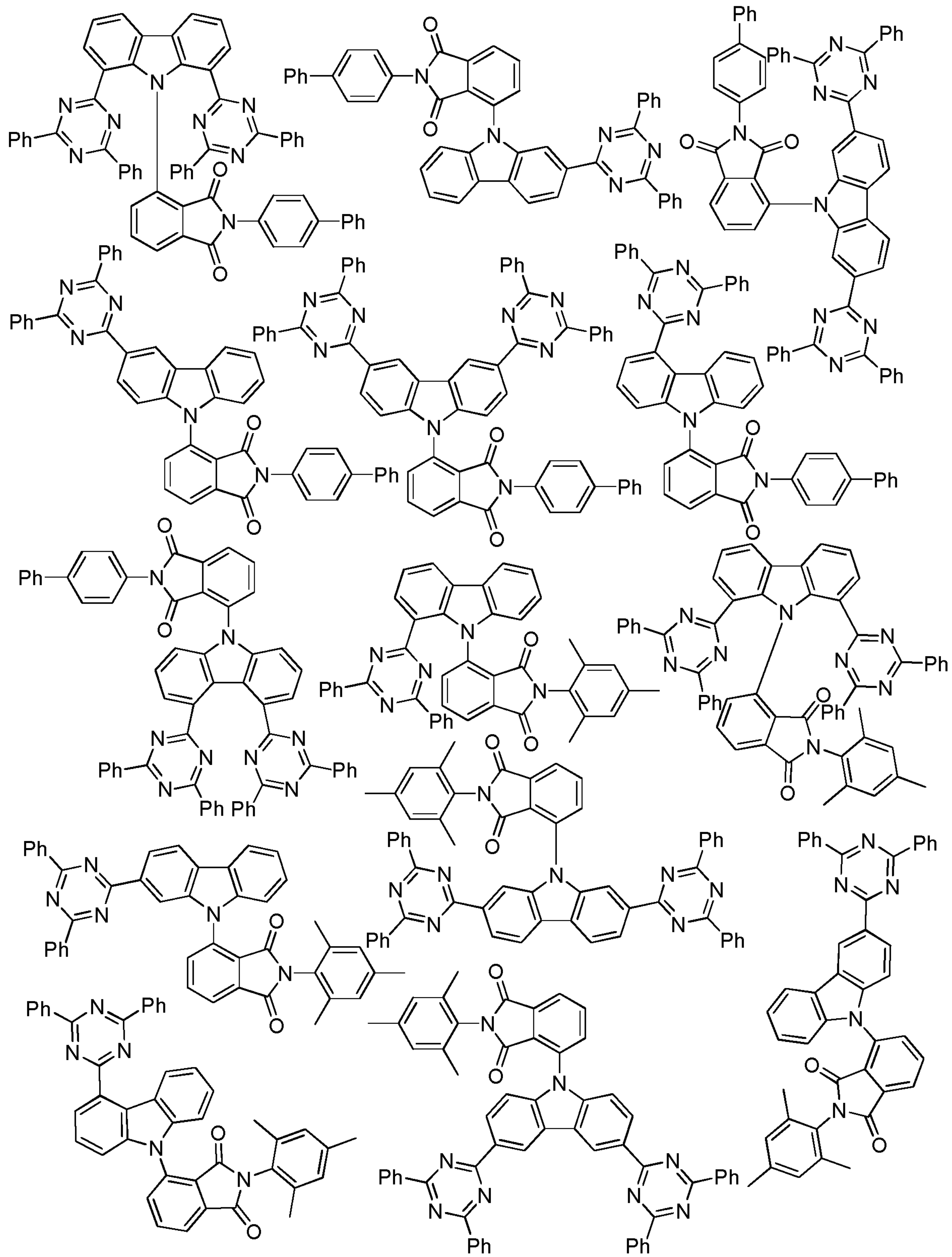

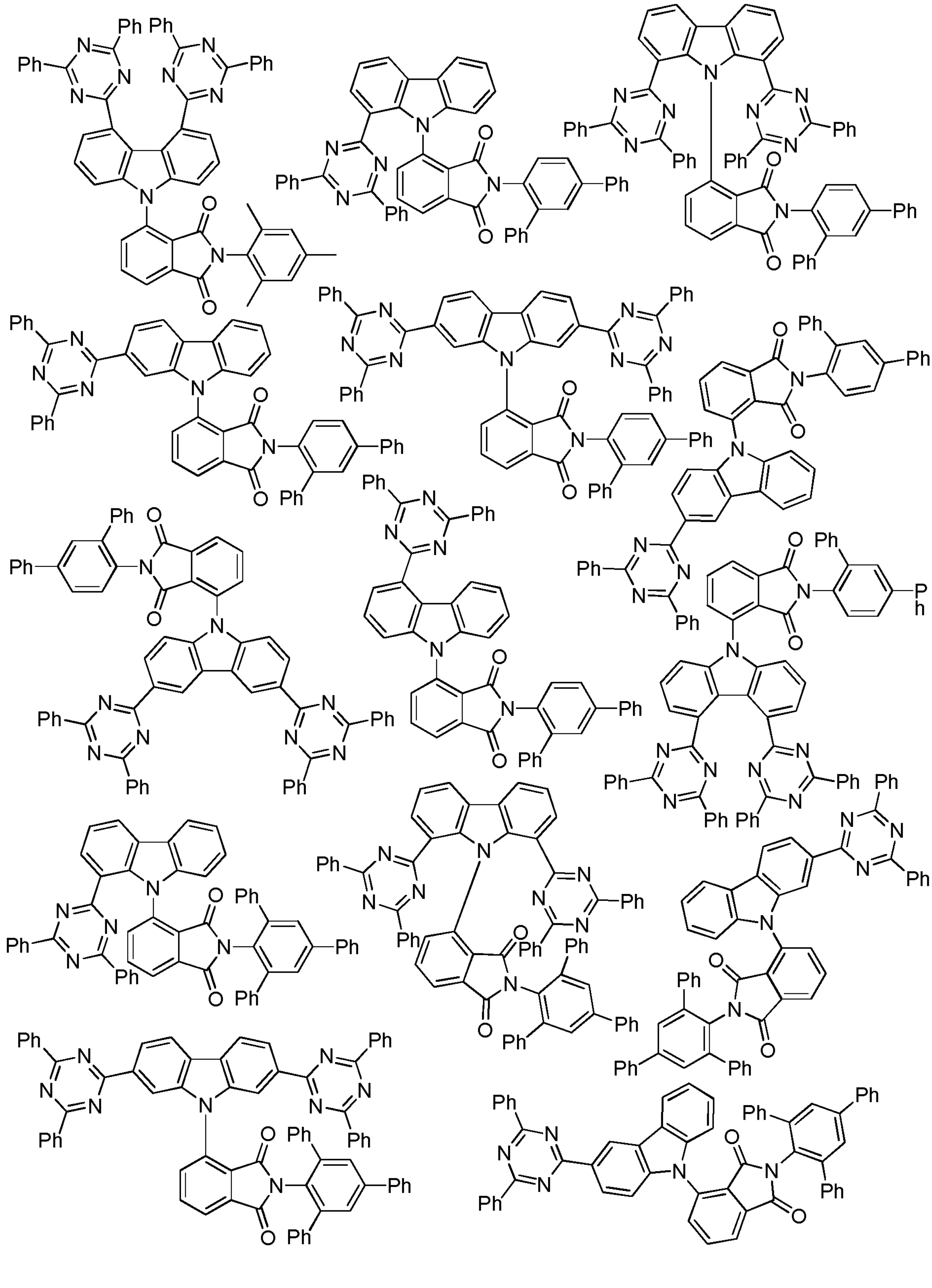

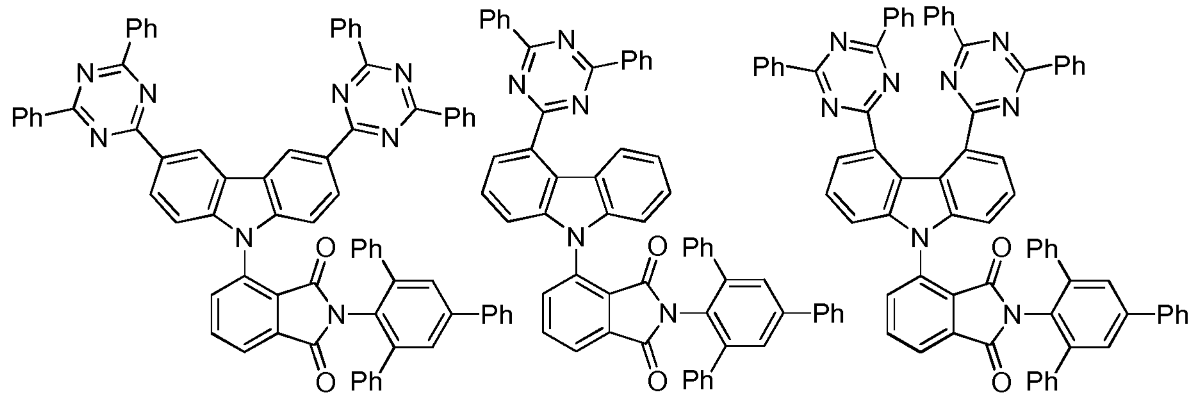

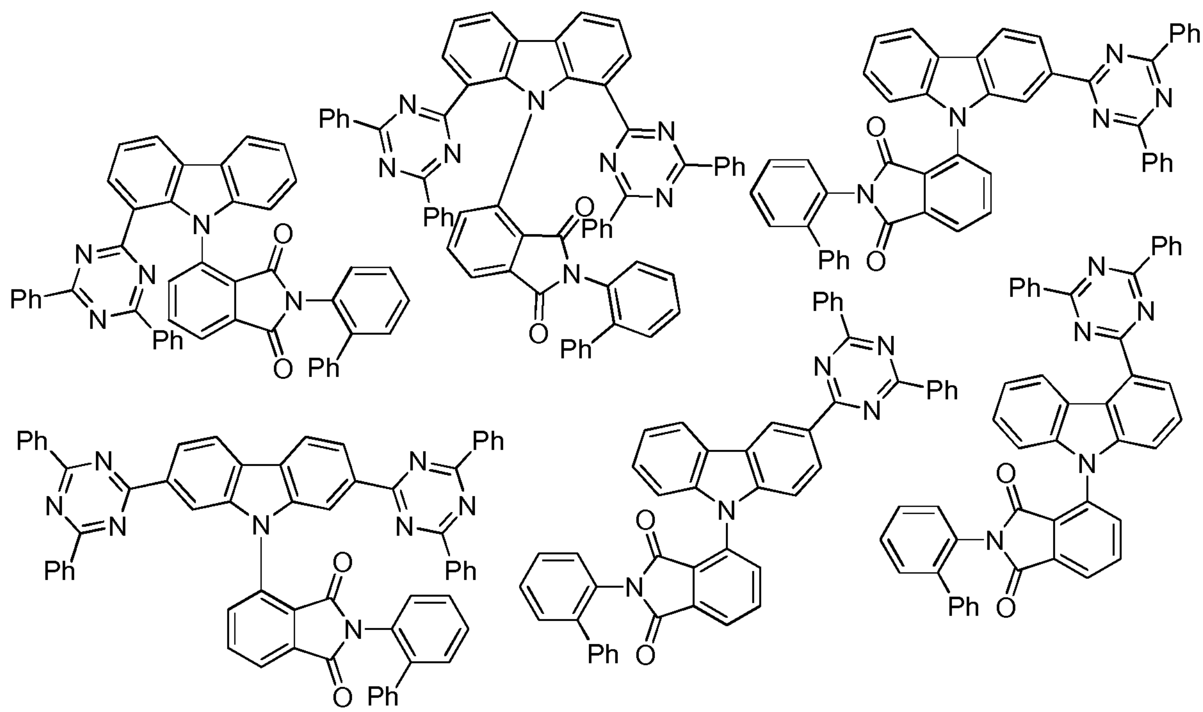

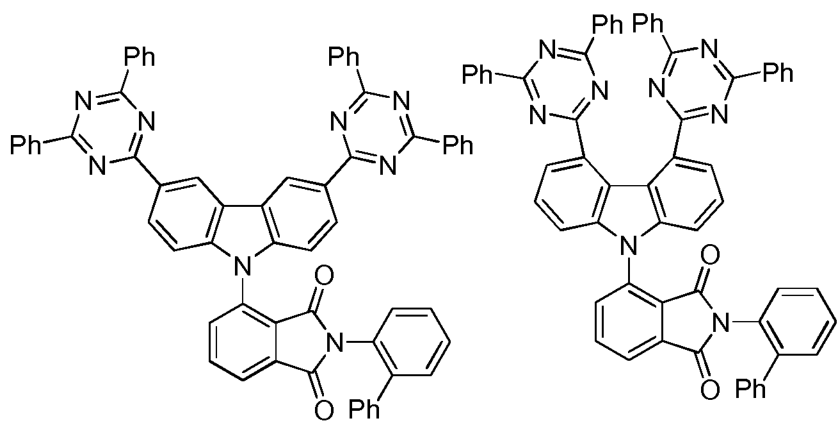

## Figuren

Es zeigen:

Figur 1 Emissionsspektrum von **1** in 10 % PMMA.

Figur 2 Emissionsspektrum von **2** in 10 % PMMA.
Figur 3 Emissionsspektrum von **3** in 10 % PMMA.
Figur 4 Emissionsspektrum von **4** in 10 % PMMA.
Figur 5 Emissionsspektrum von **5** in 10 % PMMA.
Figur 6 Emissionsspektrum von **6** in 10 % PMMA.
Figur 7 Emissionsspektrum von **7** in 10 % PMMA.
Figur 8 Emissionsspektrum von **8** in 10 % PMMA.
Figur 9 Emissionsspektrum von **9** in 10 % PMMA.
Figur 10 Emissionsspektrum von **10** in 10 % PMMA.
Figur 11 Emissionsspektrum von **11** in 10 % PMMA.
Figur 12 Emissionsspektrum von **12** in 10 % PMMA.
Figur 13 Emissionsspektrum von **13** in 10 % PMMA.
Figur 14 Emissionsspektrum von **14** in 10 % PMMA.
Figur 15 Emissionsspektrum von **15** in 10 % PMMA.
Figur 16 Emissionsspektrum von **16** in 10 % PMMA.
Figur 17 Emissionsspektrum von **17** in 10 % PMMA.
Figur 18 Emissionsspektrum von **18** in 10 % PMMA.
Figur 19 Emissionsspektrum von **19** in 10 % PMMA.
Figur 20 Emissionsspektrum von **20** in 10 % PMMA.
Figur 21 Emissionsspektrum von **21** in 10 % PMMA.

## Patentansprüche

1. Organisches Molekül, aufweisend eine Struktur der Formel A1

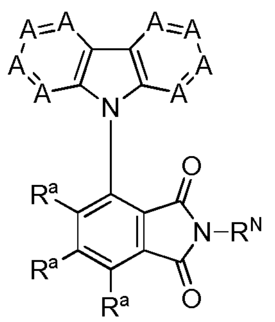

Formel A1

mit

A ist bei jedem Auftreten unabhängig voneinander $CR^b$ oder N;
$R^N$ ist Methyl, Phenyl, Xylyl, Mesityl, Naphtyl, Biphenyl, Naphthylphenyl, Terphenyl oder 2,4,6-Triphenylphenyl;
$R^a$ ist bei jedem Auftreten unabhängig voneinander H, Deuterium, eine Alkylgruppe oder eine Arylgruppe;

$R^b$ ist bei jedem Auftreten unabhängig voneinander ausgewählt aus der Gruppe bestehend aus:

- H,
- Deuterium,
- $CF_3$,
- $C(=O)R^1$,
- CN,
- einer unsubstituierten oder mit einem oder mehreren $R^2$ substituierten Alkylgruppe,
- einer unsubstituierten oder mit einem oder mehreren $R^2$ substituierten Arylgruppe, die optional zusätzlich mit einer oder mehreren unsubstituierten Alkylgruppe und/oder mit einer oder mehreren unsubstituierten oder Alkyl-substituierten Arylgruppen substituiert ist,
- einer unsubstituierten oder mit $R^2$ und/oder mit einer oder mehreren unsubstituierten Alkylgruppe und/oder

mit einer oder mehreren unsubstituierten oder Alkyl-substituierten Arylgruppe substituierten Heteroarylgruppe und

- einer Gruppe der Unterformel T1 oder einer Gruppe der Unterformel T2:

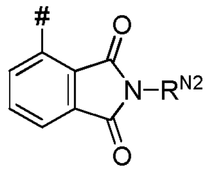

Unterformel T1

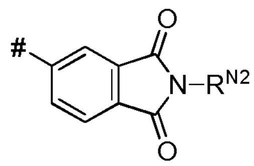

Unterformel T2

wobei gilt:

$R^{N2}$ ist eine Alkylgruppe, eine unsubstituierte oder mit einem oder mehreren $R^{N3}$ substituierte Arylgruppe oder eine unsubstituierte oder mit einem oder mehreren $R^{N3}$ substituierte Heteroarylgruppe;

$R^{N3}$ ist eine Alkylgruppe, eine Arylgruppe oder eine Heteroarylgruppe;
$R^1$ ist bei jedem Auftreten eine unsubstituierte oder mit einem oder mehreren $R^2$ substituierte Arylgruppe;
$R^2$ ist bei jedem Auftreten unabhängig voneinander F, $CF_3$ oder CN;

wobei ein bis vier A gleich N sind oder mindestens ein $R^b$ ausgewählt ist aus der Gruppe bestehend aus:

- $CF_3$,
- $C(=O)R^1$,
- CN,
- einer mit einem oder mehreren $R^2$ substituierten Alkylgruppe,
- einer mit einem oder mehreren $R^2$ substituierten Arylgruppe, die optional zusätzlich mit einer oder mehreren unsubstituierten Alkylgruppe und/oder mit einer oder mehreren unsubstituierten oder Alkyl-substituierten Arylgruppen substituiert ist,
- einer unsubstituierten oder mit einem oder mehreren $R^2$ und/ oder mit einer oder mehreren unsubstituierten Alkylgruppe und/oder mit einer oder mehreren unsubstituierten oder Alkyl-substituierten Arylgruppe substituierten Heteroarylgruppe und
- einer Gruppe der Unterformel T1 oder einer Gruppe der Unterformel T2:

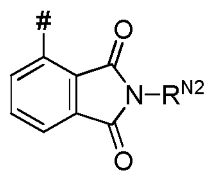

Unterformel T1

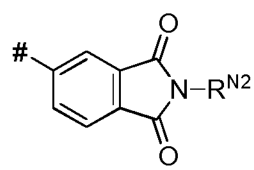

Unterformel T2

**2.** Organisches Molekül nach Anspruch 1, aufweisend eine Struktur der Formel A2

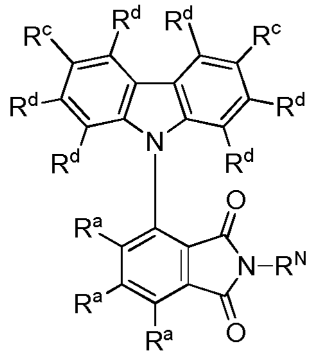

Formel A2

wobei

$R^c$ bei jedem Auftreten unabhängig voneinander ausgewählt ist aus der Gruppe bestehend aus:

- $CF_3$,
- $C(=O)R^1$,
- CN,
- einer mit einem oder mehreren $R^2$ substituierten Alkylgruppe,
- einer mit einem oder mehreren $R^2$ substituierten Arylgruppe die optional zusätzlich mit einer oder mehreren unsubstituierten Alkylgruppe und/oder mit einer oder mehreren unsubstituierten oder Alkyl-substituierten Arylgruppen substituiert ist,
- einer unsubstituierten oder mit einem oder mehreren $R^2$ und/oder mit einer oder mehreren unsubstituierten Alkylgruppe und/oder mit einer oder mehreren unsubstituierten oder Alkyl-substituierten Arylgruppe substituierten Heteroarylgruppe und
- einer Gruppe der Unterformel T1 oder einer Gruppe der Unterformel T2:

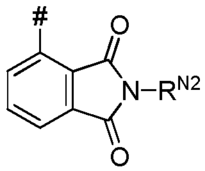

Unterformel T1

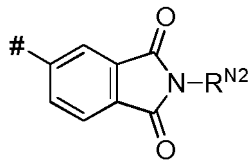

Unterformel T2

wobei $R^{N2}$ eine Alkylgruppe, eine unsubstituierte oder mit einem oder mehreren $R^{N3}$ substituierte Arylgruppe oder eine unsubstituierte oder mit einem oder mehreren $R^{N3}$ substituierte Heteroarylgruppe ist;

$R^d$ ist bei jedem Auftreten unabhängig voneinander ausgewählt aus der Gruppe bestehend aus:

- H,
- Deuterium,
- $CF_3$,
- $C(=O)R^1$,
- CN,
- einer unsubstituierten oder mit einem oder mehreren $R^2$ substituierten Alkylgruppe,
- einer unsubstituierten oder mit einem oder mehreren $R^2$ und/oder mit einer oder mehreren unsubstituierten Alkylgruppe und/oder mit einer oder mehreren unsubstituierten oder Alkyl-substituierten Arylgruppe substituierten Arylgruppe,
- einer unsubstituierten oder mit einem oder mehreren $R^2$ und/oder mit einer oder mehreren unsubstituierten Alkylgruppe und/oder mit einer oder mehreren unsubstituierten oder Alkyl-substituierten Arylgruppe substituierten Heteroarylgruppe, und
- einer Gruppe der Unterformel T1 oder einer Gruppe der Unterformel T2:

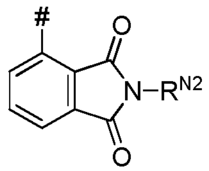
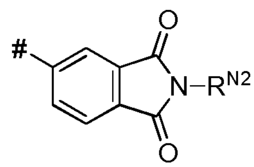

Unterformel T1 Unterformel T2

wobei $R^{N2}$ eine Alkylgruppe, eine unsubstituierte oder mit einem oder mehreren $R^{N3}$ substituierte Arylgruppe oder eine unsubstituierte oder mit einem oder mehreren $R^{N3}$ substituierte Heteroarylgruppe ist;

und im Übrigen die in Anspruch 1 angegebenen Definitionen gelten.

**3.** Organisches Molekül nach Anspruch 1, aufweisend eine Struktur der Formel A3

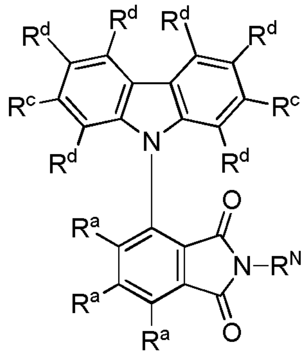

Formel A3

wobei

$R^c$ bei jedem Auftreten unabhängig voneinander ausgewählt ist aus der Gruppe bestehend aus:

- $CF_3$,
- $C(=O)R^1$,
- CN,
- einer mit einem oder mehreren $R^2$ substituierten Alkylgruppe,
- einer mit einem oder mehreren $R^2$ substituierten Arylgruppe, die optional zusätzlich mit einer oder mehreren unsubstituierten Alkylgruppe und/oder mit einer oder mehreren unsubstituierten oder Alkyl-substituierten Arylgruppen substituiert ist,
- einer unsubstituierten oder mit einem oder mehreren $R^2$ und/oder mit einer oder mehreren unsubstituierten Alkylgruppe und/oder mit einer oder mehreren unsubstituierten oder Alkyl-substituierten Arylgruppe substituierten Heteroarylgruppe und
- einer Gruppe der Unterformel T1 oder einer Gruppe der Unterformel T2:

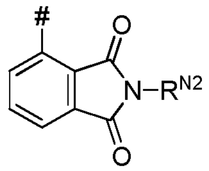
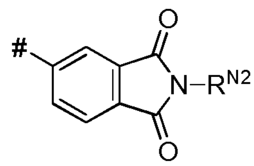

Unterformel T1 Unterformel T2

wobei $R^{N2}$ eine Alkylgruppe, eine unsubstituierte oder mit einem oder mehreren $R^{N3}$ substituierte Arylgruppe oder eine unsubstituierte oder mit einem oder mehreren $R^{N3}$ substituierte Heteroarylgruppe ist;

$R^d$ ist bei jedem Auftreten gleich oder verschieden unabhängig voneinander ausgewählt aus der Gruppe bestehend aus:

- H,
- Deuterium,
- $CF_3$,
- $C(=O)R^1$,
- CN,
- einer unsubstituierten oder mit einem oder mehreren $R^2$ substituierten Alkylgruppe,
- einer unsubstituierten oder mit einem oder mehreren $R^2$ und/oder mit einer oder mehreren unsubstituierten Alkylgruppe und/oder mit einer oder mehreren unsubstituierten oder Alkyl-substituierten Arylgruppe substituierten Arylgruppe,
- einer unsubstituierten oder mit $R^2$ und/oder mit einer oder mehreren unsubstituierten Alkylgruppe und/oder mit einer oder mehreren unsubstituierten oder Alkyl-substituierten Arylgruppe substituierten Heteroarylgruppe und
- einer Gruppe der Unterformel T1 oder einer Gruppe der Unterformel T2:

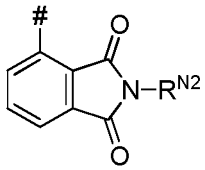

Unterformel T1

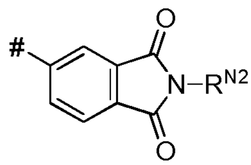

Unterformel T2

wobei $R^{N2}$ eine Alkylgruppe, eine unsubstituierte oder mit einem oder mehreren $R^{N3}$ substituierte Arylgruppe oder eine unsubstituierte oder mit einem oder mehreren $R^{N3}$ substituierte Heteroarylgruppe ist;

und im Übrigen die in Anspruch 1 angegebenen Definitionen gelten.

**4.** Organisches Molekül nach Anspruch 1, aufweisend eine Struktur der Formel A4

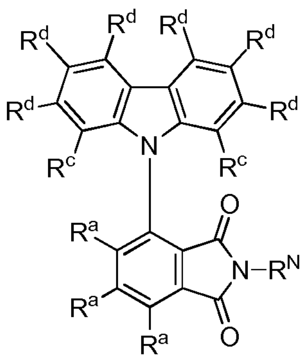

Formel A4

wobei

$R^c$ bei jedem Auftreten unabhängig voneinander ausgewählt ist aus der Gruppe bestehend aus:

- $CF_3$,
- $C(=O)R^1$,
- CN,
- einer mit einem oder mehreren $R^2$ substituierten Alkylgruppe,
- einer mit einem oder mehreren $R^2$ substituierten Arylgruppe, die optional zusätzlich mit einer oder mehreren unsubstituierten Alkylgruppe und/oder mit einer oder mehreren unsubstituierten oder Alkyl-substituierten Arylgruppen substituiert ist,

- einer unsubstituierten oder mit einem oder mehreren $R^2$ und/oder mit einer oder mehreren unsubstituierten Alkylgruppe und/oder mit einer oder mehreren unsubstituierten oder Alkyl-substituierten Arylgruppe substituierten Heteroarylgruppe und
- einer Gruppe der Unterformel T1 oder einer Gruppe der Unterformel T2:

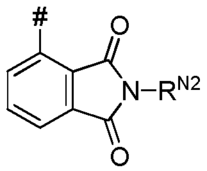
Unterformel T1

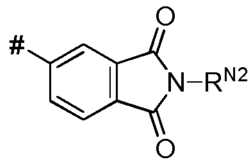
Unterformel T2

wobei $R^{N2}$ eine Alkylgruppe, eine unsubstituierte oder mit einem oder mehreren $R^{N3}$ substituierte Arylgruppe oder eine unsubstituierte oder mit einem oder mehreren $R^{N3}$ substituierte Heteroarylgruppe ist;

$R^d$ ist bei jedem Auftreten gleich oder verschieden unabhängig voneinander ausgewählt aus der Gruppe bestehend aus:

- H,
- Deuterium,
- $CF_3$,
- $C(=O)R^1$,
- CN,
- einer unsubstituierten oder mit einem oder mehreren $R^2$ substituierten Alkylgruppe,
- einer unsubstituierten oder mit einem oder mehreren $R^2$ und/oder mit einer oder mehreren unsubstituierten Alkylgruppe und/oder mit einer oder mehreren unsubstituierten oder Alkyl-substituierten Arylgruppe substituierten Arylgruppe,
- einer unsubstituierten oder mit $R^2$ und/oder mit einer oder mehreren unsubstituierten Alkylgruppe und/oder mit einer oder mehreren unsubstituierten oder Alkyl-substituierten Arylgruppe substituierten Heteroarylgruppe und
- einer Gruppe der Unterformel T1 oder einer Gruppe der Unterformel T2:

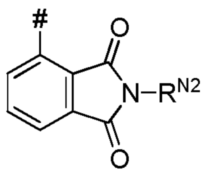
Unterformel T1

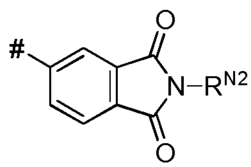
Unterformel T2

wobei $R^{N2}$ eine Alkylgruppe, eine unsubstituierte oder mit einem oder mehreren $R^{N3}$ substituierte Arylgruppe oder eine unsubstituierte oder mit einem oder mehreren $R^{N3}$ substituierte Heteroarylgruppe ist;

und im Übrigen die in Anspruch 1 angegebenen Definitionen gelten.

**5.** Organisches Molekül nach Anspruch 1 bis 4, wobei das Molekül mindestens eine CN-Gruppe aufweist.

**6.** Verwendung eines organischen Moleküls nach Anspruch 1 bis 5 als Emitter und/oder Host.

**7.** Zusammensetzung, aufweisend oder bestehend aus:

(a) mindestens einem organischen Molekül nach einem der Ansprüche 1 bis 5, insbesondere als Emitter und/oder Host, und
(b) einer oder mehrerer von dem Molekül nach einem der Ansprüche 1 bis 5 verschiedenen Emitter- und/oder Hostmaterialien und

(c) optional einem oder mehreren Farbstoffen und/oder einem oder mehreren Lösungsmitteln.

**8.** Optoelektronisches Bauelement, aufweisend ein organisches Molekül nach Anspruch 1 bis 5 oder eine Zusammensetzung nach Anspruch 7.

**9.** Optoelektronisches Bauelement nach Anspruch 8, aufweisend

- ein Substrat,
- eine Anode und
- eine Kathode, wobei die Anode oder die Kathode auf das Substrat aufgebracht sind, und
- mindestens eine lichtemittierende Schicht, die zwischen Anode und Kathode angeordnet ist und die das organisches Molekül nach Anspruch 1 bis 5 oder eine Zusammensetzung nach Anspruch 7 aufweist.

**10.** Verfahren zur Herstellung eines optoelektronischen Bauelements, wobei ein organisches Molekül nach Anspruch 1 bis 5 verwendet wird.

**11.** Verfahren nach Anspruch 10, wobei das organische Molekül durch ein Verdampfungsverfahren oder aus einer Lösung aufgebracht wird.

## Claims

**1.** Organic molecule comprising a structure of formula A1

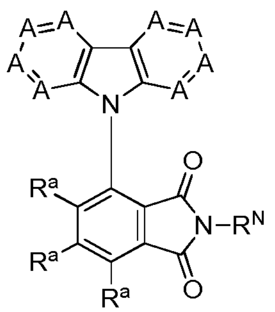

Formula A1

wherein

A at each instance is independently $CR^b$ or N;
$R^N$ is methyl, phenyl, xylyl, mesityl, naphthyl, biphenyl, naphthylphenyl, terphenyl or 2,4,6-triphenylphenyl;
$R^a$ at each instance is independently H, deuterium, an alkyl group or an aryl group;

$R^b$ at each instance is independently selected from the group consisting of:

- H,
- deuterium,
- $CF_3$,
- $C(=O)R^1$,
- CN,
- an alkyl group which is unsubstituted or substituted by one or more $R^2$,
- an aryl group which is unsubstituted or substituted by one or more $R^2$ and is optionally additionally substituted by one or more unsubstituted alkyl groups and/or by one or more unsubstituted or alkyl-substituted aryl groups,
- a heteroaryl group which is unsubstituted or substituted by $R^2$ and/or by one or more unsubstituted alkyl groups and/or by one or more unsubstituted or alkyl-substituted aryl groups and
- a group of the sub-formula T1 or a group of the sub-formula T2:

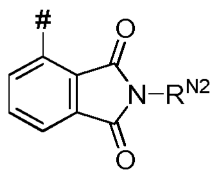

Sub-formula T1

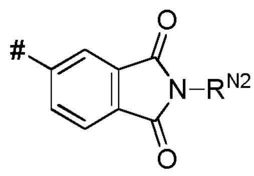

Sub-formula T2

wherein:

$R^{N2}$ is an alkyl group, an aryl group which is unsubstituted or substituted by one or more $R^{N3}$ or a heteroaryl group which is unsubstituted or substituted by one or more $R^{N3}$;

$R^{N3}$ is an alkyl group, an aryl group or a heteroaryl group;
$R^1$ at each instance is an aryl group which is unsubstituted or substituted by one or more $R^2$;
$R^2$ at each instance is independently F, $CF_3$ or CN;

wherein one to four A are N or at least one $R^b$ is selected from the group consisting of:

- $CF_3$,
- $C(=O)R^1$,
- CN,
- an alkyl group substituted by one or more $R^2$,
- an aryl group substituted by one or more $R^2$, and optionally additionally substituted by one or more unsubstituted alkyl groups and/or by one or more unsubstituted or alkyl-substituted aryl groups,
- a heteroaryl group which is unsubstituted or substituted by one or more $R^2$ and/or by one or more unsubstituted alkyl groups and/or by one or more unsubstituted or alkyl-substituted aryl groups and
- a group of the sub-formula T1 or a group of the sub-formula T2:

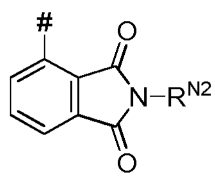

Sub-formula T1

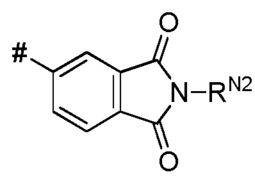

Sub-formula T2

**2.** Organic molecule according to Claim 1, comprising a structure of formula A2

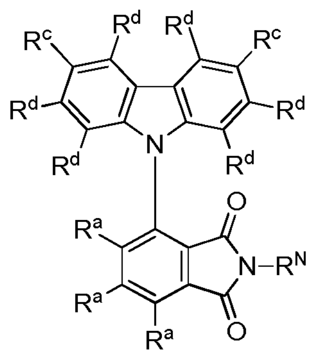

Formula A2

wherein

$R^c$ at each instance is independently selected from the group consisting of:

- $CF_3$,
- $C(=O)R^1$,
- CN,
- an alkyl group substituted by one or more $R^2$,
- an aryl group substituted by one or more $R^2$, and optionally additionally substituted by one or more unsubstituted alkyl groups and/or by one or more unsubstituted or alkyl-substituted aryl groups,
- a heteroaryl group which is unsubstituted or substituted by one or more $R^2$ and/or by one or more unsubstituted alkyl groups and/or by one or more unsubstituted or alkyl-substituted aryl groups and
- a group of the sub-formula T1 or a group of the sub-formula T2:

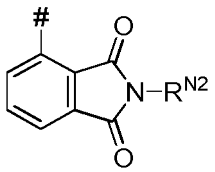

Sub-formula T1

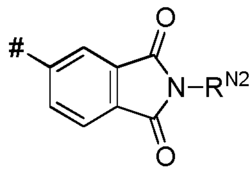

Sub-formula T2

wherein $R^{N2}$ is an alkyl group, an aryl group which is unsubstituted or substituted by one or more $R^{N3}$ or a heteroaryl group which is unsubstituted or substituted by one or more $R^{N3}$;

$R^d$ at each instance is independently selected from the group consisting of:

- H,
- deuterium,
- $CF_3$,
- $C(=O)R^1$,
- CN,
- an alkyl group which is unsubstituted or substituted by one or more $R^2$,
- an aryl group which is unsubstituted or substituted by one or more $R^2$ and/or by one or more unsubstituted alkyl groups and/or by one or more unsubstituted or alkyl-substituted aryl groups,
- a heteroaryl group which is unsubstituted or substituted by one or more $R^2$ and/or by one or more unsubstituted alkyl groups and/or by one or more unsubstituted or alkyl-substituted aryl groups, and
- a group of the sub-formula T1 or a group of the sub-formula T2:

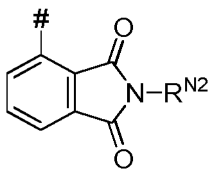

Sub-formula T1

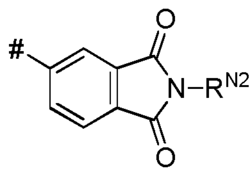

Sub-formula T2

wherein $R^{N2}$ is an alkyl group, an aryl group which is unsubstituted or substituted by one or more $R^{N3}$ or a heteroaryl group which is unsubstituted or substituted by one or more $R^{N3}$;

and for the rest the definitions given in Claim 1 are applicable.

**3.** Organic molecule according to Claim 1, comprising a structure of formula A3

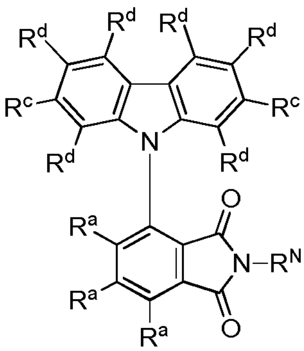

Formula A3

wherein

$R^c$ at each instance is independently selected from the group consisting of:

- $CF_3$,
- $C(=O)R^1$,
- CN,
- an alkyl group substituted by one or more $R^2$,
- an aryl group substituted by one or more $R^2$, and optionally additionally substituted by one or more unsubstituted alkyl groups and/or by one or more unsubstituted or alkyl-substituted aryl groups,
- a heteroaryl group which is unsubstituted or substituted by one or more $R^2$ and/or by one or more unsubstituted alkyl groups and/or by one or more unsubstituted or alkyl-substituted aryl groups and
- a group of the sub-formula T1 or a group of the sub-formula T2:

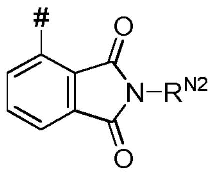

Sub-formula T1

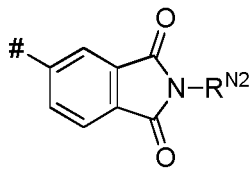

Sub-formula T2

wherein $R^{N2}$ is an alkyl group, an aryl group which is unsubstituted or substituted by one or more $R^{N3}$ or a heteroaryl group which is unsubstituted or substituted by one or more $R^{N3}$;

$R^d$ is the same or different at each instance and is independently selected from the group consisting of:

- H,
- deuterium,
- $CF_3$,
- $C(=O)R^1$,
- CN,
- an alkyl group which is unsubstituted or substituted by one or more $R^2$,
- an aryl group which is unsubstituted or substituted by one or more $R^2$ and/or by one or more unsubstituted alkyl groups and/or by one or more unsubstituted or alkyl-substituted aryl groups,
- a heteroaryl group which is unsubstituted or substituted by $R^2$ and/or by one or more unsubstituted alkyl groups and/or by one or more unsubstituted or alkyl-substituted aryl groups and
- a group of the sub-formula T1 or a group of the sub-formula T2:

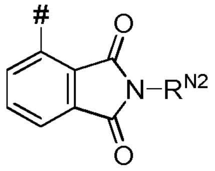

Sub-formula T1

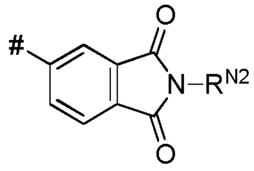

Sub-formula T2

wherein $R^{N2}$ is an alkyl group, an aryl group which is unsubstituted or substituted by one or more $R^{N3}$ or a heteroaryl group which is unsubstituted or substituted by one or more $R^{N3}$;

and for the rest the definitions given in Claim 1 are applicable.

**4.** Organic molecule according to Claim 1, comprising a structure of formula A4

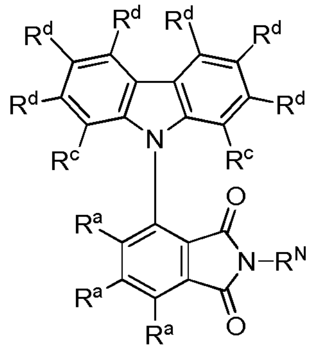

Formula A4

wherein

$R^c$ at each instance is independently selected from the group consisting of:

- $CF_3$,
- $C(=O)R^1$,
- CN,
- an alkyl group substituted by one or more $R^2$,
- an aryl group substituted by one or more $R^2$, and optionally additionally substituted by one or more unsubstituted alkyl groups and/or by one or more unsubstituted or alkyl-substituted aryl groups,
- a heteroaryl group which is unsubstituted or substituted by one or more $R^2$ and/or by one or more unsubstituted alkyl groups and/or by one or more unsubstituted or alkyl-substituted aryl groups and
- a group of the sub-formula T1 or a group of the sub-formula T2:

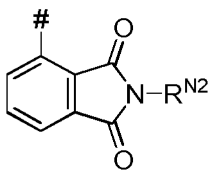

Sub-formula T1

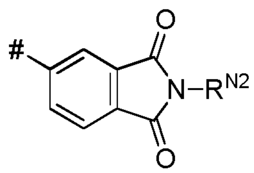

Sub-formula T2

wherein $R^{N2}$ is an alkyl group, an aryl group which is unsubstituted or substituted by one or more $R^{N3}$ or a heteroaryl group which is unsubstituted or substituted by one or more $R^{N3}$;

$R^d$ is the same or different at each instance and is independently selected from the group consisting of:

- H,
- deuterium,
- $CF_3$,
- $C(=O)R^1$,
- CN,
- an alkyl group which is unsubstituted or substituted by one or more $R^2$,
- an aryl group which is unsubstituted or substituted by one or more $R^2$ and/or by one or more unsubstituted alkyl groups and/or by one or more unsubstituted or alkyl-substituted aryl groups,
- a heteroaryl group which is unsubstituted or substituted by $R^2$ and/or by one or more unsubstituted alkyl groups and/or by one or more unsubstituted or alkyl-substituted aryl groups and
- a group of the sub-formula T1 or a group of the sub-formula T2:

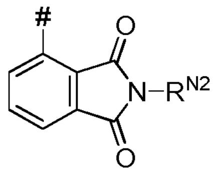

Sub-formula T1

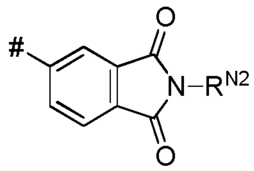

Sub-formula T2

wherein $R^{N2}$ is an alkyl group, an aryl group which is unsubstituted or substituted by one or more $R^{N3}$ or a heteroaryl group which is unsubstituted or substituted by one or more $R^{N3}$;

and for the rest the definitions given in Claim 1 are applicable.

**5.** Organic molecule according to Claims 1 to 4, wherein the molecule comprises at least one CN group.

**6.** Use of an organic molecule according to Claims 1 to 5 as an emitter and/or a host.

**7.** Composition, comprising or consisting of:

(a) at least one organic molecule according to any of Claims 1 to 5, in particular as emitter and/or host, and
(b) one or more emitter and/or host materials other than the molecule according to any of Claims 1 to 5, and
(c) optionally, one or more dyes and/or one or more solvents.

**8.** Optoelectronic component comprising an organic molecule according to Claims 1 to 5 or a composition according to Claim 7.

**9.** Optoelectronic component according to Claim 8, comprising

- a substrate,
- an anode and
- a cathode, wherein the anode or cathode has been applied to the substrate, and
- at least one light-emitting layer which is arranged between the anode and the cathode and comprises the organic molecule according to Claims 1 to 5 or a composition according to Claim 7.

**10.** Method for producing an optoelectronic component, wherein an organic molecule according to Claims 1 to 5 is used.

**11.** Method according to Claim 10, wherein the organic molecule is applied by an evaporation process or from a solution.

**Revendications**

**1.** Molécule organique, présentant une structure de formule A1

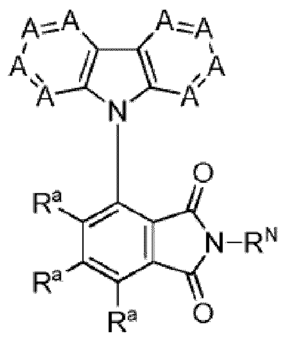

Formule A1

où

A est à chaque occurrence, chaque fois indépendamment, $CR^b$ ou N ;
$R^N$ est un groupe méthyle, phényle, xylyle, mésityle, naphtyle, biphényle, naphtylphényle, terphényle ou 2,4,6-triphénylphényle ;
$R^a$ est à chaque occurrence, chaque fois indépendamment, H, un atome de deutérium, un groupe alkyle ou un groupe aryle ;

$R^b$ est choisi à chaque occurrence, chaque fois indépendamment, dans le groupe constitué par :

- H,
- un atome de deutérium,
- $CF_3$,
- $C(=O)R^1$,
- CN,
- un groupe alkyle non substitué ou substitué par un ou plusieurs substituants $R^2$,
- un groupe aryle non substitué ou substitué par un ou plusieurs substituants $R^2$, qui est éventuellement substitué en outre par un ou plusieurs groupes alkyle non substitués et/ou par un ou plusieurs groupes aryle non substitués ou substitués par alkyle,
- un groupe hétéroaryle non substitué ou substitué par $R^2$ et/ou par un ou plusieurs groupes alkyle non substitués et/ou par un ou plusieurs groupes aryle non substitués ou substitués par alkyle et
- un groupe de sous-formule T1 ou un groupe de sous-formule T2 :

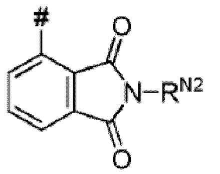

Sous-formule T1

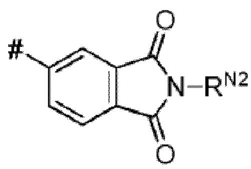

Sous-formule T2

où :

$R^{N2}$ est un groupe alkyle, un groupe aryle non substitué ou substitué par un ou plusieurs substituants $R^{N3}$ ou un groupe hétéroaryle non substitué ou substitué par un ou plusieurs substituants $R^{N3}$ ;

$R^{N3}$ est un groupe alkyle, un groupe aryle ou un groupe hétéroaryle ;
$R^1$ est à chaque occurrence un groupe aryle non substitué ou substitué par un ou plusieurs substituants $R^2$ ;
$R^2$ est à chaque occurrence, chaque fois indépendamment, F, $CF_3$ ou CN ;

un à quatre A représentant N ou au moins un $R^b$ étant choisi dans le groupe constitué par :

- $CF_3$,
- $C(=O)R^1$,
- CN,
- un groupe alkyle substitué par un ou plusieurs substituants $R^2$,
- un groupe aryle substitué par un ou plusieurs substituants $R^2$, qui est éventuellement substitué en outre par un ou plusieurs groupes alkyle non substitués et/ou par un ou plusieurs groupes aryle non substitués ou substitués par alkyle,
- un groupe hétéroaryle non substitué ou substitué par un ou plusieurs substituants $R^2$ et/ou par un ou plusieurs groupes alkyle non substitués et/ou par un ou plusieurs groupes aryle non substitués ou substitués par alkyle et
- un groupe de sous-formule T1 ou un groupe de sous-formule T2 :

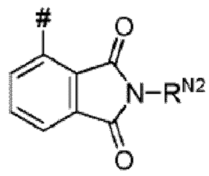

Sous-formule T1

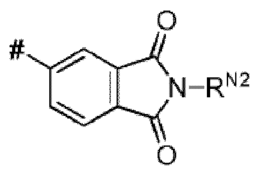

Sous-formule T2.

**2.** Molécule organique selon la revendication 1, présentant une structure de formule A2

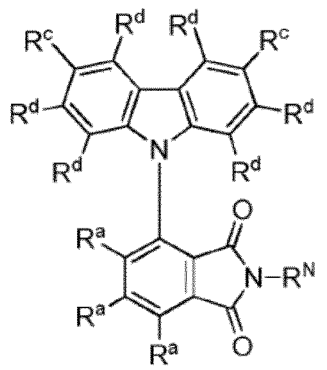

Formule A2

où

$R^c$ est choisi à chaque occurrence, chaque fois indépendamment, dans le groupe constitué par :

- $CF_3$,
- $C(=O)R^1$,
- CN,
- un groupe alkyle substitué par un ou plusieurs substituants $R^2$,
- un groupe aryle substitué par un ou plusieurs substituants $R^2$, qui est éventuellement substitué en outre par un ou plusieurs groupes alkyle non substitués et/ou par un ou plusieurs groupes aryle non substitués ou substitués par alkyle,
- un groupe hétéroaryle non substitué ou substitué par un ou plusieurs substituants $R^2$ et/ou par un ou plusieurs groupes alkyle non substitués et/ou par un ou plusieurs groupes aryle non substitués ou substitués par alkyle et
- un groupe de sous-formule T1 ou un groupe de sous-formule T2 :

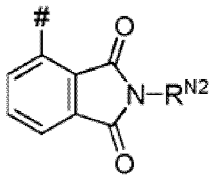 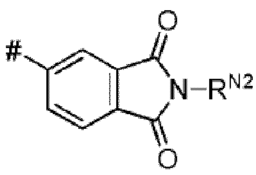

Sous-formule T1 Sous-formule T2

où $R^{N2}$ est un groupe alkyle, un groupe aryle non substitué ou substitué par un ou plusieurs substituants $R^{N3}$ ou un groupe hétéroaryle non substitué ou substitué par un ou plusieurs substituants $R^{N3}$ ;

$R^d$ est choisi à chaque occurrence, chaque fois indépendamment, dans le groupe constitué par :

- H,
- un atome de deutérium,
- $CF_3$,
- $C(=O)R^1$,
- CN,
- un groupe alkyle non substitué ou substitué par un ou plusieurs substituants $R^2$,
- un groupe aryle non substitué ou substitué par un ou plusieurs substituants $R^2$ et/ou par un ou plusieurs groupes alkyle non substitués et/ou par un ou plusieurs groupes aryle non substitués ou substitués par alkyle,
- un groupe hétéroaryle non substitué ou substitué par un ou plusieurs substituants $R^2$ et/ou par un ou plusieurs groupes alkyle non substitués et/ou par un ou plusieurs groupes aryle non substitués ou substitués par alkyle et
- un groupe de sous-formule T1 ou un groupe de sous-formule T2 :

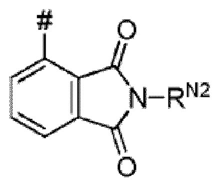 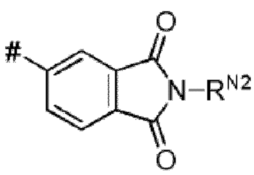

Sous-formule T1 Sous-formule T2

où $R^{N2}$ est un groupe alkyle, un groupe aryle non substitué ou substitué par un ou plusieurs substituants $R^{N3}$ ou un groupe hétéroaryle non substitué ou substitué par un ou plusieurs substituants $R^{N3}$ ;

et pour le reste s'appliquent les définitions indiquées dans la revendication 1.

**3.** Molécule organique selon la revendication 1, présentant une structure de formule A3

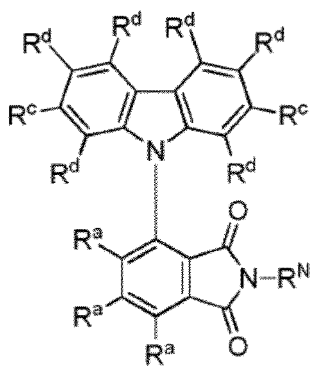

Formule A3

où

$R^c$ est choisi à chaque occurrence, chaque fois indépendamment, dans le groupe constitué par :

- $CF_3$,
- $C(=O)R^1$,
- CN,
- un groupe alkyle substitué par un ou plusieurs substituants $R^2$,
- un groupe aryle substitué par un ou plusieurs substituants $R^2$, qui est éventuellement substitué en outre par un ou plusieurs groupes alkyle non substitués et/ou par un ou plusieurs groupes aryle non substitués ou substitués par alkyle,
- un groupe hétéroaryle non substitué ou substitué par un ou plusieurs substituants $R^2$ et/ou par un ou plusieurs groupes alkyle non substitués et/ou par un ou plusieurs groupes aryle non substitués ou substitués par alkyle et
- un groupe de sous-formule T1 ou un groupe de sous-formule T2 :

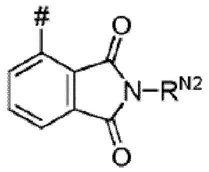

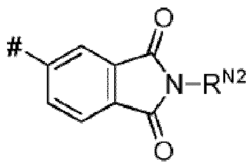

Sous-formule T1 Sous-formule T2

où $R^{N2}$ est un groupe alkyle, un groupe aryle non substitué ou substitué par un ou plusieurs substituants $R^{N3}$ ou un groupe hétéroaryle non substitué ou substitué par un ou plusieurs substituants $R^{N3}$ ;

$R^d$ est le même ou différent, choisi à chaque occurrence, chaque fois indépendamment, dans le groupe constitué par :

- H,
- un atome de deutérium,
- $CF_3$,
- $C(=O)R^1$,
- CN,
- un groupe alkyle non substitué ou substitué par un ou plusieurs substituants $R^2$,
- un groupe aryle non substitué ou substitué par un ou plusieurs substituants $R^2$ et/ou par un ou plusieurs groupes alkyle non substitués et/ou par un ou plusieurs groupes aryle non substitués ou substitués par alkyle,
- un groupe hétéroaryle non substitué ou substitué par $R^2$ et/ou par un ou plusieurs groupes alkyle non substitués et/ou par un ou plusieurs groupes aryle non substitués ou substitués par alkyle et
- un groupe de sous-formule T1 ou un groupe de sous-formule T2 :

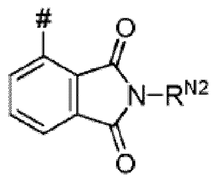

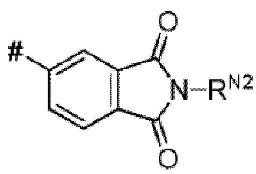

Sous-formule T1 Sous-formule T2

où $R^{N2}$ est un groupe alkyle, un groupe aryle non substitué ou substitué par un ou plusieurs substituants $R^{N3}$ ou un groupe hétéroaryle non substitué ou substitué par un ou plusieurs substituants $R^{N3}$ ;

et pour le reste s'appliquent les définitions indiquées dans la revendication 1.

**4.** Molécule organique selon la revendication 1, présentant une structure de formule A4

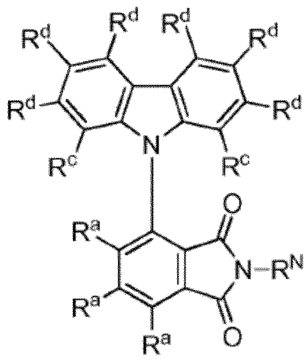

Formule A4

où

$R^c$ est choisi à chaque occurrence, chaque fois indépendamment, dans le groupe constitué par :

- $CF_3$,
- $C(=O)R^1$,
- CN,
- un groupe alkyle substitué par un ou plusieurs substituants $R^2$,
- un groupe aryle substitué par un ou plusieurs substituants $R^2$, qui est éventuellement substitué en outre par un ou plusieurs groupes alkyle non substitués et/ou par un ou plusieurs groupes aryle non substitués ou substitués par alkyle,
- un groupe hétéroaryle non substitué ou substitué par un ou plusieurs substituants $R^2$ et/ou par un ou plusieurs groupes alkyle non substitués et/ou par un ou plusieurs groupes aryle non substitués ou substitués par alkyle et
- un groupe de sous-formule T1 ou un groupe de sous-formule T2 :

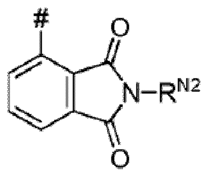

Sous-formule T1

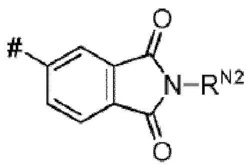

Sous-formule T2

où $R^{N2}$ est un groupe alkyle, un groupe aryle non substitué ou substitué par un ou plusieurs substituants $R^{N3}$ ou un groupe hétéroaryle non substitué ou substitué par un ou plusieurs substituants $R^{N3}$ ;

$R^d$ est le même ou différent, choisi à chaque occurrence, chaque fois indépendamment, dans le groupe constitué par :

- H,
- un atome de deutérium,
- $CF_3$,
- $C(=O)R^1$,
- CN,
- un groupe alkyle non substitué ou substitué par un ou plusieurs substituants $R^2$,
- un groupe aryle non substitué ou substitué par un ou plusieurs substituants $R^2$ et/ou par un ou plusieurs groupes alkyle non substitués et/ou par un ou plusieurs groupes aryle non substitués ou substitués par alkyle,
- un groupe hétéroaryle non substitué ou substitué par $R^2$ et/ou par un ou plusieurs groupes alkyle non substitués et/ou par un ou plusieurs groupes aryle non substitués ou substitués par alkyle et
- un groupe de sous-formule T1 ou un groupe de sous-formule T2 :

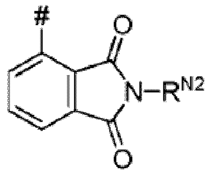 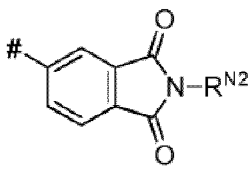

Sous-formule T1 Sous-formule T2

où $R^{N2}$ est un groupe alkyle, un groupe aryle non substitué ou substitué par un ou plusieurs substituants $R^{N3}$ ou un groupe hétéroaryle non substitué ou substitué par un ou plusieurs substituants $R^{N3}$ ;

et pour le reste s'appliquent les définitions indiquées dans la revendication 1.

**5.** Molécule organique selon l'une quelconque des revendications 1 à 4, où la molécule comporte au moins un groupe CN.

**6.** Utilisation d'une molécule organique selon l'une quelconque des revendications 1 à 5, en tant qu'émetteur et/ou hôte.

**7.** Composition comportant ou consistant en :

(a) au moins une molécule organique selon l'une quelconque des revendications 1 à 5, en particulier en tant qu'émetteur et/ou hôte, et
(b) un ou plusieurs matériaux émetteurs et/ou hôtes différents de la molécule selon l'une quelconque des revendications 1 à 5.
(c) éventuellement un ou plusieurs colorants et/ou un ou plusieurs solvants.

**8.** Composant optoélectronique, comportant une molécule organique selon l'une quelconque des revendications 1 à 5 ou une composition selon la revendication 7.

**9.** Composant optoélectronique selon la revendication 8, comportant

- un substrat,
- une anode et
- une cathode, l'anode ou la cathode étant appliquée sur le substrat, et
- au moins une couche électroluminescente, qui est disposée entre anode et cathode et qui comporte la molécule organique selon l'une quelconque des revendications 1 à 5 ou une composition selon la revendication 7.

**10.** Procédé pour la fabrication d'un composant optoélectronique, dans lequel on utilise une molécule organique selon l'une quelconque des revendications 1 à 5.

**11.** Procédé selon la revendication 10, dans lequel on applique la molécule organique par un procédé de vaporisation ou à partir d'une solution.

Figur 1

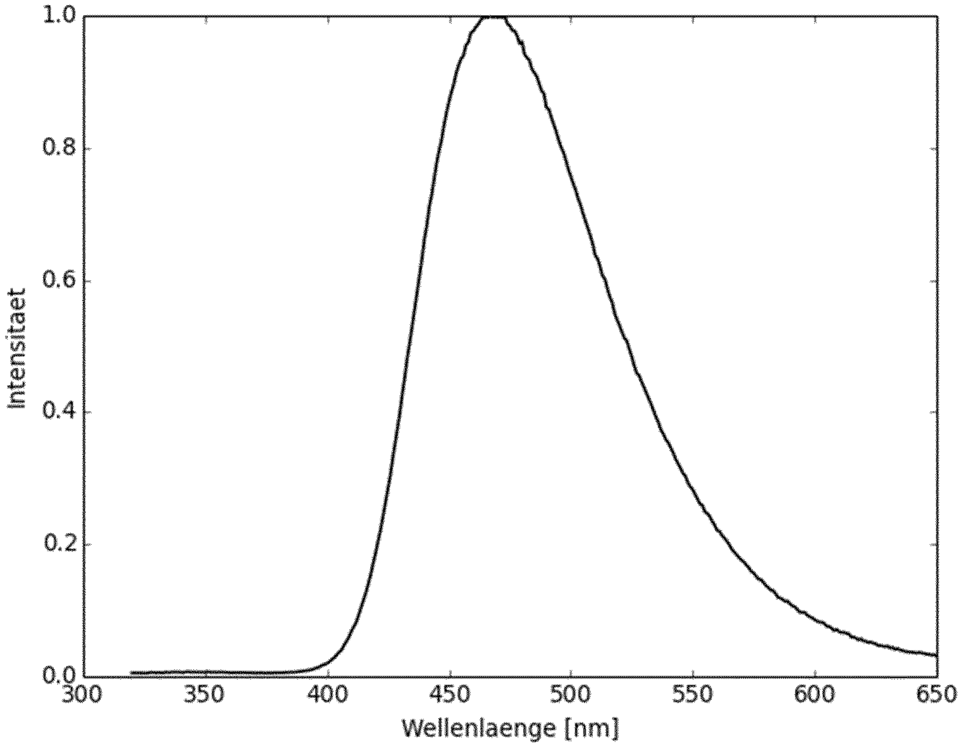


Figur 2

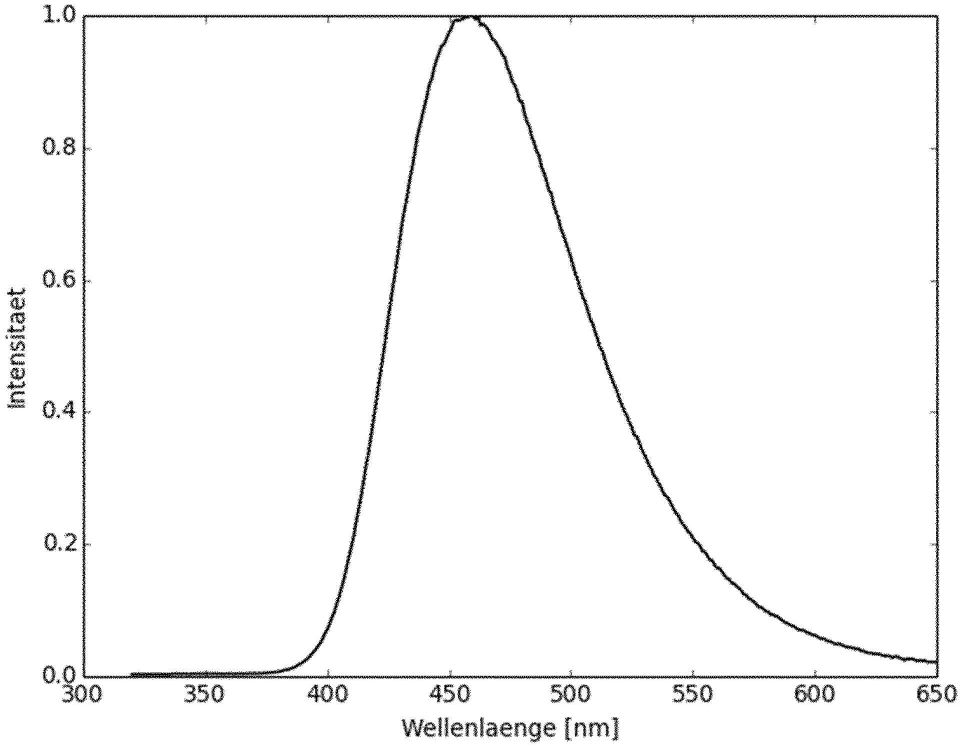

Figur 3

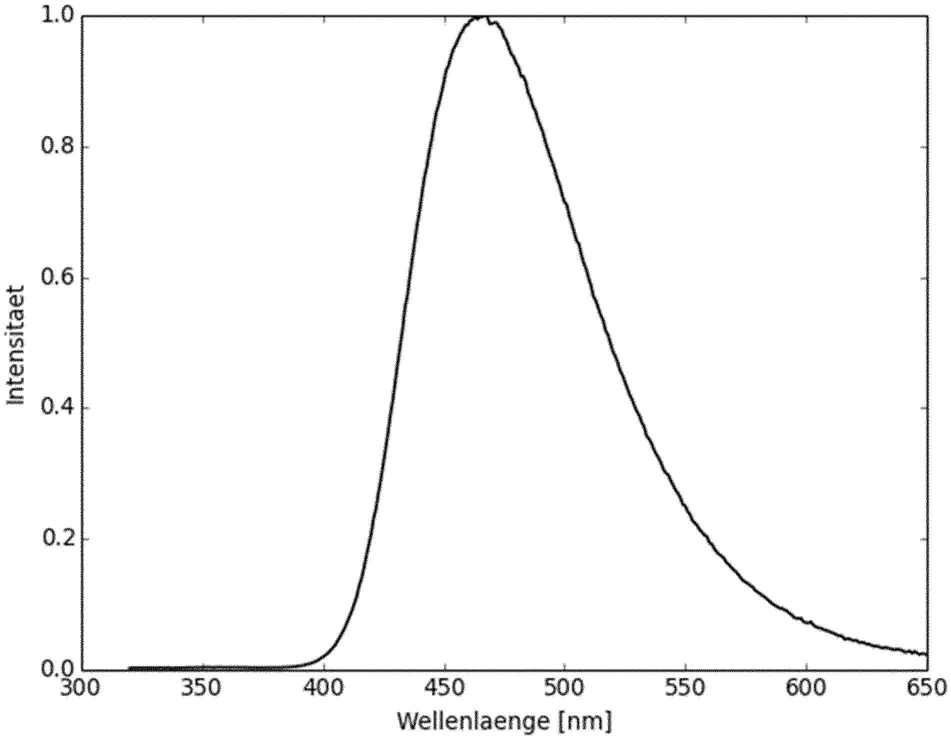


Figur 4

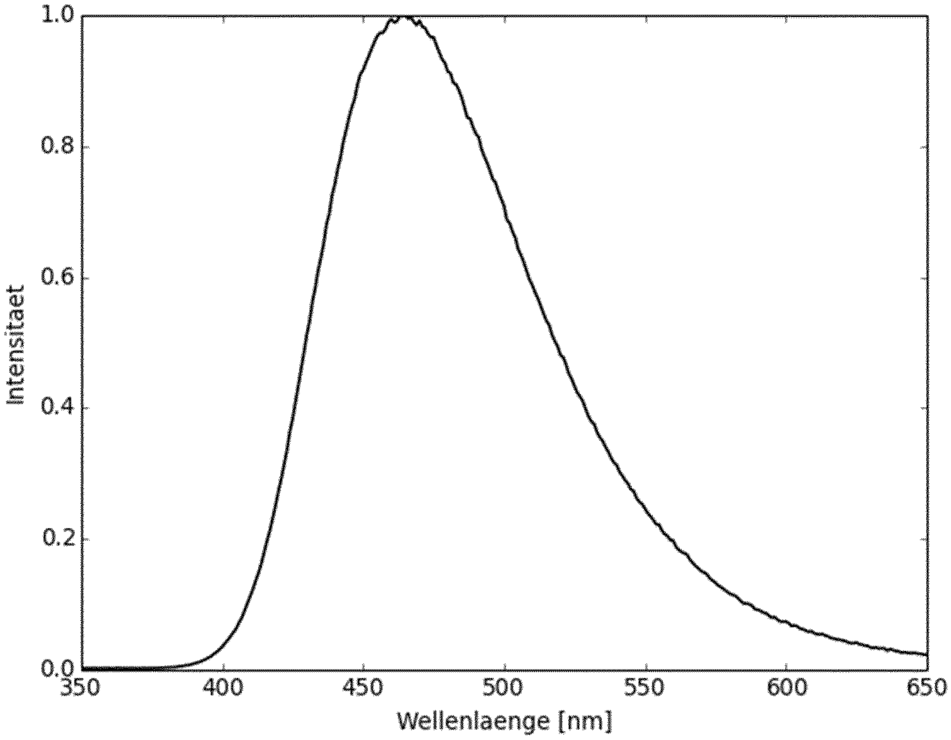

Figur 5

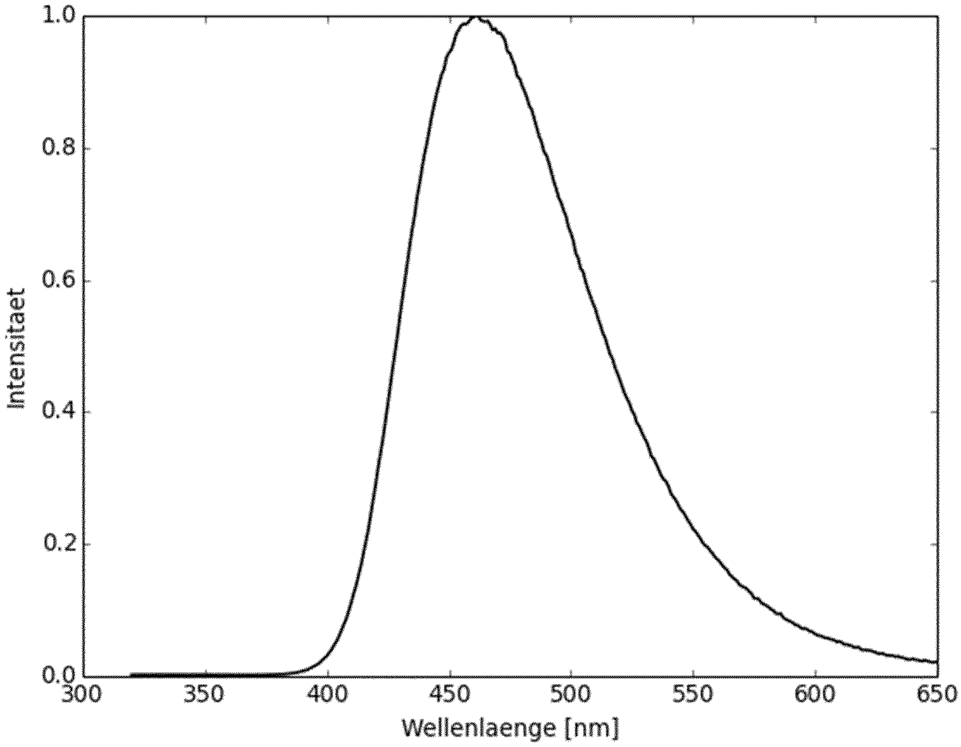


Figur 6

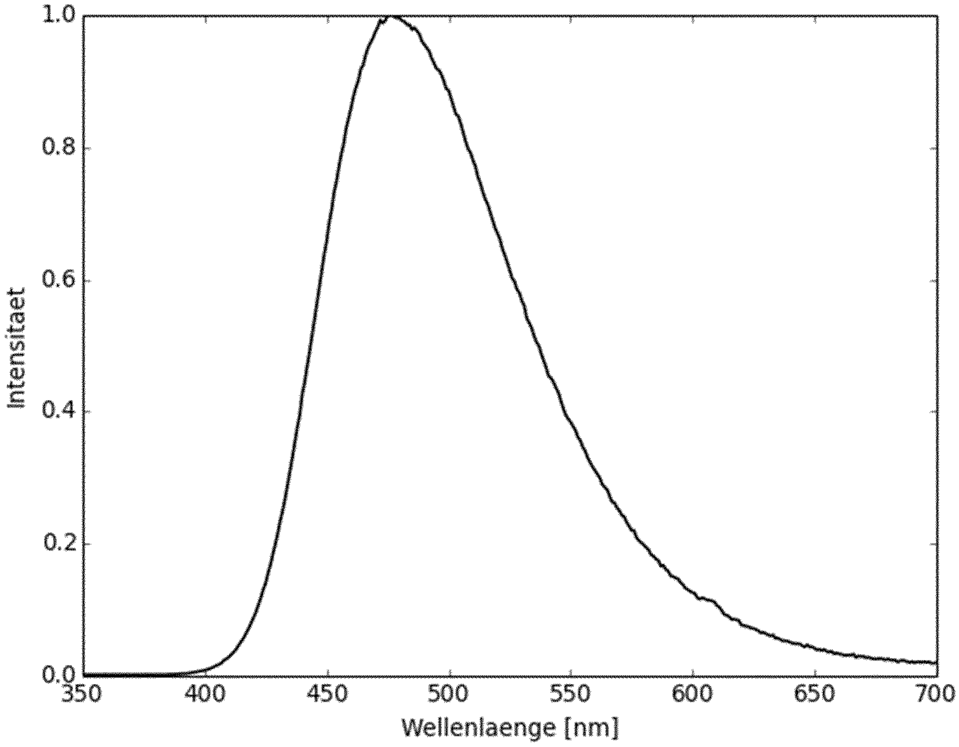

**Figur 7**

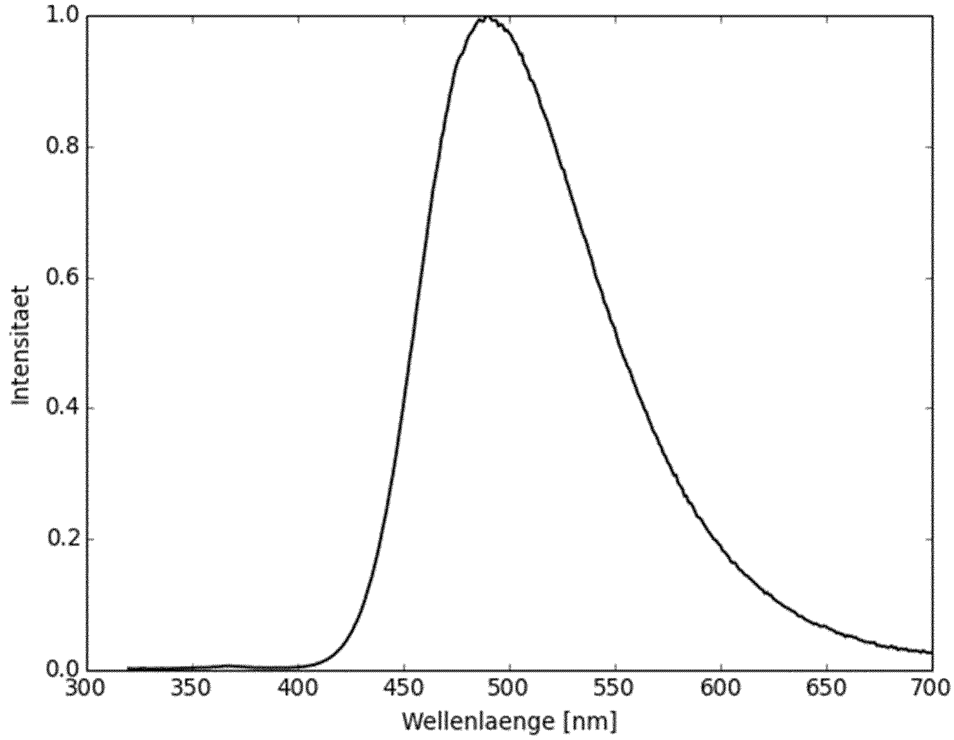


**Figur 8**

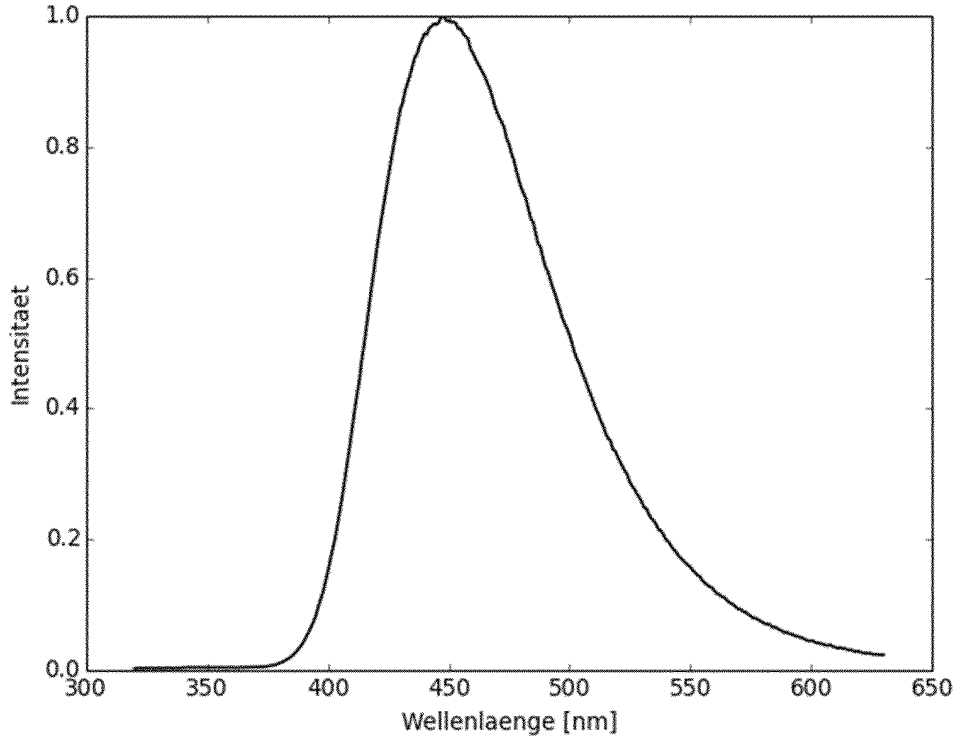

Figur 9

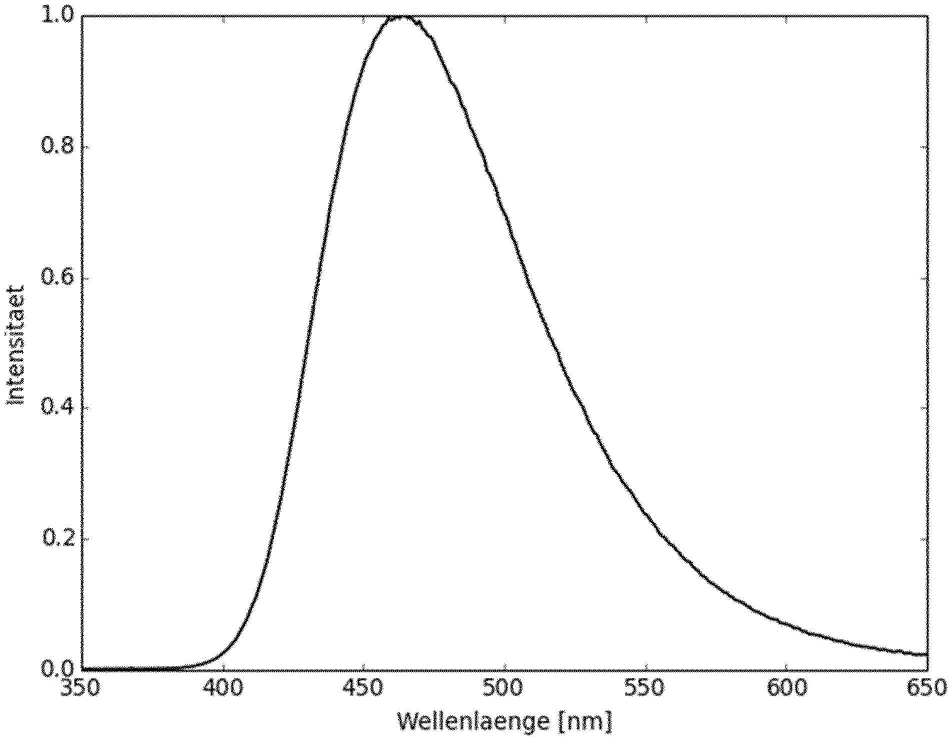


Figur 10

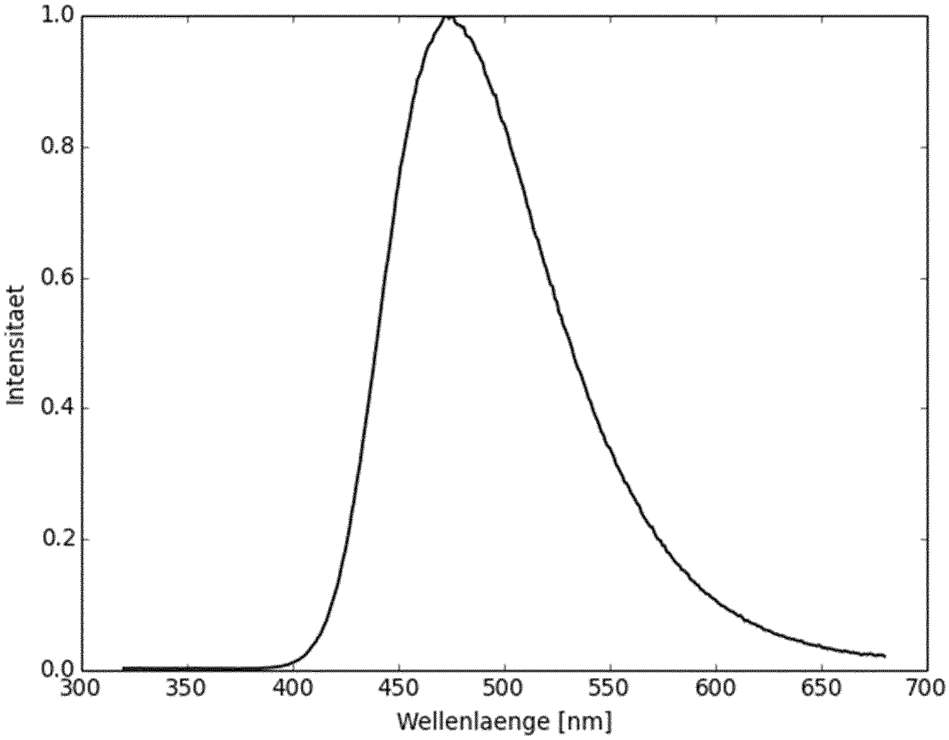

Figur 11

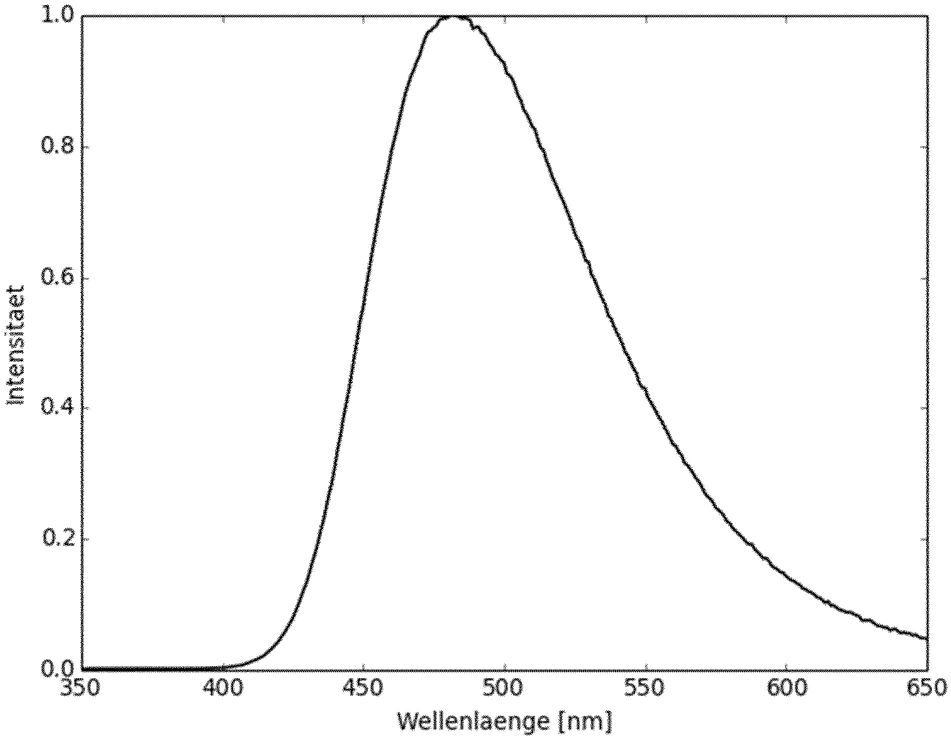


Figur 12

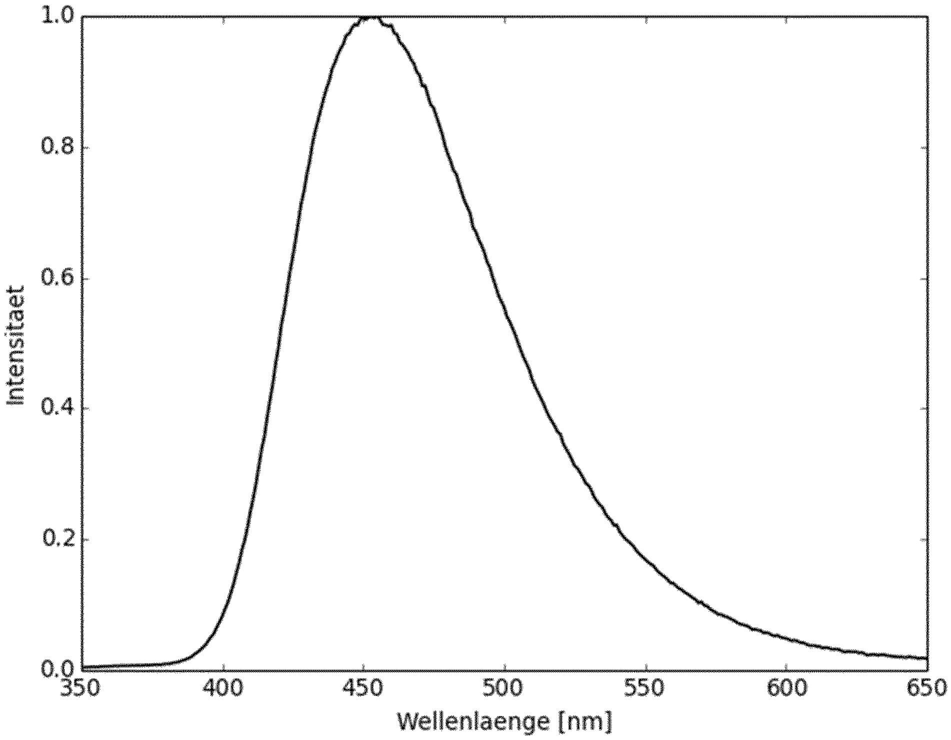

Figur 13

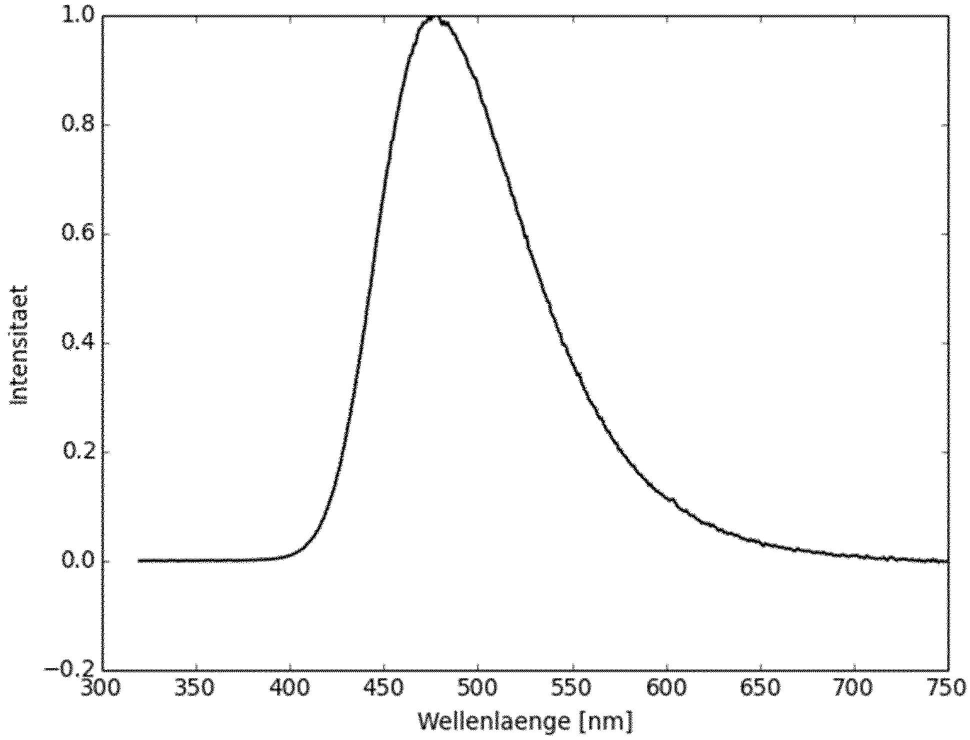


Figur 14

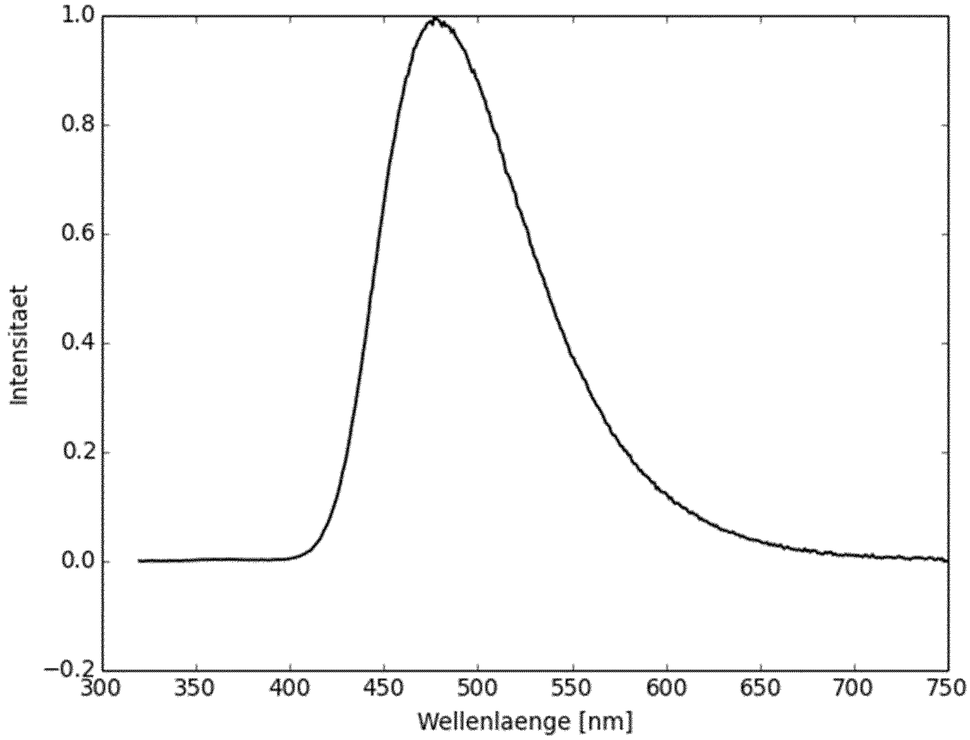

Figur 15

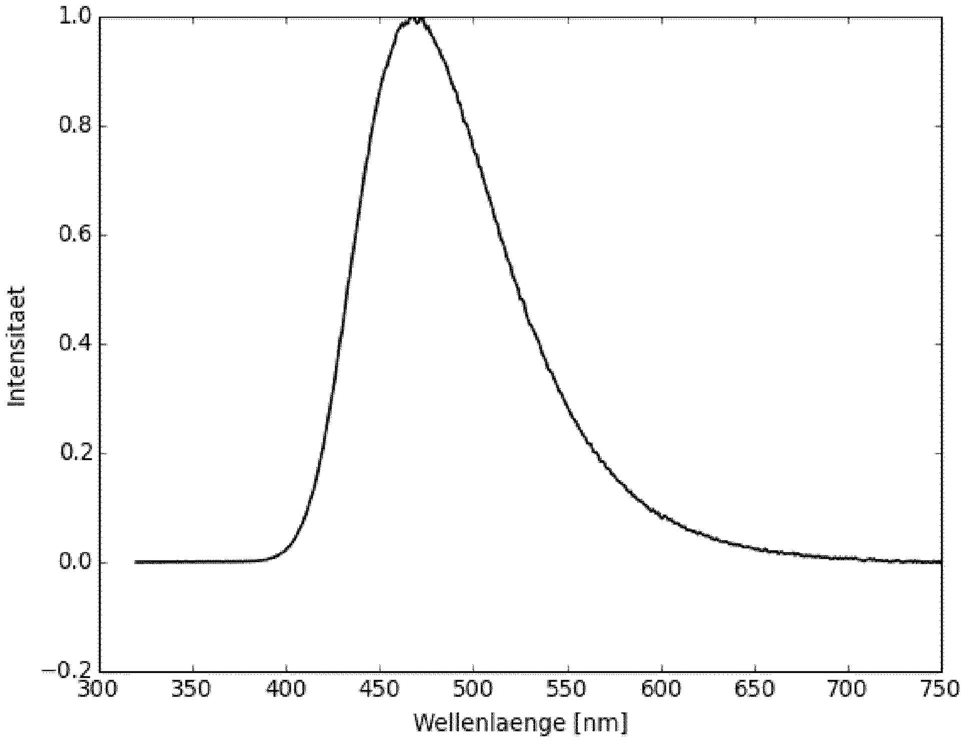


Figur 16

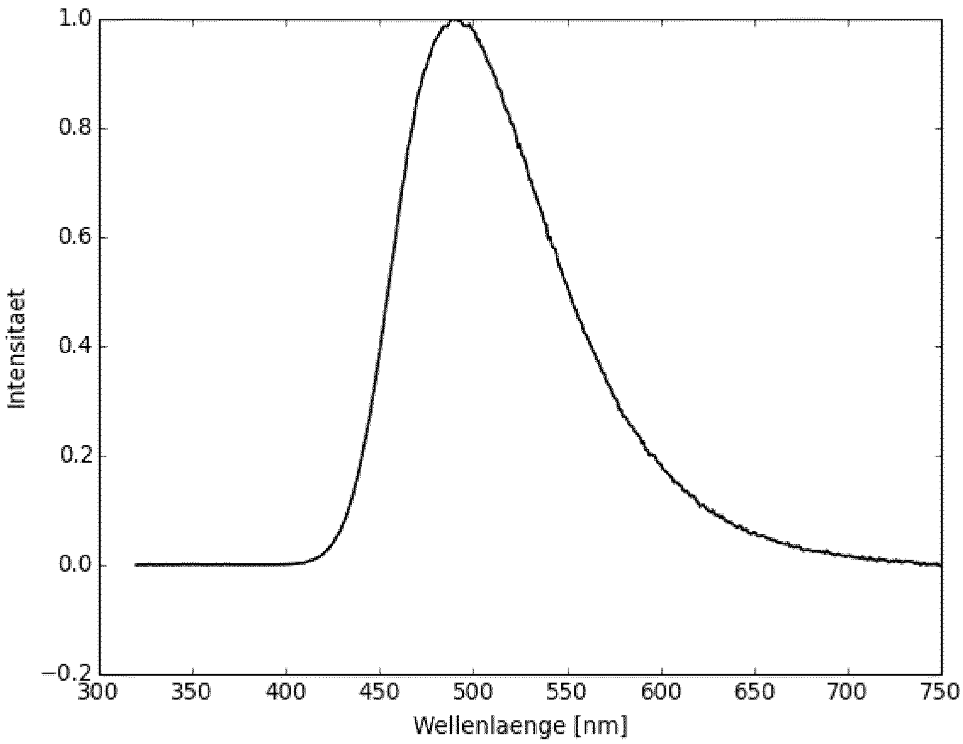

**Figur 17**

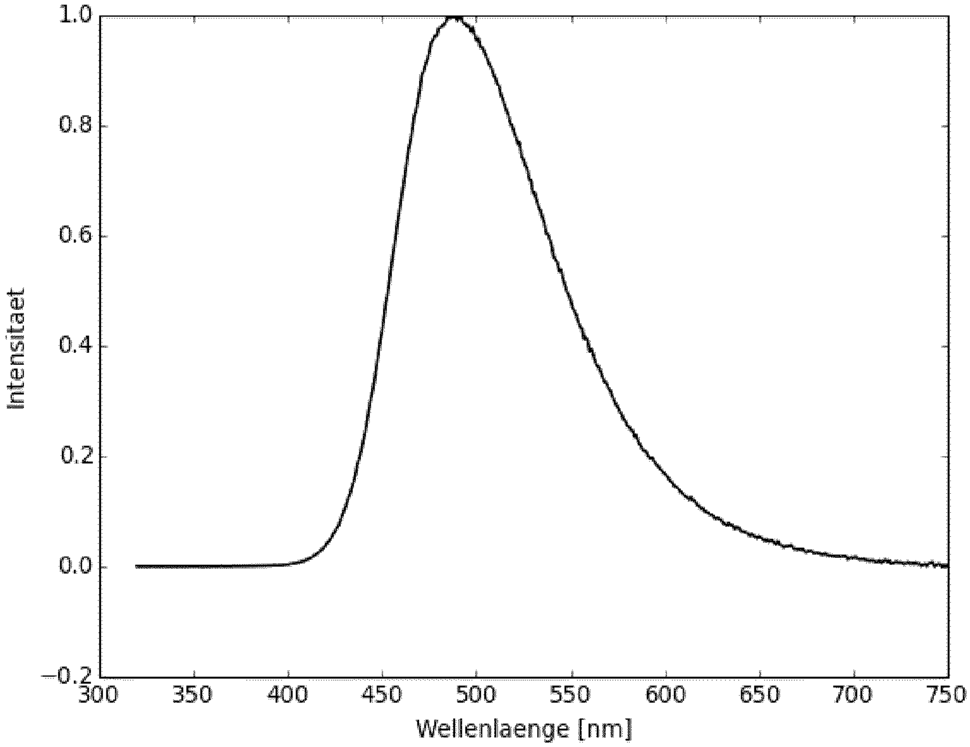


**Figur 18**

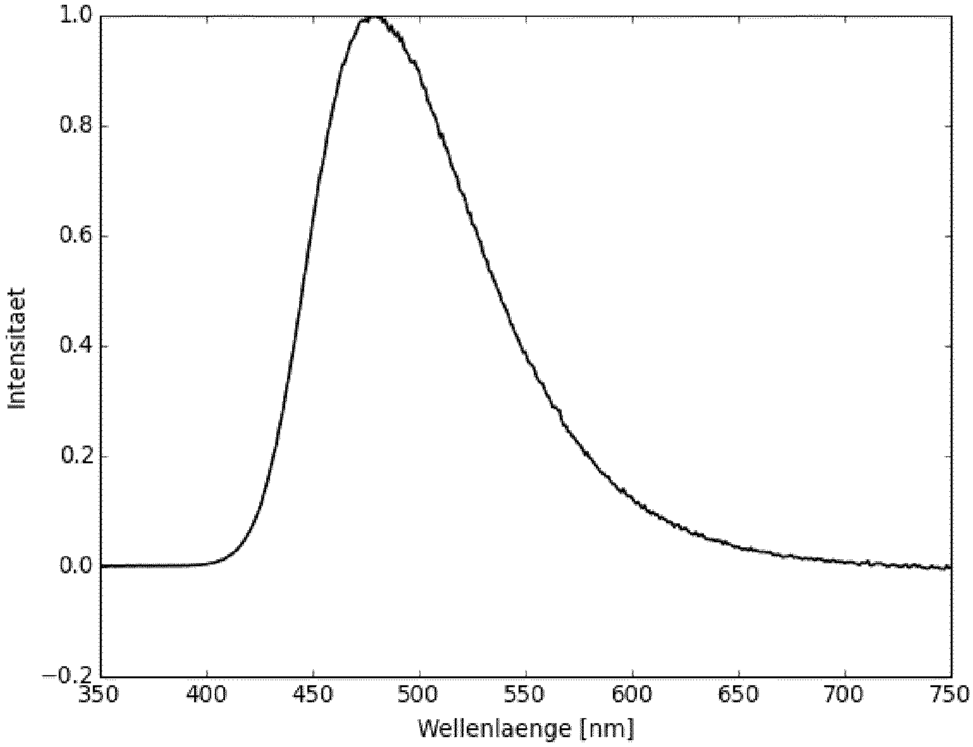

Figur 19

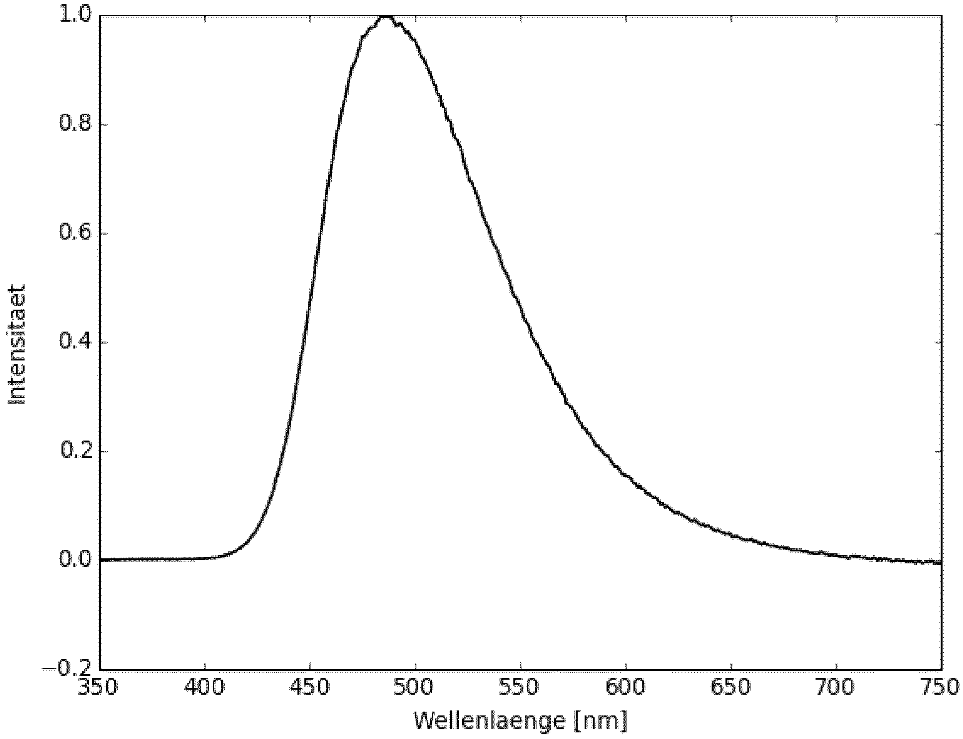


Figur 20

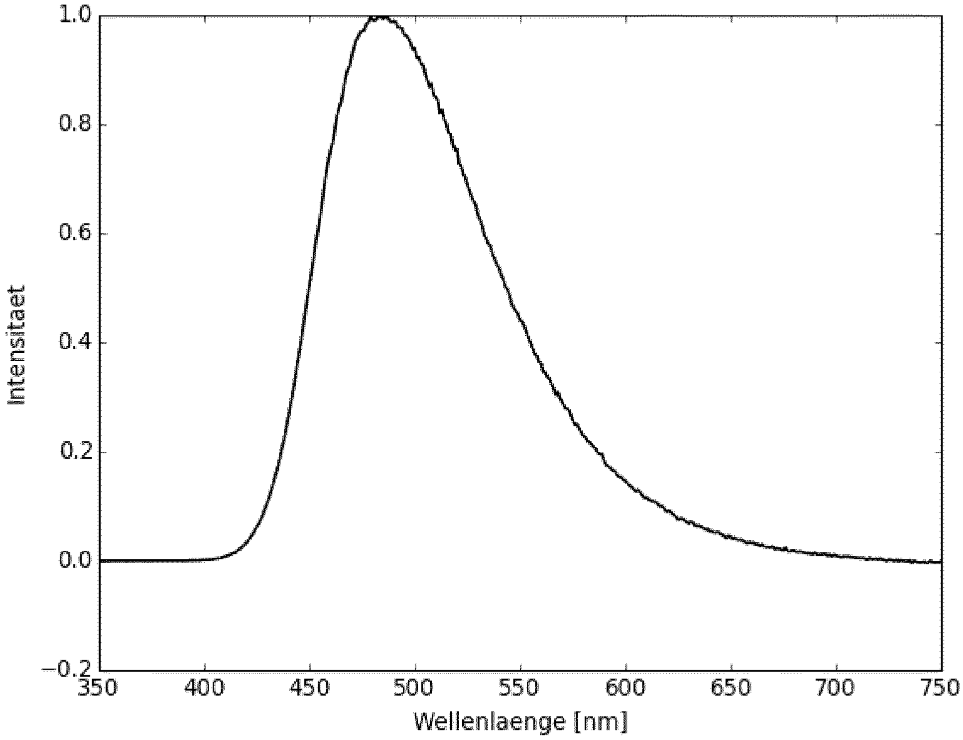

**Figur 21**

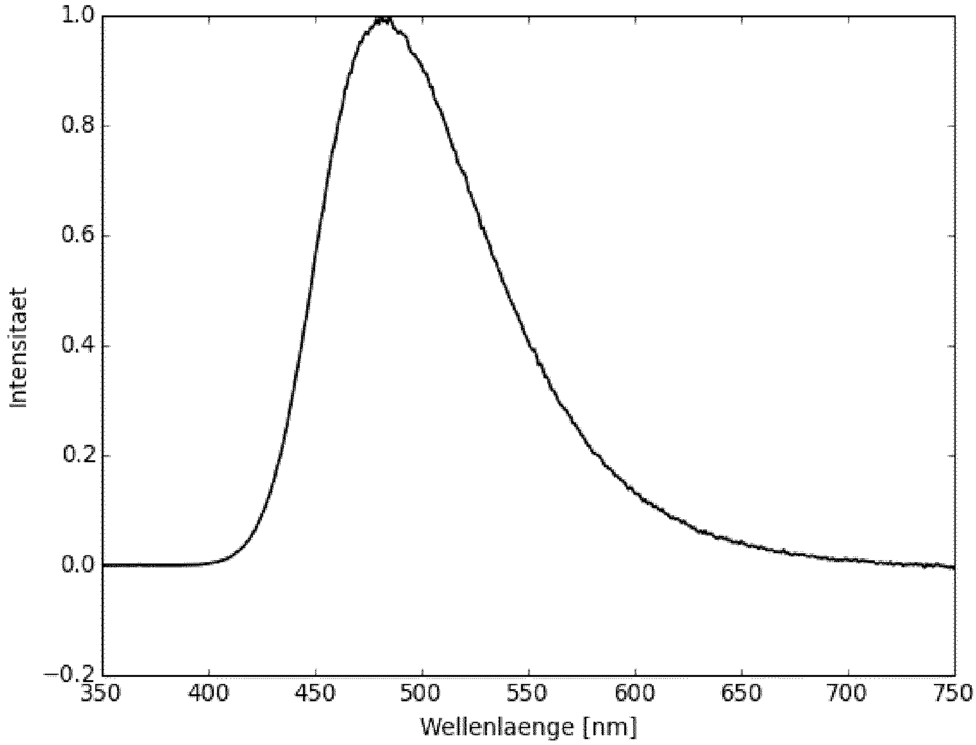
1.0
0.8
0.6
0.4
0.2
0.0
−0.2
Intensitaet
350
400
450
500
550
600
650
700
750
Wellenlaenge [nm]

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2010149748 A1, H. Yersin, U. Monkowius, T. Fischer, T. Hofbeck
- WO 2013161437 A1


### In der Beschreibung aufgeführte Nicht-Patentliteratur


- **Q. ZHANG et al.** *J. Am. Chem. Soc.,* 2012, vol. 134, 14706
- **B. M.E. THOMPSON et al.** *Chem. Mater.,* 2004, vol. 16, 4743
- **BECKE, A. D.** *Phys. Rev. A1988,* vol. 38, 3098-3100
- **PERDEW.** *J. P. Phys. Rev. B,* 1986, vol. 33, 8822-8827
- **SIERKA, M. ; HOGEKAMP, A. ; AHLRICHS, R.** *J. Chem. Phys.,* 2003, vol. 118, 9136-9148
- **BECKE, A.D.** *J.Chem.Phys.,* 1993, vol. 98, 5648-5652
- **LEE, C ; YANG, W ; PARR, R.G.** *Phys. Rev. B,* 1988, vol. 37, 785-789
- **VOSKO, S. H. ; WILK, L. ; NUSAIR, M.** *Can. J. Phys.,* 1980, vol. 58, 1200-1211
- **STEPHENS, P. J. ; DEVLIN, F. J. ; CHABALOWSKI, C. F. ; FRISCH, M. J.** *J.Phys.Chem.,* 1994, vol. 98, 11623-11627
- **WEIGEND, F. ; AHLRICHS, R.** *Phys. Chem. Chem. Phys.,* 2005, vol. 7, 3297-3305
- **RAPPOPORT, D. ; FURCHE, F.** *J. Chem. Phys.,* 2010, vol. 133, 134105, , 1-134105, 11